# EUROPEAN PATENT APPLICATION

(11) **EP 4 545 563 A1**
(43) Date of publication of application: **30.04.2025**
(21) Application number: 23829854.1
(22) Date of filing: 02.06.2023
(51) Int. Cl.: C07K 16/28, A61K 39/00, A61K 39/395, A61P 35/00

(54) **ANTIBODIES TARGETING ITGA2 AND ANTIBODY-DRUG CONJUGATES COMPRISING SAME**

(30) Priority: 27.06.2022 CN 202210743539
(71) Applicant: Foreseen Technology (Beijing) Co., Ltd., Beijing 100192 (CN); Foreseen Biotechnology (Shanghai) Co., Ltd., Shanghai 201210 (CN)
(72) Inventor: WONG, Catherine C., Beijing 100192 (CN); LI, Wei, Shanghai 201210 (CN); HAN, Pei, Beijing 100192 (CN)
(74) Representative: Mathys & Squire
(86) International application number: PCT/CN2023/098076
(87) International publication number: WO 2024/001669

(57) **Abstract**

The present invention provides a series of antibodies or antigen-binding fragments thereof with high specific binding ability to tumor cells expressing the ITGA2 protein. The antibody or the antigen-binding fragment thereof according to the present invention may achieve excellent killing effect on tumor cells after being conjugated with a drug to form an antibody-drug conjugate.

## Description

This application claims the priority for the Chinese patent application 202210743539.0 filed on June 27, 2022 entitled "ANTIBODY TARGETING ITGA2 AND ANTIBODY-DRUG CONJUGATE COMPRISING THE SAME". The priority application is hereby incorporated by reference in its entirety.

### Technical Field

The invention relates to the bio-pharmaceutical field. Specifically, the invention relates to an antibody targeting ITGA2 or an antigen-binding fragment thereof, an antibody-drug conjugate comprising the antibody, and use of the antibody and/or the antibody-drug conjugate in treating related diseases.

### Background Art

Malignant tumors (cancers) have become the "number one killer" threatening the lives and health of people around the world. There are more than 14 million cancer cases per year in the world, among which more than 3 million new cancer patients occur in China alone each year.

The root cause for high mortality of cancer is the spread, metastasis of cancer cells and the relapse and drug resistance of most patients after treatment. Among the present clinically available treatments, surgery, radiotherapy, and chemotherapy have little effect on metastasis of cancer cells, relapse, and drug resistance, or have only short-term effect, which makes it unable to change the long-term survival of the patients. At present, excision is effective for about 10-20% of early patients, but it is almost ineffective for patients who have undergone spread and metastasis. As radiotherapy can only treat local lesions, it is often used as adjunctive therapy before and after surgery and radical treatment of a few types of cancers. Though chemotherapy can be used for patients who have cancer spread and metastasis, but due to its strong toxic and side effects and its tendency to induce short-term or long-term drug resistance, it can only have a significant short-term therapeutic effect on about 20-30% of patients. Even with a comprehensive treatment measure integrating the surgery, radiotherapy and chemotherapy, its five-year survival long term therapeutic effect has been kept from 20% to 30% for many years, where about 70% to 80% patients have died within 5 years after treatment due to metastasis, relapse, and drug resistance. Even in early cancer patients who do not have metastasis at the time of the visit, some also have died due to metastasis and relapse after treatment.

At present, it is effective to treat tumor by identifying a tumor-associated antigen, preparing a drug in the form of an antibody-drug conjugate, and then administering the drug to the tumor cells. This drug form has the both the advantage of monoclonal antibody targeting tumor cells and the advantage of micromolecule cytotoxins that inflict strong killing on tumors, thereby not only significantly reducing the toxic and side effects of micromolecule cytotoxins, but also improving the efficacy of the drug, which results in significant treatment advantages.

### Summary of the Invention

The inventors found that ITGA2 is a tumor-associated antigen that is specifically overexpressed by tumor cells, and it is up-regulated in most solid tumors, and is overexpressed especially in cancers such as colorectal cancer, bile duct cancer, pancreatic cancer, and skin cancer. However, the existing antibodies that can target ITGA2 alone do not have a good inhibition effect on the growth of tumor cells. Therefore, the inventors developed a series of antibodies with high specific binding ability to tumor cells expressing the ITGA2 protein, and the antibodies target ITGA2-positive tumor cells in the form of antibody-drug conjugates to achieve excellent killing effect on cells.

In one aspect, the present invention provides an isolated antibody specifically binding to an ITGA2 protein or an antigen-binding fragment thereof, comprising a heavy chain variable region comprising HCDR1, HCDR2, and HCDR3 sequences, and a light chain variable region comprising LCDR1, LCDR2, and LCDR3 sequences, wherein the antibody or the antigen-binding fragment thereof comprises a heavy chain variable region and a light chain variable region selected from at least one of the following groups:
1) a heavy chain variable region that comprises (a) HCDR1 as set forth in SEQ ID NO: 112, (b) HCDR2 as set forth in SEQ ID NO: 113, and (c) HCDR3 as set forth in SEQ ID NO: 114, and a light chain variable region that comprises (d) LCDR1 as set forth in SEQ ID NO: 38, (e) LCDR2 as set forth in SEQ ID NO: 39, and (f) LCDR3 as set forth in SEQ ID NO: 40;
2) a heavy chain variable region that comprises (a) HCDR1 as set forth in SEQ ID NO: 116, (b) HCDR2 as set forth in SEQ ID NO: 117, and (c) HCDR3 as set forth in SEQ ID NO: 118, and a light chain variable region that comprises (d) LCDR1 as set forth in SEQ ID NO: 42, (e) LCDR2 as set forth in SEQ ID NO: 43, and (f) LCDR3 as set forth in SEQ ID NO: 44;
3) a heavy chain variable region that comprises (a) HCDR1 as set forth in SEQ ID NO: 120, (b) HCDR2 as set forth in SEQ ID NO: 121, and (c) HCDR3 as set forth in SEQ ID NO: 122, and a light chain variable region that comprises (d) LCDR1 as set forth in SEQ ID NO: 46, (e) LCDR2 as set forth in SEQ ID NO: 47, and (f) LCDR3 as set forth in SEQ ID NO: 48;
4) a heavy chain variable region that comprises (a) HCDR1 as set forth in SEQ ID NO: 124, (b) HCDR2 as set forth in SEQ ID NO: 125, and (c) HCDR3 as set forth in SEQ ID NO: 126, and a light chain variable region that comprises (d) LCDR1 as set forth in SEQ ID NO: 50, (e) LCDR2 as set forth in SEQ ID NO: 51, and (f) LCDR3 as set forth in SEQ ID NO: 52;
5) a heavy chain variable region that comprises (a) HCDR1 as set forth in SEQ ID NO: 128, (b) HCDR2 as set forth in SEQ ID NO: 129, and (c) HCDR3 as set forth in SEQ ID NO: 130, and a light chain variable region that comprises (d) LCDR1 as set forth in SEQ ID NO: 54, (e) LCDR2 as set forth in SEQ ID NO: 55, and (f) LCDR3 as set forth in SEQ ID NO: 56;
6) a heavy chain variable region that comprises (a) HCDR1 as set forth in SEQ ID NO: 132, (b) HCDR2 as set forth in SEQ ID NO: 133, and (c) HCDR3 as set forth in SEQ ID NO: 134, and a light chain variable region that comprises (d) LCDR1 as set forth in SEQ ID NO: 58, (e) LCDR2 as set forth in SEQ ID NO: 59, and (f) LCDR3 as set forth in SEQ ID NO: 60;
7) a heavy chain variable region that comprises (a) HCDR1 as set forth in SEQ ID NO: 136, (b) HCDR2 as set forth in SEQ ID NO: 137, and (c) HCDR3 as set forth in SEQ ID NO: 138, and a light chain variable region that comprises (d) LCDR1 as set forth in SEQ ID NO: 62, (e) LCDR2 as set forth in SEQ ID NO: 63, and (f) LCDR3 as set forth in SEQ ID NO: 64;
8) a heavy chain variable region that comprises (a) HCDR1 as set forth in SEQ ID NO: 140, (b) HCDR2 as set forth in SEQ ID NO: 141, and (c) HCDR3 as set forth in SEQ ID NO: 142, and a light chain variable region that comprises (d) LCDR1 as set forth in SEQ ID NO: 66, (e) LCDR2 as set forth in SEQ ID NO: 67, and (f) LCDR3 as set forth in SEQ ID NO: 68;
9) a heavy chain variable region that comprises (a) HCDR1 as set forth in SEQ ID NO: 144, (b) HCDR2 as set forth in SEQ ID NO: 145, and (c) HCDR3 as set forth in SEQ ID NO: 146, and a light chain variable region that comprises (d) LCDR1 as set forth in SEQ ID NO: 70, (e) LCDR2 as set forth in SEQ ID NO: 71, and (f) LCDR3 as set forth in SEQ ID NO: 72;
10) a heavy chain variable region that comprises (a) HCDR1 as set forth in SEQ ID NO: 148, (b) HCDR2 as set forth in SEQ ID NO: 149, and (c) HCDR3 as set forth in SEQ ID NO: 150, and a light chain variable region that comprises (d) LCDR1 as set forth in SEQ ID NO: 74, (e) LCDR2 as set forth in SEQ ID NO: 75, and (f) LCDR3 as set forth in SEQ ID NO: 76;
11) a heavy chain variable region that comprises (a) HCDR1 as set forth in SEQ ID NO: 152, (b) HCDR2 as set forth in SEQ ID NO: 153, and (c) HCDR3 as set forth in SEQ ID NO: 154, and a light chain variable region that comprises (d) LCDR1 as set forth in SEQ ID NO: 78, (e) LCDR2 as set forth in SEQ ID NO: 79, and (f) LCDR3 as set forth in SEQ ID NO: 80;
12) a heavy chain variable region that comprises (a) HCDR1 as set forth in SEQ ID NO: 156, (b) HCDR2 as set forth in SEQ ID NO: 157, and (c) HCDR3 as set forth in SEQ ID NO: 158, and a light chain variable region that comprises (d) LCDR1 as set forth in SEQ ID NO: 82, (e) LCDR2 as set forth in SEQ ID NO: 83, and (f) LCDR3 as set forth in SEQ ID NO: 84;
13) a heavy chain variable region that comprises (a) HCDR1 as set forth in SEQ ID NO: 160, (b) HCDR2 as set forth in SEQ ID NO: 161, and (c) HCDR3 as set forth in SEQ ID NO: 162, and a light chain variable region that comprises (d) LCDR1 as set forth in SEQ ID NO: 86, (e) LCDR2 as set forth in SEQ ID NO: 87, and (f) LCDR3 as set forth in SEQ ID NO: 88;
14) a heavy chain variable region that comprises (a) HCDR1 as set forth in SEQ ID NO: 164, (b) HCDR2 as set forth in SEQ ID NO: 165, and (c) HCDR3 as set forth in SEQ ID NO: 166, and a light chain variable region that comprises (d) LCDR1 as set forth in SEQ ID NO: 90, (e) LCDR2 as set forth in SEQ ID NO: 91, and (f) LCDR3 as set forth in SEQ ID NO: 92;
15) a heavy chain variable region that comprises (a) HCDR1 as set forth in SEQ ID NO: 168, (b) HCDR2 as set forth in SEQ ID NO: 169, and (c) HCDR3 as set forth in SEQ ID NO: 170, and a light chain variable region that comprises (d) LCDR1 as set forth in SEQ ID NO: 94, (e) LCDR2 as set forth in SEQ ID NO: 95, and (f) LCDR3 as set forth in SEQ ID NO: 96;
16) a heavy chain variable region that comprises (a) HCDR1 as set forth in SEQ ID NO: 172, (b) HCDR2 as set forth in SEQ ID NO: 173, and (c) HCDR3 as set forth in SEQ ID NO: 174, and a light chain variable region that comprises (d) LCDR1 as set forth in SEQ ID NO: 98, (e) LCDR2 as set forth in SEQ ID NO: 99, and (f) LCDR3 as set forth in SEQ ID NO: 100;
17) a heavy chain variable region that comprises (a) HCDR1 as set forth in SEQ ID NO: 176, (b) HCDR2 as set forth in SEQ ID NO: 177, and (c) HCDR3 as set forth in SEQ ID NO: 178, and a light chain variable region that comprises (d) LCDR1 as set forth in SEQ ID NO: 102, (e) LCDR2 as set forth in SEQ ID NO: 103, and (f) LCDR3 as set forth in SEQ ID NO: 104; and
18) a heavy chain variable region that comprises (a) HCDR1 as set forth in SEQ ID NO: 180, (b) HCDR2 as set forth in SEQ ID NO: 181, and (c) HCDR3 as set forth in SEQ ID NO: 182, and a light chain variable region that comprises (d) LCDR1 as set forth in SEQ ID NO: 106, (e) LCDR2 as set forth in SEQ ID NO: 107, and (f) LCDR3 as set forth in SEQ ID NO: 108.

In another aspect, the present invention provides an isolated antibody specifically binding to an ITGA2 protein or an antigen-binding fragment thereof, wherein the isolated antibody or the antigen-binding fragment thereof comprises a heavy chain and a light chain selected from at least one of the following groups:
1) a heavy chain that comprises an amino acid sequence encoded by a nucleotide sequence as set forth in SEQ ID NO: 19, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO: 19, or consists of an amino acid sequence encoded by the nucleotide sequence as set forth in SEQ ID NO: 19, and
   a light chain that comprises an amino acid sequence encoded by a nucleotide sequence as set forth in SEQ ID NO:1, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:1, or consists of an amino acid sequence encoded by the nucleotide sequence as set forth in SEQ ID NO: 1;
2) a heavy chain that comprises an amino acid sequence encoded by a nucleotide sequence as set forth in SEQ ID NO:20, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:20, or consists of an amino acid sequence encoded by the nucleotide sequence as set forth in SEQ ID NO:20, and
   a light chain that comprises an amino acid sequence encoded by a nucleotide sequence as set forth in SEQ ID NO:2, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:2, or consists of an amino acid sequence encoded by the nucleotide sequence as set forth in SEQ ID NO:2;
3) a heavy chain that comprises an amino acid sequence encoded by a nucleotide sequence as set forth in SEQ ID NO:21, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:21, or consists of an amino acid sequence encoded by the nucleotide sequence as set forth in SEQ ID NO:21, and
   a light chain that comprises an amino acid sequence encoded by a nucleotide sequence as set forth in SEQ ID NO:3, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:3, or consists of an amino acid sequence encoded by the nucleotide sequence as set forth in SEQ ID NO:3;
4) a heavy chain that comprises an amino acid sequence encoded by a nucleotide sequence as set forth in SEQ ID NO:22, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:22, or consists of an amino acid sequence encoded by the nucleotide sequence as set forth in SEQ ID NO:22, and
   a light chain that comprises an amino acid sequence encoded by a nucleotide sequence as set forth in SEQ ID NO:4, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:4, or consists of an amino acid sequence encoded by the nucleotide sequence as set forth in SEQ ID NO:4;
5) a heavy chain that comprises an amino acid sequence encoded by a nucleotide sequence as set forth in SEQ ID NO:23, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:23, or consists of an amino acid sequence encoded by the nucleotide sequence as set forth in SEQ ID NO:23, and
   a light chain that comprises an amino acid sequence encoded by a nucleotide sequence as set forth in SEQ ID NO:5, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:5, or consists of an amino acid sequence encoded by the nucleotide sequence as set forth in SEQ ID NO:5;
6) a heavy chain that comprises an amino acid sequence encoded by a nucleotide sequence as set forth in SEQ ID NO:24, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:24, or consists of an amino acid sequence encoded by the nucleotide sequence as set forth in SEQ ID NO:24, and
   a light chain that comprises an amino acid sequence encoded by a nucleotide sequence as set forth in SEQ ID NO:6, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:6, or consists of an amino acid sequence encoded by the nucleotide sequence as set forth in SEQ ID NO:6;
7) a heavy chain that comprises an amino acid sequence encoded by a nucleotide sequence as set forth in SEQ ID NO:25, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:25, or consists of an amino acid sequence encoded by the nucleotide sequence as set forth in SEQ ID NO:25, and
   a light chain that comprises an amino acid sequence encoded by a nucleotide sequence as set forth in SEQ ID NO:7, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:7, or consists of an amino acid sequence encoded by the nucleotide sequence as set forth in SEQ ID NO:7;
8) a heavy chain that comprises an amino acid sequence encoded by a nucleotide sequence as set forth in SEQ ID NO:26, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:26, or consists of an amino acid sequence encoded by the nucleotide sequence as set forth in SEQ ID NO:26, and
   a light chain that comprises an amino acid sequence encoded by a nucleotide sequence as set forth in SEQ ID NO:8, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:8, or consists of an amino acid sequence encoded by the nucleotide sequence as set forth in SEQ ID NO:8;
9) a heavy chain that comprises an amino acid sequence encoded by a nucleotide sequence as set forth in SEQ ID NO:27, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:27, or consists of an amino acid sequence encoded by the nucleotide sequence as set forth in SEQ ID NO:27, and
   a light chain that comprises an amino acid sequence encoded by a nucleotide sequence as set forth in SEQ ID NO:9, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:9, or consists of an amino acid sequence encoded by the nucleotide sequence as set forth in SEQ ID NO:9;
10) a heavy chain that comprises an amino acid sequence encoded by a nucleotide sequence as set forth in SEQ ID NO:28, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:28, or consists of an amino acid sequence encoded by the nucleotide sequence as set forth in SEQ ID NO:28, and
   a light chain that comprises an amino acid sequence encoded by a nucleotide sequence as set forth in SEQ ID NO:10, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:10, or consists of an amino acid sequence encoded by the nucleotide sequence as set forth in SEQ ID NO:10;
11) a heavy chain that comprises an amino acid sequence encoded by a nucleotide sequence as set forth in SEQ ID NO:29, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:29, or consists of an amino acid sequence encoded by the nucleotide sequence as set forth in SEQ ID NO:29, and
   a light chain that comprises an amino acid sequence encoded by a nucleotide sequence as set forth in SEQ ID NO: 11, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:11, or consists of an amino acid sequence encoded by the nucleotide sequence as set forth in SEQ ID NO: 11;
12) a heavy chain that comprises an amino acid sequence encoded by a nucleotide sequence as set forth in SEQ ID NO:30, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:30, or consists of an amino acid sequence encoded by the nucleotide sequence as set forth in SEQ ID NO:30, and
   a light chain that comprises an amino acid sequence encoded by a nucleotide sequence as set forth in SEQ ID NO:12, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:12, or consists of an amino acid sequence encoded by the nucleotide sequence as set forth in SEQ ID NO:12;
13) a heavy chain that comprises an amino acid sequence encoded by a nucleotide sequence as set forth in SEQ ID NO:31, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:31, or consists of an amino acid sequence encoded by the nucleotide sequence as set forth in SEQ ID NO:31, and
   a light chain that comprises an amino acid sequence encoded by a nucleotide sequence as set forth in SEQ ID NO:13, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:13, or consists of an amino acid sequence encoded by the nucleotide sequence as set forth in SEQ ID NO: 13;
14) a heavy chain that comprises an amino acid sequence encoded by a nucleotide sequence as set forth in SEQ ID NO:32, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:32, or consists of an amino acid sequence encoded by the nucleotide sequence as set forth in SEQ ID NO:32, and
   a light chain that comprises an amino acid sequence encoded by a nucleotide sequence as set forth in SEQ ID NO:14, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:14, or consists of an amino acid sequence encoded by the nucleotide sequence as set forth in SEQ ID NO: 14;
15) a heavy chain that comprises an amino acid sequence encoded by a nucleotide sequence as set forth in SEQ ID NO:33, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:33, or consists of an amino acid sequence encoded by the nucleotide sequence as set forth in SEQ ID NO:33, and
   a light chain that comprises an amino acid sequence encoded by a nucleotide sequence as set forth in SEQ ID NO:15, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:15, or consists of an amino acid sequence encoded by the nucleotide sequence as set forth in SEQ ID NO:15;
16) a heavy chain that comprises an amino acid sequence encoded by a nucleotide sequence as set forth in SEQ ID NO:34, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:34, or consists of an amino acid sequence encoded by the nucleotide sequence as set forth in SEQ ID NO:34, and
   a light chain that comprises an amino acid sequence encoded by a nucleotide sequence as set forth in SEQ ID NO:16, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:16, or consists of an amino acid sequence encoded by the nucleotide sequence as set forth in SEQ ID NO:16;
17) a heavy chain that comprises an amino acid sequence encoded by a nucleotide sequence as set forth in SEQ ID NO:35, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:35, or consists of an amino acid sequence encoded by the nucleotide sequence as set forth in SEQ ID NO:35, and
   a light chain that comprises an amino acid sequence encoded by a nucleotide sequence as set forth in SEQ ID NO:17, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:17, or consists of an amino acid sequence encoded by the nucleotide sequence as set forth in SEQ ID NO:17; and
18) a heavy chain that comprises an amino acid sequence encoded by a nucleotide sequence as set forth in SEQ ID NO:36, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:36, or consists of an amino acid sequence encoded by the nucleotide sequence as set forth in SEQ ID NO:36, and
   a light chain that comprises an amino acid sequence encoded by a nucleotide sequence as set forth in SEQ ID NO:18, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:18, or consists of an amino acid sequence encoded by the nucleotide sequence as set forth in SEQ ID NO:18.

In yet another aspect, the present invention provides an isolated nucleic acid molecule encoding a monoclonal antibody or an antigen-binding fragment thereof according to the present invention. In yet another aspect, the present invention provides an expression vector comprising the nucleic acid molecule according to the present invention.

In yet another aspect, the present invention provides a host cell comprising the nucleic acid molecule according to the present invention or the expression vector according to the invention.

In yet another aspect, the present invention provides a hybridoma cell expressing a monoclonal antibody or an antigen-binding fragment thereof according to the present invention.

In yet another aspect, the present invention provides use of the monoclonal antibody or the antigen-binding fragment thereof according to the present invention or the antibody-drug conjugate according to the present invention in preparing a pharmaceutical composition; optionally, the pharmaceutical composition is used to treat a malignant tumor, or prevent and/or treat metastasis or relapse of a malignant tumor in a patient.

In yet another aspect, the present invention provides a method for treating a malignant tumor, preventing and/or treating metastasis or relapse of a malignant tumor in a patient, the method comprising administering to the patient an effective amount of the monoclonal antibody or the antigen-binding fragment thereof according to the present invention, the antibody-drug conjugate according to the present invention, or the pharmaceutical composition according to the present invention.

In yet another aspect, the present invention provides use of the monoclonal antibody or the antigen-binding fragment thereof according to the present invention or the antibody-drug conjugate according to the present invention in preparing a diagnostic/prognostic composition; optionally, the diagnostic/prognostic composition is used to detect presence of a malignant tumor in a patient; optionally, the diagnostic/prognostic composition is used to prognosticate relapse or progression of a malignant tumor in a patient.

In yet another aspect, the invention provides a method for prognosticating relapse or progression of a malignant tumor in a patient, the method comprising:
(a) isolating a biological sample comprising tumor cells from the patient;
(b) contacting the biological sample comprising the tumor cells with the monoclonal antibody or the antigen-binding fragment thereof according to the present invention; and
(c) identifying the presence of the tumor cells that bind to the monoclonal antibody or the antigen-binding fragment thereof,
thereby prognosticating the relapse or progression of the malignant tumor in the patient.

In yet another aspect, the present invention provides a method for detecting/diagnosing presence of a malignant tumor in a patient, comprising:
a) contacting a biological sample obtained from the patient with the monoclonal antibody or the antigen-binding fragment thereof according to the invention or the antibody-drug conjugate according to the present invention;
b) detecting binding of the monoclonal antibody or the antigen-binding fragment thereof to a target antigen in the biological sample, wherein the detected binding represents the presence of the malignant tumor in the patient.

In yet another aspect, the invention provides a method for detecting the presence of tumor cells in a biological sample, comprising:
a) contacting the biological sample with the monoclonal antibody or the antigen-binding fragment thereof according to the invention or the antibody-drug conjugate according to the present invention;
b) detecting binding of the monoclonal antibody or the antigen-binding fragment thereof or the antibody-drug conjugate to a target antigen in the biological sample,
wherein the detected binding represents presence of the tumor cells in the biological sample.

In yet another aspect, the present invention provides a method for isolating tumor cells, the method comprising:
(a) providing a cell population suspected of comprising the tumor cells;
(b) identifying a subpopulation of the cells that bind to the monoclonal antibody or the antigen-binding fragment thereof or the antibody-drug conjugate according to the present invention; and
(c) isolating the subpopulation.

In yet another aspect, the present invention provides a method for producing a monoclonal antibody or an antigen-binding fragment thereof targeting human tumor cells that express ITGA2, the method comprising:
(i) culturing the host cell of the present invention in a condition suitable for expressing the nucleic acid molecule or the expression vector, and
(ii) isolating and purifying the antibody or the antigen-binding fragment thereof expressed by the nucleic acid molecule or the expression vector.

The antibody according to the present invention has an excellent binding ability to the ITGA2 protein, and is capable of binding to tumor cells that express ITGA2, especially colorectal cancer cells, bile duct cancer cells, pancreatic cancer cells and skin cancer cells. The antibody-drug conjugate according to the present invention has an excellent killing effect on tumor cells that express ITGA2, especially colorectal cancer cells, bile duct cancer cells, pancreatic cancer cells and skin cancer cells.

### Sequence Information:

In the following tables, the first left column lists the sequence numbering (SEQ ID NO:) of the present invention.

**Table 1a: Nucleic acid coding sequences SEQ ID NO:1-36 of the light and heavy chains of the antibody according to the present invention**

| **No.** | **Description** | **Nucleic Acid Sequence** |
|---|---|---|
| 1 | Humanized antibody FS002-A1 light chain coding sequence | GACATCCAGTTGACCCAGTCTCCATCCTCCCTGTC TGCATCTGTAGGAGACAGAGTCACCATCACTTGC CGGGCAAGTCAGAGCATTATCAGCTATTTAAATTG GTATCAGCAGAAACCAGGGAAAGCCCCTAAGCTC CTGATCTATGCTGCATCCAGTTTGCAAAGTGGGGT CCCATCAAGGTTCAGTGGCAGTGGATCTGGGACA GATTTCACTCTCACCATCAGCAGTCTGCAACCTGA AGATTTTGCAACTTACTACTGTCAACAGAGTTACA GTACCCCTGACACTTTTGGCCAGGGGACCAAGCT GGAGATCAAAAGGACCGTGGCTGCCCCCAGCGTG TTCATCTTCCCTCCTAGCGACGAGCAGCTGAAGA GCGGCACCGCTAGCGTGGTGTGTCTGCTGAATAA CTTCTATCCCAGGGAGGCCAAGGTGCAGTGGAAG GTGGATAACGCCCTGCAGAGCGGCAACTCCCAGG AGTCCGTGACCGAGCAGGACTCCAAGGACAGCA CCTACTCCCTGAGCTCCACCCTGACCCTGTCCAAG GCTGATTATGAGAAGCACAAGGTGTATGCTTGCGA GGTGACACACCAGGGCCTGTCCAGCCCTGTGACC AAGAGCTTCAACCGGGGCGAGTGC |
| 2 | Humanized antibody FS002-A5 light chain coding sequence | GAAATTGTGTTGACACAGTCTCCAGCCACCCTGT CTTTGTCTCCAGGGGAAAGAGCCACCCTCTCCTG CAGGGCCAGTCAGAGTGTTAGCAGCAACTTAGGC TGGTACCAACAGAAACCTGGCCAGGCTCCCAGCC TCCTCATCTATGGTGCATCCAACAGGGCCACTGGC ATCCCAGCCAGGTTCAGTGGCAGTGGGTCTGGGA CAGACTTCACTCTCACCATCAGCAGCCTAGAGCC TGAAGATTTTGCAGTTTATTACTGTCAGCAGCGTG ACACCTGGCCCCTCACTTTCGGCGGAGGGACCAA GGTGGAGATCAAAAGGACCGTGGCTGCCCCCAGC GTGTTCATCTTCCCTCCTAGCGACGAGCAGCTGAA GAGCGGCACCGCTAGCGTGGTGTGTCTGCTGAAT AACTTCTATCCCAGGGAGGCCAAGGTGCAGTGGA AGGTGGATAACGCCCTGCAGAGCGGCAACTCCCA GGAGTCCGTGACCGAGCAGGACTCCAAGGACAG CACCTACTCCCTGAGCTCCACCCTGACCCTGTCCA AGGCTGATTATGAGAAGCACAAGGTGTATGCTTGC GAGGTGACACACCAGGGCCTGTCCAGCCCTGTGA CCAAGAGCTTCAACCGGGGCGAGTGC |
| 3 | Humanized antibody FS002-A15 light chain coding sequence | GAAATTGTGTTGACACAGTCTCCAGCCACCCTGC CTTTGTCTCCGGGGGAAAGAGCCACCCTCTCCTG CAGGGCCAGTCAGAGTGTTGGCTTCTACTTAGCCT GGTACCAACAGAAACCTGGCCAGCCTCCCAGACT CCTCTTCTATGATGCATCCAAGAGGGCCACTGGCA TCCCAGCCAGGTTCAGCGCCAGTGGGTCTGGGAC AGACTTCACTCTCACCATCAGCGGCCTAGAGCCT GAAGATTTTGCAGTTTATTACTGTCAGCACCGCAT AAGCTGGCCTCTGACGTTCGGCCAAGGGACCAAG GTGGAAGTGAAGAGGACCGTGGCTGCCCCCAGC GTGTTCATCTTCCCTCCTAGCGACGAGCAGCTGAA GAGCGGCACCGCTAGCGTGGTGTGTCTGCTGAAT AACTTCTATCCCAGGGAGGCCAAGGTGCAGTGGA AGGTGGATAACGCCCTGCAGAGCGGCAACTCCCA GGAGTCCGTGACCGAGCAGGACTCCAAGGACAG |
| | | CACCTACTCCCTGAGCTCCACCCTGACCCTGTCCA AGGCTGATTATGAGAAGCACAAGGTGTATGCTTGC GAGGTGACACACCAGGGCCTGTCCAGCCCTGTGA CCAAGAGCTTCAACCGGGGCGAGTGC |
| 4 | Humanized antibody FS002-A36 light chain coding sequence | GAAATTGTGTTGACACAGTCTCCAGCCACCCTGT CTTTGTCTCCAGGGGAAAGAGCCACCCTCTCCTG CAGGGCCAGTCAGAGTGTTAGTACTTACTTAGCCT GGTACCAACAGAAACCTGGCCAGGCTCCCAGGCT CCTCATCTATGACACATCCATCAGGGCCACTGGCA TCCCAGCCAGGTTCAGTGGCAGTGGGTCTGGGAC AGACTTCACTCTCACCATCAGCAGCCTAGAGCCT GAAGATTTTGCAGTTTATTACTGTCAGCAGCGTAG CAACTGGCCGCTCACTTTCGGCGGAGGGACCAAG GTGGAGATCAAAAGGACCGTGGCTGCCCCCAGCG TGTTCATCTTCCCTCCTAGCGACGAGCAGCTGAAG AGCGGCACCGCTAGCGTGGTGTGTCTGCTGAATA ACTTCTATCCCAGGGAGGCCAAGGTGCAGTGGAA GGTGGATAACGCCCTGCAGAGCGGCAACTCCCAG GAGTCCGTGACCGAGCAGGACTCCAAGGACAGC ACCTACTCCCTGAGCTCCACCCTGACCCTGTCCAA GGCTGATTATGAGAAGCACAAGGTGTATGCTTGCG AGGTGACACACCAGGGCCTGTCCAGCCCTGTGAC CAAGAGCTTCAACCGGGGCGAGTGC |
| 5 | Humanized antibody FS002-A66 light chain coding sequence | GAAATTGTGTTGACACAGTCTCCAGCCATCCTGTC TTTGTCTCCAGGGGAAAGAGCCACCCTCTCCTGC AGGGCCAGTCAGAGTGTTAGCGAGTACTTAGCCT GGTACCAACAAAAACCTGGCCAGGCTCCCAGGCT CCTCATCTATGATATATCTAACAGGGCCACTGGCAT CCCAGCCAGGTTCAGTGGCAGTGGGTCTGAGACA GACTTCACTCTCACCATCAGCAGCCTAGAGCCTG AAGATTTTGCAGTTTATTACTGTCAACAACGTCGC ACCTGGCCTCTCACTTTCGGCCCTGGGACCAAAG TGGACATCAAAAGGACCGTGGCTGCCCCCAGCGT GTTCATCTTCCCTCCTAGCGACGAGCAGCTGAAG AGCGGCACCGCTAGCGTGGTGTGTCTGCTGAATA ACTTCTATCCCAGGGAGGCCAAGGTGCAGTGGAA GGTGGATAACGCCCTGCAGAGCGGCAACTCCCAG GAGTCCGTGACCGAGCAGGACTCCAAGGACAGC ACCTACTCCCTGAGCTCCACCCTGACCCTGTCCAA GGCTGATTATGAGAAGCACAAGGTGTATGCTTGCG AGGTGACACACCAGGGCCTGTCCAGCCCTGTGAC CAAGAGCTTCAACCGGGGCGAGTGC |
| 6 | Humanized antibody FS002-A10 light chain coding sequence | AACATCCAGTTGACCCAGTCTCCACCCTTCCTGTC TGCATCTGTAGGAGACAGAGTCACCATCACTTGC CGGGCCAGCCAGGACATTAGCAGTTTTTTAGCCTG GTATCAGCAAAAACCAGGGAAAGCCCCTAACCTC CTGATCCATGCTGCAACCACTCTGCAAAGTGGGG TCCCATCAAGGTTCAGCGGCAGTGGATCTGGGAC GGAGTTCACTCTCACAATCAGCAGCCTGCGGCCA GAAGATTTTGCCACTTACTACTGTCAACAACTTCA TACTTACCCGATCACCTTCGGCCAAGGGACACGA CTGGAGATTAAACGAACTGTGGCTGCACCATCCG TGTTCATCTTCCCTCCTAGCGACGAGCAGCTGAAG AGCGGCACCGCTAGCGTGGTGTGTCTGCTGAATA ACTTCTATCCCAGGGAGGCCAAGGTGCAGTGGAA GGTGGATAACGCCCTGCAGAGCGGCAACTCCCAG GAGTCCGTGACCGAGCAGGACTCCAAGGACAGC |
| | | ACCTACTCCCTGAGCTCCACCCTGACCCTGTCCAA GGCTGATTATGAGAAGCACAAGGTGTATGCTTGCG AGGTGACACACCAGGGCCTGTCCAGCCCTGTGAC CAAGAGCTTCAACCGGGGCGAGTGC |
| 7 | Humanized antibody FS002-C9 light chain coding sequence | AACATCCAGATGACCCAGTCTCCACCCTCCCTGTC TGCATCTGTGGGAGACAGAGTCGCCATCACTTGC CGGGCGAGTCAGGACATTAGCAATTCCTTAGCCTG GTATCAGCAGAAGTCAGGGCAACCCCCTAAACTC CTGCTCTATGATGTGTCGAAATTGGAAAGGGGGG TCCCATCCAGGTTCAGTGGCAGTGGATCTCGGAC GGATTACACTCTCACCATCAGCAGCCTGCAGCCTG AAGATTTTGCAACTTATTACTGTCAACAGTATTCTA GTGACCCCACTTTTGGACAGGGGAGTAAGCTGGA GTTCAAACGAACTGTGGCTGCACCATCCGTGTTCA TCTTCCCTCCTAGCGACGAGCAGCTGAAGAGCGG CACCGCTAGCGTGGTGTGTCTGCTGAATAACTTCT ATCCCAGGGAGGCCAAGGTGCAGTGGAAGGTGG ATAACGCCCTGCAGAGCGGCAACTCCCAGGAGTC CGTGACCGAGCAGGACTCCAAGGACAGCACCTAC TCCCTGAGCTCCACCCTGACCCTGTCCAAGGCTG ATTATGAGAAGCACAAGGTGTATGCTTGCGAGGT GACACACCAGGGCCTGTCCAGCCCTGTGACCAAG AGCTTCAACCGGGGCGAGTGC |
| 8 | Humanized antibody FS002-C11 light chain coding sequence | GACATCCAGATGATCCAGTCTCCTTCTTCCGTGTC TGCTTCTGTGGGAGACAGAGTCACCATCACTTGT CGGGCGAGTCAGGGTATCAGCAACTCGTTAGCCT GGTATCAGCACAAACCAGGTACAACCCCTAAGCT CCTGATCTATGCTGCATCCAGTTTGCAAGGTGGGG TCCCATCCAGGTTCAGCGGCAGTTCATCTGAGAC AGATTTCACTCTCACCATCAGCAGCCTTCAGCCTG AAGATTTTGCAACTTACTACTGTCAGCAGGCCAAC AGTTTCCCGATCACCTTCGGCCAGGGGACACGAC TGGATATTAAACGAACTGTGGCTGCACCATCCGTG TTCATCTTCCCTCCTAGCGACGAGCAGCTGAAGA GCGGCACCGCTAGCGTGGTGTGTCTGCTGAATAA CTTCTATCCCAGGGAGGCCAAGGTGCAGTGGAAG GTGGATAACGCCCTGCAGAGCGGCAACTCCCAGG AGTCCGTGACCGAGCAGGACTCCAAGGACAGCA CCTACTCCCTGAGCTCCACCCTGACCCTGTCCAAG GCTGATTATGAGAAGCACAAGGTGTATGCTTGCGA GGTGACACACCAGGGCCTGTCCAGCCCTGTGACC AAGAGCTTCAACCGGGGCGAGTGC |
| 9 | Humanized antibody FS002-C216 light chain coding sequence | GAAATTGTGTTGACACAGTCTCCAGCCACTCTGTC TTTGTCTCCAGGGGAAAGAGCCACCCTCTCCTGC AGGGCCAGTCAGAGTGTTAGCAGCTACTTAGCCT GGTACCAACAAAAACCTGGCCAGGCTCCCAGACT CCTCATCTATGATGCATCCAACAGGGCCACTGGCA TCCCAGCCAGGTTCAGTGGCAGTGGGTCTGGGAC AGACTTCACTCTCACCATCAGCAGCCTAGAGCCT GAAGATTTTGCAGTTTATTACTGTCAGCAGCGTAC CAACTGGCTTATGTACACTTTTGGCCAGGGGACCA AGCTGGAGATCAAACGAACTGTGGCTGCACCATC CGTGTTCATCTTCCCTCCTAGCGACGAGCAGCTGA AGAGCGGCACCGCTAGCGTGGTGTGTCTGCTGAA TAACTTCTATCCCAGGGAGGCCAAGGTGCAGTGG AAGGTGGATAACGCCCTGCAGAGCGGCAACTCCC AGGAGTCCGTGACCGAGCAGGACTCCAAGGACA |
| | | GCACCTACTCCCTGAGCTCCACCCTGACCCTGTCC AAGGCTGATTATGAGAAGCACAAGGTGTATGCTT GCGAGGTGACACACCAGGGCCTGTCCAGCCCTGT GACCAAGAGCTTCAACCGGGGCGAGTGC |
| 10 | Humanized antibody FS002-C375 light chain coding sequence | GAAATTGTGTTGACACAGTCTCCAGCCACCCTGT CTTTGTCTCCAGGGGAAAGAGCCACCCTCTCCTG CAGGGCCAGTCAGAGTGTTAGCAGCTACTTAGCC TGGTACCAACAGAAACCTGGCCAGGCTCCCAGGC TCCTCATCTATGATGCATCCAACAGGGCCACTGGC ATCCCAGCCAGGTTCAGTGGCAGTGGGTCTGGGA CAGACTTCACTCTCACCATCAGCAGCCTAGAGCC TGAAGATTTTGCAGTTTATTACTGTCAGCAGCGTA GCAACTGGCCCCTCACTTTCGGCGGAGGGACCAA GGTGGAGATCAAACGAACTGTGGCTGCACCATCC GTGTTCATCTTCCCTCCTAGCGACGAGCAGCTGAA GAGCGGCACCGCTAGCGTGGTGTGTCTGCTGAAT AACTTCTATCCCAGGGAGGCCAAGGTGCAGTGGA AGGTGGATAACGCCCTGCAGAGCGGCAACTCCCA GGAGTCCGTGACCGAGCAGGACTCCAAGGACAG CACCTACTCCCTGAGCTCCACCCTGACCCTGTCCA AGGCTGATTATGAGAAGCACAAGGTGTATGCTTGC GAGGTGACACACCAGGGCCTGTCCAGCCCTGTGA CCAAGAGCTTCAACCGGGGCGAGTGC |
| 11 | Humanized antibody FS002-C417 light chain coding sequence | AACATCCAGATGACCCAGTCTCCATCCTCCCTGTC TGCATCTGTAGGAGACAGAGTCACCATCACTTGC CGGGCAAGTCAGAGCATTAGCAGCTATTTAAATTG GTATCAGCAGAAACCAGGGAAAGCCCCTAAGCTC CTGATCTATGCTGCATCCAGTTTGCAAAGTGGGGT CCCATCAAGGTTCAGTGGCAGTGGATCTGGGACA GATTTCACTCTCACCATCAGCAGTCTGCAACCTGA AGATTTTGCATCTTACTACTGTCAACAGAGTTACA GTTCGTGGGCGTTCGGCCAAGGGACCAAGGTGGA AATCAAACGAACTGTGGCTGCACCATCCGTGTTCA TCTTCCCTCCTAGCGACGAGCAGCTGAAGAGCGG CACCGCTAGCGTGGTGTGTCTGCTGAATAACTTCT ATCCCAGGGAGGCCAAGGTGCAGTGGAAGGTGG ATAACGCCCTGCAGAGCGGCAACTCCCAGGAGTC CGTGACCGAGCAGGACTCCAAGGACAGCACCTAC TCCCTGAGCTCCACCCTGACCCTGTCCAAGGCTG ATTATGAGAAGCACAAGGTGTATGCTTGCGAGGT GACACACCAGGGCCTGTCCAGCCCTGTGACCAAG AGCTTCAACCGGGGCGAGTGC |
| 12 | Humanized antibody FS002-B27 light chain coding sequence | AACATCCAGTTGACCCAGTCTCCATCTTCCGTGTC TGCATCTGTAGGAGACAGAGTCACCATCACTTGTC GGGCGAGTCAGGGTTTTAGTAGGTGGTTAGCCTG GTATCAGCAGAAACCAGGGAAAGCCCCTAAGCTC CTGATCTATGGTGCATCCAGTTTGCAAAGTGGGGT CCCATCAAGATTCAGCGGCAGTGGATCTGGGACA GATTTCACTCTCACCATCAGCAGCCTGCAGCCTGA AGATTTTGCAACTTACTATTGTCAACAGGCTAACA GTTTCCCTCTCACTTTCGGCGGAGGGACCAAGCT GGAGATCAAACGAACTGTGGCTGCACCATCCGTG TTCATCTTCCCTCCTAGCGACGAGCAGCTGAAGA GCGGCACCGCTAGCGTGGTGTGTCTGCTGAATAA CTTCTATCCCAGGGAGGCCAAGGTGCAGTGGAAG GTGGATAACGCCCTGCAGAGCGGCAACTCCCAGG AGTCCGTGACCGAGCAGGACTCCAAGGACAGCA |
| | | CCTACTCCCTGAGCTCCACCCTGACCCTGTCCAAG GCTGATTATGAGAAGCACAAGGTGTATGCTTGCGA GGTGACACACCAGGGCCTGTCCAGCCCTGTGACC AAGAGCTTCAACCGGGGCGAGTGC |
| 13 | Humanized antibody FS002-C330 light chain coding sequence | AACATCCAGTTGACCCAGTCTCCATCCTCCCTGTC TGCATCTGTAGGAGACAGAGTCACCATCACTTGC CGGGCAAGTCAGAGCATTAGCAGCTATTTAAATTG GTATCAGCAGAAACCAGGGAAAGCCCCTAAGCTC CTGATCTATGCTGCATCCAGTTTGCAAAGTGGGGT CCCATCAAGGTTCAGTGGCAGTGGATCTGGGACA GATTTCACTCTCACCATCAGCAGTCTGCAACCTGA AGATTTTGCAACTTACTACTGTCAACAGAGTTACA GTACCCCAGACACTTTCGGCCCTGGGACCAAAGT GGATATCAAACGAACTGTGGCTGCACCATCCGTGT TCATCTTCCCTCCTAGCGACGAGCAGCTGAAGAG CGGCACCGCTAGCGTGGTGTGTCTGCTGAATAACT TCTATCCCAGGGAGGCCAAGGTGCAGTGGAAGGT GGATAACGCCCTGCAGAGCGGCAACTCCCAGGAG TCCGTGACCGAGCAGGACTCCAAGGACAGCACCT ACTCCCTGAGCTCCACCCTGACCCTGTCCAAGGC TGATTATGAGAAGCACAAGGTGTATGCTTGCGAG GTGACACACCAGGGCCTGTCCAGCCCTGTGACCA AGAGCTTCAACCGGGGCGAGTGC |
| 14 | Humanized antibody FS002-C153 light chain coding sequence | CAGTCTGCCCTGACTCAGCCTGCCTCCGTGTCTGG GTCTCCTGGACAGTCGATCACCATCTCCTGCACTG GAACCAGCAGTGACGTTGGTGGTTATAACTATGTC TCCTGGTACCAACAACACCCAGGCAAAGCCCCCA AACTCATGATTTATGATGTCAGTAATCGGCCCTCA GGGGTTTCTAATCGCTTCTCTGGCTCCAAGTCTGG CAACACGGCCTCCCTGACCATCTCTGGGCTCCAG GCTGAGGACGAGGCTGATTATTATTGCAGCTCATT TACAACCATCACTACGAGGGTGTTCGGCGGAGGG ACCAAGCTGACCGTCCTAGGTCAGCCCAAGGCTG CCCCCTCGGTGACACTGTTCCCTCCATCCTCTGAG GAACTGCAGGCCAACAAGGCTACCCTCGTGTGCC TGATCTCCGACTTTTACCCTGGCGCTGTGACCGTG GCCTGGAAGGCTGATAGTTCTCCTGTGAAGGCCG GCGTGGAAACCACCACACCTTCCAAGCAGTCCAA CAACAAATACGCCGCCTCCTCCTACCTGTCTCTGA CCCCTGAACAGTGGAAGTCCCACCGGTCCTACAG CTGCCAAGTGACCCATGAGGGCTCCACCGTGGAA AAGACCGTGGCTCCTACCGAGTGCTCT |
| 15 | Humanized antibody FS002-C241 light chain coding sequence | CAGTCTGCCCTGACTCAGCCTGCCTCCGTGTCTGG GTCTCCTGGACAGTCGATCACCATCTCCTGCAGTG GAACCAGCAGTGACATTGGTCGTTATAACTATGTC TCCTGGTACCAACAACACCCAGGCAAAGCCCCCA AACTCCTGATTTTTGATGTCAGTAATCGGCCCTCA GGGGTTTCTAATCGTTTCTCTGGCTCCAAGTCTGG CAACACGGCCTCCCTGACCATCTCTGGGCTCCAG GCTGAGGACGAGGCTGATTATTACTGCAGCTCATA TACAAGCACCGTCCCTTATGTCTTCGGAACTGGGA CGCGGGTCGCCGTCCAAGGTCAGCCCAAGGCTGC CCCCTCGGTGACACTGTTCCCTCCATCCTCTGAGG AACTGCAGGCCAACAAGGCTACCCTCGTGTGCCT GATCTCCGACTTTTACCCTGGCGCTGTGACCGTGG CCTGGAAGGCTGATAGTTCTCCTGTGAAGGCCGG CGTGGAAACCACCACACCTTCCAAGCAGTCCAAC |
| | | AACAAATACGCCGCCTCCTCCTACCTGTCTCTGAC CCCTGAACAGTGGAAGTCCCACCGGTCCTACAGC TGCCAAGTGACCCATGAGGGCTCCACCGTGGAAA AGACCGTGGCTCCTACCGAGTGCTCT |
| 16 | Humanized antibody FS002-C15 light chain coding sequence | GAAATTGTATTGACACAGTCTCCAGCCACCCTGTC TTTGTCTCCAGGGGAAAGAGCCACCCTCTCCTGC CAGGCCAGTCAGACTTTTAGCGATTACTTAGCCTG GTACCAACAGAAACCTGGCCAGGCTCCCAGGCTC CTCATCTATGATGCATCCACCAGGGCCACTGGCAT CCCAGCCAGGTTCAGTGGCAGTGGGTCTGGGACA GACTTCACTCTCACCATCAGCAGCCTAGAGCCTG AAGATTTTGCAGTTTATTACTGTCAGCAGCGTAGC CACTGGCCTCCAGGGTACACTTTTGGCCAAGGGA CCAGGGTGGAGATCAAACGAACTGTGGCTGCACC ATCCGTGTTCATCTTCCCTCCTAGCGACGAGCAGC TGAAGAGCGGCACCGCTAGCGTGGTGTGTCTGCT GAATAACTTCTATCCCAGGGAGGCCAAGGTGCAG TGGAAGGTGGATAACGCCCTGCAGAGCGGCAACT CCCAGGAGTCCGTGACCGAGCAGGACTCCAAGG ACAGCACCTACTCCCTGAGCTCCACCCTGACCCT GTCCAAGGCTGATTATGAGAAGCACAAGGTGTAT GCTTGCGAGGTGACACACCAGGGCCTGTCCAGCC CTGTGACCAAGAGCTTCAACCGGGGCGAGTGC |
| 17 | Humanized antibody FS002-C58 light chain coding sequence | GAAATTGTGTTGACACAGTCTCCAGCCACCCTGT CTTTGTCTCCAGGGGAAAGAGCCACCCTCTCCTG CAGGGCCAGTCAGAGTGTTTACAGTTCTTTAGCCT GGTACCAACAGAAACCTGGCCAGGCTCCCAAGCT CCTCATCTATGATGCATCCAACAGGGCCACTGGCG TCCCAGCCAGGTTCAGTGGCAGTGGGTCTGGGAC AGACTTCACTCTCACCATCAGCAGCCTAGAGCCT GAGGATTTTGCACTTTATTACTGTCAGCACCGTCA GCATTGGCCTCTCACTTTCGGCGGAGGGACCAAG GTGGAGATCAAACGAACTGTGGCTGCACCATCCG TGTTCATCTTCCCTCCTAGCGACGAGCAGCTGAAG AGCGGCACCGCTAGCGTGGTGTGTCTGCTGAATA ACTTCTATCCCAGGGAGGCCAAGGTGCAGTGGAA GGTGGATAACGCCCTGCAGAGCGGCAACTCCCAG GAGTCCGTGACCGAGCAGGACTCCAAGGACAGC ACCTACTCCCTGAGCTCCACCCTGACCCTGTCCAA GGCTGATTATGAGAAGCACAAGGTGTATGCTTGCG AGGTGACACACCAGGGCCTGTCCAGCCCTGTGAC CAAGAGCTTCAACCGGGGCGAGTGC |
| 18 | Humanized antibody FS002-A72 light chain coding sequence | GAAATTGTGATGACGCAGTCTCCAGTCACCCTGTC TGTCTCTCCAGGGGACTCAGCCACCCTCTCCTGC AGGGCCAGTCAGAGTGTTGATAAAAACTTAGCCT GGTACCAGCAGAAAACTGGCCAGGCTCCCAGGCT CCTCATCTATGGTGCAGCCACCAGGGCCACTGGTA TCCCAGCCAGGTTCAGTGGCAGTGGGTCTGGGAC AGAATTCACTCTCACCATCAGCAGCCTGCGGTCTG AAGATTTTGCAGTTTATTACTGTCAGCAGTATAGA AACTGGCCTTGGACGTTCGGCCTAGGGACCAAGG TGGAGATCAAACGAACTGTGGCTGCACCATCCGT GTTCATCTTCCCTCCTAGCGACGAGCAGCTGAAG AGCGGCACCGCTAGCGTGGTGTGTCTGCTGAATA ACTTCTATCCCAGGGAGGCCAAGGTGCAGTGGAA GGTGGATAACGCCCTGCAGAGCGGCAACTCCCAG GAGTCCGTGACCGAGCAGGACTCCAAGGACAGC |
| | | ACCTACTCCCTGAGCTCCACCCTGACCCTGTCCAA GGCTGATTATGAGAAGCACAAGGTGTATGCTTGCG AGGTGACACACCAGGGCCTGTCCAGCCCTGTGAC CAAGAGCTTCAACCGGGGCGAGTGC |
| 19 | Humanized antibody FS002-A1 heavy chain coding sequence | CAGGTGCAGCTGGTGCAATCTGGGGCTGAGGTGA AGAAGCCTGGGGCCTCAGTGAAGGTTTCCTGCAA GGCATCTGGATACACCTTCACCAGCTACTATATGC ACTGGGTGCGACAGGCCCCTGGACAAGGGCTTGA GTGGATGGGAATAATCAACCCTAGTGGTGGTAGC ACAAGCTACGCACAGAAGTTCCAGGGCAGAGTC ACCATGACCAGGGACACGTCCACGAGCACAGTCT ACATGGAGCTGAGCAGCCTGAGATCTGAGGACAC GGCCGTGTATTACTGTGCGAGAGATATTAGTGGGA GCTACCAAACTGATGCTTTTGATATCTGGGGCCAA GGGACAATGGTCACCGTCTCATCAGCTTCCACCA AGGGCCCCTCCGTGTTCCCCCTGGCTCCCTCTTCC AAGAGCACCAGCGGCGGCACCGCTGCTCTGGGAT GTCTGGTGAAGGACTACTTCCCTGAGCCTGTGAC CGTGTCCTGGAATTCCGGCGCCCTGACCTCCGGC GTGCACACATTCCCTGCTGTGCTGCAGTCCTCCGG CCTGTATAGCCTGTCCTCCGTGGTGACAGTGCCTA GCTCCAGCCTGGGCACCCAGACCTATATCTGCAAC GTGAACCACAAGCCTAGCAATACCAAGGTGGACA AGAAGGTGGAGCCTAAGAGCTGCGACAAGACCC ACACCTGTCCTCCATGTCCTGCTCCAGAACTGCTC GGCGGACCTTCCGTGTTCCTGTTTCCTCCAAAGCC TAAGGACACCCTGATGATCAGCAGAACCCCTGAA GTGACCTGCGTGGTGGTGGATGTGTCCCACGAGG ATCCCGAAGTGAAGTTCAATTGGTACGTGGACGG CGTGGAAGTGCACAACGCCAAGACCAAGCCTAG AGAGGAACAGTACAACAGCACCTACAGAGTGGT GTCCGTGCTGACCGTGCTGCACCAGGATTGGCTG AACGGCAAAGAGTACAAGTGCAAGGTGTCCAAC AAGGCCCTGCCTGCTCCTATCGAGAAAACCATCA GCAAGGCCAAGGGCCAGCCTAGGGAACCCCAGG TTTACACACTGCCTCCAAGCAGGGACGAGCTGAC CAAGAATCAGGTGTCCCTGACCTGCCTGGTCAAG GGCTTCTACCCTTCCGATATCGCCGTGGAATGGGA GAGCAATGGCCAGCCTGAGAACAACTACAAGAC AACCCCTCCTGTGCTGGACAGCGACGGCTCATTC TTCCTGTACAGCAAGCTGACAGTGGACAAGAGCA GATGGCAGCAGGGCAACGTGTTCAGCTGCAGCGT GATGCACGAGGCCCTGCACAACCACTACACCCAG AAGTCCCTGAGCCTGTCTCCTGGCAAA |
| 20 | Humanized antibody FS002-A5 heavy chain coding sequence | CAGGTGCAGCTGCAGGACTCGGGCCCAGGACTG GTGAAGCCTTCACAGACCCTGTCCCTCACCTGCA CTGTCTCTGGTGGCTCCATCAGCAGTGGTGGTTAC TACTGGAGCTGGATCCGCCAGCACCCAGGGAAGG GCCTGGAGTGGATTGGGTACATCTATTACAGTGGG AGCACCTACTACAACCCGTCCCTCAAGAGTCGAG TTACCATATCAGTAGACACGTCTAAGAACCAGTTC TCCCTGAAGCTGAGCTCTGTGACTGCCGCGGACA CGGCCGTGTATTACTGTGCGAGAGTGCCTGTCCCA CTGGTGGGGGCTTTTGATATCTGGGGCCAAGGGA CAATGGTCACCGTCTCATCAGCTTCCACCAAGGG CCCCTCCGTGTTCCCCCTGGCTCCCTCTTCCAAGA GCACCAGCGGCGGCACCGCTGCTCTGGGATGTCT |
| | | GGTGAAGGACTACTTCCCTGAGCCTGTGACCGTG TCCTGGAATTCCGGCGCCCTGACCTCCGGCGTGC ACACATTCCCTGCTGTGCTGCAGTCCTCCGGCCTG TATAGCCTGTCCTCCGTGGTGACAGTGCCTAGCTC CAGCCTGGGCACCCAGACCTATATCTGCAACGTG AACCACAAGCCTAGCAATACCAAGGTGGACAAGA AGGTGGAGCCTAAGAGCTGCGACAAGACCCACA CCTGTCCTCCATGTCCTGCTCCAGAACTGCTCGGC GGACCTTCCGTGTTCCTGTTTCCTCCAAAGCCTAA GGACACCCTGATGATCAGCAGAACCCCTGAAGTG ACCTGCGTGGTGGTGGATGTGTCCCACGAGGATC CCGAAGTGAAGTTCAATTGGTACGTGGACGGCGT GGAAGTGCACAACGCCAAGACCAAGCCTAGAGA GGAACAGTACAACAGCACCTACAGAGTGGTGTCC GTGCTGACCGTGCTGCACCAGGATTGGCTGAACG GCAAAGAGTACAAGTGCAAGGTGTCCAACAAGG CCCTGCCTGCTCCTATCGAGAAAACCATCAGCAA GGCCAAGGGCCAGCCTAGGGAACCCCAGGTTTAC ACACTGCCTCCAAGCAGGGACGAGCTGACCAAG AATCAGGTGTCCCTGACCTGCCTGGTCAAGGGCT TCTACCCTTCCGATATCGCCGTGGAATGGGAGAGC AATGGCCAGCCTGAGAACAACTACAAGACAACCC CTCCTGTGCTGGACAGCGACGGCTCATTCTTCCTG TACAGCAAGCTGACAGTGGACAAGAGCAGATGG CAGCAGGGCAACGTGTTCAGCTGCAGCGTGATGC ACGAGGCCCTGCACAACCACTACACCCAGAAGTC CCTGAGCCTGTCTCCTGGCAAA |
| 21 | Humanized antibody FS002-A15 heavy chain coding sequence | CGGCTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTAC TACTGGAGCTGGATCCGCCAGCACCCAGGGAAGG GCCTGGAGTGGATTGGGTACATCTATTACAGTGGG AGCACCTACTACAACCCGTCCCTCAAGAGTCGAG TTACCATATCAGTAGACACGTCTAAGAACCAGTTC TCCCTGAAGCTGAGCTCTGTGACTGCCGCGGACA CGGCCGTGTATTACTGTGCGAGAGCAACCATCTAC GGTTACTTTGACTACTGGGGCCAGGGAACCCTGG TCACCGTCTCATCAGCTTCCACCAAGGGCCCCTCC GTGTTCCCCCTGGCTCCCTCTTCCAAGAGCACCA GCGGCGGCACCGCTGCTCTGGGATGTCTGGTGAA GGACTACTTCCCTGAGCCTGTGACCGTGTCCTGG AATTCCGGCGCCCTGACCTCCGGCGTGCACACATT CCCTGCTGTGCTGCAGTCCTCCGGCCTGTATAGCC TGTCCTCCGTGGTGACAGTGCCTAGCTCCAGCCT GGGCACCCAGACCTATATCTGCAACGTGAACCAC AAGCCTAGCAATACCAAGGTGGACAAGAAGGTGG AGCCTAAGAGCTGCGACAAGACCCACACCTGTCC TCCATGTCCTGCTCCAGAACTGCTCGGCGGACCTT CCGTGTTCCTGTTTCCTCCAAAGCCTAAGGACACC CTGATGATCAGCAGAACCCCTGAAGTGACCTGCG TGGTGGTGGATGTGTCCCACGAGGATCCCGAAGT GAAGTTCAATTGGTACGTGGACGGCGTGGAAGTG CACAACGCCAAGACCAAGCCTAGAGAGGAACAG TACAACAGCACCTACAGAGTGGTGTCCGTGCTGA CCGTGCTGCACCAGGATTGGCTGAACGGCAAAGA GTACAAGTGCAAGGTGTCCAACAAGGCCCTGCCT GCTCCTATCGAGAAAACCATCAGCAAGGCCAAGG |
| | | GCCAGCCTAGGGAACCCCAGGTTTACACACTGCC TCCAAGCAGGGACGAGCTGACCAAGAATCAGGT GTCCCTGACCTGCCTGGTCAAGGGCTTCTACCCTT CCGATATCGCCGTGGAATGGGAGAGCAATGGCCA GCCTGAGAACAACTACAAGACAACCCCTCCTGTG CTGGACAGCGACGGCTCATTCTTCCTGTACAGCA AGCTGACAGTGGACAAGAGCAGATGGCAGCAGG GCAACGTGTTCAGCTGCAGCGTGATGCACGAGGC CCTGCACAACCACTACACCCAGAAGTCCCTGAGC CTGTCTCCTGGCAAA |
| 22 | Humanized antibody FS002-A36 heavy chain coding sequence | CAGGTGCAGCTGCAGGAGTCGGGCCCAGGACTG GTGAAGCCTTCACAGACCCTGTCCCTCACCTGCA CTGTCTCTGGTGGCTCCATCAGCAGTGGTGGTTAC TACTGGAGCTGGATCCGCCAGCACCCAGGGAAGG GCCTGGAGTGGATTGGGTACATCTATTACAGTGGG AGCACCTACTACAACCCGTCCCTCAAGAGTCGAG TTACCATATCAGTAGACACGTCTAAGAACCAGTTC TCCCTGAAGCTGAGCTCTGTGACTGCCGCGGACA CGGCCGTGTACTACTGTGCGAGAGTCCGCCTATTG GGTGGTGGTTTTGATATCTGGGGCCAAGGGACCA CGGTCACCGTCTCATCAGCTTCCACCAAGGGCCC CTCCGTGTTCCCCCTGGCTCCCTCTTCCAAGAGCA CCAGCGGCGGCACCGCTGCTCTGGGATGTCTGGT GAAGGACTACTTCCCTGAGCCTGTGACCGTGTCC TGGAATTCCGGCGCCCTGACCTCCGGCGTGCACA CATTCCCTGCTGTGCTGCAGTCCTCCGGCCTGTAT AGCCTGTCCTCCGTGGTGACAGTGCCTAGCTCCA GCCTGGGCACCCAGACCTATATCTGCAACGTGAA CCACAAGCCTAGCAATACCAAGGTGGACAAGAAG GTGGAGCCTAAGAGCTGCGACAAGACCCACACCT GTCCTCCATGTCCTGCTCCAGAACTGCTCGGCGG ACCTTCCGTGTTCCTGTTTCCTCCAAAGCCTAAGG ACACCCTGATGATCAGCAGAACCCCTGAAGTGAC CTGCGTGGTGGTGGATGTGTCCCACGAGGATCCC GAAGTGAAGTTCAATTGGTACGTGGACGGCGTGG AAGTGCACAACGCCAAGACCAAGCCTAGAGAGG AACAGTACAACAGCACCTACAGAGTGGTGTCCGT GCTGACCGTGCTGCACCAGGATTGGCTGAACGGC AAAGAGTACAAGTGCAAGGTGTCCAACAAGGCC CTGCCTGCTCCTATCGAGAAAACCATCAGCAAGG CCAAGGGCCAGCCTAGGGAACCCCAGGTTTACAC ACTGCCTCCAAGCAGGGACGAGCTGACCAAGAAT CAGGTGTCCCTGACCTGCCTGGTCAAGGGCTTCT ACCCTTCCGATATCGCCGTGGAATGGGAGAGCAAT GGCCAGCCTGAGAACAACTACAAGACAACCCCTC CTGTGCTGGACAGCGACGGCTCATTCTTCCTGTAC AGCAAGCTGACAGTGGACAAGAGCAGATGGCAG CAGGGCAACGTGTTCAGCTGCAGCGTGATGCACG AGGCCCTGCACAACCACTACACCCAGAAGTCCCT GAGCCTGTCTCCTGGCAAA |
| 23 | Humanized antibody FS002-A66 heavy chain coding sequence | - |
| | | CTGCAAATGAACAGCCTGAGAGCTGAGGACACG GCTGTGTATTACTGTGCGAGAGATCGTATAACGTA TCTTGACTACTACTACTACGGTATGGACGTCTGGG GCCAAGGGACCACGGTCACCGTCTCATCAGCTTC CACCAAGGGCCCCTCCGTGTTCCCCCTGGCTCCCT CTTCCAAGAGCACCAGCGGCGGCACCGCTGCTCT GGGATGTCTGGTGAAGGACTACTTCCCTGAGCCT GTGACCGTGTCCTGGAATTCCGGCGCCCTGACCT CCGGCGTGCACACATTCCCTGCTGTGCTGCAGTCC TCCGGCCTGTATAGCCTGTCCTCCGTGGTGACAGT GCCTAGCTCCAGCCTGGGCACCCAGACCTATATCT GCAACGTGAACCACAAGCCTAGCAATACCAAGGT GGACAAGAAGGTGGAGCCTAAGAGCTGCGACAA GACCCACACCTGTCCTCCATGTCCTGCTCCAGAAC TGCTCGGCGGACCTTCCGTGTTCCTGTTTCCTCCA AAGCCTAAGGACACCCTGATGATCAGCAGAACCC CTGAAGTGACCTGCGTGGTGGTGGATGTGTCCCA CGAGGATCCCGAAGTGAAGTTCAATTGGTACGTG GACGGCGTGGAAGTGCACAACGCCAAGACCAAG CCTAGAGAGGAACAGTACAACAGCACCTACAGAG TGGTGTCCGTGCTGACCGTGCTGCACCAGGATTG GCTGAACGGCAAAGAGTACAAGTGCAAGGTGTC CAACAAGGCCCTGCCTGCTCCTATCGAGAAAACC ATCAGCAAGGCCAAGGGCCAGCCTAGGGAACCCC AGGTTTACACACTGCCTCCAAGCAGGGACGAGCT GACCAAGAATCAGGTGTCCCTGACCTGCCTGGTC AAGGGCTTCTACCCTTCCGATATCGCCGTGGAATG GGAGAGCAATGGCCAGCCTGAGAACAACTACAA GACAACCCCTCCTGTGCTGGACAGCGACGGCTCA TTCTTCCTGTACAGCAAGCTGACAGTGGACAAGA GCAGATGGCAGCAGGGCAACGTGTTCAGCTGCAG CGTGATGCACGAGGCCCTGCACAACCACTACACC CAGAAGTCCCTGAGCCTGTCTCCTGGCAAA |
| 24 | Humanized antibody FS002-A10 heavy chain coding sequence | CAGGTACAGCTGCAGCAGTCAGGTCCAGGACTGG TGAAGCCCTCGCAGACCCTCTCACTTACCTGTGCC ATCTCCGGGGACAGTGTCTCTAGCGACAGTGCTG CTTGGAACTGGATCAGGCAGTCCCCATCGAGAGG CCTTGAGTGGCTGGGAAGGACATACTACAGGTCC AAGTGGTATAATGATTATGCAGTATCTGTGAAAAG TCGAATAACCATCAACCCAGACACATCCAAGAAC CAGTTCTCCCTGCAGCTAAATTCTGTGACTTCCGA CGACACGGCTGTGTATTACTGTGCAAGAGGCTCTT GGGCGGCTCCATGGTTGTGGGGCCAGGGAACCCT GGTCACCGTCTCATCAGCTTCCACCAAGGGCCCC TCCGTGTTCCCCCTGGCTCCCTCTTCCAAGAGCAC CAGCGGCGGCACCGCTGCTCTGGGATGTCTGGTG AAGGACTACTTCCCTGAGCCTGTGACCGTGTCCT GGAATTCCGGCGCCCTGACCTCCGGCGTGCACAC ATTCCCTGCTGTGCTGCAGTCCTCCGGCCTGTATA GCCTGTCCTCCGTGGTGACAGTGCCTAGCTCCAG CCTGGGCACCCAGACCTATATCTGCAACGTGAACC ACAAGCCTAGCAATACCAAGGTGGACAAGAAGGT GGAGCCTAAGAGCTGCGACAAGACCCACACCTGT CCTCCATGTCCTGCTCCAGAACTGCTCGGCGGAC CTTCCGTGTTCCTGTTTCCTCCAAAGCCTAAGGAC ACCCTGATGATCAGCAGAACCCCTGAAGTGACCT GCGTGGTGGTGGATGTGTCCCACGAGGATCCCGA |
| | | AGTGAAGTTCAATTGGTACGTGGACGGCGTGGAA GTGCACAACGCCAAGACCAAGCCTAGAGAGGAA CAGTACAACAGCACCTACAGAGTGGTGTCCGTGC TGACCGTGCTGCACCAGGATTGGCTGAACGGCAA AGAGTACAAGTGCAAGGTGTCCAACAAGGCCCTG CCTGCTCCTATCGAGAAAACCATCAGCAAGGCCA AGGGCCAGCCTAGGGAACCCCAGGTTTACACACT GCCTCCAAGCAGGGACGAGCTGACCAAGAATCA GGTGTCCCTGACCTGCCTGGTCAAGGGCTTCTAC CCTTCCGATATCGCCGTGGAATGGGAGAGCAATG GCCAGCCTGAGAACAACTACAAGACAACCCCTCC TGTGCTGGACAGCGACGGCTCATTCTTCCTGTACA GCAAGCTGACAGTGGACAAGAGCAGATGGCAGC AGGGCAACGTGTTCAGCTGCAGCGTGATGCACGA GGCCCTGCACAACCACTACACCCAGAAGTCCCTG AGCCTGTCTCCTGGCAAA |
| 25 | Humanized antibody FS002-C9 heavy chain coding sequence | CAGGTGCAGCTGTTGGAGTCTGGCCCAGGACTGG TGAAGCCTTCACAGACCGTGTCCCTCACCTGCAC TGTCTCTGGTGTCTCCATCAGCAGAGGTGTTTACT CCTGGAGCTGGATCCGCCAGCACCCAGGGAAGGG CCTCGAATGGATTGGGTTCATCGATTACAATGGGA GAACCGACTACAACCCGTCCCTCAAGAGTCGAGT TACCATATCACAAGACACGTCTAAGAACGACTTCT CCCTGAAGCTGACGTCTGTGACTGCCGCGGACAC GGCCATATATTACTGTGCGAGAGGTTTGCATGCTT TTGATATCTGGGGCCAAGGGACAATGGTCACCGT CTCATCAGCTTCCACCAAGGGCCCCTCCGTGTTCC CCCTGGCTCCCTCTTCCAAGAGCACCAGCGGCGG CACCGCTGCTCTGGGATGTCTGGTGAAGGACTAC TTCCCTGAGCCTGTGACCGTGTCCTGGAATTCCGG CGCCCTGACCTCCGGCGTGCACACATTCCCTGCTG TGCTGCAGTCCTCCGGCCTGTATAGCCTGTCCTCC GTGGTGACAGTGCCTAGCTCCAGCCTGGGCACCC AGACCTATATCTGCAACGTGAACCACAAGCCTAG CAATACCAAGGTGGACAAGAAGGTGGAGCCTAAG AGCTGCGACAAGACCCACACCTGTCCTCCATGTC CTGCTCCAGAACTGCTCGGCGGACCTTCCGTGTT CCTGTTTCCTCCAAAGCCTAAGGACACCCTGATGA TCAGCAGAACCCCTGAAGTGACCTGCGTGGTGGT GGATGTGTCCCACGAGGATCCCGAAGTGAAGTTC AATTGGTACGTGGACGGCGTGGAAGTGCACAACG CCAAGACCAAGCCTAGAGAGGAACAGTACAACA GCACCTACAGAGTGGTGTCCGTGCTGACCGTGCT GCACCAGGATTGGCTGAACGGCAAAGAGTACAA GTGCAAGGTGTCCAACAAGGCCCTGCCTGCTCCT ATCGAGAAAACCATCAGCAAGGCCAAGGGCCAG CCTAGGGAACCCCAGGTTTACACACTGCCTCCAA GCAGGGACGAGCTGACCAAGAATCAGGTGTCCCT GACCTGCCTGGTCAAGGGCTTCTACCCTTCCGATA TCGCCGTGGAATGGGAGAGCAATGGCCAGCCTGA GAACAACTACAAGACAACCCCTCCTGTGCTGGAC AGCGACGGCTCATTCTTCCTGTACAGCAAGCTGA CAGTGGACAAGAGCAGATGGCAGCAGGGCAACG TGTTCAGCTGCAGCGTGATGCACGAGGCCCTGCA CAACCACTACACCCAGAAGTCCCTGAGCCTGTCT CCTGGCAAA |
| 26 | Humanized antibody | CGGCTGCAGCTGCAGGAGTCGGGCCCACGACTGG |
| | FS002-C11 heavy chain coding sequence | TGAAGCCTTCGGAGACCCTGTCCCTCACCTGCAG CGTCTCTGGTGGCTCCATCAGCAGTAGGAATCACT ACTGGGCCTGGATCCGCCAGCCCCCAGGGAAGGG CCTGGAGTGGATTGGGTACATCTATTACAGTGGGA GCACCTACTACAACCCGTCCCTCAAGAGTCGAGT TACCATATCAGTAGACACGTCTAAGAACCAGTTCT CCCTGAAGCTGAGCTCTGTGACTGCCGCGGACAC GGCCGTGTATTACTGTGCGAGAGTAGGGCTGCTG CTCCGGGACTTTGACTACTGGGGCCAGGGAACCC TGGTCACCGTCTCATCAGCTTCCACCAAGGGCCC CTCCGTGTTCCCCCTGGCTCCCTCTTCCAAGAGCA CCAGCGGCGGCACCGCTGCTCTGGGATGTCTGGT GAAGGACTACTTCCCTGAGCCTGTGACCGTGTCC TGGAATTCCGGCGCCCTGACCTCCGGCGTGCACA CATTCCCTGCTGTGCTGCAGTCCTCCGGCCTGTAT AGCCTGTCCTCCGTGGTGACAGTGCCTAGCTCCA GCCTGGGCACCCAGACCTATATCTGCAACGTGAA CCACAAGCCTAGCAATACCAAGGTGGACAAGAAG GTGGAGCCTAAGAGCTGCGACAAGACCCACACCT GTCCTCCATGTCCTGCTCCAGAACTGCTCGGCGG ACCTTCCGTGTTCCTGTTTCCTCCAAAGCCTAAGG ACACCCTGATGATCAGCAGAACCCCTGAAGTGAC CTGCGTGGTGGTGGATGTGTCCCACGAGGATCCC GAAGTGAAGTTCAATTGGTACGTGGACGGCGTGG AAGTGCACAACGCCAAGACCAAGCCTAGAGAGG AACAGTACAACAGCACCTACAGAGTGGTGTCCGT GCTGACCGTGCTGCACCAGGATTGGCTGAACGGC AAAGAGTACAAGTGCAAGGTGTCCAACAAGGCC CTGCCTGCTCCTATCGAGAAAACCATCAGCAAGG CCAAGGGCCAGCCTAGGGAACCCCAGGTTTACAC ACTGCCTCCAAGCAGGGACGAGCTGACCAAGAAT CAGGTGTCCCTGACCTGCCTGGTCAAGGGCTTCT ACCCTTCCGATATCGCCGTGGAATGGGAGAGCAAT GGCCAGCCTGAGAACAACTACAAGACAACCCCTC CTGTGCTGGACAGCGACGGCTCATTCTTCCTGTAC AGCAAGCTGACAGTGGACAAGAGCAGATGGCAG CAGGGCAACGTGTTCAGCTGCAGCGTGATGCACG AGGCCCTGCACAACCACTACACCCAGAAGTCCCT GAGCCTGTCTCCTGGCAAA |
| 27 | Humanized antibody FS002-C216 heavy chain coding sequence | GAGGTGCAGCTGGTGGAGTCTGGGGGAGGCGTG GTCCAGCCTGGGAGGTCCCTGAGACTCTCCTGTG CAGCCTCTGGATTCACCTTCAGTAGCTATGCTATG CACTGGGTCCGCCAGGCTCCAGGCAAGGGGCTGG AGTGGGTGGCAGTTATATCATATGATGGAAGTAAT AAATACTACGCAGACTCCGTGAAGGGCCGATTCA CCATCTCCAGAGACAATTCCAAGAACACGCTGTAT CTGCAAATGAACAGCCTGAGAGCTGAGGACACG GCTGTGTATTACTGTGCGAGGGATTGTAGTGGTGG TAGCTGCTATGATGCTTTTGATATCTGGGGCCAAG GGACAATGGTCACCGTCTCATCAGCTTCCACCAA GGGCCCCTCCGTGTTCCCCCTGGCTCCCTCTTCCA AGAGCACCAGCGGCGGCACCGCTGCTCTGGGATG TCTGGTGAAGGACTACTTCCCTGAGCCTGTGACC GTGTCCTGGAATTCCGGCGCCCTGACCTCCGGCG TGCACACATTCCCTGCTGTGCTGCAGTCCTCCGGC CTGTATAGCCTGTCCTCCGTGGTGACAGTGCCTAG CTCCAGCCTGGGCACCCAGACCTATATCTGCAACG |
| | | TGAACCACAAGCCTAGCAATACCAAGGTGGACAA GAAGGTGGAGCCTAAGAGCTGCGACAAGACCCA CACCTGTCCTCCATGTCCTGCTCCAGAACTGCTCG GCGGACCTTCCGTGTTCCTGTTTCCTCCAAAGCCT AAGGACACCCTGATGATCAGCAGAACCCCTGAAG TGACCTGCGTGGTGGTGGATGTGTCCCACGAGGA TCCCGAAGTGAAGTTCAATTGGTACGTGGACGGC GTGGAAGTGCACAACGCCAAGACCAAGCCTAGA GAGGAACAGTACAACAGCACCTACAGAGTGGTGT CCGTGCTGACCGTGCTGCACCAGGATTGGCTGAA CGGCAAAGAGTACAAGTGCAAGGTGTCCAACAA GGCCCTGCCTGCTCCTATCGAGAAAACCATCAGC AAGGCCAAGGGCCAGCCTAGGGAACCCCAGGTTT ACACACTGCCTCCAAGCAGGGACGAGCTGACCA AGAATCAGGTGTCCCTGACCTGCCTGGTCAAGGG CTTCTACCCTTCCGATATCGCCGTGGAATGGGAGA GCAATGGCCAGCCTGAGAACAACTACAAGACAAC CCCTCCTGTGCTGGACAGCGACGGCTCATTCTTCC TGTACAGCAAGCTGACAGTGGACAAGAGCAGATG GCAGCAGGGCAACGTGTTCAGCTGCAGCGTGATG CACGAGGCCCTGCACAACCACTACACCCAGAAGT CCCTGAGCCTGTCTCCTGGCAAA |
| 28 | Humanized antibody FS002-C375 heavy chain coding sequence | CAGGTGCAGCTACAGGAGTCGGGCCCAGGACTG GTGAAGCCTTCACAGACCCTGTCCCTCACCTGCA CTGTCTCTGGTGGCTCCATCAGCAGTGGTGGTTAC TACTGGAGCTGGATCCGCCAGCACCCAGGGAAGG GCCTGGAGTGGATTGGGTACATCTATTACAGTGGG AGCACCTACTACAACCCGTCCCTCAAGAGTCGAG TTACCATATCAGTAGACACGTCTAAGAACCAGTTC TCCCTGAAGCTGAGCTCTGTGACTGCCGCGGACA CGGCCGTGTATTACTGTGCGAGAGTTGGAGTAGC CCTTCACTACGGTATGGACGTCTGGGGCCAAGGG ACCACGGTCACCGTCTCATCAGCTTCCACCAAGG GCCCCTCCGTGTTCCCCCTGGCTCCCTCTTCCAAG AGCACCAGCGGCGGCACCGCTGCTCTGGGATGTC TGGTGAAGGACTACTTCCCTGAGCCTGTGACCGT GTCCTGGAATTCCGGCGCCCTGACCTCCGGCGTG CACACATTCCCTGCTGTGCTGCAGTCCTCCGGCCT GTATAGCCTGTCCTCCGTGGTGACAGTGCCTAGCT CCAGCCTGGGCACCCAGACCTATATCTGCAACGTG AACCACAAGCCTAGCAATACCAAGGTGGACAAGA AGGTGGAGCCTAAGAGCTGCGACAAGACCCACA CCTGTCCTCCATGTCCTGCTCCAGAACTGCTCGGC GGACCTTCCGTGTTCCTGTTTCCTCCAAAGCCTAA GGACACCCTGATGATCAGCAGAACCCCTGAAGTG ACCTGCGTGGTGGTGGATGTGTCCCACGAGGATC CCGAAGTGAAGTTCAATTGGTACGTGGACGGCGT GGAAGTGCACAACGCCAAGACCAAGCCTAGAGA GGAACAGTACAACAGCACCTACAGAGTGGTGTCC GTGCTGACCGTGCTGCACCAGGATTGGCTGAACG GCAAAGAGTACAAGTGCAAGGTGTCCAACAAGG CCCTGCCTGCTCCTATCGAGAAAACCATCAGCAA GGCCAAGGGCCAGCCTAGGGAACCCCAGGTTTAC ACACTGCCTCCAAGCAGGGACGAGCTGACCAAG AATCAGGTGTCCCTGACCTGCCTGGTCAAGGGCT TCTACCCTTCCGATATCGCCGTGGAATGGGAGAGC AATGGCCAGCCTGAGAACAACTACAAGACAACCC |
| | | CTCCTGTGCTGGACAGCGACGGCTCATTCTTCCTG TACAGCAAGCTGACAGTGGACAAGAGCAGATGG CAGCAGGGCAACGTGTTCAGCTGCAGCGTGATGC ACGAGGCCCTGCACAACCACTACACCCAGAAGTC CCTGAGCCTGTCTCCTGGCAAA |
| 29 | Humanized antibody FS002-C417 heavy chain coding sequence | GAGGTGCAGCTGGTGGAGTCTGGGGGAGTCGTG GTACAGCCTGGGGGGTCCCTGAGACTCTCCTGTG CAGCCTCTGGATTCACCTTTGATGATTATACCATGC ACTGGGTCCGTCAAGCTCCGGGGAAGGGTCTGGA GTGGGTCTCTCTTATTAGTTGGGATGGTGGTAGCA CATACTATGCAGACTCTGTGAAGGGCCGATTCACC ATCTCCAGAGACAACGCCAAGAACACGCTGTATC TGCAAATGAACAGTCTGAGAGCCGAGGACACGG CTGTGTATTACTGTGCAAGAGATACGGGGTACGCT ATGGATGTCTGGGGCCAAGGGACAATGGTCACCG TCTCATCAGCTTCCACCAAGGGCCCCTCCGTGTTC CCCCTGGCTCCCTCTTCCAAGAGCACCAGCGGCG GCACCGCTGCTCTGGGATGTCTGGTGAAGGACTA CTTCCCTGAGCCTGTGACCGTGTCCTGGAATTCCG GCGCCCTGACCTCCGGCGTGCACACATTCCCTGCT GTGCTGCAGTCCTCCGGCCTGTATAGCCTGTCCTC CGTGGTGACAGTGCCTAGCTCCAGCCTGGGCACC CAGACCTATATCTGCAACGTGAACCACAAGCCTA GCAATACCAAGGTGGACAAGAAGGTGGAGCCTAA GAGCTGCGACAAGACCCACACCTGTCCTCCATGT CCTGCTCCAGAACTGCTCGGCGGACCTTCCGTGT TCCTGTTTCCTCCAAAGCCTAAGGACACCCTGATG ATCAGCAGAACCCCTGAAGTGACCTGCGTGGTGG TGGATGTGTCCCACGAGGATCCCGAAGTGAAGTT CAATTGGTACGTGGACGGCGTGGAAGTGCACAAC GCCAAGACCAAGCCTAGAGAGGAACAGTACAAC AGCACCTACAGAGTGGTGTCCGTGCTGACCGTGC TGCACCAGGATTGGCTGAACGGCAAAGAGTACAA GTGCAAGGTGTCCAACAAGGCCCTGCCTGCTCCT ATCGAGAAAACCATCAGCAAGGCCAAGGGCCAG CCTAGGGAACCCCAGGTTTACACACTGCCTCCAA GCAGGGACGAGCTGACCAAGAATCAGGTGTCCCT GACCTGCCTGGTCAAGGGCTTCTACCCTTCCGATA TCGCCGTGGAATGGGAGAGCAATGGCCAGCCTGA GAACAACTACAAGACAACCCCTCCTGTGCTGGAC AGCGACGGCTCATTCTTCCTGTACAGCAAGCTGA CAGTGGACAAGAGCAGATGGCAGCAGGGCAACG TGTTCAGCTGCAGCGTGATGCACGAGGCCCTGCA CAACCACTACACCCAGAAGTCCCTGAGCCTGTCT CCTGGCAAA |
| 30 | Humanized antibody FS002-B27 heavy chain coding sequence | CAGGTCCAGCTGGTGCAGTCTGGGGCTGAGGTGA AGAAGCCTGGGTCCTCGGTGAAGGTCTCCTGCAA GGCTTCTGGAGGCACCTTCAGCAGCTATGCTATCA GCTGGGTGCGACAGGCCCCTGGACAAGGGCTTGA GTGGATGGGAAGGATCATCCCTATCCTTGGTATAG CAAACTACGCACAGAAGTTCCAGGGCAGAGTCAC GATTACCGCGGACAAATCCACGAGCACAGCCTAC ATGGAGCTGAGCAGCCTGAGATCTGAGGACACGG CCGTGTATTACTGTGCGAGAGCGGAGGGCAGCAG CTGGTACTACTTTGACTACTGGGGCCAGGGAACC CTGGTCACCGTCTCATCAGCTTCCACCAAGGGCC CCTCCGTGTTCCCCCTGGCTCCCTCTTCCAAGAGC |
| | | ACCAGCGGCGGCACCGCTGCTCTGGGATGTCTGG TGAAGGACTACTTCCCTGAGCCTGTGACCGTGTC CTGGAATTCCGGCGCCCTGACCTCCGGCGTGCAC ACATTCCCTGCTGTGCTGCAGTCCTCCGGCCTGTA TAGCCTGTCCTCCGTGGTGACAGTGCCTAGCTCCA GCCTGGGCACCCAGACCTATATCTGCAACGTGAA CCACAAGCCTAGCAATACCAAGGTGGACAAGAAG GTGGAGCCTAAGAGCTGCGACAAGACCCACACCT GTCCTCCATGTCCTGCTCCAGAACTGCTCGGCGG ACCTTCCGTGTTCCTGTTTCCTCCAAAGCCTAAGG ACACCCTGATGATCAGCAGAACCCCTGAAGTGAC CTGCGTGGTGGTGGATGTGTCCCACGAGGATCCC GAAGTGAAGTTCAATTGGTACGTGGACGGCGTGG AAGTGCACAACGCCAAGACCAAGCCTAGAGAGG AACAGTACAACAGCACCTACAGAGTGGTGTCCGT GCTGACCGTGCTGCACCAGGATTGGCTGAACGGC AAAGAGTACAAGTGCAAGGTGTCCAACAAGGCC CTGCCTGCTCCTATCGAGAAAACCATCAGCAAGG CCAAGGGCCAGCCTAGGGAACCCCAGGTTTACAC ACTGCCTCCAAGCAGGGACGAGCTGACCAAGAAT CAGGTGTCCCTGACCTGCCTGGTCAAGGGCTTCT ACCCTTCCGATATCGCCGTGGAATGGGAGAGCAAT GGCCAGCCTGAGAACAACTACAAGACAACCCCTC CTGTGCTGGACAGCGACGGCTCATTCTTCCTGTAC AGCAAGCTGACAGTGGACAAGAGCAGATGGCAG CAGGGCAACGTGTTCAGCTGCAGCGTGATGCACG AGGCCCTGCACAACCACTACACCCAGAAGTCCCT GAGCCTGTCTCCTGGCAAA |
| 31 | Humanized antibody FS002-C330 heavy chain coding sequence | CAGCTGCAGCTGCAGGAGTCGGGCCCAGGACTG GTGAAGCCTTCGGGGACCCTGTCCCTCACCTGCG CTGTCTCTGGTGGCTCCATCAGCAGTAGTAACTGG TGGAGTTGGGTCCGCCAGCCCCCAGGGAAGGGG CTGGAGTGGATTGGGGAAATCTATCATAGTGGGAG CACCAACTACAACCCGTCCCTCAAGAGTCGAGTC ACCATATCAGTAGACAAGTCCAAGAACCAGTTCT CCCTGAAGCTGAGCTCTGTGACCGCCGCGGACAC GGCCGTGTATTACTGTGCGAGAGCCCACTCCCGAT ATTACTATGGCCTTGACTACTGGGGCCAGGGAACC CTGGTCACCGTCTCATCAGCTTCCACCAAGGGCC CCTCCGTGTTCCCCCTGGCTCCCTCTTCCAAGAGC ACCAGCGGCGGCACCGCTGCTCTGGGATGTCTGG TGAAGGACTACTTCCCTGAGCCTGTGACCGTGTC CTGGAATTCCGGCGCCCTGACCTCCGGCGTGCAC ACATTCCCTGCTGTGCTGCAGTCCTCCGGCCTGTA TAGCCTGTCCTCCGTGGTGACAGTGCCTAGCTCCA GCCTGGGCACCCAGACCTATATCTGCAACGTGAA CCACAAGCCTAGCAATACCAAGGTGGACAAGAAG GTGGAGCCTAAGAGCTGCGACAAGACCCACACCT GTCCTCCATGTCCTGCTCCAGAACTGCTCGGCGG ACCTTCCGTGTTCCTGTTTCCTCCAAAGCCTAAGG ACACCCTGATGATCAGCAGAACCCCTGAAGTGAC CTGCGTGGTGGTGGATGTGTCCCACGAGGATCCC GAAGTGAAGTTCAATTGGTACGTGGACGGCGTGG AAGTGCACAACGCCAAGACCAAGCCTAGAGAGG AACAGTACAACAGCACCTACAGAGTGGTGTCCGT GCTGACCGTGCTGCACCAGGATTGGCTGAACGGC AAAGAGTACAAGTGCAAGGTGTCCAACAAGGCC |
| | | CTGCCTGCTCCTATCGAGAAAACCATCAGCAAGG CCAAGGGCCAGCCTAGGGAACCCCAGGTTTACAC ACTGCCTCCAAGCAGGGACGAGCTGACCAAGAAT CAGGTGTCCCTGACCTGCCTGGTCAAGGGCTTCT ACCCTTCCGATATCGCCGTGGAATGGGAGAGCAAT GGCCAGCCTGAGAACAACTACAAGACAACCCCTC CTGTGCTGGACAGCGACGGCTCATTCTTCCTGTAC AGCAAGCTGACAGTGGACAAGAGCAGATGGCAG CAGGGCAACGTGTTCAGCTGCAGCGTGATGCACG AGGCCCTGCACAACCACTACACCCAGAAGTCCCT GAGCCTGTCTCCTGGCAAA |
| 32 | Humanized antibody FS002-C153 heavy chain coding sequence | CAGGTGCAGCTACAACAGTGGGGGGCAGAGCTT GTGAAGCCAGGGGCCTCAGTCAAGTTGTCCTGCA CAGCTTCTGGCTTCAACATTAAAGACACCTATATG CACTGGGTGAAGCAGAGGCCTGAACAGGGCCTG GAGTGGATTGGAAGGATTGATCCTGCGAATGGTAA TACTAAATATGACCCGAAGTTCCAGGGCAAGGCC ACTATAACAGCAGACACATCCTCCAACACAGCCT ACCTGCAGCTCAGCAGCCTGACATCTGAGGACAC TGCCGTCTATTACTGTGCTAGATCCTACTATAGGTA CGACGGGTACTTCGATGTCTGGGGCGCAGGGACA ATGGTCACCGTCTCATCAGCTTCCACCAAGGGCCC CTCCGTGTTCCCCCTGGCTCCCTCTTCCAAGAGCA CCAGCGGCGGCACCGCTGCTCTGGGATGTCTGGT GAAGGACTACTTCCCTGAGCCTGTGACCGTGTCC TGGAATTCCGGCGCCCTGACCTCCGGCGTGCACA CATTCCCTGCTGTGCTGCAGTCCTCCGGCCTGTAT AGCCTGTCCTCCGTGGTGACAGTGCCTAGCTCCA GCCTGGGCACCCAGACCTATATCTGCAACGTGAA CCACAAGCCTAGCAATACCAAGGTGGACAAGAAG GTGGAGCCTAAGAGCTGCGACAAGACCCACACCT GTCCTCCATGTCCTGCTCCAGAACTGCTCGGCGG ACCTTCCGTGTTCCTGTTTCCTCCAAAGCCTAAGG ACACCCTGATGATCAGCAGAACCCCTGAAGTGAC CTGCGTGGTGGTGGATGTGTCCCACGAGGATCCC GAAGTGAAGTTCAATTGGTACGTGGACGGCGTGG AAGTGCACAACGCCAAGACCAAGCCTAGAGAGG AACAGTACAACAGCACCTACAGAGTGGTGTCCGT GCTGACCGTGCTGCACCAGGATTGGCTGAACGGC AAAGAGTACAAGTGCAAGGTGTCCAACAAGGCC CTGCCTGCTCCTATCGAGAAAACCATCAGCAAGG CCAAGGGCCAGCCTAGGGAACCCCAGGTTTACAC ACTGCCTCCAAGCAGGGACGAGCTGACCAAGAAT CAGGTGTCCCTGACCTGCCTGGTCAAGGGCTTCT ACCCTTCCGATATCGCCGTGGAATGGGAGAGCAAT GGCCAGCCTGAGAACAACTACAAGACAACCCCTC CTGTGCTGGACAGCGACGGCTCATTCTTCCTGTAC AGCAAGCTGACAGTGGACAAGAGCAGATGGCAG CAGGGCAACGTGTTCAGCTGCAGCGTGATGCACG AGGCCCTGCACAACCACTACACCCAGAAGTCCCT GAGCCTGTCTCCTGGCAAA |
| 33 | Humanized antibody FS002-C241 heavy chain coding sequence | CGGCTGCAGCTGCAGGAGTCGGGCCCAGGACTG GTGAAGCCTTCGGAGACCCTGTCCCTCACCTGCA CTGTCTCTGGTGGCTCCATCAGCAGTAGTAGTTAC TACTGGGGCTGGATCCGCCAGCCCCCAGGGAAGG GGCTGGAGTGGATTGGGAGTATCTATTATAGTGGG AGCACCTACTACAACCCGTCCCTCAAGAGTCGAG |
| | | TCACCATATCCGTAGACACGTCCAAGAACCAGTTC TCCCTGAAGCTGAGCTCTGTGACCGCCGCAGACA CGGCTGTGTATTACTGTGCGAGACGGTACAGCTAT GGTAGAGATGCTTTTGATATCTGGGGCCAAGGGAC AATGGTCACCGTCTCATCAGCTTCCACCAAGGGC CCCTCCGTGTTCCCCCTGGCTCCCTCTTCCAAGAG CACCAGCGGCGGCACCGCTGCTCTGGGATGTCTG GTGAAGGACTACTTCCCTGAGCCTGTGACCGTGT CCTGGAATTCCGGCGCCCTGACCTCCGGCGTGCA CACATTCCCTGCTGTGCTGCAGTCCTCCGGCCTGT ATAGCCTGTCCTCCGTGGTGACAGTGCCTAGCTCC AGCCTGGGCACCCAGACCTATATCTGCAACGTGA ACCACAAGCCTAGCAATACCAAGGTGGACAAGAA GGTGGAGCCTAAGAGCTGCGACAAGACCCACAC CTGTCCTCCATGTCCTGCTCCAGAACTGCTCGGCG GACCTTCCGTGTTCCTGTTTCCTCCAAAGCCTAAG GACACCCTGATGATCAGCAGAACCCCTGAAGTGA CCTGCGTGGTGGTGGATGTGTCCCACGAGGATCC CGAAGTGAAGTTCAATTGGTACGTGGACGGCGTG GAAGTGCACAACGCCAAGACCAAGCCTAGAGAG GAACAGTACAACAGCACCTACAGAGTGGTGTCCG TGCTGACCGTGCTGCACCAGGATTGGCTGAACGG CAAAGAGTACAAGTGCAAGGTGTCCAACAAGGC CCTGCCTGCTCCTATCGAGAAAACCATCAGCAAG GCCAAGGGCCAGCCTAGGGAACCCCAGGTTTACA CACTGCCTCCAAGCAGGGACGAGCTGACCAAGAA TCAGGTGTCCCTGACCTGCCTGGTCAAGGGCTTCT ACCCTTCCGATATCGCCGTGGAATGGGAGAGCAAT GGCCAGCCTGAGAACAACTACAAGACAACCCCTC CTGTGCTGGACAGCGACGGCTCATTCTTCCTGTAC AGCAAGCTGACAGTGGACAAGAGCAGATGGCAG CAGGGCAACGTGTTCAGCTGCAGCGTGATGCACG AGGCCCTGCACAACCACTACACCCAGAAGTCCCT GAGCCTGTCTCCTGGCAAA |
| 34 | Humanized antibody FS002-C15 heavy chain coding sequence | CAGCTGCAGCTGCAGGAGTCGGGCCCAGGACTG GTGAAGCCTTCGGAGACCCTGTCCCTCACCTGCA CTGTCTCTGGTGGCTCCATCAGCAGTAGTAGTTAC TACTGGGGCTGGATCCGCCAGCCCCCAGGGAAGG GGCTGGAGTGGATTGGGAGTATCTATTATAGTGGG AGCACCTACTACAACCCGTCCCTCAAGAGTCGAG TCACCATATCCGTAGACACGTCCAAGAACCAGTTC TCCCTGAAGCTGAGCTCTGTGACCGCCGCAGACA CGGCTGTGTATTACTGTGCGAGACTGACGATTTTT GGAGTGGTGCTCTACTGGGGCCAGGGAACCCTGG TCACCGTCTCATCAGCTTCCACCAAGGGCCCCTCC GTGTTCCCCCTGGCTCCCTCTTCCAAGAGCACCA GCGGCGGCACCGCTGCTCTGGGATGTCTGGTGAA GGACTACTTCCCTGAGCCTGTGACCGTGTCCTGG AATTCCGGCGCCCTGACCTCCGGCGTGCACACATT CCCTGCTGTGCTGCAGTCCTCCGGCCTGTATAGCC TGTCCTCCGTGGTGACAGTGCCTAGCTCCAGCCT GGGCACCCAGACCTATATCTGCAACGTGAACCAC AAGCCTAGCAATACCAAGGTGGACAAGAAGGTGG AGCCTAAGAGCTGCGACAAGACCCACACCTGTCC TCCATGTCCTGCTCCAGAACTGCTCGGCGGACCTT CCGTGTTCCTGTTTCCTCCAAAGCCTAAGGACACC CTGATGATCAGCAGAACCCCTGAAGTGACCTGCG |
| | | TGGTGGTGGATGTGTCCCACGAGGATCCCGAAGT GAAGTTCAATTGGTACGTGGACGGCGTGGAAGTG CACAACGCCAAGACCAAGCCTAGAGAGGAACAG TACAACAGCACCTACAGAGTGGTGTCCGTGCTGA CCGTGCTGCACCAGGATTGGCTGAACGGCAAAGA GTACAAGTGCAAGGTGTCCAACAAGGCCCTGCCT GCTCCTATCGAGAAAACCATCAGCAAGGCCAAGG GCCAGCCTAGGGAACCCCAGGTTTACACACTGCC TCCAAGCAGGGACGAGCTGACCAAGAATCAGGT GTCCCTGACCTGCCTGGTCAAGGGCTTCTACCCTT CCGATATCGCCGTGGAATGGGAGAGCAATGGCCA GCCTGAGAACAACTACAAGACAACCCCTCCTGTG CTGGACAGCGACGGCTCATTCTTCCTGTACAGCA AGCTGACAGTGGACAAGAGCAGATGGCAGCAGG GCAACGTGTTCAGCTGCAGCGTGATGCACGAGGC CCTGCACAACCACTACACCCAGAAGTCCCTGAGC CTGTCTCCTGGCAAA |
| 35 | Humanized antibody FS002-C58 heavy chain coding sequence | GAGGTGCAGCTGTTGGAGTCTGGGGGAGGCGTG GTCCAGCCTGGGAGGTCCCTGAGACTCTCCTGTG CAGCCTCTGGATTCACCTTCAGTAGCTATGCTATG CACTGGGTCCGCCAGGCTCCAGGCAAGGGGCTGG AGTGGGTGGCAGTTATATCATATGATGGAAGTAAT AAATACTACGCAGACTCCGTGAAGGGCCGATTCA CCATCTCCAGAGACAATTCCAAGAACACGCTGTAT CTGCAAATGAACAGCCTGAGAGCTGAGGACACG GCTGTGTATTACTGTGCGAGAGGAAAGTACAACT GGTTCGACCCCTGGGGCCAGGGCACCCTGGTCAC CGTCTCATCAGCTTCCACCAAGGGCCCCTCCGTGT TCCCCCTGGCTCCCTCTTCCAAGAGCACCAGCGG CGGCACCGCTGCTCTGGGATGTCTGGTGAAGGAC TACTTCCCTGAGCCTGTGACCGTGTCCTGGAATTC CGGCGCCCTGACCTCCGGCGTGCACACATTCCCT GCTGTGCTGCAGTCCTCCGGCCTGTATAGCCTGTC CTCCGTGGTGACAGTGCCTAGCTCCAGCCTGGGC ACCCAGACCTATATCTGCAACGTGAACCACAAGC CTAGCAATACCAAGGTGGACAAGAAGGTGGAGCC TAAGAGCTGCGACAAGACCCACACCTGTCCTCCA TGTCCTGCTCCAGAACTGCTCGGCGGACCTTCCG TGTTCCTGTTTCCTCCAAAGCCTAAGGACACCCTG ATGATCAGCAGAACCCCTGAAGTGACCTGCGTGG TGGTGGATGTGTCCCACGAGGATCCCGAAGTGAA GTTCAATTGGTACGTGGACGGCGTGGAAGTGCAC AACGCCAAGACCAAGCCTAGAGAGGAACAGTAC AACAGCACCTACAGAGTGGTGTCCGTGCTGACCG TGCTGCACCAGGATTGGCTGAACGGCAAAGAGTA CAAGTGCAAGGTGTCCAACAAGGCCCTGCCTGCT CCTATCGAGAAAACCATCAGCAAGGCCAAGGGCC AGCCTAGGGAACCCCAGGTTTACACACTGCCTCC AAGCAGGGACGAGCTGACCAAGAATCAGGTGTC CCTGACCTGCCTGGTCAAGGGCTTCTACCCTTCCG ATATCGCCGTGGAATGGGAGAGCAATGGCCAGCC TGAGAACAACTACAAGACAACCCCTCCTGTGCTG GACAGCGACGGCTCATTCTTCCTGTACAGCAAGC TGACAGTGGACAAGAGCAGATGGCAGCAGGGCA ACGTGTTCAGCTGCAGCGTGATGCACGAGGCCCT GCACAACCACTACACCCAGAAGTCCCTGAGCCTG TCTCCTGGCAAA |
| 36 | Humanized antibody FS002-A72 heavy chain coding sequence | CAGGTGCAGCTGGTGGACTCTGGGGGGGGCTTGG TACAGCCTGGAGGGTCCCTGAGACTCTCCTGTGT AGCCTCTGGATTCGCCTTCAGTAATTATGAGATGA ACTGGGTCCGCCAGGCTCCAGGGAAGGGGCTGG AGTGGGTTTCATACATTAGTAGTAGTAGTAGTTAC ACAAACTACGCAGACTCTGTGAAGGGCCGATTCA CCATCTCCAGAGACAACGCCAAGAACTCACTGTA TCTGCAAATGAACAGCCTGAGAGCCGAGGACACG GCCGTGTATTACTGTGCGAGAGCCCAGGTTCGGG ATGCTTTTGATATCTGGGGCCAAGGGACCACGGTC ACCGTCTCATCAGCTTCCACCAAGGGCCCCTCCGT GTTCCCCCTGGCTCCCTCTTCCAAGAGCACCAGC GGCGGCACCGCTGCTCTGGGATGTCTGGTGAAGG ACTACTTCCCTGAGCCTGTGACCGTGTCCTGGAAT TCCGGCGCCCTGACCTCCGGCGTGCACACATTCC CTGCTGTGCTGCAGTCCTCCGGCCTGTATAGCCTG TCCTCCGTGGTGACAGTGCCTAGCTCCAGCCTGG GCACCCAGACCTATATCTGCAACGTGAACCACAA GCCTAGCAATACCAAGGTGGACAAGAAGGTGGAG CCTAAGAGCTGCGACAAGACCCACACCTGTCCTC CATGTCCTGCTCCAGAACTGCTCGGCGGACCTTCC GTGTTCCTGTTTCCTCCAAAGCCTAAGGACACCCT GATGATCAGCAGAACCCCTGAAGTGACCTGCGTG GTGGTGGATGTGTCCCACGAGGATCCCGAAGTGA AGTTCAATTGGTACGTGGACGGCGTGGAAGTGCA CAACGCCAAGACCAAGCCTAGAGAGGAACAGTA CAACAGCACCTACAGAGTGGTGTCCGTGCTGACC GTGCTGCACCAGGATTGGCTGAACGGCAAAGAGT ACAAGTGCAAGGTGTCCAACAAGGCCCTGCCTGC TCCTATCGAGAAAACCATCAGCAAGGCCAAGGGC CAGCCTAGGGAACCCCAGGTTTACACACTGCCTC CAAGCAGGGACGAGCTGACCAAGAATCAGGTGT CCCTGACCTGCCTGGTCAAGGGCTTCTACCCTTCC GATATCGCCGTGGAATGGGAGAGCAATGGCCAGC CTGAGAACAACTACAAGACAACCCCTCCTGTGCT GGACAGCGACGGCTCATTCTTCCTGTACAGCAAG CTGACAGTGGACAAGAGCAGATGGCAGCAGGGC AACGTGTTCAGCTGCAGCGTGATGCACGAGGCCC TGCACAACCACTACACCCAGAAGTCCCTGAGCCT GTCTCCTGGCAAA |

**Table 1b: Amino acid sequences (SEQ ID NO: 37-183) of the variable regions, CDRs, and constant regions of the light and heavy chains of the antibody according to the present invention**

| No. | Description | Amino Acid Sequence |
|---|---|---|
| 37 | Humanized antibody FS002-A1 light chain variable region | DIQLTQSPSSLSASVGDRVTITC*RASQSIISYLN*WYQ QKPGKAPKLLIY*AASSLQS*GVPSRFSGSGSGTDFTLT ISSLQPEDFATYYC*QQSYSTPDT*FGQGTKLEIK |
| 38 | Humanized antibody FS002-A1VLCDR1 | ***RASQSIISYLN*** |
| 39 | Humanized antibody FS002-A1VLCDR2 | ***AASSLQS*** |
| 40 | Humanized antibody FS002-A1VLCDR3 | ***QQSYSTPDT*** |
| 41 | Humanized antibody FS002-A5 light chain variable | EIVLTQSPATLSLSPGERATLSCRASQSVSSNLGWYQ QKPGQAPSLLIYGASNRATGIPARFSGSGSGTDFTLTI |
| | region | SSLEPEDFAVYYCQQRDTWPLTFGGGTKVEIK |
| 42 | Humanized antibody FS002-A5VLCDR1 | ***RASQSVSSNLG*** |
| 43 | Humanized antibody FS002-A5VLCDR2 | ***GASNRAT*** |
| 44 | Humanized antibody FS002-A5VLCDR3 | ***QQRDTWPLT*** |
| 45 | Humanized antibody FS002-A15 light chain variable region | EIVLTQSPATLPLSPGERATLSCRASQSVGFYLAWYQ QKPGQPPRLLFYDASKRATGIPARFSASGSGTDFTLTI SGLEPEDFAVYYCQHRISWPLTFGQGTKVEVK |
| 46 | Humanized antibody FS002-A15VLCDR1 | ***RASQSVGFYLA*** |
| 47 | Humanized antibody FS002-A15VLCDR2 | ***DASKRAT*** |
| 48 | Humanized antibody F5002-A15VLCDR3 | ***QHRISWPLT*** |
| 49 | Humanized antibody FS002-A36 light chain variable region | EIVLTQSPATLSLSPGERATLSCRASQSVSTYLAWYQ QKPGQAPRLLIYDTSIRATGIPARFSGSGSGTDFTLTI SSLEPEDFAVYYCQQRSNWPLTFGGGTKVEIK |
| 50 | Humanized antibody FS002-A36VLCDR1 | ***RASQSVSTYLA*** |
| 51 | Humanized antibody FS002-A36VLCDR2 | ***DTSIRAT*** |
| 52 | Humanized antibody FS002-A36VLCDR3 | ***QQRSNWPLT*** |
| 53 | Humanized antibody FS002-A66 light chain variable region | EIVLTQSPAILSLSPGERATLSCRASQSVSEYLAWYQ QKPGQAPRLLIYDISNRATGIPARFSGSGSETDFTLTI SSLEPEDFAVYYCQQRRTWPLTFGPGTKVDIK |
| 54 | Humanized antibody FS002-A66VLCDR1 | ***RASQSVSEYLA*** |
| 55 | Humanized antibody FS002-A66VLCDR2 | ***DISNRAT*** |
| 56 | Humanized antibody FS002-A66VLCDR3 | ***QQRRTWPLT*** |
| 57 | Humanized antibody FS002-A10 light chain variable region | NIQLTQSPPFLSASVGDRVTITCRASQDISSFLAWYQ QKPGKAPNLLIHAATTLQSGVPSRFSGSGSGTEFTLT ISSLRPEDFATYYCQQLHTYPITFGQGTRLEIK |
| 58 | Humanized antibody FS002-A10VLCDR1 | ***RASQDISSFLA*** |
| 59 | Humanized antibody FS002-A10VLCDR2 | ***AATTLQS*** |
| 60 | Humanized antibody FS002-A10VLCDR3 | ***QQLHTYPIT*** |
| 61 | Humanized antibody FS002-C9 light chain variable region | NIQMTQSPPSLSASVGDRVAITC**RASQDISNSLA**WY QQKSGQPPKLLLY**DVSKLER**GVPSRFSGSGSRTDYT LTISSLQPEDFATYYC**QQYSSDPT**FGQGSKLEFK |
| 62 | Humanized antibody FS002-C9VLCDR1 | ***RASQDISNSLA*** |
| 63 | Humanized antibody FS002-C9VLCDR2 | ***DVSKLER*** |
| 64 | Humanized antibody FS002-C9VLCDR3 | ***QQYSSDPT*** |
| 65 | Humanized antibody FS002-C11 light chain variable region | DIQMIQSPSSVSASVGDRVTITC RASQGISNSLA WYQ HKPGTTPKLLIY AASSLQG GVPSRFSGSSSETDFTLTI SSLQPEDFATYYC QQANSFPIT FGQGTRLDIK |
| 66 | Humanized antibody FS002-C11VLCDR1 | ***RASQGISNSLA*** |
| 67 | Humanized antibody F5002-C11VLCDR2 | ***AASSLQG*** |
| 68 | Humanized antibody FS002-C11VLCDR3 | ***QQANSFPIT*** |
| 69 | Humanized antibody FS002-C216 light chain variable region | EIVLTQSPATLSLSPGERATLSC**RASQSVSSYLA**WYQ QKPGQAPRLLIY**DASNRAT**GIPARFSGSGSGTDFTLTI SSLEPEDFAVYYC**QQRTNWLMYT**FGQGTKLEIK |
| 70 | Humanized antibody FS002-C216VLCDR1 | ***RASQSVSSYLA*** |
| 71 | Humanized antibody FS002-C216VLCDR2 | ***DASNRAT*** |
| 72 | Humanized antibody FS002-C216VLCDR3 | ***QQRTNWLMYT*** |
| 73 | Humanized antibody FS002-C375 light chain variable region | EIVLTQSPATLSLSPGERATLSCRASQSVSSYLAWYQ QKPGQAPRLLIYDASNRATGIPARFSGSGSGTDFTLTI SSLEPEDFAVYYCQQRSNWPLTFGGGTKVEIK |
| 74 | Humanized antibody FS002-C375VLCDR1 | ***RASQSVSSYLA*** |
| 75 | Humanized antibody FS002-C375VLCDR2 | ***DASNRAT*** |
| 76 | Humanized antibody FS002-C375VLCDR3 | ***QQRSNWPLT*** |
| 77 | Humanized antibody FS002-C417 light chain variable region | NIQMTQSPSSLSASVGDRVTITCRASQSISSYLNWYQ QKPGKAPKLLIYAASSLQSGVPSRFSGSGSGTDFTLT ISSLQPEDFASYYCQQSYSSWAFGQGTKVEIK |
| 78 | Humanized antibody FS002-C417VLCDR1 | ***RASQSISSYLN*** |
| 79 | Humanized antibody FS002-C417VLCDR2 | ***AASSLQS*** |
| 80 | Humanized antibody FS002-C417VLCDR3 | ***QQSYSSWA*** |
| 81 | Humanized antibody FS002-B27 light chain variable region | NIQLTQSPSSVSASVGDRVTITC*RASQGFSRWLA*WY QQKPGKAPKLLIY*GASSLQS*GVPSRFSGSGSGTDFTL TISSLQPEDFATYYC*QQANSFPLT*FGGGTKLEIK |
| 82 | Humanized antibody FS002-B27VLCDR1 | ***RASQGFSRWLA*** |
| 83 | Humanized antibody FS002-B27VLCDR2 | ***GASSLQS*** |
| 84 | Humanized antibody FS002-B27VLCDR3 | ***QQANSFPLT*** |
| 85 | Humanized antibody FS002-C330 light chain variable region | NIQLTQSPSSLSASVGDRVTITCRASQSISSYLNWYQ QKPGKAPKLLIYAASSLQSGVPSRFSGSGSGTDFTLT ISSLQPEDFATYYCQQSYSTPDTFGPGTKVDIK |
| 86 | Humanized antibody FS002-C330VLCDR1 | ***RASQSISSYLN*** |
| 87 | Humanized antibody FS002-C330VLCDR2 | ***AASSLQS*** |
| 88 | Humanized antibody FS002-C330VLCDR3 | ***QQSYSTQDT*** |
| 89 | Humanized antibody FS002-C153 light chain variable region | QSALTQPASVSGSPGQSITISCTGTSSDVGGYNYVSW YQQHPGKAPKLMIYDVSNRPSGVSNRFSGSKSGNT ASLTISGLQAEDEADYYCSSFTTITTRVFGGGTKLTV L |
| 90 | Humanized antibody FS002-C153VLCDR1 | ***TGTSSDVGGYNYVS*** |
| 91 | Humanized antibody FS002-C153VLCDR2 | ***DVSNRPS*** |
| 92 | Humanized antibody FS002-C153VLCDR3 | ***SSFTTITTRV*** |
| 93 | Humanized antibody FS002-C241 light chain variable region | QSALTQPASVSGSPGQSITISCSGTSSDIGRYNYVSW YQQHPGKAPKLLIFDVSNRPSGVSNRFSGSKSGNTA SLTISGLQAEDEADYYCSSYTSTVPYVFGTGTRVAVQ |
| 94 | Humanized antibody FS002-C241VLCDR1 | ***SGTSSDIGRYNYVS*** |
| 95 | Humanized antibody FS002-C241VLCDR2 | ***DVSNRPS*** |
| 96 | Humanized antibody FS002-C241VLCDR3 | ***SSYTSTVPYV*** |
| 97 | Humanized antibody FS002-C15 light chain variable region | EIVLTQSPATLSLSPGERATLSCQASQTFSDYLAWYQ QKPGQAPRLLIYDASTRATGIPARFSGSGSGTDFTLTI SSLEPEDFAVYYCQQRSHWPPGYTFGQGTRVEIK |
| 98 | Humanized antibody FS002-C15VLCDR1 | ***QASQTFSDYLA*** |
| 99 | Humanized antibody FS002-C15VLCDR2 | ***DASTRAT*** |
| 100 | Humanized antibody FS002-C15VLCDR3 | ***QQRSHWPPGYT*** |
| 101 | Humanized antibody FS002-C58 light chain variable region | EIVLTQSPATLSLSPGERATLSCRASQSVYSSLAWYQ QKPGQAPKLLIYDASNRATGVPARFSGSGSGTDFTL TISSLEPEDFALYYCQHRQHWPLTFGGGTKVEIK |
| 102 | Humanized antibody FS002-C58VLCDR1 | ***RASQSVYSSLA*** |
| 103 | Humanized antibody FS002-C58VLCDR2 | ***DASNRAT*** |
| 104 | Humanized antibody FS002-C58VLCDR3 | ***QHRQHWPLT*** |
| 105 | Humanized antibody FS002-A72 light chain variable region | EIVMTQSPVTLSVSPGDSATLSCRASQSVDKNLAWY QQKTGQAPRLLIYGAATRATGIPARFSGSGSGTEFTL TISSLRSEDFAVYYCQQYRNWPWTFGLGTKVEIK |
| 106 | Humanized antibody FS002-A72VLCDR1 | ***RASQSVDKNLA*** |
| 107 | Humanized antibody FS002-A72VLCDR2 | ***GAATRAT*** |
| 108 | Humanized antibody FS002-A72VLCDR3 | ***QQYRNWPWT*** |
| 109 | Kappa light chain constant region sequence | |
| 110 | Lambda light chain constant region sequence | GQPKAAPSVTLFPPSSEELQANKATLVCLISDFYPGA VTVAWKADSSPVKAGVETTTPSKQSNNKYAASSYL SLTPEQWKSHRSYSCQVTHEGSTVEKTVAPTECS |
| 111 | Humanized antibody FS002-A1 heavy chain variable region | QVQLVQSGAEVKKPGASVKVSCKASGYTFTSYYM HWVRQAPGQGLEWMGIINPSGGSTSYAQKFQGRV TMTRDTSTSTVYMELSSLRSEDTAVYYCARDISGSY QTDAFDIWGQGTMVTVSS |
| 112 | Humanized antibody FS002-A1VHCDR1 | ***GYTFTSYYMH*** |
| 113 | Humanized antibody FS002-A1VHCDR2 | ***IINPSGGSTS*** |
| 114 | Humanized antibody FS002-A1VHCDR3 | ***DISGSYQTDAFDI*** |
| 115 | Humanized antibody FS002-A5 heavy chain variable region | QVQLQDSGPGLVKPSQTLSLTCTVSGGSISSGGYYW SWIRQHPGKGLEWIGYIYYSGSTYYNPSLKSRVTISV DTSKNQFSLKLSSVTAADTAVYYCARVPVPLVGAF DIWGQGTMVTVSS |
| 116 | Humanized antibody FS002-A5VHCDR1 | ***GGSISSGGYYWS*** |
| 117 | Humanized antibody FS002-A5VHCDR2 | ***YIYYSGSTY*** |
| 118 | Humanized antibody FS002-A5VHCDR3 | ***VPVPLVGAFDI*** |
| 119 | Humanized antibody FS002-A15 heavy chain variable region | RLQLQESGPGLVKPSQTLSLTCTVSGGSISSGGYYWS WIRQHPGKGLEWIGYIYYSGSTYYNPSLKSRVTISVD TSKNQFSLKLSSVTAADTAVYYCARATIYGYFDYWG QGTLVTVSS |
| 120 | Humanized antibody FS002-A15VHCDR1 | ***GGSISSGGYYWS*** |
| 121 | Humanized antibody FS002-A15VHCDR2 | ***YIYYSGSTY*** |
| 122 | Humanized antibody FS002-A15VHCDR3 | ***ATIYGYFDY*** |
| 123 | Humanized antibody FS002-A36 heavy chain variable region | QVQLQESGPGLVKPSQTLSLTCTVSGGSISSGGYYW SWIRQHPGKGLEWIGYIYYSGSTYYNPSLKSRVTISV DTSKNQFSLKLSSVTAADTAVYYCARVRLLGGGFDI WGQGTTVTVSS |
| 124 | Humanized antibody FS002-A36VHCDR1 | ***GGSISSGGYYWS*** |
| 125 | Humanized antibody FS002-A36VHCDR2 | ***YIYYSGSTY*** |
| 126 | Humanized antibody FS002-A36VHCDR3 | ***VRLLGGGFDI*** |
| 127 | Humanized antibody FS002-A66 heavy chain variable region | EVQLVESGGGVVQPGRSLRLSCAASGFTFSSYAMH WVRQAPGKGLEWVAVISYDGSNKYYVDSVKGRFTI SRDNSKNTLYLQMNSLRAEDTAVYYCARDRITYLD YYYYGMDVWGQGTTVTVSS |
| 128 | Humanized antibody FS002-A66VHCDR1 | ***GFTFSSYAMH*** |
| 129 | Humanized antibody FS002-A66VHCDR2 | ***VISYDGSNKY*** |
| 130 | Humanized antibody FS002-A66VHCDR3 | ***DRITYLDYYYYGMDV*** |
| 131 | Humanized antibody FS002-A10 heavy chain variable region | QVQLQQSGPGLVKPSQTLSLTCAIS GDSVSSDSAAW N WIRQSPSRGLEWLG RTYYRSKWYND YAVSVKSRIT INPDTSKNQFSLQLNSVTSDDTAVYYCAR GSWAAP WL WGQGTLVTVSS |
| 132 | Humanized antibody FS002-A10VHCDR1 | ***GDSVSSDSAAWN*** |
| 133 | Humanized antibody FS002-A10VHCDR2 | ***RTYYRSKWYND*** |
| 134 | Humanized antibody FS002-A10VHCDR3 | ***GSWAAPWL*** |
| 135 | Humanized antibody FS002-C9 heavy chain | QVQLLESGPGLVKPSQTVSLTCTVSGVSISRGVYSWS WIRQHPGKGLEWIGFIDYNGRTDYNPSLKSRVTISQ |
| | variable region | DTSKNDFSLKLTSVTAADTAIYYCARGLHAFDIWG QGTMVTVSS |
| 136 | Humanized antibody FS002-C9VHCDR1 | ***GVSISRGVYSWS*** |
| 137 | Humanized antibody FS002-C9VHCDR2 | ***FIDYNGRTD*** |
| 138 | Humanized antibody FS002-C9VHCDR3 | ***GLHAFDI*** |
| 139 | Humanized antibody FS002-C11 heavy chain variable region | RLQLQESGPRLVKPSETLSLTCSVSGGSISSRNHYWA WIRQPPGKGLEWIGYIYYSGSTYYNPSLKSRVTISVD TSKNQFSLKLSSVTAADTAVYYCARVGLLLRDFDY WGQGTLVTVSS |
| 140 | Humanized antibody FS002-C11VHCDR1 | ***GGSISSRNHYWA*** |
| 141 | Humanized antibody FS002-C11VHCDR2 | ***YIYYSGSTY*** |
| 142 | Humanized antibody FS002-C11VHCDR3 | ***VGLLLRDFDY*** |
| 143 | Humanized antibody FS002-C216 heavy chain variable region | EVQLVESGGGVVQPGRSLRLSCAASGFTFSSYAMH WVRQAPGKGLEWVAVISYDGSNKYYADSVKGRFTI SRDNSKNTLYLQMNSLRAEDTAVYYCARDCSGGSC YDAFDIWGQGTMVTVSS |
| 144 | Humanized antibody FS002-C216VHCDR1 | ***GFTFSSYAMH*** |
| 145 | Humanized antibody FS002-C216VHCDR2 | ***VISYDGSNKY*** |
| 146 | Humanized antibody FS002-C216VHCDR3 | ***DCSGGSCYDAFDI*** |
| 147 | Humanized antibody FS002-C375 heavy chain variable region | QVQLQESGPGLVKPSQTLSLTCTVSGGSISSGGYYW SWIRQHPGKGLEWIGYIYYSGSTYYNPSLKSRVTISV DTSKNQFSLKLSSVTAADTAVYYCARVGVALHYGM DVWGQGTTVTVSS |
| 148 | Humanized antibody FS002-C375VHCDR1 | ***GGSISSGGYYWS*** |
| 149 | Humanized antibody FS002-C375VHCDR2 | ***YIYYSGSTY*** |
| 150 | Humanized antibody FS002-C375VHCDR3 | ***VGVALHYGMDV*** |
| 151 | Humanized antibody FS002-C417 heavy chain variable region | EVQLVESGGVVVQPGGSLRLSCAASGFTFDDYTMH WVRQAPGKGLEWVSLISWDGGSTYYADSVKGRFTI SRDNAKNTLYLQMNSLRAEDTAVYYCARDTGYAM DVWGQGTMVTVSS |
| 152 | Humanized antibody FS002-C417VHCDR1 | ***GFTFDDYTMH*** |
| 153 | Humanized antibody FS002-C417VHCDR2 | ***LISWDGGSTY*** |
| 154 | Humanized antibody FS002-C417VHCDR3 | ***DTGYAMDV*** |
| 155 | Humanized antibody FS002-B27 heavy chain variable region | QVQLVQSGAEVKKPGSSVKVSCKASGGTFSSYAIS WVRQAPGQGLEWMGRIIPILGIANYAQKFQGRVTIT ADKSTSTAYMELSSLRSEDTAVYYCARAEGSSWYYF DYWGQGTLVTVSS |
| 156 | Humanized antibody FS002-B27VHCDR1 | ***GGTFSSYAIS*** |
| 157 | Humanized antibody FS002-B27VHCDR2 | ***RIIPILGIAN*** |
| 158 | Humanized antibody FS002-B27VHCDR3 | ***AEGSSWYYFDY*** |
| 159 | Humanized antibody FS002-C330 heavy chain variable region | QLQLQESGPGLVKPSGTLSLTCAVSGGSISSSNWWS WVRQPPGKGLEWIGEIYHSGSTNYNPSLKSRVTISV DKSKNQFSLKLSSVTAADTAVYYCARAHSRYYYGL DYWGQGTLVTVSS |
| 160 | Humanized antibody FS002-C330VHCDR1 | ***GGSISSSNWWS*** |
| 161 | Humanized antibody FS002-C330VHCDR2 | ***EIYHSGSTN*** |
| 162 | Humanized antibody FS002-C330VHCDR3 | ***4HSRYYYGLDY*** |
| 163 | Humanized antibody FS002-C153 heavy chain variable region | QVQLQQWGAELVKPGASVKLSCTASGFNIKDTYM HWVKQRPEQGLEWIGRIDPANGNTKYDPKFQGKAT ITADTSSNTAYLQLSSLTSEDTAVYYCARSYYRYDGY FDVWGAGTMVTVSS |
| 164 | Humanized antibody FS002-C153VHCDR1 | ***GFNIKDTYMH*** |
| 165 | Humanized antibody FS002-C153VHCDR2 | ***RIDPANGNTK*** |
| 166 | Humanized antibody FS002-C153VHCDR3 | ***SYYRYDGYFDV*** |
| 167 | Humanized antibody FS002-C241 heavy chain variable region | RLQLQESGPGLVKPSETLSLTCTVSGGSISSSSYYWG WIRQPPGKGLEWIGSIYYSGSTYYNPSLKSRVTISVD TSKNQFSLKLSSVTAADTAVYYCARRYSYGRDAFDI WGQGTMVTVSS |
| 168 | Humanized antibody FS002-C241VHCDR1 | ***GGSISSSSYYWG*** |
| 169 | Humanized antibody FS002-C241VHCDR2 | ***SIYYSGSTY*** |
| 170 | Humanized antibody FS002-C241VHCDR3 | ***RYSYGRDAFDI*** |
| 171 | Humanized antibody FS002-C15 heavy chain variable region | QLQLQESGPGLVKPSETLSLTCTVS**GGSISSSSYYWG** WIRQPPGKGLEWIG**SIYYSGSTY**YNPSLKSRVTISVD TSKNQFSLKLSSVTAADTAVYYCAR**LTIFGVVLY**WG QGTLVTVSS |
| 172 | Humanized antibody FS002-C15VHCDR1 | ***GGSISSSSYYWG*** |
| 173 | Humanized antibody FS002-C15VHCDR2 | ***SIYYSGSTY*** |
| 174 | Humanized antibody FS002-C15VHCDR3 | ***LTIFGVVLY*** |
| 175 | Humanized antibody FS002-C58 heavy chain variable region | EVQLLESGGGVVQPGRSLRLSCAASGFTFSSYAMH WVRQAPGKGLEWVAVISYDGSNKYYADSVKGRFTI SRDNSKNTLYLQMNSLRAEDTAVYYCARGKYNWF DPWGQGTLVTVSS |
| 176 | Humanized antibody FS002-C58VHCDR1 | ***GFTFSSYAMH*** |
| 177 | Humanized antibody FS002-C58VHCDR2 | ***VISYDGSNKY*** |
| 178 | Humanized antibody FS002-C58VHCDR3 | ***GKYNWFDP*** |
| 179 | Humanized antibody FS002-A72 heavy chain | QVQLVDSGGGLVQPGGSLRLSCVASGFAFSNYEMN WVRQAPGKGLEWVSYISSSSSYTNYADSVKGRFTIS |
| | variable region | RDNAKNSLYLQMNSLRAEDTAVYYCARAQVRDAF DIWGQGTTVTVSS |
| 180 | Humanized antibody FS002-A72VHCDR1 | ***GFAFSNYEMN*** |
| 181 | Humanized antibody FS002-A72VHCDR2 | ***YISSSSSYTN*** |
| 182 | Humanized antibody FS002-A72VHCDR3 | ***AQVRDAFDI*** |
| 183 | IgG1 heavy chain constant region | |

| | | |
|---|---|---|
| Note: The bold and underlined parts of amino acid sequences in the table are the corresponding CDR sequences. | | |

**Table 1c: Amino acid sequences (SEQ ID NO: 184-219) of the light and heavy chains of the antibody according to the present invention**

| No. | Description | Amino Acid Sequence |
|---|---|---|
| 184 | Humanized antibody FS002-A1 light chain | DIQLTQSPSSLSASVGDRVTITCRASQSIISYLNWYQ QKPGKAPKLLIYAASSLQSGVPSRFSGSGSGTDFTL TISSLQPEDFATYYCQQSYSTPDTFGQGTKLEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 185 | Humanized antibody FS002-A5 light chain | EIVLTQSPATLSLSPGERATLSCRASQSVSSNLGWYQ QKPGQAPSLLIYGASNRATGIPARFSGSGSGTDFTLT ISSLEPEDFAVYYCQQRDTWPLTFGGGTKVEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 186 | Humanized antibody FS002-A15 light chain | EIVLTQSPATLPLSPGERATLSCRASQSVGFYLAWY QQKPGQPPRLLFYDASKRATGIPARFSASGSGTDFT LTISGLEPEDFAVYYCQHRISWPLTFGQGTKVEVKR TVAAPSVFIFPPSDEQLKSGTASVVCLLNNFYPREAK VQWKVDNALQSGNSQESVTEQDSKDSTYSLSSTLT LSKADYEKHKVYACEVTHQGLSSPVTKSFNRGEC |
| 187 | Humanized antibody FS002-A36 light chain | EIVLTQSPATLSLSPGERATLSC**RASQSVSTYLA**WYQ QKPGQAPRLLIY**DTSIRAT**GIPARFSGSGSGTDFTLTI SSLEPEDFAVYYC**QQRSNWPLT**FGGGTKVEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 188 | Humanized antibody FS002-A66 light chain | EIVLTQSPAILSLSPGERATLSCRASQSVSEYLAWYQ QKPGQAPRLLIYDISNRATGIPARFSGSGSETDFTLTI SSLEPEDFAVYYCQQRRTWPLTFGPGTKVDIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 189 | Humanized antibody | NIQLTQSPPFLSASVGDRVTITCRASQDISSFLAWYQ |
| | FS002-A10 light chain | QKPGKAPNLLIHAATTLQSGVPSRFSGSGSGTEFTL TISSLRPEDFATYYCQQLHTYPITFGQGTRLEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 190 | Humanized antibody FS002-C9 light chain | NIQMTQSPPSLSASVGDRVAITCRASQDISNSLAWY QQKSGQPPKLLLYDVSKLERGVPSRFSGSGSRTDYT LTISSLQPEDFATYYCQQYSSDPTFGQGSKLEFKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 191 | Humanized antibody FS002-C11 light chain | DIQMIQSPSSVSASVGDRVTITCRASQGISNSLAWY QHKPGTTPKLLIYAASSLQGGVPSRFSGSSSETDFTL TISSLQPEDFATYYCQQANSFPITFGQGTRLDIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 192 | Humanized antibody FS002-C216 light chain | EIVLTQSPATLSLSPGERATLSCRASQSVSSYLAWYQ QKPGQAPRLLIYDASNRATGIPARFSGSGSGTDFTLT ISSLEPEDFAVYYCQQRTNWLMYTFGQGTKLEIKR TVAAPSVFIFPPSDEQLKSGTASVVCLLNNFYPREAK VQWKVDNALQSGNSQESVTEQDSKDSTYSLSSTLT LSKADYEKHKVYACEVTHQGLSSPVTKSFNRGEC |
| 193 | Humanized antibody FS002-C375 light chain | EIVLTQSPATLSLSPGERATLSCRASQSVSSYLAWYQ QKPGQAPRLLIYDASNRATGIPARFSGSGSGTDFTLT ISSLEPEDFAVYYCQQRSNWPLTFGGGTKVEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 194 | Humanized antibody FS002-C417 light chain | NIQMTQSPSSLSASVGDRVTITCRASQSISSYLNWY QQKPGKAPKLLIYAASSLQSGVPSRFSGSGSGTDFT LTISSLQPEDFASYYCQQSYSSWAFGQGTKVEIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 195 | Humanized antibody FS002-B27 light chain | NIQLTQSPSSVSASVGDRVTITCRASQGFSRWLAW YQQKPGKAPKLLIYGASSLQSGVPSRFSGSGSGTDF TLTISSLQPEDFATYYCQQANSFPLTFGGGTKLEIKR TVAAPSVFIFPPSDEQLKSGTASVVCLLNNFYPREAK VQWKVDNALQSGNSQESVTEQDSKDSTYSLSSTLT LSKADYEKHKVYACEVTHQGLSSPVTKSFNRGEC |
| 196 | Humanized antibody FS002-C330 light chain | NIQLTQSPSSLSASVGDRVTITC**RASQSISSYLN**WYQ QKPGKAPKLLIY**AASSLQS**GVPSRFSGSGSGTDFTL TISSLQPEDFATYYC**QQSYSTPDT**FGPGTKVDIKRTV AAPSVFIFPPSDEQLKSGTASVVCLLNNFYPREAKV QWKVDNALQSGNSQESVTEQDSKDSTYSLSSTLTL SKADYEKHKVYACEVTHQGLSSPVTKSFNRGEC |
| 197 | Humanized antibody FS002-C153 light chain | QSALTQPASVSGSPGQSITISCTGTSSDVGGYNYVS WYQQHPGKAPKLMIYDVSNRPSGVSNRFSGSKSGN TASLTISGLQAEDEADYYCSSFTTITTRVFGGGTKLT VLGQPKAAPSVTLFPPSSEELQANKATLVCLISDFYP GAVTVAWKADSSPVKAGVETTTPSKQSNNKYAASS YLSLTPEQWKSHRSYSCQVTHEGSTVEKTVAPTECS |
| 198 | Humanized antibody FS002-C241 light chain | QSALTQPASVSGSPGQSITISCSGTSSDIGRYNYVSW YQQHPGKAPKLLIFDVSNRPSGVSNRFSGSKSGNTA SLTISGLQAEDEADYYCSSYTSTVPYVFGTGTRVAV |
| | | QGQPKAAPSVTLFPPSSEELQANKATLVCLISDFYPG AVTVAWKADSSPVKAGVETTTPSKQSNNKYAASSY LSLTPEQWKSHRSYSCQVTHEGSTVEKTVAPTECS |
| 199 | Humanized antibody FS002-C15 light chain | EIVLTQSPATLSLSPGERATLSCQASQTFSDYLAWY QQKPGQAPRLLIYDASTRATGIPARFSGSGSGTDFTL TISSLEPEDFAVYYCQQRSHWPPGYTFGQGTRVEIK RTVAAPSVFIFPPSDEQLKSGTASVVCLLNNFYPREA KVQWKVDNALQSGNSQESVTEQDSKDSTYSLSSTL TLSKADYEKHKVYACEVTHQGLSSPVTKSFNRGEC |
| 200 | Humanized antibody FS002-C58 light chain | EIVLTQSPATLSLSPGERATLSCRASQSVYSSLAWYQ QKPGQAPKLLIYDASNRATGVPARFSGSGSGTDFTL TISSLEPEDFALYYCQHRQHWPLTFGGGTKVEIKRT VAAPSVFIFPPSDEQLKSGTASVVCLLNNFYPREAK VQWKVDNALQSGNSQESVTEQDSKDSTYSLSSTLT LSKADYEKHKVYACEVTHQGLSSPVTKSFNRGEC |
| 201 | Humanized antibody FS002-A72 light chain | EIVMTQSPVTLSVSPGDSATLSCRASQSVDKNLAW YQQKTGQAPRLLIYGAATRATGIPARFSGSGSGTEF TLTISSLRSEDFAVYYCQQYRNWPWTFGLGTKVEI KRTVAAPSVFIFPPSDEQLKSGTASVVCLLNNFYPRE AKVQWKVDNALQSGNSQESVTEQDSKDSTYSLSST LTLSKADYEKHKVYACEVTHQGLSSPVTKSFNRGE C |
| 202 | Humanized antibody FS002-A1 heavy chain | QVQLVQSGAEVKKPGASVKVSCKASGYTFTSYYM HWVRQAPGQGLEWMGIINPSGGSTSYAQKFQGRV TMTRDTSTSTVYMELSSLRSEDTAVYYCARDISGSY QTDAFDIWGQGTMVTVSSASTKGPSVFPLAPSSKS TSGGTAALGCLVKDYFPEPVTVSWNSGALTSGVHT FPAVLQSSGLYSLSSVVTVPSSSLGTQTYICNVNHKP SNTKVDKKVEPKSCDKTHTCPPCPAPELLGGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPSRDELTKNQVSLTCLVKGFYPSDIAVEWE SNGQPENNYKTTPPVLDSDGSFFLYSKLTVDKSRWQ QGNVFSCSVMHEALHNHYTQKSLSLSPGK |
| 203 | Humanized antibody FS002-A5 heavy chain | QVQLQDSGPGLVKPSQTLSLTCTVSGGSISSGGYY WSWIRQHPGKGLEWIGYIYYSGSTYYNPSLKSRVTI SVDTSKNQFSLKLSSVTAADTAVYYCARVPVPLVG AFDIWGQGTMVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSRDELTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 204 | Humanized antibody FS002-A15 heavy chain | RLQLQESGPGLVKPSQTLSLTCTVSGGSISSGGYYW SWIRQHPGKGLEWIGYIYYSGSTYYNPSLKSRVTIS VDTSKNQFSLKLSSVTAADTAVYYCARATIYGYFD YWGQGTLVTVSSASTKGPSVFPLAPSSKSTSGGTAA LGCLVKDYFPEPVTVSWNSGALTSGVHTFPAVLQSS GLYSLSSVVTVPSSSLGTQTYICNVNHKPSNTKVDK KVEPKSCDKTHTCPPCPAPELLGGPSVFLFPPKPKDT LMISRTPEVTCVVVDVSHEDPEVKFNWYVDGVEVH NAKTKPREEQYNSTYRVVSVLTVLHQDWLNGKEY |
| | | KCKVSNKALPAPIEKTISKAKGQPREPQVYTLPPSR DELTKNQVSLTCLVKGFYPSDIAVEWESNGQPENNY KTTPPVLDSDGSFFLYSKLTVDKSRWQQGNVFSCSV MHEALHNHYTQKSLSLSPGK |
| 205 | Humanized antibody FS002-A36 heavy chain | QVQLQESGPGLVKPSQTLSLTCTVSGGSISSGGYY WSWIRQHPGKGLEWIGYIYYSGSTYYNPSLKSRVTI SVDTSKNQFSLKLSSVTAADTAVYYCARVRLLGGG FDIWGQGTTVTVSSASTKGPSVFPLAPSSKSTSGGT AALGCLVKDYFPEPVTVSWNSGALTSGVHTFPAVL QSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSRDELTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 206 | Humanized antibody FS002-A66 heavy chain | EVQLVESGGGVVQPGRSLRLSCAASGFTFSSYAMH WVRQAPGKGLEWVAVISYDGSNKYYVDSVKGRFT ISRDNSKNTLYLQMNSLRAEDTAVYYCARDRITYL DYYYYGMDVWGQGTTVTVSSASTKGPSVFPLAPS SKSTSGGTAALGCLVKDYFPEPVTVSWNSGALTSG VHTFPAVLQSSGLYSLSSVVTVPSSSLGTQTYICNVN HKPSNTKVDKKVEPKSCDKTHTCPPCPAPELLGGPS VFLFPPKPKDTLMISRTPEVTCVVVDVSHEDPEVKF NWYVDGVEVHNAKTKPREEQYNSTYRVVSVLTVL HQDWLNGKEYKCKVSNKALPAPIEKTISKAKGQPR EPQVYTLPPSRDELTKNQVSLTCLVKGFYPSDIAVE WESNGQPENNYKTTPPVLDSDGSFFLYSKLTVDKSR WQQGNVFSCSVMHEALHNHYTQKSLSLSPGK |
| 207 | Humanized antibody FS002-A10 heavy chain | QVQLQQSGPGLVKPSQTLSLTCAISGDSVSSDSAAW NWIRQSPSRGLEWLGRTYYRSKWYNDYAVSVKSRI TINPDTSKNQFSLQLNSVTSDDTAVYYCARGSWAA PWLWGQGTLVTVSSASTKGPSVFPLAPSSKSTSGGT AALGCLVKDYFPEPVTVSWNSGALTSGVHTFPAVL QSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSRDELTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 208 | Humanized antibody FS002-C9 heavy chain | QVQLLESGPGLVKPSQTVSLTCTVSGVSISRGVYSW SWIRQHPGKGLEWIGFIDYNGRTDYNPSLKSRVTIS QDTSKNDFSLKLTSVTAADTAIYYCARGLHAFDIW GQGTMVTVSSASTKGPSVFPLAPSSKSTSGGTAALG CLVKDYFPEPVTVSWNSGALTSGVHTFPAVLQSSGL YSLSSVVTVPSSSLGTQTYICNVNHKPSNTKVDKKV EPKSCDKTHTCPPCPAPELLGGPSVFLFPPKPKDTLM ISRTPEVTCVVVDVSHEDPEVKFNWYVDGVEVHNA KTKPREEQYNSTYRVVSVLTVLHQDWLNGKEYKC KVSNKALPAPIEKTISKAKGQPREPQVYTLPPSRDEL TKNQVSLTCLVKGFYPSDIAVEWESNGQPENNYKTT PPVLDSDGSFFLYSKLTVDKSRWQQGNVFSCSVMH EALHNHYTQKSLSLSPGK |
| 209 | Humanized antibody | RLQLQESGPRLVKPSETLSLTCSVSGGSISSRNHYW |
| | FS002-C11 heavy chain | AWIRQPPGKGLEWIGYIYYSGSTYYNPSLKSRVTIS VDTSKNQFSLKLSSVTAADTAVYYCARVGLLLRDF DYWGQGTLVTVSSASTKGPSVFPLAPSSKSTSGGTA ALGCLVKDYFPEPVTVSWNSGALTSGVHTFPAVLQS SGLYSLSSVVTVPSSSLGTQTYICNVNHKPSNTKVD KKVEPKSCDKTHTCPPCPAPELLGGPSVFLFPPKPK DTLMISRTPEVTCVVVDVSHEDPEVKFNWYVDGVE VHNAKTKPREEQYNSTYRVVSVLTVLHQDWLNGK EYKCKVSNKALPAPIEKTISKAKGQPREPQVYTLPPS RDELTKNQVSLTCLVKGFYPSDIAVEWESNGQPENN YKTTPPVLDSDGSFFLYSKLTVDKSRWQQGNVFSCS VMHEALHNHYTQKSLSLSPGK |
| 210 | Humanized antibody FS002-C216 heavy chain | EVQLVESGGGVVQPGRSLRLSCAASGFTFSSYAMH WVRQAPGKGLEWVAVISYDGSNKYYADSVKGRFT ISRDNSKNTLYLQMNSLRAEDTAVYYCARDCSGGS CYDAFDIWGQGTMVTVSSASTKGPSVFPLAPSSKS TSGGTAALGCLVKDYFPEPVTVSWNSGALTSGVHT FPAVLQSSGLYSLSSVVTVPSSSLGTQTYICNVNHKP SNTKVDKKVEPKSCDKTHTCPPCPAPELLGGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPSRDELTKNQVSLTCLVKGFYPSDIAVEWE SNGQPENNYKTTPPVLDSDGSFFLYSKLTVDKSRWQ QGNVFSCSVMHEALHNHYTQKSLSLSPGK |
| 211 | Humanized antibody FS002-C375 heavy chain | QVQLQESGPGLVKPSQTLSLTCTVSGGSISSGGYY WSWIRQHPGKGLEWIGYIYYSGSTYYNPSLKSRVTI SVDTSKNQFSLKLSSVTAADTAVYYCARVGVALHY GMDVWGQGTTVTVSSASTKGPSVFPLAPSSKSTSG GTAALGCLVKDYFPEPVTVSWNSGALTSGVHTFPAV LQSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSRDELTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 212 | Humanized antibody FS002-C417 heavy chain | EVQLVESGGVVVQPGGSLRLSCAASGFTFDDYTM HWVRQAPGKGLEWVSLISWDGGSTYYADSVKGR FTISRDNAKNTLYLQMNSLRAEDTAVYYCARDTGY AMDVWGQGTMVTVSSASTKGPSVFPLAPSSKSTSG GTAALGCLVKDYFPEPVTVSWNSGALTSGVHTFPAV LQSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSRDELTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 213 | Humanized antibody FS002-B27 heavy chain | QVQLVQSGAEVKKPGSSVKVSCKASGGTFSSYAIS WVRQAPGQGLEWMGRIIPILGIANYAQKFQGRVTI TADKSTSTAYMELSSLRSEDTAVYYCARAEGSSWY YFDYWGQGTLVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK |
| | | VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSRDELTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 214 | Humanized antibody FS002-C330 heavy chain | QLQLQESGPGLVKPSGTLSLTCAVSGGSISSSNWWS WVRQPPGKGLEWIGEIYHSGSTNYNPSLKSRVTISV DKSKNQFSLKLSSVTAADTAVYYCARAHSRYYYGL DYWGQGTLVTVSSASTKGPSVFPLAPSSKSTSGGTA ALGCLVKDYFPEPVTVSWNSGALTSGVHTFPAVLQS SGLYSLSSVVTVPSSSLGTQTYICNVNHKPSNTKVD KKVEPKSCDKTHTCPPCPAPELLGGPSVFLFPPKPK DTLMISRTPEVTCVVVDVSHEDPEVKFNWYVDGVE VHNAKTKPREEQYNSTYRVVSVLTVLHQDWLNGK EYKCKVSNKALPAPIEKTISKAKGQPREPQVYTLPPS RDELTKNQVSLTCLVKGFYPSDIAVEWESNGQPENN YKTTPPVLDSDGSFFLYSKLTVDKSRWQQGNVFSCS VMHEALHNHYTQKSLSLSPGK |
| 215 | Humanized antibody FS002-C153 heavy chain | QVQLQQWGAELVKPGASVKLSCTASGFNIKDTYM HWVKQRPEQGLEWIGRIDPANGNTKYDPKFQGKA TITADTSSNTAYLQLSSLTSEDTAVYYCARSYYRYD GYFDVWGAGTMVTVSSASTKGPSVFPLAPSSKSTS GGTAALGCLVKDYFPEPVTVSWNSGALTSGVHTFPA VLQSSGLYSLSSVVTVPSSSLGTQTYICNVNHKPSNT KVDKKVEPKSCDKTHTCPPCPAPELLGGPSVFLFPP KPKDTLMISRTPEVTCVVVDVSHEDPEVKFNWYVD GVEVHNAKTKPREEQYNSTYRVVSVLTVLHQDWL NGKEYKCKVSNKALPAPIEKTISKAKGQPREPQVYT LPPSRDELTKNQVSLTCLVKGFYPSDIAVEWESNGQ PENNYKTTPPVLDSDGSFFLYSKLTVDKSRWQQGN VFSCSVMHEALHNHYTQKSLSLSPGK |
| 216 | Humanized antibody FS002-C241 heavy chain | RLQLQESGPGLVKPSETLSLTCTVSGGSISSSSYYW GWIRQPPGKGLEWIGSIYYSGSTYYNPSLKSRVTIS VDTSKNQFSLKLSSVTAADTAVYYCARRYSYGRDA FDIWGQGTMVTVSSASTKGPSVFPLAPSSKSTSGGT AALGCLVKDYFPEPVTVSWNSGALTSGVHTFPAVL QSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSRDELTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 217 | Humanized antibody FS002-C15 heavy chain | QLQLQESGPGLVKPSETLSLTCTVSGGSISSSSYYW GWIRQPPGKGLEWIGSIYYSGSTYYNPSLKSRVTIS VDTSKNQFSLKLSSVTAADTAVYYCARLTIFGVVLY WGQGTLVTVSSASTKGPSVFPLAPSSKSTSGGTAAL GCLVKDYFPEPVTVSWNSGALTSGVHTFPAVLQSSG LYSLSSVVTVPSSSLGTQTYICNVNHKPSNTKVDKK VEPKSCDKTHTCPPCPAPELLGGPSVFLFPPKPKDTL MISRTPEVTCVVVDVSHEDPEVKFNWYVDGVEVH NAKTKPREEQYNSTYRVVSVLTVLHQDWLNGKEY KCKVSNKALPAPIEKTISKAKGQPREPQVYTLPPSR DELTKNQVSLTCLVKGFYPSDIAVEWESNGQPENNY |
| | | KTTPPVLDSDGSFFLYSKLTVDKSRWQQGNVFSCSV MHEALHNHYTQKSLSLSPGK |
| 218 | Humanized antibody FS002-C58 heavy chain | EVQLLESGGGVVQPGRSLRLSCAASGFTFSSYAMH WVRQAPGKGLEWVAVISYDGSNKYYADSVKGRFT ISRDNSKNTLYLQMNSLRAEDTAVYYCARGKYNW FDPWGQGTLVTVSSASTKGPSVFPLAPSSKSTSGGT AALGCLVKDYFPEPVTVSWNSGALTSGVHTFPAVL QSSGLYSLSSVVTVPSSSLGTQTYICNVNHKPSNTK VDKKVEPKSCDKTHTCPPCPAPELLGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSRDELTKNQVSLTCLVKGFYPSDIAVEWESNGQP ENNYKTTPPVLDSDGSFFLYSKLTVDKSRWQQGNV FSCSVMHEALHNHYTQKSLSLSPGK |
| 219 | Humanized antibody FS002-A72 heavy chain | QVQLVDSGGGLVQPGGSLRLSCVASGFAFSNYEMN WVRQAPGKGLEWVSYISSSSSYTNYADSVKGRFTIS RDNAKNSLYLQMNSLRAEDTAVYYCARAQVRDAF DIWGQGTTVTVSSASTKGPSVFPLAPSSKSTSGGTA ALGCLVKDYFPEPVTVSWNSGALTSGVHTFPAVLQS SGLYSLSSVVTVPSSSLGTQTYICNVNHKPSNTKVD KKVEPKSCDKTHTCPPCPAPELLGGPSVFLFPPKPK DTLMISRTPEVTCVVVDVSHEDPEVKFNWYVDGVE VHNAKTKPREEQYNSTYRVVSVLTVLHQDWLNGK EYKCKVSNKALPAPIEKTISKAKGQPREPQVYTLPPS RDELTKNQVSLTCLVKGFYPSDIAVEWESNGQPENN YKTTPPVLDSDGSFFLYSKLTVDKSRWQQGNVFSCS VMHEALHNHYTQKSLSLSPGK |

| | | |
|---|---|---|
| Note: The bold and underlined parts of amino acid sequences in the table are the corresponding CDR sequences. | | |

### Detailed Description of the Invention

### I. General Definitions

In the present invention, the scientific and technical terms used herein have the meanings commonly understood by those skilled in the art, unless otherwise stated. Also, terms, and laboratory procedures associated to the protein and nucleic acid chemistry, molecular biology, cell and tissue culture, microbiology, immunology used herein are terms and common procedures widely used in the corresponding fields. Also, the following definitions and explanations of related terms are provided for a better understanding of the present invention.

As used herein, "antibody" refers to immunoglobulin and immunoglobulin fragments, naturally or partially or fully synthetically produced (e.g., recombinantly produced), comprising any fragment comprising at least a portion of the variable region of an immunoglobulin molecule and retaining the binding specificity of the full length immunoglobulin. Thus, an antibody includes any protein having a binding structural domain homologous or substantially identical to an immunoglobulin antigen-binding structural domain (antibody-binding site). An antibody includes an antibody fragment, such as an antibody fragment against tumor stem cells. As used herein, the term "antibody" thus includes a synthetic antibody, a recombinantly produced antibody, a multispecific antibody (e.g., a bispecific antibody), a human antibody, a non-human antibody, a humanized antibody, a chimeric antibody, an intracellular antibody, and antibody fragments, for example, but not limited to, Fab fragment, Fab' fragment, F(ab')₂ fragment, Fv fragment, disulfide-linked Fv (dsFv), Fd fragment, Fd' fragment, single-chain Fv (scFv), single-chain Fab (scFab), a double antibody, an anti-idiotypic (anti-Id) antibody, or an antigen-binding fragment of any of the above antibodies. The antibody provided herein includes members of any immunoglobulin class (e.g., IgG, IgM, IgD, IgE, IgA, and IgY), any class (e.g., IgG1, IgG2, IgG3, IgG4, IgA1, and IgA2) or subclass (e.g., IgG2a and IgG2b).

As used herein, an "antibody fragment" or "antigen-binding fragment" of an antibody refers to any portion of a full length antibody that is less than the full length, but includes at least a portion of the variable region of the antibody that binds to the antigen (e.g., one or more CDRs and/or one or more antibody-binding sites), and thus retains the binding specificity as well as at least part of the specific binding capability of the full length antibody. Thus, an antigen-binding fragment refers to an antibody fragment comprising an antigen-binding portion that binds to the same antigen as the antibody deriving the antibody fragment. The antibody fragment includes an antibody derivative produced by enzymatic treatment of the full length antibody, as well as a synthetically produced derivative, such as a recombinantly produced derivative. The antibody includes an antibody fragment. Examples of the antibody fragment include, but are not limited to, Fab, Fab', F(ab')₂, single chain Fv (scFv), Fv, dsFv, double antibody, Fd and Fd' fragments, and other fragments, comprising modified fragments (see, for example, Methods in Molecular Biology, Vol 207: Recombinant Antibodies for Cancer Therapy Methods and Protocols (2003); Chapter 1; p 3-25, Kipriyanov). The fragment may include a plurality of chains joined together, for example by a disulfide bond and/or by a peptide linker. The antibody fragment typically includes at least or about 50 amino acids, and typically at least or about 200 amino acids. The antigen-binding fragment includes any antibody fragment that, when being inserted into an antibody framework (e.g., by substituting a corresponding region), obtains an antibody that immunospecifically binds to the antigen (i.e., exhibits at least or at least about 10⁷-10⁸ M⁻¹ Ka).

As used herein, a "monoclonal antibody" refers to a population of identical antibodies, where each antibody molecule is identical to other antibody molecules in the population of monoclonal antibodies. In contrast to the properties of a polyclonal population of antibodies, the polyclonal population of antibodies include antibodies having a plurality of different sequences. The monoclonal antibody may be prepared by a number of well-known methods (Smith et al. (2004) J. Clin. Pathol. 57, 912-917; and Nelson et al., J Clin Pathol (2000), 53, 111-117). For example, the monoclonal antibody may be prepared by immortalized B cells, for example by fusing with myeloma cells to produce a hybridoma cell line or by infecting B cells with a virus such as EBV. Recombinant techniques may also be used to prepare an antibody from a clonal population of host cells in vitro by transforming the host cells with a plasmid carrying an artificial sequence of a nucleotide encoding the antibody.

As used herein, the term "hybridoma" or "hybridoma cell" refers to a cell or cell line (typically a myeloma or lymphoma cell) produced by fusing antibody-producing lymphocytes and non-antibody-producing cancer cells. As known to those of ordinary skill in the art, hybridoma may proliferate and continue to be supplied to produce specific monoclonal antibodies. Methods for producing a hybridoma are known in the art (see, for example, Harlow & Lane, 1988). When referring to the term "hybridoma" or "hybridoma cell", it also includes subcloned and progeny cells of the hybridoma.

As used herein, a "conventional antibody" refers to an antibody comprising two heavy chains (which may be marked as H and H'), two light chains (which may be marked as L and L'), and two antigen-binding sites, where each heavy chain may be a full length immunoglobulin heavy chain or any of functional regions thereof that retains the antigen binding ability (e.g., heavy chains comprising, but are not limited to, V_{H} chains, V_{H}-C_{H}1 chains, and V_{H}-C_{H}1-C_{H}2-C_{H}3 chains), and each light chain may be a full length light chain or any functional region (e.g., light chains comprising, but not limited to, V_{L} chain and V_{L}-C_{L} chain). Each heavy chain (H and H') is paired with a light chain (L and L', respectively).

As used herein, a full length antibody is an antibody having two full length heavy chains (e.g., V_{H}-C_{H}1-C_{H}2-C_{H}3 or V_{H}-C_{H}1-C_{H}2-C_{H}3-C_{H}4), two full length light chains (V_{L}-C_{L}), and a hinge region, for example an antibody naturally produced by secretion of B cells by an antibody, and an synthetically produced antibody with the same structural domain.

As used herein, dsFv refers to an Fv having an engineered intermolecular disulfide bond that stabilizes a V_{H}-V_{L} pair.

As used herein, a Fab fragment is an antibody fragment obtained by papain digestion of a full length immunoglobulin, or a fragment having the same structure produced, for example, by recombinant methods. The Fab fragment includes a light chain (comprising V_{L} and C_{L}) and another chain comprising a variable structural domain (V_{H}) of the heavy chain and a constant region structural domain (C_{H}1) of the heavy chain.

As used herein, an F(ab')₂ fragment is an antibody fragment resulting from pepsin digestion of immunoglobulin at pH 4.0-4.5, or a fragment of the same structure produced, for example, by recombinant methods. The F(ab')₂ fragment substantially includes two Fab fragments, where each heavy chain moiety includes an additional several amino acids, comprising a cysteine that forms a disulfide bond linking the two fragments.

As used herein, a Fab' fragment is a fragment comprising a half of a F(ab')₂ fragment (one heavy chain and one light chain).

As used herein, a scFv fragment refers to an antibody fragment comprising a variable light chain (V_{L}) and a variable heavy chain (V_{H}) covalently linked in any sequence by a polypeptide linker. The length of the linker makes the two variable structural domains bridged substantially without interference. An exemplary linker is a (Gly-Ser)ₙ residue with some Glu or Lys residues dispersed to increase solubility.

The term "chimeric antibody" refers to such an antibody wherein the variable region sequence is derived from one species and the constant region sequence is derived from another species, such as an antibody wherein the variable region sequence is derived from a mouse antibody and the constant region sequence is derived from a human antibody.

A "humanized" antibody refers to a non-human (e.g., mouse) antibody form that is a chimeric immunoglobulin, immunoglobulin chain or fragment thereof (e.g., Fv, Fab, Fab', F(ab')₂ or other antigen-binding subsequences of the antibody), comprising minimal sequences derived from non-human immunoglobulins. Preferably, the humanized antibody is a human immunoglobulin (recipient antibody) where residues of complementarity determining regions (CDRs) of the recipient antibody are substituted by CDR residues of non-human species (donor antibody) having the expected specificity, affinity and ability, such as a mouse, rat or rabbit.

Furthermore, in humanization, it is also possible to mutate the amino acid residues within the CDR1, CDR2 and/or CDR3 regions of VH and/or VL, thereby improving one or more binding properties (e.g., affinity) of the antibody. A mutation, such as a PCR-mediated mutation, can be introduced, wherein the effect of the mutation on antibody binding or other functional properties can be assessed using in vitro or in vivo tests as described herein. Typically, a conservative mutation is introduced. Such mutation can be substitution, addition or deletion of an amino acid. In addition, there are typically a maximum of one or two mutations within the CDRs. Thus, the humanized antibody according to the present invention also include the antibody comprising one or two amino acid mutations within the CDRs.

As used herein, the term "epitope" refers to any antigen determining cluster on an antigen to which the paratope of an antibody binds. The epitopic determining cluster typically includes a chemically active surface typing of molecules, such as an amino acid or sugar side chain, and typically has specific three dimensional structural characteristics as well as specific charge characteristics.

As used herein, a variable structural domain or variable region is a specific Ig structural domain of an antibody heavy or light chain that comprises an amino acid sequence that varies between different antibodies. Each light chain and each heavy chain has variable region structural domains V_{L} and V_{H}, respectively. The variable structural domain provides antigen specificity and is therefore responsible for antigen recognition. Each variable region comprises a CDR and a framework region (FR), wherein the CDR is the moiety of an antigen-binding site structural domain.

As used herein, an "antigen binding structural domain" and "antigen-binding site" are used synonymously to refer to a structural domain within an antibody that recognizes and physically interacts with a cognate antigen. Native common full length antibody molecules have two common antigen-binding sites, each of which includes a heavy chain variable region moiety and a light chain variable region moiety. Common antigen binding sites include a loop joining the antiparallel beta (β) chains within the variable region structural domain. The antigen-binding site may include other moieties of the variable region structural domain. Each common antigen-binding site includes 3 hypervariable regions from the heavy chain and 3 hypervariable regions from the light chain. The hypervariable region is also referred to as a complementarity determining region (CDR).

As used herein, the "hypervariable region", "HV", "complementarity determining region", "CDR", and "antibody CDR" may be used interchangeably to refer to one of the multiple moieties of each variable region of an antigen-binding site that together form an antibody. Each variable region structural domain includes three CDRs, named as CDR1, CDR2, and CDR3. For example, the light chain variable region structural domain includes three CDRs named as VL CDR1, VL CDR2, and VL CDR3; the heavy chain variable region structural domain includes three CDRs named as VH CDR1, VH CDR2, and VH CDR3. The three CDRs in the variable region are discontinuous along the linear amino acid sequence, but are close in the folded polypeptide. The CDRs are located within the loop joining the parallel chains within a β-sheet of the variable structural domain. As described herein, those skilled in the art are aware of and can identify CDRs based on Kabat or Chothia numbers (see, e.g., Kabat, E.A. et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917). In the present invention, the abbreviations "VH CDR", "HCDR", "VL CDR", and "LCDR" have the same meaning.

As used herein, a framework region (FR) is a structural domain within an antibody variable region structural domain within a β-sheet. In terms of amino acid sequence, the FR region is relatively more conservative than the hypervariable region.

As used herein, a "constant region" structural domain is a structural domain in an antibody heavy or light chain that comprises an amino acid sequence that is relatively more conservative than the amino acid sequence of the variable region structural domain. In a common full length antibody molecule, each light chain has a single light chain constant region (C_{L}) structural domain, and each heavy chain includes one or more heavy chain constant region (C_{H}) structural domains, comprising C_{H}1, C_{H}2, C_{H}3, and C_{H}4. The full length IgA, IgD, and IgG isotypes include C_{H}1, C_{H}2, C_{H}3, and a hinge region, while IgE and IgM include C_{H}1, C_{H}2, C_{H}3, and C_{H}4. The C_{H}1 and C_{L} structural domains extend the Fab arm of the antibody molecule, thus facilitating interaction with the antigen and rotation of the antibody arm. The antibody constant region may serve to an effector function such as, but not limited to, clearing the antigens, pathogens, and toxins to which the antibody specifically binds, such as by interaction with various cells, biomolecules, and tissues.

As used herein, a functional region of an antibody is an antibody moiety comprising at least V_{H}, V_{L}, C_{H} (e.g., C_{H}1, C_{H}2 or C_{H}3), C_{L} or a hinge region structural domain or at least a functional region thereof of the antibody.

As used herein, a functional region of a V_{H} structural domain is at least a portion of a complete V_{H} structural domain that retains at least a part of the binding specificity of the complete V_{H} structural domain (e.g., by retaining one or more CDRs of the complete V_{H} structural domain) such that the functional region of the V_{H} structural domain binds to the antigen either alone or in combination with another antibody structural domain (e.g., a V_{L} structural domain) or a region thereof. A functional region of an exemplary V_{H} structural domain is a region that contains CDR1, CDR2, and/or CDR3 of the V_{H} structural domain.

As used herein, a functional region of a V_{L} structural domain is at least a portion of a complete V_{L} structural domain that retains at least a part of the binding specificity of the complete V_{L} structural domain (e.g., by retaining one or more CDRs of the complete V_{L} structural domain) such that the functional region of the V_{L} structural domain binds to the antigen either alone or in combination with another antibody structural domain (e.g., a V_{H} structural domain) or a region thereof. A functional region of an exemplary V_{L} structural domain is a region that contains CDR1, CDR2 and/or CDR3 of the V_{L} structural domain.

As used herein, the "specific binding" or "immunospecific binding" with respect to an antibody or an antigen-binding fragment thereof is used interchangeably herein and refers to the capability of an antibody or an antigen-binding fragment thereof to form one or more non-covalent bonds with an alloantigen through the non-covalent interaction between antibody binding sites of the antibody and the antigen. The antigen may be an isolated antigen or present in a tumor cell.

The term "Kassoc" or "Ka" used herein is intended to indicate the binding rate of a specific antibody-antigen interaction, while the term "Kdis" or "Kd" is intended to indicate the dissociation rate of a specific antibody-antigen interaction. The term "KD" used herein is intended to represent a dissociation constant, which is derived from the ratio of Kd to Ka (i.e., Kd/Ka) and is expressed as a molar concentration (M). The KD value of an antibody may be determined using methods known in the art. The KD of an antibody may be determined by for example surface plasmon resonance (SPR, Rich and Myszka (2000) Curr. Opin. Biotechnol 11:54; Englebienne (1998) Analyst. 123:1599), isothermal titration calorimetry (ITC) or other kinetic interaction determination methods known in the art (see, for example, Paul, ed., Fundamental Immunology, 2nd ed., Raven Press, New York, pages 332-336 (1989); see also US Patent No. 7,229,619 that describes exemplary SPR and ITC methods for calculating the binding affinity of antibodies). Instruments and methods for detecting and monitoring the binding rate in real time are known and commercially available (see BiaCore 2000, Biacore AB, Upsala, Sweden and GE Healthcare Life Sciences; Malmqvist (2000) Biochem. Soc. Trans. 27:335).

The terms "conserved sequence modification" or "conserved amino acid modification" or "conserved amino acid substitution" as used herein refer to an amino acid modification that does not significantly affect or alter the binding properties of the antibody comprising that amino acid sequence. Such conserved modification includes substitution, addition, and deletion of an amino acid. Modification may be introduced into the antibodies of the present invention by means of standard techniques known in the art, such as site-directed mutagenesis and PCR-mediated mutagenesis. The "conserved amino acid modification" or "conserved amino acid substitution" refers to an substitution in which an amino acid residue is replaced with an amino acid residue of a similar property and/or structure. Families of amino acid residues with similar side chains are well defined in the art.

There are 20 common amino acids, and there are many ways to classify the amino acids. For example, based on the groups binding to the amino acids, amino acids may be divided into aliphatic amino acids, aromatic amino acids, heterocyclic amino acids, sulfur-containing amino acids, and iodine-containing amino acids. Aliphatic amino acids include alanine, valine, leucine, isoleucine, methionine, aspartic acid, glutamic acid, lysine, arginine, glycine, serine, threonine, cysteine, asparagine, and glutamine. Aromatic amino acids include: phenylalanine and tyrosine. Heterocyclic amino acids include: histidine and tryptophan. Heterocyclic amino acids include: proline. Based on the chemical properties of amino acids, amino acids may be divided into acidic amino acids, basic amino acids, and neutral amino acids. Neutral amino acids include glycine, alanine, leucine, isoleucine, valine, cystine, cysteine, methionine, threonine, serine, phenylalanine, tyrosine, tryptophan, proline, methionine, and hydroxyproline. Acidic amino acids include glutamic acid and aspartic acid. Basic amino acids include lysine, arginine, and histidine.

In addition, amino acids may also be classified as follows: amino acids with basic side chains (e.g., lysine, arginine, histidine), acidic side chains (e.g., aspartic acid, glutamic acid), amino acids with uncharged polar side chains (e.g., glycine, asparagine, glutamine, serine, threonine, tyrosine, cysteine, tryptophan), amino acids with non-polar side chains (e.g., alanine, valine, leucine, isoleucine, proline, phenylalanine, methionine), amino acids with β-branched side chains (e.g., threonine, valine, isoleucine) and amino acids with aromatic side chains (e.g., tyrosine, phenylalanine, tryptophan, histidine).

As used herein, "polypeptide" refers to two or more amino acids covalently linked. The terms "polypeptide" and "protein" are used interchangeably herein.

The terms "isolated protein", "isolated polypeptide" or "isolated antibody" means that the protein, polypeptide or antibody (1) is not associated with its naturally associated components in its natural state, (2) is free of other proteins from the same species, (3) is expressed by cells from different species, or (4) is not occurring in nature. Thus, a chemically synthesized polypeptide or a polypeptide synthesized in a cellular system other than a naturally derived cell of the polypeptide will be "isolated" from its naturally associated components. The isolation may also be employed to render the protein substantially free of naturally associated components, i.e., using protein purification techniques well known in the art.

The "substitution", "deletion", or "addition" of amino acid residues refers to the conserved amino acid modification in a peptide or protein that does not alter the function and/or biological activity of the peptide or protein. For example, the substitution of amino acid residues may be a conserved amino acid substitution. Suitable conservative amino acid substitutions are known to those skilled in the art and may generally be carried out without altering the biological activity of the resulting molecule. In general, one skilled in the art knows that a single amino acid substitution in a non-essential region of a polypeptide does not substantially alter the biological activity (see, for example, Watson et al., Molecular Biology of the Gene, 4th Edition, 1987, The Benjamin/Cummings Pub. co., p.224).

As used herein, the terms "polynucleotide" and "nucleic acid molecule" refer to an oligomer or polymer comprising at least two linked nucleotides or nucleotide derivatives, comprising deoxyribonucleic acid (DNA) and ribonucleic acid (RNA) typically linked together by phosphodiester bonds.

As used herein, an isolated nucleic acid molecule is a nucleic acid molecule isolated from other nucleic acid molecules present in a natural nucleic acid molecule source. An "isolated" nucleic acid molecule, such as a cDNA molecule, may be substantially free of other cellular material or culture medium when prepared by recombinant techniques, or substantially free of chemical precursor or other chemical components in chemical synthesis. An exemplary isolated nucleic acid molecule provided herein includes an isolated nucleic acid molecule encoding the provided antibody or antigen-binding fragment.

The "sequence identity" has the meaning well known in the art, and the percentage of sequence identity between two nucleic acid or polypeptide molecules or regions may be calculated using the disclosed technique. The sequence identity may be measured along the full length of a polynucleotide or polypeptide or along a region of the molecule. (See, for example: Computational Molecular Biology, Lesk, A.M., ed., Oxford University Press, New York, 1988; Biocomputing: Informatics and Genome Projects, Smith, D.W., ed., Academic Press, New York, 1993*;* Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many methods for measuring the identity between two polynucleotides or polypeptides, the term " sequence identity" is well known to those skilled in the art (Carrillo, H. & Lipman, D., SIAM J Applied Math 48:1073 (1988)).

As used herein, "operably linked" with respect to a nucleic acid sequence, region, element or structural domain means that the nucleic acid regions are functionally related to each other. For example, a promoter may be operably linked to a nucleic acid encoding a polypeptide such that the promoter regulates or mediates transcription of the nucleic acid.

As used herein, "expression" refers to the process by which a polypeptide is produced by transcription and translation of a polynucleotide. The level of expression of the polypeptide may be assessed using any method known in the art, comprising, for example, methods for determining the amount of polypeptide produced from a host cell. Such methods may include, but are not limited to, quantification of polypeptides in cell lysates by ELISA, Coomassie blue staining after gel electrophoresis, Lowry protein determination, and Bradford protein determination.

As used herein, a "host cell" is a cell used to receive, maintain, replicate, and amplify a vector A host cell may also be used to express the polypeptide encoded by a vector. When the host cell divides, the nucleic acid contained in the vector replicates, thereby amplifying the nucleic acid. The host cell may be an eukaryotic cell or a prokaryotic cell. Suitable host cells include, but are not limited to, CHO cells, COS cells, HeLa cells, HEK cells such as HEK 293 cells.

As used herein, a "vector" is a replicable nucleic acid from which one or more heterologous proteins may be expressed when the vector is transformed into a suitable host cell. The vectors include those into which a nucleic acid encoding a polypeptide or a fragment thereof may be introduced, typically by restricting enzyme digestion and ligation. Also included in the vectors are those vectors comprising a nucleic acid encoding a polypeptide. A vector is used to introduce a nucleic acid encoding a polypeptide into a host cell, to amplify the nucleic acid, or to express/display the polypeptide encoded by the nucleic acid. The vector typically remains free, but can be designed to integrate a gene or a part thereof into the chromosome of a genome. Vectors of artificial chromosomes, such as yeast artificial vectors and mammalian artificial chromosomes, are also contemplated. The selection and use of such vectors are well known to those skilled in the art. As used herein, an "expression vector" includes a vector capable of expressing DNA operably linked to a regulatory sequence, such as a regulatory sequence in a promoter region that is capable of affecting the expression of such a DNA fragment. Such additional fragments may include promoter and terminator sequences, and may optionally include one or more replication origins, one or more selective markers, enhancers, polyadenylation signals, and the like. An expression vector is typically derived from plasmid or viral DNA, or may include elements of both. Thus, an expression vector refers to a recombinant DNA or RNA construct, such as a plasmid, phage, recombinant virus, or other vector that, when introduced into a suitable host cell, results in expression of the cloned DNA. Suitable expression vectors are well known to those skilled in the art and include expression vectors that are replicable in eukaryotic cells and/or prokaryotic cells, as well as expression vectors that retain the free expression vector or integrate into the genome of the host cell.

As used herein, "treating" an individual having a disease or condition indicates partial or total relief of the symptoms of the individual, or no change after treatment. Thus, treatment includes prevention, therapy, and/or cure. Prevention refers to the prevention of underlying disease and/or prevention of worsening symptoms or disease progression. Treatment also includes any of the antibody or antigen-binding fragment thereof provided, as well as any pharmaceutical use of the composition provided herein.

As used herein, "therapeutic effect" refers to an effect caused by the treatment of an individual that alters, generally ameliorates or relieves the symptoms of a disease or condition, or cures the disease or condition.

As used herein, "therapeutically effective amount" or "therapeutically effective dosage" refers to an amount of a substance, compound, material, or composition comprising a compound that is at least sufficient to produce a therapeutic effect upon administration to a subject. Thus, it is an amount necessary to prevent, cure, ameliorate, block or partially block the symptoms of a disease or condition.

As used herein, "prophylactically effective amount" or "prophylactically effective dosage" refers to an amount of a substance, compound, material or a composition comprising a compound that has the desired prophylactic effect upon administration to a subject, for example, preventing or delaying the occurrence or relapse of a disease or condition, and reducing the likelihood of the occurrence or relapse of a disease or condition. The complete prophylactically effective dosage does not have to occur by administrating one dose and can occur only after administrating a series of doses. Thus, a prophylactically effective amount can be administered in one or more administrations.

As used herein, the term "patient" refers to a mammal, such as a human being.

### II. Antibody targeting the ITGA2 protein

The full name of ITGA2 is Integrin alpha-2, and the human ITGA2 protein is numbered as P17301 in the Uniprot database. See https://www.uniprot.org/uniprot/P17301.

The inventor found that ITGA2 is a tumor-associated antigen that is specifically overexpressed by tumor cells, especially in patients with cancers such as colorectal cancer, bile duct cancer, pancreatic cancer, and skin cancer. Based on this, the present inventor has designed a variety of different humanized antibodies targeting the ITGA2 protein. The experiments in examples prove that the antibody according to the present invention has excellent binding ability for the ITGA2 protein (EC50<1 nM), and also has excellent binding ability to HuCCT1 cells that highly express ITGA2 (EC50<5 nM). With the excellent binding ability of the monoclonal antibody according to the present invention, partner molecules such as cytotoxins can be effectively delivered to tumor cells.

Therefore, in one aspect, the present invention provides an isolated antibody specifically binding to an ITGA2 protein or an antigen-binding fragment thereof, wherein the isolated antibody or the antigen-binding fragment thereof comprises a heavy chain variable region that comprises HCDR1, HCDR2, and HCDR3 sequences:
1) the heavy chain variable region comprises (a) HCDR1 as set forth in SEQ ID NO: 112, (b) HCDR2 as set forth in SEQ ID NO: 113, and (c) HCDR3 as set forth in SEQ ID NO: 114;
2) the heavy chain variable region comprises (a) HCDR1 as set forth in SEQ ID NO: 116, (b) HCDR2 as set forth in SEQ ID NO: 117, and (c) HCDR3 as set forth in SEQ ID NO: 118;
3) the heavy chain variable region comprises (a) HCDR1 as set forth in SEQ ID NO: 120, (b) HCDR2 as set forth in SEQ ID NO: 121, and (c) HCDR3 as set forth in SEQ ID NO: 122;
4) the heavy chain variable region comprises (a) HCDR1 as set forth in SEQ ID NO: 124, (b) HCDR2 as set forth in SEQ ID NO: 125, and (c) HCDR3 as set forth in SEQ ID NO: 126;
5) the heavy chain variable region comprises (a) HCDR1 as set forth in SEQ ID NO: 128, (b) HCDR2 as set forth in SEQ ID NO: 129, and (c) HCDR3 as set forth in SEQ ID NO: 130;
6) the heavy chain variable region comprises (a) HCDR1 as set forth in SEQ ID NO: 132, (b) HCDR2 as set forth in SEQ ID NO: 133, and (c) HCDR3 as set forth in SEQ ID NO: 134;
7) the heavy chain variable region comprises (a) HCDR1 as set forth in SEQ ID NO: 136, (b) HCDR2 as set forth in SEQ ID NO: 137, and (c) HCDR3 as set forth in SEQ ID NO: 138;
8) the heavy chain variable region comprises (a) HCDR1 as set forth in SEQ ID NO: 140, (b) HCDR2 as set forth in SEQ ID NO: 141, and (c) HCDR3 as set forth in SEQ ID NO: 142;
9) the heavy chain variable region comprises (a) HCDR1 as set forth in SEQ ID NO: 144, (b) HCDR2 as set forth in SEQ ID NO: 145, and (c) HCDR3 as set forth in SEQ ID NO: 146;
10) the heavy chain variable region comprises (a) HCDR1 as set forth in SEQ ID NO: 148, (b) HCDR2 as set forth in SEQ ID NO: 149, and (c) HCDR3 as set forth in SEQ ID NO: 150;
11) the heavy chain variable region comprises (a) HCDR1 as set forth in SEQ ID NO: 152, (b) HCDR2 as set forth in SEQ ID NO: 153, and (c) HCDR3 as set forth in SEQ ID NO: 154;
12) the heavy chain variable region comprises (a) HCDR1 as set forth in SEQ ID NO: 156, (b) HCDR2 as set forth in SEQ ID NO: 157, and (c) HCDR3 as set forth in SEQ ID NO: 158;
13) the heavy chain variable region comprises (a) HCDR1 as set forth in SEQ ID NO: 160, (b) HCDR2 as set forth in SEQ ID NO: 161, and (c) HCDR3 as set forth in SEQ ID NO: 162;
14) the heavy chain variable region comprises (a) HCDR1 as set forth in SEQ ID NO: 164, (b) HCDR2 as set forth in SEQ ID NO: 165, and (c) HCDR3 as set forth in SEQ ID NO: 166;
15) the heavy chain variable region comprises (a) HCDR1 as set forth in SEQ ID NO: 168, (b) HCDR2 as set forth in SEQ ID NO: 169, and (c) HCDR3 as set forth in SEQ ID NO: 170;
16) the heavy chain variable region comprises (a) HCDR1 as set forth in SEQ ID NO: 172, (b) HCDR2 as set forth in SEQ ID NO: 173, and (c) HCDR3 as set forth in SEQ ID NO: 174;
17) the heavy chain variable region comprises (a) HCDR1 as set forth in SEQ ID NO: 176, (b) HCDR2 as set forth in SEQ ID NO: 177, and (c) HCDR3 as set forth in SEQ ID NO: 178; and
18) the heavy chain variable region comprises (a) HCDR1 as set forth in SEQ ID NO: 180, (b) HCDR2 as set forth in SEQ ID NO: 181, and (c) HCDR3 as set forth in SEQ ID NO: 182.

In another aspect, the present invention provides an isolated antibody specifically binding to an ITGA2 protein or an antigen-binding fragment thereof, wherein the isolated antibody or the antigen-binding fragment thereof comprises a light chain variable region that contains LCDR1, LCDR2, and LCDR3 sequences:
1) the light chain variable region comprises (d) LCDR1 as set forth in SEQ ID NO: 38, (e) LCDR2 as set forth in SEQ ID NO: 39, and (f) LCDR3 as set forth in SEQ ID NO: 40;
2) the light chain variable region includes (d) LCDR1 as set forth in SEQ ID NO: 42, (e) LCDR2 as set forth in SEQ ID NO: 43, and (f) LCDR3 as set forth in SEQ ID NO: 44;
3) the light chain variable region comprises (d) LCDR1 as set forth in SEQ ID NO: 46, (e) LCDR2 as set forth in SEQ ID NO: 47, and (f) LCDR3 as set forth in SEQ ID NO: 48;
4) the light chain variable region comprises (d) LCDR1 as set forth in SEQ ID NO: 50, (e) LCDR2 as set forth in SEQ ID NO: 51, and (f) LCDR3 as set forth in SEQ ID NO: 52;
5) the light chain variable region comprises (d) LCDR1 as set forth in SEQ ID NO: 54, (e) LCDR2 as set forth in SEQ ID NO: 55, and (f) LCDR3 as set forth in SEQ ID NO: 56;
6) the light chain variable region comprises (d) LCDR1 as set forth in SEQ ID NO: 58, (e) LCDR2 as set forth in SEQ ID NO: 59, and (f) LCDR3 as set forth in SEQ ID NO: 60;
7) the light chain variable region comprises (d) LCDR1 as set forth in SEQ ID NO: 62, (e) LCDR2 as set forth in SEQ ID NO: 63, and (f) LCDR3 as set forth in SEQ ID NO: 64;
8) the light chain variable region comprises (d) LCDR1 as set forth in SEQ ID NO: 66, (e) LCDR2 as set forth in SEQ ID NO: 67, and (f) LCDR3 as set forth in SEQ ID NO: 68;
9) the light chain variable region comprises (d) LCDR1 as set forth in SEQ ID NO: 70, (e) LCDR2 as set forth in SEQ ID NO: 71, and (f) LCDR3 as set forth in SEQ ID NO: 72;
10) the light chain variable region comprises (d) LCDR1 as set forth in SEQ ID NO: 74, (e) LCDR2 as set forth in SEQ ID NO: 75, and (f) LCDR3 as set forth in SEQ ID NO: 76;
11) the light chain variable region comprises (d) LCDR1 as set forth in SEQ ID NO: 78, (e) LCDR2 as set forth in SEQ ID NO: 79, and (f) LCDR3 as set forth in SEQ ID NO: 80;
12) the light chain variable region comprises (d) LCDR1 as set forth in SEQ ID NO: 82, (e) LCDR2 as set forth in SEQ ID NO: 83, and (f) LCDR3 as set forth in SEQ ID NO: 84;
13) the light chain variable region comprises (d) LCDR1 as set forth in SEQ ID NO: 86, (e) LCDR2 as set forth in SEQ ID NO: 87, and (f) LCDR3 as set forth in SEQ ID NO: 88;
14) the light chain variable region comprises (d) LCDR1 as set forth in SEQ ID NO: 90, (e) LCDR2 as set forth in SEQ ID NO: 91, and (f) LCDR3 as set forth in SEQ ID NO: 92;
15) the light chain variable region comprises (d) LCDR1 as set forth in SEQ ID NO: 94, (e) LCDR2 as set forth in SEQ ID NO: 95, and (f) LCDR3 as set forth in SEQ ID NO: 96;
16) the light chain variable region comprises (d) LCDR1 as set forth in SEQ ID NO: 98, (e) LCDR2 as set forth in SEQ ID NO: 99, and (f) LCDR3 as set forth in SEQ ID NO: 100;
17) the light chain variable region comprises (d) LCDR1 as set forth in SEQ ID NO: 102, (e) LCDR2 as set forth in SEQ ID NO: 103, and (f) LCDR3 as set forth in SEQ ID NO: 104; and
18) the light chain variable region comprises (d) LCDR1 as set forth in SEQ ID NO: 106, (e) LCDR2 as set forth in SEQ ID NO: 107, and (f) LCDR3 as set forth in SEQ ID NO: 108.

In some embodiments, the isolated antibody or the antigen-binding fragment thereof comprises a heavy chain variable region comprising HCDR1, HCDR2, and HCDR3 sequences, and a light chain variable region comprising LCDR1, LCDR2, and LCDR3 sequences, wherein the antibody or the antigen-binding fragment thereof comprises a heavy chain variable region and a light chain variable region selected from at least one of the following groups:
1) a heavy chain variable region that comprises (a) HCDR1 as set forth in SEQ ID NO: 112, (b) HCDR2 as set forth in SEQ ID NO: 113, and (c) HCDR3 as set forth in SEQ ID NO: 114, and a light chain variable region that comprises (d) LCDR1 as set forth in SEQ ID NO: 38, (e) LCDR2 as set forth in SEQ ID NO: 39, and (f) LCDR3 as set forth in SEQ ID NO: 40;
2) a heavy chain variable region that comprises (a) HCDR1 as set forth in SEQ ID NO: 116, (b) HCDR2 as set forth in SEQ ID NO: 117, and (c) HCDR3 as set forth in SEQ ID NO: 118, and a light chain variable region that comprises (d) LCDR1 as set forth in SEQ ID NO: 42, (e) LCDR2 as set forth in SEQ ID NO: 43, and (f) LCDR3 as set forth in SEQ ID NO: 44;
3) a heavy chain variable region that comprises (a) HCDR1 as set forth in SEQ ID NO: 120, (b) HCDR2 as set forth in SEQ ID NO: 121, and (c) HCDR3 as set forth in SEQ ID NO: 122, and a light chain variable region that comprises (d) LCDR1 as set forth in SEQ ID NO: 46, (e) LCDR2 as set forth in SEQ ID NO: 47, and (f) LCDR3 as set forth in SEQ ID NO: 48;
4) a heavy chain variable region that comprises (a) HCDR1 as set forth in SEQ ID NO: 124, (b) HCDR2 as set forth in SEQ ID NO: 125, and (c) HCDR3 as set forth in SEQ ID NO: 126, and a light chain variable region that comprises (d) LCDR1 as set forth in SEQ ID NO: 50, (e) LCDR2 as set forth in SEQ ID NO: 51, and (f) LCDR3 as set forth in SEQ ID NO: 52;
5) a heavy chain variable region that comprises (a) HCDR1 as set forth in SEQ ID NO: 128, (b) HCDR2 as set forth in SEQ ID NO: 129, and (c) HCDR3 as set forth in SEQ ID NO: 130, and a light chain variable region that comprises (d) LCDR1 as set forth in SEQ ID NO: 54, (e) LCDR2 as set forth in SEQ ID NO: 55, and (f) LCDR3 as set forth in SEQ ID NO: 56;
6) a heavy chain variable region that comprises (a) HCDR1 as set forth in SEQ ID NO: 132, (b) HCDR2 as set forth in SEQ ID NO: 133, and (c) HCDR3 as set forth in SEQ ID NO: 134, and a light chain variable region that comprises (d) LCDR1 as set forth in SEQ ID NO: 58, (e) LCDR2 as set forth in SEQ ID NO: 59, and (f) LCDR3 as set forth in SEQ ID NO: 60;
7) a heavy chain variable region that comprises (a) HCDR1 as set forth in SEQ ID NO: 136, (b) HCDR2 as set forth in SEQ ID NO: 137, and (c) HCDR3 as set forth in SEQ ID NO: 138, and a light chain variable region that comprises (d) LCDR1 as set forth in SEQ ID NO: 62, (e) LCDR2 as set forth in SEQ ID NO: 63, and (f) LCDR3 as set forth in SEQ ID NO: 64;
8) a heavy chain variable region that comprises (a) HCDR1 as set forth in SEQ ID NO: 140, (b) HCDR2 as set forth in SEQ ID NO: 141, and (c) HCDR3 as set forth in SEQ ID NO: 142, and a light chain variable region that comprises (d) LCDR1 as set forth in SEQ ID NO: 66, (e) LCDR2 as set forth in SEQ ID NO: 67, and (f) LCDR3 as set forth in SEQ ID NO: 68;
9) the heavy chain variable region comprises (a) HCDR1 as set forth in SEQ ID NO: 144, (b) HCDR2 as set forth in SEQ ID NO: 145, and (c) HCDR3 as set forth in SEQ ID NO: 146, and the light chain variable region comprises (d) LCDR1 as set forth in SEQ ID NO: 70, (e) LCDR2 as set forth in SEQ ID NO: 71, and (f) LCDR3 as set forth in SEQ ID NO: 72;
10) a heavy chain variable region that comprises (a) HCDR1 as set forth in SEQ ID NO: 148, (b) HCDR2 as set forth in SEQ ID NO: 149, and (c) HCDR3 as set forth in SEQ ID NO: 150, and a light chain variable region that comprises (d) LCDR1 as set forth in SEQ ID NO: 74, (e) LCDR2 as set forth in SEQ ID NO: 75, and (f) LCDR3 as set forth in SEQ ID NO: 76;
11) a heavy chain variable region that comprises (a) HCDR1 as set forth in SEQ ID NO: 152, (b) HCDR2 as set forth in SEQ ID NO: 153, and (c) HCDR3 as set forth in SEQ ID NO: 154, and a light chain variable region that comprises (d) LCDR1 as set forth in SEQ ID NO: 78, (e) LCDR2 as set forth in SEQ ID NO: 79, and (f) LCDR3 as set forth in SEQ ID NO: 80;
12) a heavy chain variable region that comprises (a) HCDR1 as set forth in SEQ ID NO: 156, (b) HCDR2 as set forth in SEQ ID NO: 157, and (c) HCDR3 as set forth in SEQ ID NO: 158, and a light chain variable region that comprises (d) LCDR1 as set forth in SEQ ID NO: 82, (e) LCDR2 as set forth in SEQ ID NO: 83, and (f) LCDR3 as set forth in SEQ ID NO: 84;
13) the heavy chain variable region comprises (a) HCDR1 as set forth in SEQ ID NO: 160, (b) HCDR2 as set forth in SEQ ID NO: 161, and (c) HCDR3 as set forth in SEQ ID NO: 162, and the light chain variable region comprises (d) LCDR1 as set forth in SEQ ID NO: 86, (e) LCDR2 as set forth in SEQ ID NO: 87, and (f) LCDR3 as set forth in SEQ ID NO: 88;
14) a heavy chain variable region that comprises (a) HCDR1 as set forth in SEQ ID NO: 164, (b) HCDR2 as set forth in SEQ ID NO: 165, and (c) HCDR3 as set forth in SEQ ID NO: 166, and a light chain variable region that comprises (d) LCDR1 as set forth in SEQ ID NO: 90, (e) LCDR2 as set forth in SEQ ID NO: 91, and (f) LCDR3 as set forth in SEQ ID NO: 92;
15) a heavy chain variable region that comprises (a) HCDR1 as set forth in SEQ ID NO: 168, (b) HCDR2 as set forth in SEQ ID NO: 169, and (c) HCDR3 as set forth in SEQ ID NO: 170, and a light chain variable region that comprises (d) LCDR1 as set forth in SEQ ID NO: 94, (e) LCDR2 as set forth in SEQ ID NO: 95, and (f) LCDR3 as set forth in SEQ ID NO: 96;
16) a heavy chain variable region that comprises (a) HCDR1 as set forth in SEQ ID NO: 172, (b) HCDR2 as set forth in SEQ ID NO: 173, and (c) HCDR3 as set forth in SEQ ID NO: 174, and a light chain variable region that comprises (d) LCDR1 as set forth in SEQ ID NO: 98, (e) LCDR2 as set forth in SEQ ID NO: 99, and (f) LCDR3 as set forth in SEQ ID NO: 100;
17) the heavy chain variable region comprises (a) HCDR1 as set forth in SEQ ID NO: 176, (b) HCDR2 as set forth in SEQ ID NO: 177, and (c) HCDR3 as set forth in SEQ ID NO: 178, and the light chain variable region comprises (d) LCDR1 as set forth in SEQ ID NO: 102, (e) LCDR2 as set forth in SEQ ID NO: 103, and (f) LCDR3 as set forth in SEQ ID NO: 104; and
18) a heavy chain variable region that comprises (a) HCDR1 as set forth in SEQ ID NO: 180, (b) HCDR2 as set forth in SEQ ID NO: 181, and (c) HCDR3 as set forth in SEQ ID NO: 182, and a light chain variable region that comprises (d) LCDR1 as set forth in SEQ ID NO: 106, (e) LCDR2 as set forth in SEQ ID NO: 107, and (f) LCDR3 as set forth in SEQ ID NO: 108.

In some embodiments, the isolated antibody or the antigen binding fragment thereof comprises a heavy chain variable region selected from at least one of the following groups:
1) a heavy chain variable region that comprises an amino acid sequence as set forth in SEQ ID NO:111, or an amino acid sequence that is at least 85%, at least 90%, at least 95%, at least 96%, at least 97%, at least 98%, or at least 99% identical to the amino acid sequence as set forth in SEQ ID NO:111;
2) a heavy chain variable region that comprises an amino acid sequence as set forth in SEQ ID NO:115, or an amino acid sequence that is at least 85%, at least 90%, at least 95%, at least 96%, at least 97%, at least 98%, or at least 99% identical to the amino acid sequence as set forth in SEQ ID NO:115;
3) a heavy chain variable region that comprises an amino acid sequence as set forth in SEQ ID NO:119, or an amino acid sequence that is at least 85%, at least 90%, at least 95%, at least 96%, at least 97%, at least 98%, or at least 99% identical to the amino acid sequence as set forth in SEQ ID NO:119;
4) a heavy chain variable region that comprises an amino acid sequence as set forth in SEQ ID NO:123, or an amino acid sequence that is at least 85%, at least 90%, at least 95%, at least 96%, at least 97%, at least 98%, or at least 99% identical to the amino acid sequence as set forth in SEQ ID NO:123;
5) a heavy chain variable region that comprises an amino acid sequence as set forth in SEQ ID NO:127, or an amino acid sequence that is at least 85%, at least 90%, at least 95%, at least 96%, at least 97%, at least 98%, or at least 99% identical to the amino acid sequence as set forth in SEQ ID NO:127;
6) a heavy chain variable region that comprises an amino acid sequence as set forth in SEQ ID NO:131, or an amino acid sequence that is at least 85%, at least 90%, at least 95%, at least 96%, at least 97%, at least 98%, or at least 99% identical to the amino acid sequence as set forth in SEQ ID NO:131;
7) a heavy chain variable region that comprises an amino acid sequence as set forth in SEQ ID NO:135, or an amino acid sequence that is at least 85%, at least 90%, at least 95%, at least 96%, at least 97%, at least 98%, or at least 99% identical to the amino acid sequence as set forth in SEQ ID NO:135;
8) a heavy chain variable region that comprises an amino acid sequence as set forth in SEQ ID NO:139, or an amino acid sequence that is at least 85%, at least 90%, at least 95%, at least 96%, at least 97%, at least 98%, or at least 99% identical to the amino acid sequence as set forth in SEQ ID NO:139;
9) a heavy chain variable region that comprises an amino acid sequence as set forth in SEQ ID NO:143, or an amino acid sequence that is at least 85%, at least 90%, at least 95%, at least 96%, at least 97%, at least 98%, or at least 99% identical to the amino acid sequence as set forth in SEQ ID NO:143;
10) a heavy chain variable region that comprises an amino acid sequence as set forth in SEQ ID NO:147, or an amino acid sequence that is at least 85%, at least 90%, at least 95%, at least 96%, at least 97%, at least 98%, or at least 99% identical to the amino acid sequence as set forth in SEQ ID NO:147;
11) a heavy chain variable region that comprises an amino acid sequence as set forth in SEQ ID NO:151, or an amino acid sequence that is at least 85%, at least 90%, at least 95%, at least 96%, at least 97%, at least 98%, or at least 99% identical to the amino acid sequence as set forth in SEQ ID NO:151;
12) a heavy chain variable region that comprises an amino acid sequence as set forth in SEQ ID NO:155, or an amino acid sequence that is at least 85%, at least 90%, at least 95%, at least 96%, at least 97%, at least 98%, or at least 99% identical to the amino acid sequence as set forth in SEQ ID NO:155;
13) a heavy chain variable region that comprises an amino acid sequence as set forth in SEQ ID NO:159, or an amino acid sequence that is at least 85%, at least 90%, at least 95%, at least 96%, at least 97%, at least 98%, or at least 99% identical to the amino acid sequence as set forth in SEQ ID NO:159;
14) a heavy chain variable region that comprises an amino acid sequence as set forth in SEQ ID NO:163, or an amino acid sequence that is at least 85%, at least 90%, at least 95%, at least 96%, at least 97%, at least 98%, or at least 99% identical to the amino acid sequence as set forth in SEQ ID NO:163;
15) a heavy chain variable region that comprises an amino acid sequence as set forth in SEQ ID NO:167, or an amino acid sequence that is at least 85%, at least 90%, at least 95%, at least 96%, at least 97%, at least 98%, or at least 99% identical to the amino acid sequence as set forth in SEQ ID NO:167;
16) a heavy chain variable region that comprises an amino acid sequence as set forth in SEQ ID NO:171, or an amino acid sequence that is at least 85%, at least 90%, at least 95%, at least 96%, at least 97%, at least 98%, or at least 99% identical to the amino acid sequence as set forth in SEQ ID NO:171;
17) a heavy chain variable region that comprises an amino acid sequence as set forth in SEQ ID NO:175, or an amino acid sequence that is at least 85%, at least 90%, at least 95%, at least 96%, at least 97%, at least 98%, or at least 99% identical to the amino acid sequence as set forth in SEQ ID NO:175; and
18) a heavy chain variable region that comprises an amino acid sequence as set forth in SEQ ID NO:179, or an amino acid sequence that is at least 85%, at least 90%, at least 95%, at least 96%, at least 97%, at least 98%, or at least 99% identical to the amino acid sequence as set forth in SEQ ID NO:179.

In some embodiments, the isolated antibody or the antigen binding fragment thereof comprises a light chain variable region selected from at least one of the following groups:
1) a light chain variable region that comprises an amino acid sequence as set forth in SEQ ID NO:37, or an amino acid sequence that is at least 85%, at least 90%, at least 95%, at least 96%, at least 97%, at least 98%, or at least 99% identical to the amino acid sequence as set forth in SEQ ID NO:37;
2) a light chain variable region that comprises an amino acid sequence as set forth in SEQ ID NO:41, or an amino acid sequence that is at least 85%, at least 90%, at least 95%, at least 96%, at least 97%, at least 98%, or at least 99% identical to the amino acid sequence as set forth in SEQ ID NO:41;
3) a light chain variable region that comprises an amino acid sequence as set forth in SEQ ID NO:45, or an amino acid sequence that is at least 85%, at least 90%, at least 95%, at least 96%, at least 97%, at least 98%, or at least 99% identical to the amino acid sequence as set forth in SEQ ID NO:45;
4) a light chain variable region that comprises an amino acid sequence as set forth in SEQ ID NO:49, or an amino acid sequence that is at least 85%, at least 90%, at least 95%, at least 96%, at least 97%, at least 98%, or at least 99% identical to the amino acid sequence as set forth in SEQ ID NO:49;
5) a light chain variable region that comprises an amino acid sequence as set forth in SEQ ID NO:53, or an amino acid sequence that is at least 85%, at least 90%, at least 95%, at least 96%, at least 97%, at least 98%, or at least 99% identical to the amino acid sequence as set forth in SEQ ID NO:53;
6) a light chain variable region that comprises an amino acid sequence as set forth in SEQ ID NO:57, or an amino acid sequence that is at least 85%, at least 90%, at least 95%, at least 96%, at least 97%, at least 98%, or at least 99% identical to the amino acid sequence as set forth in SEQ ID NO:57;
7) a light chain variable region that comprises an amino acid sequence as set forth in SEQ ID NO:61, or an amino acid sequence that is at least 85%, at least 90%, at least 95%, at least 96%, at least 97%, at least 98%, or at least 99% identical to the amino acid sequence as set forth in SEQ ID NO:61;
8) a light chain variable region that comprises an amino acid sequence as set forth in SEQ ID NO:65, or an amino acid sequence that is at least 85%, at least 90%, at least 95%, at least 96%, at least 97%, at least 98%, or at least 99% identical to the amino acid sequence as set forth in SEQ ID NO:65;
9) a light chain variable region that comprises an amino acid sequence as set forth in SEQ ID NO:69, or an amino acid sequence that is at least 85%, at least 90%, at least 95%, at least 96%, at least 97%, at least 98%, or at least 99% identical to the amino acid sequence as set forth in SEQ ID NO:69;
10) a light chain variable region that comprises an amino acid sequence as set forth in SEQ ID NO:73, or an amino acid sequence that is at least 85%, at least 90%, at least 95%, at least 96%, at least 97%, at least 98%, or at least 99% identical to the amino acid sequence as set forth in SEQ ID NO:73;
11) a light chain variable region that comprises an amino acid sequence as set forth in SEQ ID NO:77, or an amino acid sequence that is at least 85%, at least 90%, at least 95%, at least 96%, at least 97%, at least 98%, or at least 99% identical to the amino acid sequence as set forth in SEQ ID NO:77;
12) a light chain variable region that comprises an amino acid sequence as set forth in SEQ ID NO:81, or an amino acid sequence that is at least 85%, at least 90%, at least 95%, at least 96%, at least 97%, at least 98%, or at least 99% identical to the amino acid sequence as set forth in SEQ ID NO:81;
13) a light chain variable region that comprises an amino acid sequence as set forth in SEQ ID NO:85, or an amino acid sequence that is at least 85%, at least 90%, at least 95%, at least 96%, at least 97%, at least 98%, or at least 99% identical to the amino acid sequence as set forth in SEQ ID NO:85;
14) a light chain variable region that comprises an amino acid sequence as set forth in SEQ ID NO:89, or an amino acid sequence that is at least 85%, at least 90%, at least 95%, at least 96%, at least 97%, at least 98%, or at least 99% identical to the amino acid sequence as set forth in SEQ ID NO:89;
15) a light chain variable region that comprises an amino acid sequence as set forth in SEQ ID NO:93, or an amino acid sequence that is at least 85%, at least 90%, at least 95%, at least 96%, at least 97%, at least 98%, or at least 99% identical to the amino acid sequence as set forth in SEQ ID NO:93;
16) a light chain variable region that comprises an amino acid sequence as set forth in SEQ ID NO:97, or an amino acid sequence that is at least 85%, at least 90%, at least 95%, at least 96%, at least 97%, at least 98%, or at least 99% identical to the amino acid sequence as set forth in SEQ ID NO:97;
17) a light chain variable region that comprises an amino acid sequence as set forth in SEQ ID NO:101, or an amino acid sequence that is at least 85%, at least 90%, at least 95%, at least 96%, at least 97%, at least 98%, or at least 99% identical to the amino acid sequence as set forth in SEQ ID NO:101; and
18) a light chain variable region that comprises an amino acid sequence as set forth in SEQ ID NO:105, or an amino acid sequence that is at least 85%, at least 90%, at least 95%, at least 96%, at least 97%, at least 98%, or at least 99% identical to the amino acid sequence as set forth in SEQ ID NO:105.

In some embodiments, the isolated antibody or the antigen binding fragment thereof comprises a heavy chain variable region and a light chain variable region selected from at least one of the following groups:
1) a heavy chain variable region that comprises an amino acid sequence as set forth in SEQ ID NO:111, or an amino acid sequence that is at least 85%, at least 90%, at least 95%, at least 96%, at least 97%, at least 98%, or at least 99% identical to the amino acid sequence as set forth in SEQ ID NO:111, and a light chain variable region that comprises an amino acid sequence as set forth in SEQ ID NO:37, or an amino acid sequence that is at least 85%, at least 90%, at least 95%, at least 96%, at least 97%, at least 98%, or at least 99% identical to the amino acid sequence as set forth in SEQ ID NO:37;
2) a heavy chain variable region that comprises an amino acid sequence as set forth in SEQ ID NO:115, or an amino acid sequence that is at least 85%, at least 90%, at least 95%, at least 96%, at least 97%, at least 98%, or at least 99% identical to the amino acid sequence as set forth in SEQ ID NO:115, and a light chain variable region that comprises an amino acid sequence as set forth in SEQ ID NO:41, or an amino acid sequence that is at least 85%, at least 90%, at least 95%, at least 96%, at least 97%, at least 98%, or at least 99% identical to the amino acid sequence as set forth in SEQ ID NO:41;
3) a heavy chain variable region that comprises an amino acid sequence as set forth in SEQ ID NO:119, or an amino acid sequence that is at least 85%, at least 90%, at least 95%, at least 96%, at least 97%, at least 98%, or at least 99% identical to the amino acid sequence as set forth in SEQ ID NO:119, and a light chain variable region that comprises an amino acid sequence as set forth in SEQ ID NO:45, or an amino acid sequence that is at least 85%, at least 90%, at least 95%, at least 96%, at least 97%, at least 98%, or at least 99% identical to the amino acid sequence as set forth in SEQ ID NO:45;
4) a heavy chain variable region that comprises an amino acid sequence as set forth in SEQ ID NO:123, or an amino acid sequence that is at least 85%, at least 90%, at least 95%, at least 96%, at least 97%, at least 98%, or at least 99% identical to the amino acid sequence as set forth in SEQ ID NO: 123, and a light chain variable region that comprises an amino acid sequence as set forth in SEQ ID NO:49, or an amino acid sequence that is at least 85%, at least 90%, at least 95%, at least 96%, at least 97%, at least 98%, or at least 99% identical to the amino acid sequence as set forth in SEQ ID NO:49;
5) a heavy chain variable region that comprises an amino acid sequence as set forth in SEQ ID NO:127, or an amino acid sequence that is at least 85%, at least 90%, at least 95%, at least 96%, at least 97%, at least 98%, or at least 99% identical to the amino acid sequence as set forth in SEQ ID NO: 127, and a light chain variable region that comprises an amino acid sequence as set forth in SEQ ID NO:53, or an amino acid sequence that is at least 85%, at least 90%, at least 95%, at least 96%, at least 97%, at least 98%, or at least 99% identical to the amino acid sequence as set forth in SEQ ID NO:53;
6) a heavy chain variable region that comprises an amino acid sequence as set forth in SEQ ID NO:131, or an amino acid sequence that is at least 85%, at least 90%, at least 95%, at least 96%, at least 97%, at least 98%, or at least 99% identical to the amino acid sequence as set forth in SEQ ID NO:131, and a light chain variable region that comprises an amino acid sequence as set forth in SEQ ID NO:57, or an amino acid sequence that is at least 85%, at least 90%, at least 95%, at least 96%, at least 97%, at least 98%, or at least 99% identical to the amino acid sequence as set forth in SEQ ID NO:57;
7) a heavy chain variable region that comprises an amino acid sequence as set forth in SEQ ID NO:135, or an amino acid sequence that is at least 85%, at least 90%, at least 95%, at least 96%, at least 97%, at least 98%, or at least 99% identical to the amino acid sequence as set forth in SEQ ID NO: 135, and a light chain variable region that comprises an amino acid sequence as set forth in SEQ ID NO:61, or an amino acid sequence that is at least 85%, at least 90%, at least 95%, at least 96%, at least 97%, at least 98%, or at least 99% identical to the amino acid sequence as set forth in SEQ ID NO:61;
8) a heavy chain variable region that comprises an amino acid sequence as set forth in SEQ ID NO:139, or an amino acid sequence that is at least 85%, at least 90%, at least 95%, at least 96%, at least 97%, at least 98%, or at least 99% identical to the amino acid sequence as set forth in SEQ ID NO: 139, and a light chain variable region that comprises an amino acid sequence as set forth in SEQ ID NO:65, or an amino acid sequence that is at least 85%, at least 90%, at least 95%, at least 96%, at least 97%, at least 98%, or at least 99% identical to the amino acid sequence as set forth in SEQ ID NO:65;
9) a heavy chain variable region that comprises an amino acid sequence as set forth in SEQ ID NO:143, or an amino acid sequence that is at least 85%, at least 90%, at least 95%, at least 96%, at least 97%, at least 98%, or at least 99% identical to the amino acid sequence as set forth in SEQ ID NO: 143, and a light chain variable region that comprises an amino acid sequence as set forth in SEQ ID NO:69, or an amino acid sequence that is at least 85%, at least 90%, at least 95%, at least 96%, at least 97%, at least 98%, or at least 99% identical to the amino acid sequence as set forth in SEQ ID NO:69;
10) a heavy chain variable region that comprises an amino acid sequence as set forth in SEQ ID NO:147, or an amino acid sequence that is at least 85%, at least 90%, at least 95%, at least 96%, at least 97%, at least 98%, or at least 99% identical to the amino acid sequence as set forth in SEQ ID NO: 147, and a light chain variable region that comprises an amino acid sequence as set forth in SEQ ID NO:73, or an amino acid sequence that is at least 85%, at least 90%, at least 95%, at least 96%, at least 97%, at least 98%, or at least 99% identical to the amino acid sequence as set forth in SEQ ID NO:73;
11) a heavy chain variable region that comprises an amino acid sequence as set forth in SEQ ID NO:151, or an amino acid sequence that is at least 85%, at least 90%, at least 95%, at least 96%, at least 97%, at least 98%, or at least 99% identical to the amino acid sequence as set forth in SEQ ID NO: 151, and a light chain variable region that comprises an amino acid sequence as set forth in SEQ ID NO:77, or an amino acid sequence that is at least 85%, at least 90%, at least 95%, at least 96%, at least 97%, at least 98%, or at least 99% identical to the amino acid sequence as set forth in SEQ ID NO:77;
12) a heavy chain variable region that comprises an amino acid sequence as set forth in SEQ ID NO:155, or an amino acid sequence that is at least 85%, at least 90%, at least 95%, at least 96%, at least 97%, at least 98%, or at least 99% identical to the amino acid sequence as set forth in SEQ ID NO:155, and a light chain variable region that comprises an amino acid sequence as set forth in SEQ ID NO:81, or an amino acid sequence that is at least 85%, at least 90%, at least 95%, at least 96%, at least 97%, at least 98%, or at least 99% identical to the amino acid sequence as set forth in SEQ ID NO:81;
13) a heavy chain variable region that comprises an amino acid sequence as set forth in SEQ ID NO:159, or an amino acid sequence that is at least 85%, at least 90%, at least 95%, at least 96%, at least 97%, at least 98%, or at least 99% identical to the amino acid sequence as set forth in SEQ ID NO:159, and a light chain variable region that comprises an amino acid sequence as set forth in SEQ ID NO:85, or an amino acid sequence that is at least 85%, at least 90%, at least 95%, at least 96%, at least 97%, at least 98%, or at least 99% identical to the amino acid sequence as set forth in SEQ ID NO:85;
14) a heavy chain variable region that comprises an amino acid sequence as set forth in SEQ ID NO:163, or an amino acid sequence that is at least 85%, at least 90%, at least 95%, at least 96%, at least 97%, at least 98%, or at least 99% identical to the amino acid sequence as set forth in SEQ ID NO: 163, and a light chain variable region that comprises an amino acid sequence as set forth in SEQ ID NO:89, or an amino acid sequence that is at least 85%, at least 90%, at least 95%, at least 96%, at least 97%, at least 98%, or at least 99% identical to the amino acid sequence as set forth in SEQ ID NO:89;
15) a heavy chain variable region that comprises an amino acid sequence as set forth in SEQ ID NO:167, or an amino acid sequence that is at least 85%, at least 90%, at least 95%, at least 96%, at least 97%, at least 98%, or at least 99% identical to the amino acid sequence as set forth in SEQ ID NO: 167, and a light chain variable region that comprises an amino acid sequence as set forth in SEQ ID NO:93, or an amino acid sequence that is at least 85%, at least 90%, at least 95%, at least 96%, at least 97%, at least 98%, or at least 99% identical to the amino acid sequence as set forth in SEQ ID NO:93;
16) a heavy chain variable region that comprises an amino acid sequence as set forth in SEQ ID NO:171, or an amino acid sequence that is at least 85%, at least 90%, at least 95%, at least 96%, at least 97%, at least 98%, or at least 99% identical to the amino acid sequence as set forth in SEQ ID NO: 171, and a light chain variable region that comprises an amino acid sequence as set forth in SEQ ID NO:97, or an amino acid sequence that is at least 85%, at least 90%, at least 95%, at least 96%, at least 97%, at least 98%, or at least 99% identical to the amino acid sequence as set forth in SEQ ID NO:97;
17) a heavy chain variable region that comprises an amino acid sequence as set forth in SEQ ID NO:175, or an amino acid sequence that is at least 85%, at least 90%, at least 95%, at least 96%, at least 97%, at least 98%, or at least 99% identical to the amino acid sequence as set forth in SEQ ID NO: 175, and a light chain variable region that comprises an amino acid sequence as set forth in SEQ ID NO:101, or an amino acid sequence that is at least 85%, at least 90%, at least 95%, at least 96%, at least 97%, at least 98%, or at least 99% identical to the amino acid sequence as set forth in SEQ ID NO:101; and
18) a heavy chain variable region that comprises an amino acid sequence as set forth in SEQ ID NO:179, or an amino acid sequence that is at least 85%, at least 90%, at least 95%, at least 96%, at least 97%, at least 98%, or at least 99% identical to the amino acid sequence as set forth in SEQ ID NO: 179, and a light chain variable region that comprises an amino acid sequence as set forth in SEQ ID NO:105, or an amino acid sequence that is at least 85%, at least 90%, at least 95%, at least 96%, at least 97%, at least 98%, or at least 99% identical to the amino acid sequence as set forth in SEQ ID NO:105.

In some embodiments, an antibody that is at least 85%, at least 90%, at least 95%, at least 96%, at least 97%, at least 98%, or at least 99% identical to the amino acid sequences of the heavy chain variable region (SEQ ID NOs: 111, 115, 119, 123, 127, 131, 135, 139, 143, 147, 151, 155, 159, 163, 167, 171, 175, 179) and/or amino acid sequences of the light chain variable region (SEQ ID NOs: 37, 41, 45, 49, 53, 57, 61, 65, 69, 73, 77, 81, 85, 89, 93, 97, 101, 105) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the heavy chain variable region (SEQ ID NOs: 111, 115, 119, 123, 127, 131, 135, 139, 143, 147, 151, 155, 159, 163, 167, 171, 175, 179) and/or amino acid sequences of the light chain variable region (SEQ ID NOs: 37, 41, 45, 49, 53, 57, 61, 65, 69, 73, 77, 81, 85, 89, 93, 97, 101, 105) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the heavy chain variable region (SEQ ID NOs: 111, 115, 119, 123, 127, 131, 135, 139, 143, 147, 151, 155, 159, 163, 167, 171, 175, 179) and/or amino acid sequences of the light chain variable region (SEQ ID NOs: 37, 41, 45, 49, 53, 57, 61, 65, 69, 73, 77, 81, 85, 89, 93, 97, 101, 105) of the antibody according to the present invention has a same CDR of the heavy chain and/or light chain variable region as the antibody according to the present invention, and has a conserved amino acid substitution only at locations (such as a framework region) of the heavy chain and/or light chain variable region other than the CDR, wherein the conserved amino acid substitution does not change the biological function and/or activity of the corresponding antibody.

Optionally, the antibody that is at least 85%, at least 90%, at least 95%, at least 96%, at least 97%, at least 98%, or at least 99% identical to the amino acid sequences of the heavy chain variable region (SEQ ID NOs: 111, 115, 119, 123, 127, 131, 135, 139, 143, 147, 151, 155, 159, 163, 167, 171, 175, 179) and/or amino acid sequences of the light chain variable region (SEQ ID NOs: 37, 41, 45, 49, 53, 57, 61, 65, 69, 73, 77, 81, 85, 89, 93, 97, 101, 105) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the heavy chain variable region (SEQ ID NOs: 111, 115, 119, 123, 127, 131, 135, 139, 143, 147, 151, 155, 159, 163, 167, 171, 175, 179) and/or amino acid sequences of the light chain variable region (SEQ ID NOs: 37, 41, 45, 49, 53, 57, 61, 65, 69, 73, 77, 81, 85, 89, 93, 97, 101, 105) of the antibody according to the present invention has a same CDR of the heavy chain and/or light chain variable region as the antibody according to the present invention, and has a conserved amino acid substitution only at a framework region of the heavy chain and/or light chain variable region, wherein the conserved amino acid substitution does not change the biological function and/or activity of the corresponding antibody, and the antibody may also specifically bind to the ITGA2 protein.

In some embodiments, the isolated antibody or the antigen-binding fragment thereof further comprises a heavy chain constant region, wherein the heavy chain constant region comprises an amino acid sequence as set forth in SEQ ID NO: 183, or an amino acid sequence that is at least 85%, at least 90%, at least 95%, at least 96%, at least 97%, at least 98%, or at least 99% identical to the amino acid sequence as set forth in SEQ ID NO: 183.

In some embodiments, the isolated antibody or the antigen-binding fragment thereof further comprises a light chain constant region, wherein the light chain constant region comprises an amino acid sequence as set forth in SEQ ID NO: 109, or an amino acid sequence that is at least 85%, at least 90%, at least 95%, at least 96%, at least 97%, at least 98%, or at least 99% identical to the amino acid sequence as set forth in SEQ ID NO: 109, or the light chain constant region comprises an amino acid sequence as set forth in SEQ ID NO: 110, or an amino acid sequence that is at least 85%, at least 90%, at least 95%, at least 96%, at least 97%, at least 98%, or at least 99% identical to the amino acid sequence as set forth in SEQ ID NO: 110

In some embodiments, the isolated antibody or the antigen binding fragment thereof further comprises a heavy chain constant region and/or a light chain constant regio,
the heavy chain constant region comprises an amino acid sequence as set forth in SEQ ID NO: 183, or an amino acid sequence that is at least 85%, at least 90%, at least 95%, at least 96%, at least 97%, at least 98%, or at least 99% identical to the amino acid sequence as set forth in SEQ ID NO:183, and
the light chain constant region comprises an amino acid sequence as set forth in SEQ ID NO: 109, or an amino acid sequence that is at least 85%, at least 90%, at least 95%, at least 96%, at least 97%, at least 98%, or at least 99% identical to the amino acid sequence as set forth in SEQ ID NO: 109, or the light chain constant region comprises an amino acid sequence as set forth in SEQ ID NO:110, or an amino acid sequence that is at least 85%, at least 90%, at least 95%, at least 96%, at least 97%, at least 98%, or at least 99% identical to the amino acid sequence as set forth in SEQ ID NO: 110

In some embodiments, the isolated antibody or the antigen binding fragment thereof comprises a heavy chain selected from at least one of the following groups:
1) a heavy chain that comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202;
2) a heavy chain that comprises an amino acid sequence as set forth in SEQ ID NO:203, or an amino acid sequence that is at least 85%, at least 90%, at least 95%, at least 96%, at least 97%, at least 98%, or at least 99% identical to the amino acid sequence as set forth in SEQ ID NO:203,
3) a heavy chain that comprises an amino acid sequence as set forth in SEQ ID NO:204, or an amino acid sequence that is at least 85%, at least 90%, at least 95%, at least 96%, at least 97%, at least 98%, or at least 99% identical to the amino acid sequence as set forth in SEQ ID NO:204;
4) a heavy chain that comprises an amino acid sequence as set forth in SEQ ID NO:205, or an amino acid sequence that is at least 85%, at least 90%, at least 95%, at least 96%, at least 97%, at least 98%, or at least 99% identical to the amino acid sequence as set forth in SEQ ID NO:205;
5) a heavy chain that comprises an amino acid sequence as set forth in SEQ ID NO:206, or an amino acid sequence that is at least 85%, at least 90%, at least 95%, at least 96%, at least 97%, at least 98%, or at least 99% identical to the amino acid sequence as set forth in SEQ ID NO:206;
6) a heavy chain that comprises an amino acid sequence as set forth in SEQ ID NO:207, or an amino acid sequence that is at least 85%, at least 90%, at least 95%, at least 96%, at least 97%, at least 98%, or at least 99% identical to the amino acid sequence as set forth in SEQ ID NO:207;
7) a heavy chain that comprises an amino acid sequence as set forth in SEQ ID NO:208, or an amino acid sequence that is at least 85%, at least 90%, at least 95%, at least 96%, at least 97%, at least 98%, or at least 99% identical to the amino acid sequence as set forth in SEQ ID NO:208;
8) a heavy chain that comprises an amino acid sequence as set forth in SEQ ID NO:209, or an amino acid sequence that is at least 85%, at least 90%, at least 95%, at least 96%, at least 97%, at least 98%, or at least 99% identical to the amino acid sequence as set forth in SEQ ID NO:209;
9) a heavy chain that comprises an amino acid sequence as set forth in SEQ ID NO:210, or an amino acid sequence that is at least 85%, at least 90%, at least 95%, at least 96%, at least 97%, at least 98%, or at least 99% identical to the amino acid sequence as set forth in SEQ ID NO:210;
10) a heavy chain that comprises an amino acid sequence as set forth in SEQ ID NO:211, or an amino acid sequence that is at least 85%, at least 90%, at least 95%, at least 96%, at least 97%, at least 98%, or at least 99% identical to the amino acid sequence as set forth in SEQ ID NO:211;
11) a heavy chain that comprises an amino acid sequence as set forth in SEQ ID NO:212, or an amino acid sequence that is at least 85%, at least 90%, at least 95%, at least 96%, at least 97%, at least 98%, or at least 99% identical to the amino acid sequence as set forth in SEQ ID NO:212;
12) a heavy chain that comprises an amino acid sequence as set forth in SEQ ID NO:213, or an amino acid sequence that is at least 85%, at least 90%, at least 95%, at least 96%, at least 97%, at least 98%, or at least 99% identical to the amino acid sequence as set forth in SEQ ID NO:213;
13) a heavy chain that comprises an amino acid sequence as set forth in SEQ ID NO:214, or an amino acid sequence that is at least 85%, at least 90%, at least 95%, at least 96%, at least 97%, at least 98%, or at least 99% identical to the amino acid sequence as set forth in SEQ ID NO:214;
14) a heavy chain that comprises an amino acid sequence as set forth in SEQ ID NO:215, or an amino acid sequence that is at least 85%, at least 90%, at least 95%, at least 96%, at least 97%, at least 98%, or at least 99% identical to the amino acid sequence as set forth in SEQ ID NO:215;
15) a heavy chain that comprises an amino acid sequence as set forth in SEQ ID NO:216, or an amino acid sequence that is at least 85%, at least 90%, at least 95%, at least 96%, at least 97%, at least 98%, or at least 99% identical to the amino acid sequence as set forth in SEQ ID NO:216;
16) a heavy chain that comprises an amino acid sequence as set forth in SEQ ID NO:217, or an amino acid sequence that is at least 85%, at least 90%, at least 95%, at least 96%, at least 97%, at least 98%, or at least 99% identical to the amino acid sequence as set forth in SEQ ID NO:217;
17) a heavy chain that comprises an amino acid sequence as set forth in SEQ ID NO:218, or an amino acid sequence that is at least 85%, at least 90%, at least 95%, at least 96%, at least 97%, at least 98%, or at least 99% identical to the amino acid sequence as set forth in SEQ ID NO:218; and
18) a heavy chain that comprises an amino acid sequence as set forth in SEQ ID NO:219, or an amino acid sequence that is at least 85%, at least 90%, at least 95%, at least 96%, at least 97%, at least 98%, or at least 99% identical to the amino acid sequence as set forth in SEQ ID NO:219.

In some embodiments, the isolated antibody or the antigen binding fragment thereof comprises a light chain selected from at least one of the following groups:
1) a light chain that comprises an amino acid sequence as set forth in SEQ ID NO: 184, or an amino acid sequence that is at least 85%, at least 90%, at least 95%, at least 96%, at least 97%, at least 98%, or at least 99% identical to the amino acid sequence as set forth in SEQ ID NO: 184;
2) a light chain that comprises an amino acid sequence as set forth in SEQ ID NO:185, or an amino acid sequence that is at least 85%, at least 90%, at least 95%, at least 96%, at least 97%, at least 98%, or at least 99% identical to the amino acid sequence as set forth in SEQ ID NO: 185;
3) a light chain that comprises an amino acid sequence as set forth in SEQ ID NO:186, or an amino acid sequence that is at least 85%, at least 90%, at least 95%, at least 96%, at least 97%, at least 98%, or at least 99% identical to the amino acid sequence as set forth in SEQ ID NO: 186;
4) a light chain that comprises an amino acid sequence as set forth in SEQ ID NO:187, or an amino acid sequence that is at least 85%, at least 90%, at least 95%, at least 96%, at least 97%, at least 98%, or at least 99% identical to the amino acid sequence as set forth in SEQ ID NO: 187;
5) a light chain that comprises an amino acid sequence as set forth in SEQ ID NO:188, or an amino acid sequence that is at least 85%, at least 90%, at least 95%, at least 96%, at least 97%, at least 98%, or at least 99% identical to the amino acid sequence as set forth in SEQ ID NO: 188;
6) a light chain that comprises an amino acid sequence as set forth in SEQ ID NO:189, or an amino acid sequence that is at least 85%, at least 90%, at least 95%, at least 96%, at least 97%, at least 98%, or at least 99% identical to the amino acid sequence as set forth in SEQ ID NO: 189;
7) a light chain that comprises an amino acid sequence as set forth in SEQ ID NO: 190, or an amino acid sequence that is at least 85%, at least 90%, at least 95%, at least 96%, at least 97%, at least 98%, or at least 99% identical to the amino acid sequence as set forth in SEQ ID NO:190;
8) a light chain that comprises an amino acid sequence as set forth in SEQ ID NO:191, or an amino acid sequence that is at least 85%, at least 90%, at least 95%, at least 96%, at least 97%, at least 98%, or at least 99% identical to the amino acid sequence as set forth in SEQ ID NO:191;
9) a light chain that comprises an amino acid sequence as set forth in SEQ ID NO:192, or an amino acid sequence that is at least 85%, at least 90%, at least 95%, at least 96%, at least 97%, at least 98%, or at least 99% identical to the amino acid sequence as set forth in SEQ ID NO:192;
10) a light chain that comprises an amino acid sequence as set forth in SEQ ID NO: 193, or an amino acid sequence that is at least 85%, at least 90%, at least 95%, at least 96%, at least 97%, at least 98%, or at least 99% identical to the amino acid sequence as set forth in SEQ ID NO: 193;
11) a light chain that comprises an amino acid sequence as set forth in SEQ ID NO:194, or an amino acid sequence that is at least 85%, at least 90%, at least 95%, at least 96%, at least 97%, at least 98%, or at least 99% identical to the amino acid sequence as set forth in SEQ ID NO: 194;
12) a light chain that comprises an amino acid sequence as set forth in SEQ ID NO:195, or an amino acid sequence that is at least 85%, at least 90%, at least 95%, at least 96%, at least 97%, at least 98%, or at least 99% identical to the amino acid sequence as set forth in SEQ ID NO: 195;
13) a light chain that comprises an amino acid sequence as set forth in SEQ ID NO:196, or an amino acid sequence that is at least 85%, at least 90%, at least 95%, at least 96%, at least 97%, at least 98%, or at least 99% identical to the amino acid sequence as set forth in SEQ ID NO: 196;
14) a light chain that comprises an amino acid sequence as set forth in SEQ ID NO:197, or an amino acid sequence that is at least 85%, at least 90%, at least 95%, at least 96%, at least 97%, at least 98%, or at least 99% identical to the amino acid sequence as set forth in SEQ ID NO:197;
15) a light chain that comprises an amino acid sequence as set forth in SEQ ID NO:198, or an amino acid sequence that is at least 85%, at least 90%, at least 95%, at least 96%, at least 97%, at least 98%, or at least 99% identical to the amino acid sequence as set forth in SEQ ID NO: 198;
16) a light chain that comprises an amino acid sequence as set forth in SEQ ID NO:199, or an amino acid sequence that is at least 85%, at least 90%, at least 95%, at least 96%, at least 97%, at least 98%, or at least 99% identical to the amino acid sequence as set forth in SEQ ID NO:199;
17) a light chain that comprises an amino acid sequence as set forth in SEQ ID NO:200, or an amino acid sequence that is at least 85%, at least 90%, at least 95%, at least 96%, at least 97%, at least 98%, or at least 99% identical to the amino acid sequence as set forth in SEQ ID NO:200; and
18) a light chain that comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201.

In some embodiments, the isolated antibody or the antigen binding fragment thereof comprises a heavy chain and a light chain selected from at least one of the following groups:
1) a heavy chain that comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202, and a light chain that comprises an amino acid sequence as set forth in SEQ ID NO:184, or an amino acid sequence that is at least 85%, at least 90%, at least 95%, at least 96%, at least 97%, at least 98%, or at least 99% identical to the amino acid sequence as set forth in SEQ ID NO: 184;
2) a heavy chain that comprises an amino acid sequence as set forth in SEQ ID NO:203, or an amino acid sequence that is at least 85%, at least 90%, at least 95%, at least 96%, at least 97%, at least 98%, or at least 99% identical to the amino acid sequence as set forth in SEQ ID NO:203, and a light chain that comprises an amino acid sequence as set forth in SEQ ID NO:185, or an amino acid sequence that is at least 85%, at least 90%, at least 95%, at least 96%, at least 97%, at least 98%, or at least 99% identical to the amino acid sequence as set forth in SEQ ID NO: 185;
3) a heavy chain that comprises an amino acid sequence as set forth in SEQ ID NO:204, or an amino acid sequence that is at least 85%, at least 90%, at least 95%, at least 96%, at least 97%, at least 98%, or at least 99% identical to the amino acid sequence as set forth in SEQ ID NO:204, and a light chain that comprises an amino acid sequence as set forth in SEQ ID NO:186, or an amino acid sequence that is at least 85%, at least 90%, at least 95%, at least 96%, at least 97%, at least 98%, or at least 99% identical to the amino acid sequence as set forth in SEQ ID NO: 186;
4) a heavy chain that comprises an amino acid sequence as set forth in SEQ ID NO:205, or an amino acid sequence that is at least 85%, at least 90%, at least 95%, at least 96%, at least 97%, at least 98%, or at least 99% identical to the amino acid sequence as set forth in SEQ ID NO:205, and a light chain that comprises an amino acid sequence as set forth in SEQ ID NO:187, or an amino acid sequence that is at least 85%, at least 90%, at least 95%, at least 96%, at least 97%, at least 98%, or at least 99% identical to the amino acid sequence as set forth in SEQ ID NO: 187;
5) a heavy chain that comprises an amino acid sequence as set forth in SEQ ID NO:206, or an amino acid sequence that is at least 85%, at least 90%, at least 95%, at least 96%, at least 97%, at least 98%, or at least 99% identical to the amino acid sequence as set forth in SEQ ID NO:206, and a light chain that comprises an amino acid sequence as set forth in SEQ ID NO:188, or an amino acid sequence that is at least 85%, at least 90%, at least 95%, at least 96%, at least 97%, at least 98%, or at least 99% identical to the amino acid sequence as set forth in SEQ ID NO: 188;
6) a heavy chain that comprises an amino acid sequence as set forth in SEQ ID NO:207, or an amino acid sequence that is at least 85%, at least 90%, at least 95%, at least 96%, at least 97%, at least 98%, or at least 99% identical to the amino acid sequence as set forth in SEQ ID NO:207, and a light chain that comprises an amino acid sequence as set forth in SEQ ID NO:189, or an amino acid sequence that is at least 85%, at least 90%, at least 95%, at least 96%, at least 97%, at least 98%, or at least 99% identical to the amino acid sequence as set forth in SEQ ID NO: 189;
7) a heavy chain that comprises an amino acid sequence as set forth in SEQ ID NO:208, or an amino acid sequence that is at least 85%, at least 90%, at least 95%, at least 96%, at least 97%, at least 98%, or at least 99% identical to the amino acid sequence as set forth in SEQ ID NO:208, and a light chain that comprises an amino acid sequence as set forth in SEQ ID NO:190, or an amino acid sequence that is at least 85%, at least 90%, at least 95%, at least 96%, at least 97%, at least 98%, or at least 99% identical to the amino acid sequence as set forth in SEQ ID NO:190;
8) a heavy chain that comprises an amino acid sequence as set forth in SEQ ID NO:209, or an amino acid sequence that is at least 85%, at least 90%, at least 95%, at least 96%, at least 97%, at least 98%, or at least 99% identical to the amino acid sequence as set forth in SEQ ID NO:209, and a light chain that comprises an amino acid sequence as set forth in SEQ ID NO:191, or an amino acid sequence that is at least 85%, at least 90%, at least 95%, at least 96%, at least 97%, at least 98%, or at least 99% identical to the amino acid sequence as set forth in SEQ ID NO:191;
9) a heavy chain that comprises an amino acid sequence as set forth in SEQ ID NO:210, or an amino acid sequence that is at least 85%, at least 90%, at least 95%, at least 96%, at least 97%, at least 98%, or at least 99% identical to the amino acid sequence as set forth in SEQ ID NO:210, and a light chain that comprises an amino acid sequence as set forth in SEQ ID NO:192, or an amino acid sequence that is at least 85%, at least 90%, at least 95%, at least 96%, at least 97%, at least 98%, or at least 99% identical to the amino acid sequence as set forth in SEQ ID NO:192;
10) a heavy chain that comprises an amino acid sequence as set forth in SEQ ID NO:211, or an amino acid sequence that is at least 85%, at least 90%, at least 95%, at least 96%, at least 97%, at least 98%, or at least 99% identical to the amino acid sequence as set forth in SEQ ID NO:211, and a light chain that comprises an amino acid sequence as set forth in SEQ ID NO:193, or an amino acid sequence that is at least 85%, at least 90%, at least 95%, at least 96%, at least 97%, at least 98%, or at least 99% identical to the amino acid sequence as set forth in SEQ ID NO: 193;
11) a heavy chain that comprises an amino acid sequence as set forth in SEQ ID NO:212, or an amino acid sequence that is at least 85%, at least 90%, at least 95%, at least 96%, at least 97%, at least 98%, or at least 99% identical to the amino acid sequence as set forth in SEQ ID NO:212, and a light chain that comprises an amino acid sequence as set forth in SEQ ID NO:194, or an amino acid sequence that is at least 85%, at least 90%, at least 95%, at least 96%, at least 97%, at least 98%, or at least 99% identical to the amino acid sequence as set forth in SEQ ID NO:194;
12) a heavy chain that comprises an amino acid sequence as set forth in SEQ ID NO:213, or an amino acid sequence that is at least 85%, at least 90%, at least 95%, at least 96%, at least 97%, at least 98%, or at least 99% identical to the amino acid sequence as set forth in SEQ ID NO:213, and a light chain that comprises an amino acid sequence as set forth in SEQ ID NO:195, or an amino acid sequence that is at least 85%, at least 90%, at least 95%, at least 96%, at least 97%, at least 98%, or at least 99% identical to the amino acid sequence as set forth in SEQ ID NO:195;
13) a heavy chain that comprises an amino acid sequence as set forth in SEQ ID NO:214, or an amino acid sequence that is at least 85%, at least 90%, at least 95%, at least 96%, at least 97%, at least 98%, or at least 99% identical to the amino acid sequence as set forth in SEQ ID NO:214, and a light chain that comprises an amino acid sequence as set forth in SEQ ID NO:196, or an amino acid sequence that is at least 85%, at least 90%, at least 95%, at least 96%, at least 97%, at least 98%, or at least 99% identical to the amino acid sequence as set forth in SEQ ID NO:196;
14) a heavy chain that comprises an amino acid sequence as set forth in SEQ ID NO:215, or an amino acid sequence that is at least 85%, at least 90%, at least 95%, at least 96%, at least 97%, at least 98%, or at least 99% identical to the amino acid sequence as set forth in SEQ ID NO:215, and a light chain that comprises an amino acid sequence as set forth in SEQ ID NO:197, or an amino acid sequence that is at least 85%, at least 90%, at least 95%, at least 96%, at least 97%, at least 98%, or at least 99% identical to the amino acid sequence as set forth in SEQ ID NO:197;
15) a heavy chain that comprises an amino acid sequence as set forth in SEQ ID NO:216, or an amino acid sequence that is at least 85%, at least 90%, at least 95%, at least 96%, at least 97%, at least 98%, or at least 99% identical to the amino acid sequence as set forth in SEQ ID NO:216, and a light chain that comprises an amino acid sequence as set forth in SEQ ID NO:198, or an amino acid sequence that is at least 85%, at least 90%, at least 95%, at least 96%, at least 97%, at least 98%, or at least 99% identical to the amino acid sequence as set forth in SEQ ID NO: 198;
16) a heavy chain that comprises an amino acid sequence as set forth in SEQ ID NO:217, or an amino acid sequence that is at least 85%, at least 90%, at least 95%, at least 96%, at least 97%, at least 98%, or at least 99% identical to the amino acid sequence as set forth in SEQ ID NO:217, and a light chain that comprises an amino acid sequence as set forth in SEQ ID NO:199, or an amino acid sequence that is at least 85%, at least 90%, at least 95%, at least 96%, at least 97%, at least 98%, or at least 99% identical to the amino acid sequence as set forth in SEQ ID NO:199;
17) a heavy chain that comprises an amino acid sequence as set forth in SEQ ID NO:218, or an amino acid sequence that is at least 85%, at least 90%, at least 95%, at least 96%, at least 97%, at least 98%, or at least 99% identical to the amino acid sequence as set forth in SEQ ID NO:218, and a light chain that comprises an amino acid sequence as set forth in SEQ ID NO:200, or an amino acid sequence that is at least 85%, at least 90%, at least 95%, at least 96%, at least 97%, at least 98%, or at least 99% identical to the amino acid sequence as set forth in SEQ ID NO:200; and
18) a heavy chain that comprises an amino acid sequence as set forth in SEQ ID NO:219, or an amino acid sequence that is at least 85%, at least 90%, at least 95%, at least 96%, at least 97%, at least 98%, or at least 99% identical to the amino acid sequence as set forth in SEQ ID NO:219, and a light chain that comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201.

In some embodiments, an antibody that is at least 85%, at least 90%, at least 95%, at least 96%, at least 97%, at least 98%, or at least 99% identical to the amino acid sequences of the heavy chain (SEQ ID NOs: 202-219) and/or amino acid sequences of the light chain (SEQ ID Nos: 184-201) of the antibody according to the present invention has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the heavy chain (SEQ ID NOs: 202-219) and/or amino acid sequences of the light chain (SEQ ID Nos: 184-201) of the antibody according to the present invention may also specifically bind to the ITGA2 protein. In some embodiments, an antibody that is at least 85%, at least 90%, at least 95%, at least 96%, at least 97%, at least 98%, or at least 99% identical to the amino acid sequences of the heavy chain (SEQ ID NOs: 202-219) and/or amino acid sequences of the light chain (SEQ ID NOs: 184-201) of the antibody according to the present invention has a same heavy chain and/or light chain variable region as the antibody according to the present invention, and has a conserved amino acid substitution only at locations (such as a heavy chain and/or light chain constant region) of the heavy chain and/or light chain other than the heavy chain and/or light chain variable region, wherein the conserved amino acid substitution does not change the biological function and/or activity of the corresponding antibody.

Optionally, the antibody that is at least 85%, at least 90%, at least 95%, at least 96%, at least 97%, at least 98%, or at least 99% identical to the amino acid sequences of the heavy chain (SEQ ID NOs: 202-219) and/or amino acid sequences of the light chain (SEQ ID Nos: 184-201) of the antibody according to the present invention may also specifically bind to the ITGA2 protein.

In some embodiments, an antibody that is at least 85%, at least 90%, at least 95%, at least 96%, at least 97%, at least 98%, or at least 99% identical to the amino acid sequences of the heavy chain (SEQ ID NOs: 202-219) and/or amino acid sequences of the light chain (SEQ ID NOs: 184-201) of the antibody according to the present invention has a same heavy chain and/or light chain variable region as the antibody according to the present invention, and has a conserved amino acid substitution only at constant regions of the heavy chain and/or light chain, wherein the conserved amino acid substitution does not change the biological function and/or activity of the corresponding antibody, and the antibody may also specifically bind to the ITGA2 protein.

In some embodiments, the isolated antibody or the antigen binding fragment thereof consists of a heavy chain and a light chain selected from at least one of the following groups:
1) a heavy chain that consists of an amino acid sequence as set forth in SEQ ID NO:202, and a light chain that consists of an amino acid sequence as set forth in SEQ ID NO: 184;
2) a heavy chain that consists of an amino acid sequence as set forth in SEQ ID NO:203, and a light chain that consists of an amino acid sequence as set forth in SEQ ID NO: 185;
3) a heavy chain that consists of an amino acid sequence as set forth in SEQ ID NO:204, and a light chain that consists of an amino acid sequence as set forth in SEQ ID NO: 186;
4) a heavy chain that consists of an amino acid sequence as set forth in SEQ ID NO:205, and a light chain that consists of an amino acid sequence as set forth in SEQ ID NO: 187;
5) a heavy chain that consists of an amino acid sequence as set forth in SEQ ID NO:206, and a light chain that consists of an amino acid sequence as set forth in SEQ ID NO: 188;
6) a heavy chain that consists of an amino acid sequence as set forth in SEQ ID NO:207, and a light chain that consists of an amino acid sequence as set forth in SEQ ID NO: 189;
7) a heavy chain that consists of an amino acid sequence as set forth in SEQ ID NO:208, and a light chain that consists of an amino acid sequence as set forth in SEQ ID NO:190;
8) a heavy chain that consists of an amino acid sequence as set forth in SEQ ID NO:209, and a light chain that consists of an amino acid sequence as set forth in SEQ ID NO:191;
9) a heavy chain that consists of an amino acid sequence as set forth in SEQ ID NO:210, and a light chain that consists of an amino acid sequence as set forth in SEQ ID NO:192;
10) a heavy chain that consists of an amino acid sequence as set forth in SEQ ID NO:211, and a light chain that consists of an amino acid sequence as set forth in SEQ ID NO: 193;
11) a heavy chain that consists of an amino acid sequence as set forth in SEQ ID NO:212, and a light chain that consists of an amino acid sequence as set forth in SEQ ID NO: 194;
12) a heavy chain that consists of an amino acid sequence as set forth in SEQ ID NO:213, and a light chain that consists of an amino acid sequence as set forth in SEQ ID NO:195;
13) a heavy chain that consists of an amino acid sequence as set forth in SEQ ID NO:214, and a light chain that consists of an amino acid sequence as set forth in SEQ ID NO:196;
14) a heavy chain that consists of an amino acid sequence as set forth in SEQ ID NO:215, and a light chain that consists of an amino acid sequence as set forth in SEQ ID NO:197;
15) a heavy chain that consists of an amino acid sequence as set forth in SEQ ID NO:216, and a light chain that consists of an amino acid sequence as set forth in SEQ ID NO:198;
16) a heavy chain that consists of an amino acid sequence as set forth in SEQ ID NO:217, and a light chain that consists of an amino acid sequence as set forth in SEQ ID NO:199;
17) a heavy chain that consists of an amino acid sequence as set forth in SEQ ID NO:218, and a light chain that consists of an amino acid sequence as set forth in SEQ ID NO:200; and
18) a heavy chain that consists of an amino acid sequence as set forth in SEQ ID NO:219, and a light chain that consists of an amino acid sequence as set forth in SEQ ID NO:201.

The amino acid sequence numberings (SEQ ID NO:) of the antibody according to the present invention and the corresponding structure thereof are summarized in Table 2 below:

| Antibody | Light Chain | Light Chain Sequence | VL CDR1-3 | Light Chain Constant Region | Light Chain Variable Region | Heavy Chain | Heavy Chain Sequence | VH CDR1 -3 | Heavy Chain Constant Region | Heavy Chain Variable Region |
|---|---|---|---|---|---|---|---|---|---|---|
| FS002-A1 | FS002-A1-LC (Kappa) | 184 | 38-40 | 109 | 37 | FS002-Al-HC (IgG1) | 202 | 112-1 14 | 183 | 111 |
| FS002-A5 | FS002-A5-LC (Kappa) | 185 | 42-44 | 109 | 41 | FS002-A5-HC (IgG1) | 203 | 116-1 18 | 183 | 115 |
| FS002-A15 | FS002-A15-L C (Kappa) | 186 | 46-48 | 109 | 45 | FS002-A15-H C (IgG1) | 204 | 120-1 22 | 183 | 119 |
| FS002-A36 | FS002-A36-L C (Kappa) | 187 | 50-52 | 109 | 49 | FS002-A36-H C (IgG1) | 205 | 124-1 26 | 183 | 123 |
| FS002-A66 | FS002-A66-L C (Kappa) | 188 | 54-56 | 109 | 53 | FS002-A66-H C (IgG1) | 206 | 128-1 30 | 183 | 127 |
| FS002-A10 | FS002-A10-L C (Kappa-2) | 189 | 58-60 | 109 | 57 | FS002-A10-H C (IgG1) | 207 | 132-1 34 | 183 | 131 |
| FS002-C9 | FS002-C9-LC (Kappa-2) | 190 | 62-64 | 109 | 61 | FS002-C9-HC (IgG1) | 208 | 136-1 38 | 183 | 135 |
| FS002-C11 | FS002-C11-LC (Kappa-2) | 191 | 66-68 | 109 | 65 | FS002-C11-H C (IgG1) | 209 | 140-1 42 | 183 | 139 |
| FS002-C216 | FS002-C216-L C (Kappa-2) | 192 | 70-72 | 109 | 69 | FS002-C216-H C (IgG1) | 210 | 144-1 46 | 183 | 143 |
| FS002-C375 | FS002-C375-L C (Kappa-2) | 193 | 74-76 | 109 | 73 | FS002-C375-H C (IgG1) | 211 | 148-1 50 | 183 | 147 |
| FS002-C417 | FS002-C417-L C (Kappa-2) | 194 | 78-80 | 109 | 77 | FS002-C417-H C (IgG1) | 212 | 152-1 54 | 183 | 151 |
| FS002-B27 | FS002-B27-LC (Kappa-2) | 195 | 82-84 | 109 | 81 | FS002-B27-H C (IgG1) | 213 | 156-1 58 | 183 | 155 |
| FS002-C330 | FS002-C330-L C (Kappa-2) | 196 | 86-88 | 109 | 85 | FS002-C330-H C (IgG1) | 214 | 160-1 62 | 183 | 159 |
| FS002-C153 | FS002-C153-L C(Lam bdaC2)-2 | 197 | 90-92 | 110 | 89 | FS002-C153-H C (IgG1) | 215 | 164-1 66 | 183 | 163 |
| FS002-C241 | FS002-C241-L C(Lam bdaC2)-2 | 198 | 94-96 | 110 | 93 | FS002-C241-H C (IgG1) | 216 | 168-1 70 | 183 | 167 |
| FS002-C15 | FS002-C15-LC (Kappa-2) | 199 | 98-100 | 109 | 97 | FS002-C15-H C (IgG1) | 217 | 172-1 74 | 183 | 171 |
| FS002-C58 | FS002-C58-LC (Kappa-2) | 200 | 102-104 | 109 | 101 | FS002-C58-H C (IgG1) | 218 | 176-1 78 | 183 | 175 |
| FS002-A72 | FS002-A72-L C (Kappa-2) | 201 | 106-108 | 109 | 105 | FS002-A72-H C (IgG1) | 219 | 180-1 82 | 183 | 179 |

In some embodiments, the isolated antibody or the antigen binding fragment thereof consists of a heavy chain selected from at least one of the following groups:
1) a heavy chain that comprises an amino acid sequence encoded by a nucleotide sequence as set forth in SEQ ID NO: 19, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO: 19, or consists of an amino acid sequence encoded by the nucleotide sequence as set forth in SEQ ID NO: 19;
2) a heavy chain that comprises an amino acid sequence encoded by a nucleotide sequence as set forth in SEQ ID NO:20, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:20, or consists of an amino acid sequence encoded by the nucleotide sequence as set forth in SEQ ID NO:20;
3) a heavy chain that comprises an amino acid sequence encoded by a nucleotide sequence as set forth in SEQ ID NO:21, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:21, or consists of an amino acid sequence encoded by the nucleotide sequence as set forth in SEQ ID NO:21;
4) a heavy chain that comprises an amino acid sequence encoded by a nucleotide sequence as set forth in SEQ ID NO:22, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:22, or consists of an amino acid sequence encoded by the nucleotide sequence as set forth in SEQ ID NO:22;
5) a heavy chain that comprises an amino acid sequence encoded by a nucleotide sequence as set forth in SEQ ID NO:23, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:23, or consists of an amino acid sequence encoded by the nucleotide sequence as set forth in SEQ ID NO:23;
6) a heavy chain that comprises an amino acid sequence encoded by a nucleotide sequence as set forth in SEQ ID NO:24, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:24, or consists of an amino acid sequence encoded by the nucleotide sequence as set forth in SEQ ID NO:24;
7) a heavy chain that comprises an amino acid sequence encoded by a nucleotide sequence as set forth in SEQ ID NO:25, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:25, or consists of an amino acid sequence encoded by the nucleotide sequence as set forth in SEQ ID NO:25;
8) a heavy chain that comprises an amino acid sequence encoded by a nucleotide sequence as set forth in SEQ ID NO:26, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:26, or consists of an amino acid sequence encoded by the nucleotide sequence as set forth in SEQ ID NO:26;
9) a heavy chain that comprises an amino acid sequence encoded by a nucleotide sequence as set forth in SEQ ID NO:27, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:27, or consists of an amino acid sequence encoded by the nucleotide sequence as set forth in SEQ ID NO:27;
10) a heavy chain that comprises an amino acid sequence encoded by a nucleotide sequence as set forth in SEQ ID NO:28, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:28, or consists of an amino acid sequence encoded by the nucleotide sequence as set forth in SEQ ID NO:28;
11) a heavy chain that comprises an amino acid sequence encoded by a nucleotide sequence as set forth in SEQ ID NO:29, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:29, or consists of an amino acid sequence encoded by the nucleotide sequence as set forth in SEQ ID NO:29;
12) a heavy chain that comprises an amino acid sequence encoded by a nucleotide sequence as set forth in SEQ ID NO:30, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:30, or consists of an amino acid sequence encoded by the nucleotide sequence as set forth in SEQ ID NO:30;
13) a heavy chain that comprises an amino acid sequence encoded by a nucleotide sequence as set forth in SEQ ID NO:31, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:31, or consists of an amino acid sequence encoded by the nucleotide sequence as set forth in SEQ ID NO:31;
14) a heavy chain that comprises an amino acid sequence encoded by a nucleotide sequence as set forth in SEQ ID NO:32, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:32, or consists of an amino acid sequence encoded by the nucleotide sequence as set forth in SEQ ID NO:32;
15) a heavy chain that comprises an amino acid sequence encoded by a nucleotide sequence as set forth in SEQ ID NO:33, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:33, or consists of an amino acid sequence encoded by the nucleotide sequence as set forth in SEQ ID NO:33;
16) a heavy chain that comprises an amino acid sequence encoded by a nucleotide sequence as set forth in SEQ ID NO:34, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:34, or consists of an amino acid sequence encoded by the nucleotide sequence as set forth in SEQ ID NO:34;
17) a heavy chain that comprises an amino acid sequence encoded by a nucleotide sequence as set forth in SEQ ID NO:35, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:35, or consists of an amino acid sequence encoded by the nucleotide sequence as set forth in SEQ ID NO:35; and
18) a heavy chain that comprises an amino acid sequence encoded by a nucleotide sequence as set forth in SEQ ID NO:36, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:36, or consists of an amino acid sequence encoded by the nucleotide sequence as set forth in SEQ ID NO:36.

In some embodiments, the isolated antibody or the antigen binding fragment thereof consists of a light chain selected from at least one of the following groups:
1) a light chain that comprises an amino acid sequence encoded by a nucleotide sequence as set forth in SEQ ID NO: 1, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO: 1, or consists of an amino acid sequence encoded by the nucleotide sequence as set forth in SEQ ID NO: 1;
2) a light chain that comprises an amino acid sequence encoded by a nucleotide sequence as set forth in SEQ ID NO:2, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:2, or consists of an amino acid sequence encoded by the nucleotide sequence as set forth in SEQ ID NO:2;
3) a light chain that comprises an amino acid sequence encoded by a nucleotide sequence as set forth in SEQ ID NO:3, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:3, or consists of an amino acid sequence encoded by the nucleotide sequence as set forth in SEQ ID NO:3;
4) a light chain that comprises an amino acid sequence encoded by a nucleotide sequence as set forth in SEQ ID NO:4, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:4, or consists of an amino acid sequence encoded by the nucleotide sequence as set forth in SEQ ID NO:4;
5) a light chain that comprises an amino acid sequence encoded by a nucleotide sequence as set forth in SEQ ID NO:5, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:5, or consists of an amino acid sequence encoded by the nucleotide sequence as set forth in SEQ ID NO:5;
6) a light chain that comprises an amino acid sequence encoded by a nucleotide sequence as set forth in SEQ ID NO:6, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:6, or consists of an amino acid sequence encoded by the nucleotide sequence as set forth in SEQ ID NO:6;
7) a light chain that comprises an amino acid sequence encoded by a nucleotide sequence as set forth in SEQ ID NO:7, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:7, or consists of an amino acid sequence encoded by the nucleotide sequence as set forth in SEQ ID NO:7;
8) a light chain that comprises an amino acid sequence encoded by a nucleotide sequence as set forth in SEQ ID NO:8, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:8, or consists of an amino acid sequence encoded by the nucleotide sequence as set forth in SEQ ID NO:8;
9) a light chain that comprises an amino acid sequence encoded by a nucleotide sequence as set forth in SEQ ID NO:9, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:9, or consists of an amino acid sequence encoded by the nucleotide sequence as set forth in SEQ ID NO:9;
10) a light chain that comprises an amino acid sequence encoded by a nucleotide sequence as set forth in SEQ ID NO: 10, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:10, or consists of an amino acid sequence encoded by the nucleotide sequence as set forth in SEQ ID NO:10;
11) a light chain that comprises an amino acid sequence encoded by a nucleotide sequence as set forth in SEQ ID NO: 11, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:11, or consists of an amino acid sequence encoded by the nucleotide sequence as set forth in SEQ ID NO: 11;
12) a light chain that comprises an amino acid sequence encoded by a nucleotide sequence as set forth in SEQ ID NO: 12, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:12, or consists of an amino acid sequence encoded by the nucleotide sequence as set forth in SEQ ID NO:12;
13) a light chain that comprises an amino acid sequence encoded by a nucleotide sequence as set forth in SEQ ID NO: 13, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:13, or consists of an amino acid sequence encoded by the nucleotide sequence as set forth in SEQ ID NO:13;
14) a light chain that comprises an amino acid sequence encoded by a nucleotide sequence as set forth in SEQ ID NO: 14, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO: 14, or consists of an amino acid sequence encoded by the nucleotide sequence as set forth in SEQ ID NO:14;
15) a light chain that comprises an amino acid sequence encoded by a nucleotide sequence as set forth in SEQ ID NO: 15, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:15, or consists of an amino acid sequence encoded by the nucleotide sequence as set forth in SEQ ID NO:15;
16) a light chain that comprises an amino acid sequence encoded by a nucleotide sequence as set forth in SEQ ID NO: 16, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:16, or consists of an amino acid sequence encoded by the nucleotide sequence as set forth in SEQ ID NO:16;
17) a light chain that comprises an amino acid sequence encoded by a nucleotide sequence as set forth in SEQ ID NO: 17, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO: 17, or consists of an amino acid sequence encoded by the nucleotide sequence as set forth in SEQ ID NO:17; and
18) a light chain that comprises an amino acid sequence encoded by a nucleotide sequence as set forth in SEQ ID NO: 18, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:18, or consists of an amino acid sequence encoded by the nucleotide sequence as set forth in SEQ ID NO: 18.

In some embodiments, the isolated antibody or the antigen binding fragment thereof consists of a heavy chain and a light chain selected from at least one of the following groups:
1) a heavy chain that comprises an amino acid sequence encoded by a nucleotide sequence as set forth in SEQ ID NO: 19, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:19, or consists of an amino acid sequence encoded by the nucleotide sequence as set forth in SEQ ID NO:19, and
   a light chain that comprises an amino acid sequence encoded by a nucleotide sequence as set forth in SEQ ID NO:1, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:1, or consists of an amino acid sequence encoded by the nucleotide sequence as set forth in SEQ ID NO:1;
2) a heavy chain that comprises an amino acid sequence encoded by a nucleotide sequence as set forth in SEQ ID NO:20, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:20, or consists of an amino acid sequence encoded by the nucleotide sequence as set forth in SEQ ID NO:20, and
   a light chain that comprises an amino acid sequence encoded by a nucleotide sequence as set forth in SEQ ID NO:2, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:2, or consists of an amino acid sequence encoded by the nucleotide sequence as set forth in SEQ ID NO:2;
3) a heavy chain that comprises an amino acid sequence encoded by a nucleotide sequence as set forth in SEQ ID NO:21, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:21, or consists of an amino acid sequence encoded by the nucleotide sequence as set forth in SEQ ID NO:21, and
   a light chain that comprises an amino acid sequence encoded by a nucleotide sequence as set forth in SEQ ID NO:3, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:3, or consists of an amino acid sequence encoded by the nucleotide sequence as set forth in SEQ ID NO:3;
4) a heavy chain that comprises an amino acid sequence encoded by a nucleotide sequence as set forth in SEQ ID NO:22, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:22, or consists of an amino acid sequence encoded by the nucleotide sequence as set forth in SEQ ID NO:22, and
   a light chain that comprises an amino acid sequence encoded by a nucleotide sequence as set forth in SEQ ID NO:4, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:4, or consists of an amino acid sequence encoded by the nucleotide sequence as set forth in SEQ ID NO:4;
5) a heavy chain that comprises an amino acid sequence encoded by a nucleotide sequence as set forth in SEQ ID NO:23, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:23, or consists of an amino acid sequence encoded by the nucleotide sequence as set forth in SEQ ID NO:23, and
   a light chain that comprises an amino acid sequence encoded by a nucleotide sequence as set forth in SEQ ID NO:5, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:5, or consists of an amino acid sequence encoded by the nucleotide sequence as set forth in SEQ ID NO:5;
6) a heavy chain that comprises an amino acid sequence encoded by a nucleotide sequence as set forth in SEQ ID NO:24, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:24, or consists of an amino acid sequence encoded by the nucleotide sequence as set forth in SEQ ID NO:24, and
   a light chain that comprises an amino acid sequence encoded by a nucleotide sequence as set forth in SEQ ID NO:6, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:6, or consists of an amino acid sequence encoded by the nucleotide sequence as set forth in SEQ ID NO:6;
7) a heavy chain that comprises an amino acid sequence encoded by a nucleotide sequence as set forth in SEQ ID NO:25, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:25, or consists of an amino acid sequence encoded by the nucleotide sequence as set forth in SEQ ID NO:25, and
   a light chain that comprises an amino acid sequence encoded by a nucleotide sequence as set forth in SEQ ID NO:7, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:7, or consists of an amino acid sequence encoded by the nucleotide sequence as set forth in SEQ ID NO:7;
8) a heavy chain that comprises an amino acid sequence encoded by a nucleotide sequence as set forth in SEQ ID NO:26, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:26, or consists of an amino acid sequence encoded by the nucleotide sequence as set forth in SEQ ID NO:26, and
   a light chain that comprises an amino acid sequence encoded by a nucleotide sequence as set forth in SEQ ID NO:8, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:8, or consists of an amino acid sequence encoded by the nucleotide sequence as set forth in SEQ ID NO:8;
9) a heavy chain that comprises an amino acid sequence encoded by a nucleotide sequence as set forth in SEQ ID NO:27, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:27, or consists of an amino acid sequence encoded by the nucleotide sequence as set forth in SEQ ID NO:27, and
   a light chain that comprises an amino acid sequence encoded by a nucleotide sequence as set forth in SEQ ID NO:9, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:9, or consists of an amino acid sequence encoded by the nucleotide sequence as set forth in SEQ ID NO:9;
10) a heavy chain that comprises an amino acid sequence encoded by a nucleotide sequence as set forth in SEQ ID NO:28, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:28, or consists of an amino acid sequence encoded by the nucleotide sequence as set forth in SEQ ID NO:28, and
   a light chain that comprises an amino acid sequence encoded by a nucleotide sequence as set forth in SEQ ID NO:10, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:10, or consists of an amino acid sequence encoded by the nucleotide sequence as set forth in SEQ ID NO:10;
11) a heavy chain that comprises an amino acid sequence encoded by a nucleotide sequence as set forth in SEQ ID NO:29, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:29, or consists of an amino acid sequence encoded by the nucleotide sequence as set forth in SEQ ID NO:29, and
   a light chain that comprises an amino acid sequence encoded by a nucleotide sequence as set forth in SEQ ID NO: 11, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:11, or consists of an amino acid sequence encoded by the nucleotide sequence as set forth in SEQ ID NO: 11;
12) a heavy chain that comprises an amino acid sequence encoded by a nucleotide sequence as set forth in SEQ ID NO:30, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:30, or consists of an amino acid sequence encoded by the nucleotide sequence as set forth in SEQ ID NO:30, and
   a light chain that comprises an amino acid sequence encoded by a nucleotide sequence as set forth in SEQ ID NO:12, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:12, or consists of an amino acid sequence encoded by the nucleotide sequence as set forth in SEQ ID NO:12;
13) a heavy chain that comprises an amino acid sequence encoded by a nucleotide sequence as set forth in SEQ ID NO:31, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:31, or consists of an amino acid sequence encoded by the nucleotide sequence as set forth in SEQ ID NO:31, and
   a light chain that comprises an amino acid sequence encoded by a nucleotide sequence as set forth in SEQ ID NO:13, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:13, or consists of an amino acid sequence encoded by the nucleotide sequence as set forth in SEQ ID NO:13;
14) a heavy chain that comprises an amino acid sequence encoded by a nucleotide sequence as set forth in SEQ ID NO:32, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:32, or consists of an amino acid sequence encoded by the nucleotide sequence as set forth in SEQ ID NO:32, and
   a light chain that comprises an amino acid sequence encoded by a nucleotide sequence as set forth in SEQ ID NO:14, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO: 14, or consists of an amino acid sequence encoded by the nucleotide sequence as set forth in SEQ ID NO: 14;
15) a heavy chain that comprises an amino acid sequence encoded by a nucleotide sequence as set forth in SEQ ID NO:33, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:33, or consists of an amino acid sequence encoded by the nucleotide sequence as set forth in SEQ ID NO:33, and
   a light chain that comprises an amino acid sequence encoded by a nucleotide sequence as set forth in SEQ ID NO:15, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:15, or consists of an amino acid sequence encoded by the nucleotide sequence as set forth in SEQ ID NO:15;
16) a heavy chain that comprises an amino acid sequence encoded by a nucleotide sequence as set forth in SEQ ID NO:34, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:34, or consists of an amino acid sequence encoded by the nucleotide sequence as set forth in SEQ ID NO:34, and
   a light chain that comprises an amino acid sequence encoded by a nucleotide sequence as set forth in SEQ ID NO:16, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:16, or consists of an amino acid sequence encoded by the nucleotide sequence as set forth in SEQ ID NO:16;
17) a heavy chain that comprises an amino acid sequence encoded by a nucleotide sequence as set forth in SEQ ID NO:35, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:35, or consists of an amino acid sequence encoded by the nucleotide sequence as set forth in SEQ ID NO:35, and
   a light chain that comprises an amino acid sequence encoded by a nucleotide sequence as set forth in SEQ ID NO:17, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:17, or consists of an amino acid sequence encoded by the nucleotide sequence as set forth in SEQ ID NO:17; and
18) a heavy chain that comprises an amino acid sequence encoded by a nucleotide sequence as set forth in SEQ ID NO:36, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:36, or consists of an amino acid sequence encoded by the nucleotide sequence as set forth in SEQ ID NO:36, and
   a light chain that comprises an amino acid sequence encoded by a nucleotide sequence as set forth in SEQ ID NO:18, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:18, or consists of an amino acid sequence encoded by the nucleotide sequence as set forth in SEQ ID NO:18.

In some embodiments, an antibody that is at least 85%, at least 90%, at least 95%, at least 96%, at least 97%, at least 98%, or at least 99% identical to the amino acid sequences of the heavy chain of the antibody according to the present invention that is encoded by the nucleotide sequences according to the present invention (SEQ ID NOs: 19-36) and/or to the amino acid sequences of the light chain of the antibody according to the present invention that is encoded by the nucleotide sequences according to the present invention (SEQ ID Nos: 1-18) has the same or similar biological functions and/or activity as the antibody according to the present invention.

In some embodiments, an antibody that is at least 85%, at least 90%, at least 95%, at least 96%, at least 97%, at least 98%, or at least 99% identical to the amino acid sequences of the heavy chain of the antibody according to the present invention that is encoded by the nucleotide sequences according to the present invention (SEQ ID NOs: 19-36) and/or to the amino acid sequences of the light chain of the antibody according to the present invention that is encoded by the nucleotide sequences according to the present invention (SEQ ID Nos: 1-18) may also specifically bind to the ITGA2 protein.

In some embodiments, an antibody that is at least 85%, at least 90%, at least 95%, at least 96%, at least 97%, at least 98%, or at least 99% identical to the amino acid sequences of the heavy chain of the antibody according to the present invention that is encoded by the nucleotide sequences according to the present invention (SEQ ID NOs: 19-36) and/or to the amino acid sequences of the light chain of the antibody according to the present invention that is encoded by the nucleotide sequences according to the present invention (SEQ ID Nos: 1-18) has a same heavy chain and/or light chain variable region as the antibody according to the present invention, and has a conserved amino acid substitution only at locations (such as a heavy chain and/or light chain constant region) of the heavy chain and/or light chain other than the heavy chain and/or light chain variable region, wherein the conserved amino acid substitution does not change the biological function and/or activity of the corresponding antibody.

Optionally, the antibody that is at least 85%, at least 90%, at least 95%, at least 96%, at least 97%, at least 98%, or at least 99% identical to the amino acid sequences of the heavy chain of the antibody according to the present invention that is encoded by the nucleotide sequences according to the present invention (SEQ ID NOs: 19-36) and/or to the amino acid sequences of the light chain of the antibody according to the present invention that is encoded by the nucleotide sequences according to the present invention (SEQ ID Nos: 1-18) may also specifically bind to the ITGA2 protein.

In some embodiments, an antibody that is at least 85%, at least 90%, at least 95%, at least 96%, at least 97%, at least 98%, or at least 99% identical to the amino acid sequences of the heavy chain of the antibody according to the present invention that is encoded by the nucleotide sequences according to the present invention (SEQ ID NOs: 19-36) and/or to the amino acid sequences of the light chain of the antibody according to the present invention that is encoded by the nucleotide sequences according to the present invention (SEQ ID Nos: 1-18) has a same heavy chain and/or light chain variable region as the antibody according to the present invention, and has a conserved amino acid substitution only at a heavy chain and/or light chain constant region of the heavy chain and/or light chain, wherein the conserved amino acid substitution does not change the biological function and/or activity of the corresponding antibody, and the antibody may also specifically bind to the ITGA2 protein.

In some embodiments, the antibody or the antigen-binding fragment thereof is capable of binding the ITGA2 protein in vitro at an EC50 of less than 1 nM.

In some embodiments, the antibody or the antigen-binding fragment thereof is capable of binding to an ITGA2 protein on the surface of HuCCT1 cells with an EC50 of less than 5 nM.

In some embodiments, the antibody is a monoclonal antibody, a polyclonal antibody, a chimeric antibody, or a humanized antibody.

### III. Nucleic Acid, Vector, Host Cell, Hybridoma, and Method for Preparing Antibody

In one aspect, the invention provides an isolated nucleic acid molecule encoding the antibody or the antigen-binding fragment thereof according to the present invention described above. In some embodiments, the nucleic acid molecules according to the present invention encode the heavy chain and/or light chain variable region, each CDR in the heavy chain and/or light chain variable region, and the heavy chain and/or light chain constant region of the antibody according to the present invention described above. In some embodiments, the nucleotide sequence of the nucleic acid molecule is codon optimized for a host cell for expression. In some embodiments, the nucleic acid molecule according to the invention is operably linked to an expression regulatory sequence. The present invention further provides an isolated nucleic acid molecule encoding the antibody or the antigen-binding fragment thereof according to the present invention.

In some embodiments, the nucleic acid molecule comprises or consists of a nucleotide sequence selected from any of the following nucleotide sequences as set forth in SEQ ID NOs: 1-18, and/or the nucleic acid molecule comprises or consists of a nucleotide sequence selected from any of the following nucleotide sequences as set forth in SEQ ID NOs: 19-36.

In some embodiments, the nucleic acid molecule comprises or consists of a nucleotide sequence combination selected from any one of the following nucleotide sequences: SEQ ID NO: 1 and SEQ ID NO: 19, SEQ ID NO: 2 and SEQ ID NO: 20, SEQ ID NO: 3 and SEQ ID NO: 21, SEQ ID NO: 4 and SEQ ID NO: 22, SEQ ID NO: 5 and SEQ ID NO: 23, SEQ ID NO: 6 and SEQ ID NO: 24, SEQ ID NO: 7 and SEQ ID NO: 25, SEQ ID NO: 8 and SEQ ID NO: 26, SEQ ID NO: 9 and SEQ ID NO: 27, SEQ ID NO: 10 and SEQ ID NO: 28, SEQ ID NO: 11 and SEQ ID NO: 29, SEQ ID NO: 12 and SEQ ID NO: 30, SEQ ID NO: 13 and SEQ ID NO: 31, SEQ ID NO: 14 and SEQ ID NO: 32, SEQ ID NO: 15 and SEQ ID NO: 33, SEQ ID NO: 16 and SEQ ID NO: 34, SEQ ID NO: 17 and SEQ ID NO: 35, and SEQ ID NO: 18 and SEQ ID NO: 36.

In some embodiments, the nucleic acid molecule is operably linked to an expression regulatory sequence.

The nucleotide sequence numberings (SEQ ID NO:) of the antibody according to the present invention and the corresponding structure thereof are summarized in Table 3 below:

| Antibody | Light Chain | Nucleotide Sequence Encoding Light Chain | Heavy Chain | Nucleotide Sequence Encoding Heavy Chain |
|---|---|---|---|---|
| FS002-A1 | FS002-A1-LC (Kappa) | 1 | FS002-A1-HC (IgG1) | 19 |
| FS002-A5 | FS002-A5-LC (Kappa) | 2 | FS002-A5-HC (IgG1) | 20 |
| FS002-A15 | FS002-A15-LC (Kappa) | 3 | FS002-A15-HC (IgG1) | 21 |
| FS002-A36 | FS002-A36-LC (Kappa) | 4 | FS002-A36-HC (IgG1) | 22 |
| FS002-A66 | FS002-A66-LC (Kappa) | 5 | FS002-A66-HC (IgG1) | 23 |
| FS002-A10 | FS002-A10-LC (Kappa-2) | 6 | FS002-A10-HC (IgG1) | 24 |
| FS002-C9 | FS002-C9-LC (Kappa-2) | 7 | FS002-C9-HC (IgG1) | 25 |
| FS002-C11 | FS002-C11-LC (Kappa-2) | 8 | FS002-C11-HC (IgG1) | 26 |
| FS002-C216 | FS002-C216-LC (Kappa-2) | 9 | F002-C216-HC (IgG1) | 27 |
| FS002-C375 | FS002-C375-LC (Kappa-2) | 10 | FS002-C375-HC (IgG1) | 28 |
| FS002-C417 | FS002-C417-LC (Kappa-2) | 11 | FS002-C417-HC (IgG1) | 29 |
| FS002-B27 | FS002-B27-LC (Kappa-2) | 12 | FS002-B27-HC (IgG1) | 30 |
| FS002-C330 | FS002-C330-LC (Kappa-2) | 13 | FS002-C330-HC (IgG1) | 31 |
| FS002-C153 | FS002-C153-LC( LambdaC2)-2 | 14 | FS002-C153-HC (IgG1) | 32 |
| FS002-C241 | FS002-C241-LC( LambdaC2)-2 | 15 | FS002-C241-HC (IgG1) | 33 |
| FS002-C15 | FS002-C15-LC (Kappa-2) | 16 | FS002-C15-HC (IgG1) | 34 |
| FS002-C58 | FS002-C58-LC (Kappa-2) | 17 | FS002-C58-HC (IgG1) | 35 |
| FS002-A72 | FS002-A72-LC (Kappa-2) | 18 | FS002-A72-HC (IgG1) | 36 |

The invention further provides an expression vector comprising at least one of the nucleic acid molecules according to the invention described above.

The invention further provides a host cell transformed with at least one of the nucleic acid molecules or expression vectors according the invention described above.

The present invention further provides a hybridoma cell expressing at least one of the antibody or the antigen-binding fragment thereof according to the present invention described above.

The invention further provides a method for producing the antibody or the antigen-binding fragment thereof according to the invention, comprising:
(i) culturing the host cell of the present invention in a condition suitable for expressing the nucleic acid molecule or the expression vector, and
(ii) isolating and purifying the antibody or antigen-binding fragment thereof expressed by the host cell.

In some embodiments, the nucleotide sequence of the nucleic acid molecule is codon optimized for a host cell for expression. In some embodiments, the nucleic acid molecule is operably linked to an expression regulatory sequence.

The invention further provides a method for producing the antibody or the antigen-binding fragment thereof according to the invention, comprising:
(i) preparing a hybridoma cell capable of expressing the monoclonal antibody or the antigen-binding fragment thereof according to the present invention, and
(ii) isolating and purifying the antibody or antigen-binding fragment thereof expressed by the hybridoma cell.

### IV. Antibody-drug Conjugate

In one aspect, the invention provides an antibody-drug conjugate (ADC) prepared by the antibody or the antigen-binding fragment thereof according to the invention.

Specifically, the invention provides an antibody-drug conjugate of the formula Ab-L-D, where: "Ab" represents at least one monoclonal antibody or the antigen-binding fragment thereof according to the present invention described above,
"L" represents the linker that connects the antibody moiety to the partner molecule; and "D" represents the partner molecule.

As used herein, the ADC is prepared by a linker that link a partner molecule to the antibody or the antigen-binding fragment thereof. In the ADC according to the present invention, the antibody performs a targeting function: by binding to a target tissue or cell of the antigen in which it is found, and the antibody directs the conjugate to the target tissue or cell. In that place, the linker is cleaved, for example, releasing a partner molecule to perform its desired biological function.

The partner molecule may be a drug payload or a marker molecule. The drug payload may be a therapeutic agent, where for example, the therapeutic agent may be a cytotoxic agent, a chemotherapeutic agent, a cytostatic agent, an immunomodulator, and the like. In some embodiments, the therapeutic agent is a cytotoxic agent, i.e., a cytotoxin. As used herein, the terms "cytotoxic agent" and "cytotoxin" are used interchangeably.

A marker molecule may be any marker that produces a detectable signal, such as a radioactive label, a fluorescent label, or a detectable modification enzyme for a catalytic substrate.

As used herein, the terms "drug", "payload" and "drug payload" are used interchangeably. As used herein, the antibody-drug conjugate (ADC) covers a conjugate formed by connecting the antibody or the antigen-binding fragment thereof according to the present invention to a drug payload, and a conjugate formed by connecting the antibody or the antigen-binding fragment thereof according to the present invention to a marker molecule.

The proportion of partner molecules linked to the antibody may vary depending on factors such as the amount of partner molecules used during the conjugation reaction and the experimental conditions. The ratio of the partner molecule to the antibody may be 1 to 6, 1 to 5, 1 to 4, 1 to 3, 1 to 2, or 1 to 1.5.

Therefore, the present invention provides an antibody-drug conjugate of the formula Ab-(L-D)p, where:
"Ab" represents at least one monoclonal antibody or the antigen-binding fragment thereof according to the present invention described above;
"L" represents the linker that connects the antibody moiety to the partner molecule;
"D" represents the partner molecule; and
"p" represents the number of linker/partner molecules (preferably drug payloads) conjugated to the monoclonal antibody or the antigen-binding fragment thereof according to the present invention described above.

In some embodiments, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.

In some embodiments, the linker according to the present invention is selected from one of the following: ve, mc-GGFG, AcLysve, mc, MalPeg6, m(H20)c and m(H20)cvc; and/or the linker is cleavable. In some embodiments, the linker according to the present invention is vc.

In some embodiments, the partner molecule according to the present invention is a drug payload, wherein the drug payload is a therapeutic molecule. Optionally, the drug payload is selected from: a cytotoxic agent, a cytostatic agent, an immunomodulator and a chemotherapeutic agent. Optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD.

In some embodiments, the drug payload is MMAE. "MMAE" refers to monomethyl auristatin E, as shown in the figure below, wherein the wavy lines indicate that it is covalently connected to the linker (L) of the antibody-drug conjugate:

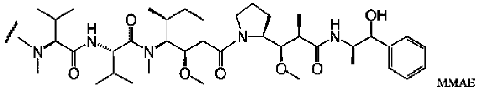

In some embodiments, the drug payload is MMAF. "MMAF" refers to monomethyl auristatin F, wherein the wavy lines indicate that it is covalently connected to the linker (L) of the antibody-drug conjugate (US2005/0238649):

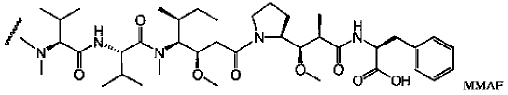

In some embodiments, the linker-partner molecule is vcMMAE. The drug linker part and conjugation method for vcMMAE are published in WO2004010957, US7659241, US7829531, US7851437, and US 11/833,028 (SeattleGenetics, Inc.), which are incorporated herein by reference. Using a method similar to that disclosed in the foregoing literatures, the drug linker of vcMMAE is conjugated with the antibody according to the present invention.

For further discussion of types of cytotoxins, linkers and methods for conjugating therapeutic agents to antibodies, see further Saito, G. et al. (2003) Adv. Drug Deliv. Rev. 55:199-215; Trail, PA et al. (2003) Cancer. Immunol. Immunother. 52:328-337; Payne, G. (2003) Cancer Cell 3: 207-212; Allen, T. M. (2002) Nat. Rev. Cancer 2: 750-763; Pastan, I. and Kreitman, R. J. (2002) Curr. Opin. Investig. Drugs 3: 1089-1091; Senter, P. D. and Springer, C. J. (2001) Adv. Drug Deliv. Rev. 53: 247-264, all of which are incorporated herein by reference.

In some embodiments, particularly, the drug payload may radioisotope, resulting in a cytotoxic radioactive drug (also known as an antibody-radioisotope conjugate). Examples of radioisotopes that kill tumor cells include, but are not limited to, iodine-131, indium-111, yttrium-90, and lutetium-177. Methods for preparing the antibody-radioisotope conjugate are known in the art.

In some embodiments, the partner molecule according to the present invention is a marker molecule. Optionally, the marker molecule is selected from one of: a radioactive label, a fluorescent label, and an enzyme that can detect a modification. In some embodiments, the radioactive label is radioisotope, such as iodine-131, indium-111, yttrium-90, and lutetium-177. Experiments have shown that the ITGA2 protein may be used as an effective target for the antibody-drug conjugate. The antibody-drug conjugate obtained by conjugating the antibody to micromolecule toxins can produce excellent killing effect on tumors expressing the ITGA2 protein.

### V. Pharmaceutical composition, Therapeutic Use and Method

The present invention provides a pharmaceutical composition comprising a therapeutic effective amount of at least one monoclonal antibody or antigen-binding fragment thereof according to the present invention described above or at least one antibody-drug conjugate according to the present invention described above, a therapeutic use and a therapeutic method of the pharmaceutical composition. In the antibody-drug conjugate according to the present invention, the antibody according to the present invention is conjugated with a drug payload.

In one aspect, the present invention provides a pharmaceutical composition comprising a therapeutic effective amount of at least one monoclonal antibody or antigen-binding fragment thereof according to the present invention described above or at least one antibody-drug conjugate according to the present invention described above. In some embodiments, the pharmaceutical composition according to the present invention may further include a pharmaceutically acceptable carrier.

A "pharmaceutical amount" of at least one monoclonal antibody or antigen-binding fragment thereof according to the present invention described above or at least one antibody-drug conjugate according to the present invention described above preferably results in decreased severity of the symptoms of a disease, an increased frequency and duration of the asymptomatic phase of a disease, or prevention of damage or disability caused by the pain of a disease. For example, for treating a tumor, a "therapeutic effective amount" of at least one monoclonal antibody or antigen-binding fragment thereof according to the present invention described above or at least one antibody-drug conjugate according to the present invention described above preferably inhibits cell growth or tumor growth by at least about 10%, preferably at least about 20%, more preferably at least about 30%, more preferably at least about 40%, more preferably at least about 50%, more preferably at least about 60%, more preferably at least about 70%, or more preferably at least about 80%, compared to a subject not receiving treatment. The ability to inhibit tumor growth may be evaluated in an animal model system that predicts the therapeutic efficacy against a human tumor. Alternatively, it may also be evaluated by examining the ability to inhibit cell growth, which may be determined in vitro by tests well known to those skilled in the art. An effective amount of the antibody or antigen-binding fragment thereof according to the invention is capable of reducing the size of a tumor, or otherwise alleviating a subject's symptoms, such as prevention and/or treatment of metastasis or relapse. One skilled in the art may determine such an amount based on factors such as the size of the subject, the severity of the subject's symptoms, and the selected particular composition or route of administration.

In another aspect, the present invention provides use of at least one monoclonal antibody or antigen-binding fragment thereof according to the present invention described above or at least one antibody-drug conjugate according to the present invention described above in preparing a pharmaceutical composition. In some embodiments, the pharmaceutical composition is used for treating a malignant tumor, preventing and/or treating metastasis or relapse of a malignant tumor in a patient.

In another aspect, the present invention provides a pharmaceutical composition comprising at least one monoclonal antibody or the antigen-binding fragment thereof according to the present invention described above or the antibody-drug conjugate according to the present invention described above, wherein the pharmaceutical composition is used for a method of treating a malignant tumor, or preventing and/or treating metastasis or relapse of a malignant tumor in a patient.

In another aspect, the present invention provides a method for treating a malignant tumor, preventing and/or treating metastasis or relapse of a malignant tumor in a patient, the method comprising administering to the patient an effective amount of at least one monoclonal antibody or the antigen-binding fragment thereof according to the present invention described above, at least one antibody-drug conjugate according to the present invention described above, and/or at least one pharmaceutical composition according to the present invention described above.

In some embodiments, the malignant tumor is a tumor expressing the ITGA2 protein. The malignant tumor is selected from a group consisting of the following: breast cancer, colorectal cancer, large intestine cancer, skin cancer, pancreatic cancer, prostate cancer, liver cancer, lung cancer, gastric cancer, leukemia, bile duct cancer, cervical cancer, endometrial cancer, esophageal cancer, head and neck cancer, lymphoma, medullary thyroid cancer, Non-Hodgkin's lymphoma, ovarian cancer, glioma, melanoma, and bladder cancer. In some embodiments, the malignant tumor is selected from a group consisting of the following: colorectal cancer, bile duct cancer, pancreatic cancer, and skin cancer. In some embodiments, the malignant tumor is colorectal cancer. In some embodiments, the malignant tumor is bile duct cancer. In some embodiments, the malignant tumor is pancreatic cancer. In some embodiments, the malignant tumor is skin cancer.

### VI. Diagnostic/Prognostic Composition, Diagnostic/Prognostic Use and Method

As described herein, ITGA2 is a tumor-associated antigen that is specifically overexpressed by tumor cells, especially in patients with cancers such as colorectal cancer, bile duct cancer, pancreatic cancer, and skin cancer, and is expressed on the surface of tumor cells.

The present invention provides a diagnostic/prognostic composition comprising at least one monoclonal antibody or antigen-binding fragment thereof according to the present invention described above or at least one antibody-drug conjugate according to the present invention described above, a diagnostic/prognostic use and a diagnostic/prognostic method of the composition. In the diagnostic/prognostic drug conjugate according to the present invention, the antibody according to the present invention is conjugated with a marker molecule. For the diagnostic/prognostic composition, the diagnostic/prognostic use and the diagnostic/prognostic method, the antibody-drug conjugate according to the present invention described above refers to a conjugate formed by connecting the antibody of the present invention or the antigen-binding fragment thereof to a marker molecule.

In one aspect, the present invention provides use of at least one monoclonal antibody or antigen-binding fragment thereof according to the present invention described above or at least one antibody-drug conjugate according to the present invention described above in preparing a diagnostic/prognostic composition. In some embodiments, the diagnostic composition is used for detecting presence of ITGA2a malignant tumor in a patient. In some embodiments, the prognostic composition is used for prognosticating relapse or progression of a malignant tumor in a patient. In another aspect, the present invention provides a method for detecting/diagnosing presence of a malignant tumor in a patient, the method comprising administering to the patient an effective amount of at least one monoclonal antibody or antigen-binding fragment thereof according to the present invention described above, at least one antibody-drug conjugate according to the present invention described above, and/or at least one diagnostic/prognostic composition according to the present invention described above.

In some embodiments, the present invention provides a method for detecting/diagnosing presence of a malignant tumor in a patient, comprising:
a) contacting a biological sample obtained from the patient with at least one monoclonal antibody or antigen-binding fragment thereof according to the present invention described above, at least one antibody-drug conjugate according to the present invention described above, and/or at least one diagnostic/prognostic composition according to the present invention described above,
b) detecting binding of at least one monoclonal antibody or antigen-binding fragment thereof according to the present invention described above, at least one antibody-drug conjugate according to the present invention described above, and/or at least one diagnostic/prognostic composition according to the present invention described above with a target antigen in the biological sample, wherein the detected binding of the antibody to the target antigen indicates presence of the malignant tumor in the patient.

In another aspect, the present invention provides a method for prognosticating relapse or progression of a malignant tumor in a patient, the method comprising administering to the patient an effective amount of at least one monoclonal antibody or antigen-binding fragment thereof according to the present invention described above, at least one antibody-drug conjugate according to the present invention described above, and/or at least one diagnostic/prognostic composition according to the present invention described above.

In some embodiments, the invention provides a method for prognosticating relapse or progression of a malignant tumor in a patient, the method comprising:
(a) isolating a biological sample comprising tumor cells from the patient,
(b) contacting the biological sample comprising tumor cells with at least one monoclonal antibody or antigen-binding fragment thereof according to the present invention described above, at least one antibody-drug conjugate according to the present invention described above, and/or at least one diagnostic/prognostic composition according to the present invention described above, and
(c) identifying the binding of the antibody to the tumor cells, which represents the presence of tumor cells,
thereby prognosticating the relapse or progression of the malignant tumor in the patient.

In some embodiments, the progression of the malignant tumor includes metastasis of the malignant tumor in the patient.

In some embodiments, the detected binding of tumor cells to at least one monoclonal antibody or antigen-binding fragment thereof according to the present invention described above, at least one antibody-drug conjugate according to the present invention described above, and/or at least one diagnostic/prognostic composition according to the present invention described above indicates high risk of relapse or progression of the malignant tumor in the patient.

In some embodiments, the partner molecule according to the present invention is a marker molecule. Optionally, the marker molecule is selected from one of: a radioactive label (for example, an isotope), a fluorescent label (for example, a fluorescent dye), a chemical substance, and an enzyme or label that can detect a modification.

In some embodiments, the biological sample is selected from one of: a blood sample, a tissue sample, and a lymph sample.

Methods for detecting binding of an antibody to an antigen are known in the art, such as ELISA and the like.

### VII. Kit

The scope of the present invention further comprises a kit for the method according to the present invention, wherein the kit includes at least one monoclonal antibody or antigen-binding fragment thereof according to the present invention described above, at least one antibody-drug conjugate according to the present invention described above, at least one pharmaceutical composition according to the present invention described above, or a diagnostic/prognostic composition according to the present invention described above, and a direction for use.

In some embodiments, the kit may further include at least one additional detection reagent used to detect presence of at least one monoclonal antibody or antigen-binding fragment thereof according to the present invention described above, at least one antibody-drug conjugate according to the present invention described above, at least one pharmaceutical composition according to the present invention described above, or a diagnostic/prognostic composition according to the present invention described above. The kit generally includes a label indicating the intended use and/or method of use of the contents of the kit. The term "label" includes any written or recorded material provided on or with the kit or otherwise provided with the kit.

### Examples

The present invention may be further understood by reference to the specific examples set forth herein, which are only intended to illustrate the invention, and are not intended to limit the scope of the invention. It is apparent that various modifications and variations may be made to the present invention without departing from the spirit of the invention, and such modifications and variations are therefore also within the patentable scope of the present invention.

### Example 1 - Specific overexpression of the ITGA2 protein on the surface of a variety of cancer cells

HuCC-T1 (human bile duct cancer cells; MeisenCTCC: CTCC-003-0103), HCCC-9810 (human bile duct cancer cells; Procell Life Science&Technology Co.,Ltd. (Wuhan): CL-0095), AsPC-1 (human metastatic pancreatic cancer cells; Procell Life Science&Technology Co.,Ltd. (Wuhan): CL-0027), BxPC-3 (human orthotopic pancreatic adenocarcinoma cells; MeisenCTCC: CTCC-001-0360), A431 (human epidermal cancer cells; Procell Life Science&Technology Co.,Ltd. (Wuhan): CL-0015), HT-29 (human colorectal adenocarcinoma cell line; Procell Life Science&Technology Co.,Ltd. (Wuhan): CL-0118) and CHO (hamster ovary cells; Procell Life Science&Technology Co.,Ltd. (Wuhan): CL-0061) cells were seeded into a deep 96-well plate. The cells were seeded into each well at 50,000/100 µL, and each cell was seeded in three wells. It was then blown evenly with PBS and washed twice. Each well was added with a PBS solution comprising 1% FBS per at an amount of 100 µL/well and the plate was then incubated in a 4°C freezer for 30 min. A FITC-labeled mouse anti-human ITGA2 antibody solution (BD Pharmingen:555498) was diluted to a working concentration of 20 µL/well, and incubated in a 4°C freezer for 30 min in the dark. After incubation, cells were washed twice with 500 ul of 1% FBS and then added with 250 ul of PBS and suspended again. The blank reference solution was a 250 µL PBS solution comprising the reference control solution. Flow cytometry was used to collect and analyze the number of ITGA2 protein copies of each cell line, in the molecules of equivalent soluble fluorochrome (MESF). The results are shown in the table 3 below.

**Table 3**

| **Cell Line** | **ITGA2 Protein Copy Number (MESF)** |
|---|---|
| Human bile duct cancer cells HuCCT1 | 2,013,058 |
| Human orthotopic pancreatic adenocarcinoma cells BxPC-3 | 1,633,773 |
| Human bile duct cancer cells HCCC-9810 | 961,428 |
| Human metastatic pancreatic cancer cells ASPC-1 | 746,355 |
| Human epidermal carcinoma cells A431 | 721,588 |
| Human colorectal adenocarcinoma cells HT-29 | 151,268 |
| CHO | 959 |

As shown in Table 3, ITGA2 protein had higher expression (MESF>150,000) on the surface of HuCCT1, BxPC-3, HCCC-9810, ASPC-1, and A431 cells, and almost no expression on the surface of the negative control cell CHO (MESF<1000, which can be regarded as the background value at the experimental technical level).

### Example 2 - Preparation of ITGA2 Antibody

### 1. Constructing a Vector

The design, vector construction, expression, purification and identification of the ITGA2 antibody sequence of the present invention were entrusted to Sanyou Biopharmaceuticals Co., Ltd. The construction process mainly includes constructing the sequence of the ITGA2 antibody on a pcDNA3.4 vector, and obtaining a plasmid through PCR, enzyme digestion, ligation, transformation, identification, sequencing, alignment, and extraction.

The vector comprising the light chain and heavy chain sequence of the ITGA2 antibody according to the present invention is shown in Table 4 below:

| **Antibody** | **Vector Comprising Heavy Chain** | **Vector Comprising Light Chain** |
|---|---|---|
| FS002-A1 | pcDNA3.4-FS002-A1-HC (IgG1) | pcDNA3.4-FS002-A1-LC (Kappa) |
| FS002-A5 | pcDNA3.4- FS002-A5-HC(IgG1 ) | pcDNA3.4-FS002-A5-LC (Kappa) |
| FS002-A15 | pcDNA3.4-FS002-A15-HC (IgG1) | pcDNA3.4-FS002-A15-LC (Kappa) |
| FS002-A36 | pcDNA3.4-FS002-A36-HC (IgG1) | pcDNA3.4-FS002-A36-LC (Kappa) |
| FS002-A66 | pcDNA3.4-FS002-A66-HC (IgG1) | pcDNA3.4-FS002-A66-LC (Kappa) |
| FS002-A10 | pcDNA3.4-FS002-A10-HC (IgG1) | pcDNA3.4-FS002-A10-LC (Kappa-2) |
| FS002-C9 | pcDNA3.4-FS002-C9-HC (IgG1) | pcDNA3.4-FS002-C9-LC (Kappa-2) |
| FS002-C11 | pcDNA3.4-FS002-C11-HC (IgG1) | pcDNA3.4-FS002-C11-LC (Kappa-2) |
| FS002-C216 | pcDNA3.4-FS002-C216-HC (IgG1) | pcDNA3.4-FS002-C216-LC (Kappa-2) |
| FS002-C375 | pcDNA3.4-FS002-C375-HC (IgG1) | pcDNA3.4-FS002-C375-LC (Kappa-2) |
| FS002-C417 | pcDNA3.4-FS002-C417-HC (IgG1) | pcDNA3.4-FS002-C417-LC (Kappa-2) |
| FS002-B27 | pcDNA3.4-FS002-B27-HC (IgG1) | pcDNA3.4-FS002-B27-LC (Kappa-2) |
| FS002-C330 | pcDNA3.4-FS002-C330-HC (IgG1) | pcDNA3.4-FS002-C330-LC (Kappa-2) |
| FS002-C153 | pcDNA3.4-FS002-C153-HC (IgG1) | pcDNA3.4-FS002-C153-LC(LambdaC2)-2 |
| FS002-C241 | pcDNA3.4-FS002-C241-HC (IgG1) | pcDNA3.4-FS002-C241-LC(LambdaC2)-2 |
| FS002-C15 | pcDNA3.4-FS002-C15-HC (IgG1) | pcDNA3.4-FS002-C15-LC (Kappa-2) |
| FS002-C58 | pcDNA3.4-FS002-C58-HC (IgG1) | pcDNA3.4-FS002-C58-LC (Kappa-2) |
| FS002-A72 | pcDNA3.4-FS002-A72-HC (IgG1) | pcDNA3.4-FS002-A72-LC (Kappa-2) |

The 16 plasmids of the light chain were of Kappa type, 2 plasmids of Lambda type; and the 18 plasmids of the heavy chain were of IgG1 type.

The coding sequences for the light and heavy chains in each plasmid vector in the table above are as follows (the shaded parts correspond to the constant region sequences):
Light chain:
   Humanized antibody light chain coding sequence: FS002-A1-LC (Kappa) (SEQ ID NO: 1), which is used to express the humanized light chain variable region + human light chain constant region 
GACATCCAGTTGACCCAGTCTCCATCCTCCCTGTCTGCATCTGTAGGAGACAGAGTCAC
CATCACTTGCCGGGCAAGTCAGAGCATTATCAGCTATTTAAATTGGTATCAGCAGAAAC
CAGGGAAAGCCCCTAAGCTCCTGATCTATGCTGCATCCAGTTTGCAAAGTGGGGTCCC
ATCAAGGTTCAGTGGCAGTGGATCTGGGACAGATTTCACTCTCACCATCAGCAGTCTG
CAACCTGAAGATTTTGCAACTTACTACTGTCAACAGAGTTACAGTACCCCTGACACTTT
TGGCCAGGGGACCAAGCTGGAGATCAAAAGGACCGTGGCTGCCCCCAGCGTGTTCAT
CTTCCCTCCTAGCGACGAGCAGCTGAAGAGCGGCACCGCTAGCGTGGTGTGTCTGCTG
AATAACTTCTATCCCAGGGAGGCCAAGGTGCAGTGGAAGGTGGATAACGCCCTGCAGA
GCGGCAACTCCCAGGAGTCCGTGACCGAGCAGGACTCCAAGGACAGCACCTACTCCC
TGAGCTCCACCCTGACCCTGTCCAAGGCTGATTATGAGAAGCACAAGGTGTATGCTTG
CGAGGTGACACACCAGGGCCTGTCCAGCCCTGTGACCAAGAGCTTCAACCGGGGCGA
GTGC
   Humanized antibody light chain coding sequence: FS002-A5-LC (Kappa) (SEQ ID NO: 2), which is used to express the humanized light chain variable region + human light chain constant region 
GAAATTGTGTTGACACAGTCTCCAGCCACCCTGTCTTTGTCTCCAGGGGAAAGAGCCA
CCCTCTCCTGCAGGGCCAGTCAGAGTGTTAGCAGCAACTTAGGCTGGTACCAACAGAA
ACCTGGCCAGGCTCCCAGCCTCCTCATCTATGGTGCATCCAACAGGGCCACTGGCATCC
CAGCCAGGTTCAGTGGCAGTGGGTCTGGGACAGACTTCACTCTCACCATCAGCAGCCT
AGAGCCTGAAGATTTTGCAGTTTATTACTGTCAGCAGCGTGACACCTGGCCCCTCACTT
 
TCGGCGGAGGGACCAAGGTGGAGATCAAAAGGACCGTGGCTGCCCCCAGCGTGTTCA
TCTTCCCTCCTAGCGACGAGCAGCTGAAGAGCGGCACCGCTAGCGTGGTGTGTCTGCT
GAATAACTTCTATCCCAGGGAGGCCAAGGTGCAGTGGAAGGTGGATAACGCCCTGCAG
AGCGGCAACTCCCAGGAGTCCGTGACCGAGCAGGACTCCAAGGACAGCACCTACTCC
CTGAGCTCCACCCTGACCCTGTCCAAGGCTGATTATGAGAAGCACAAGGTGTATGCTT
GCGAGGTGACACACCAGGGCCTGTCCAGCCCTGTGACCAAGAGCTTCAACCGGGGCG
AGTGC
   Humanized antibody light chain coding sequence: FS002-A15-LC (Kappa) (SEQ ID NO: 3), which is used to express the humanized light chain variable region + human light chain constant region 
GAAATTGTGTTGACACAGTCTCCAGCCACCCTGCCTTTGTCTCCGGGGGAAAGAGCCA
CCCTCTCCTGCAGGGCCAGTCAGAGTGTTGGCTTCTACTTAGCCTGGTACCAACAGAA
ACCTGGCCAGCCTCCCAGACTCCTCTTCTATGATGCATCCAAGAGGGCCACTGGCATCC
CAGCCAGGTTCAGCGCCAGTGGGTCTGGGACAGACTTCACTCTCACCATCAGCGGCCT
AGAGCCTGAAGATTTTGCAGTTTATTACTGTCAGCACCGCATAAGCTGGCCTCTGACGT
TCGGCCAAGGGACCAAGGTGGAAGTGAAGAGGACCGTGGCTGCCCCCAGCGTGTTCA
TCTTCCCTCCTAGCGACGAGCAGCTGAAGAGCGGCACCGCTAGCGTGGTGTGTCTGCT
GAATAACTTCTATCCCAGGGAGGCCAAGGTGCAGTGGAAGGTGGATAACGCCCTGCAG
AGCGGCAACTCCCAGGAGTCCGTGACCGAGCAGGACTCCAAGGACAGCACCTACTCC
CTGAGCTCCACCCTGACCCTGTCCAAGGCTGATTATGAGAAGCACAAGGTGTATGCTT
GCGAGGTGACACACCAGGGCCTGTCCAGCCCTGTGACCAAGAGCTTCAACCGGGGCG
AGTGC
   Humanized antibody light chain coding sequence: FS002-A36-LC (Kappa) (SEQ ID NO: 4), which is used to express the humanized light chain variable region + human light chain constant region 
GAAATTGTGTTGACACAGTCTCCAGCCACCCTGTCTTTGTCTCCAGGGGAAAGAGCCA
CCCTCTCCTGCAGGGCCAGTCAGAGTGTTAGTACTTACTTAGCCTGGTACCAACAGAA
ACCTGGCCAGGCTCCCAGGCTCCTCATCTATGACACATCCATCAGGGCCACTGGCATCC
CAGCCAGGTTCAGTGGCAGTGGGTCTGGGACAGACTTCACTCTCACCATCAGCAGCCT
AGAGCCTGAAGATTTTGCAGTTTATTACTGTCAGCAGCGTAGCAACTGGCCGCTCACTT
TCGGCGGAGGGACCAAGGTGGAGATCAAAAGGACCGTGGCTGCCCCCAGCGTGTTCA
TCTTCCCTCCTAGCGACGAGCAGCTGAAGAGCGGCACCGCTAGCGTGGTGTGTCTGCT
GAATAACTTCTATCCCAGGGAGGCCAAGGTGCAGTGGAAGGTGGATAACGCCCTGCAG
AGCGGCAACTCCCAGGAGTCCGTGACCGAGCAGGACTCCAAGGACAGCACCTACTCC
CTGAGCTCCACCCTGACCCTGTCCAAGGCTGATTATGAGAAGCACAAGGTGTATGCTT
GCGAGGTGACACACCAGGGCCTGTCCAGCCCTGTGACCAAGAGCTTCAACCGGGGCG
AGTGC
   Humanized antibody light chain coding sequence: FS002-A66-LC (Kappa) (SEQ ID NO: 5), which is used to express the humanized light chain variable region + human light chain constant region 
GAAATTGTGTTGACACAGTCTCCAGCCATCCTGTCTTTGTCTCCAGGGGAAAGAGCCA
CCCTCTCCTGCAGGGCCAGTCAGAGTGTTAGCGAGTACTTAGCCTGGTACCAACAAAA
ACCTGGCCAGGCTCCCAGGCTCCTCATCTATGATATATCTAACAGGGCCACTGGCATCC
CAGCCAGGTTCAGTGGCAGTGGGTCTGAGACAGACTTCACTCTCACCATCAGCAGCCT
AGAGCCTGAAGATTTTGCAGTTTATTACTGTCAACAACGTCGCACCTGGCCTCTCACTT
TCGGCCCTGGGACCAAAGTGGACATCAAAAGGACCGTGGCTGCCCCCAGCGTGTTCA
TCTTCCCTCCTAGCGACGAGCAGCTGAAGAGCGGCACCGCTAGCGTGGTGTGTCTGCT
GAATAACTTCTATCCCAGGGAGGCCAAGGTGCAGTGGAAGGTGGATAACGCCCTGCAG
AGCGGCAACTCCCAGGAGTCCGTGACCGAGCAGGACTCCAAGGACAGCACCTACTCC
CTGAGCTCCACCCTGACCCTGTCCAAGGCTGATTATGAGAAGCACAAGGTGTATGCTT
GCGAGGTGACACACCAGGGCCTGTCCAGCCCTGTGACCAAGAGCTTCAACCGGGGCG
AGTGC
   Humanized antibody light chain coding sequence: FS002-A10-LC (Kappa-2) (SEQ ID NO: 6), which is used to express the humanized light chain variable region + human light chain constant region 
AACATCCAGTTGACCCAGTCTCCACCCTTCCTGTCTGCATCTGTAGGAGACAGAGTCA
CCATCACTTGCCGGGCCAGCCAGGACATTAGCAGTTTTTTAGCCTGGTATCAGCAAAA
ACCAGGGAAAGCCCCTAACCTCCTGATCCATGCTGCAACCACTCTGCAAAGTGGGGTC
CCATCAAGGTTCAGCGGCAGTGGATCTGGGACGGAGTTCACTCTCACAATCAGCAGCC
TGCGGCCAGAAGATTTTGCCACTTACTACTGTCAACAACTTCATACTTACCCGATCACC
TTCGGCCAAGGGACACGACTGGAGATTAAACGAACTGTGGCTGCACCATCCGTGTTCA
TCTTCCCTCCTAGCGACGAGCAGCTGAAGAGCGGCACCGCTAGCGTGGTGTGTCTGCT
GAATAACTTCTATCCCAGGGAGGCCAAGGTGCAGTGGAAGGTGGATAACGCCCTGCAG
AGCGGCAACTCCCAGGAGTCCGTGACCGAGCAGGACTCCAAGGACAGCACCTACTCC
CTGAGCTCCACCCTGACCCTGTCCAAGGCTGATTATGAGAAGCACAAGGTGTATGCTT
GCGAGGTGACACACCAGGGCCTGTCCAGCCCTGTGACCAAGAGCTTCAACCGGGGCG
AGTGC
   Humanized antibody light chain coding sequence: FS002-C9-LC (Kappa-2) (SEQ ID NO: 7), which is used to express the humanized light chain variable region + human light chain constant region 
AACATCCAGATGACCCAGTCTCCACCCTCCCTGTCTGCATCTGTGGGAGACAGAGTCG
CCATCACTTGCCGGGCGAGTCAGGACATTAGCAATTCCTTAGCCTGGTATCAGCAGAA
GTCAGGGCAACCCCCTAAACTCCTGCTCTATGATGTGTCGAAATTGGAAAGGGGGGTC
CCATCCAGGTTCAGTGGCAGTGGATCTCGGACGGATTACACTCTCACCATCAGCAGCC
TGCAGCCTGAAGATTTTGCAACTTATTACTGTCAACAGTATTCTAGTGACCCCACTTTT
GGACAGGGGAGTAAGCTGGAGTTCAAACGAACTGTGGCTGCACCATCCGTGTTCATCT
TCCCTCCTAGCGACGAGCAGCTGAAGAGCGGCACCGCTAGCGTGGTGTGTCTGCTGAA
TAACTTCTATCCCAGGGAGGCCAAGGTGCAGTGGAAGGTGGATAACGCCCTGCAGAGC
GGCAACTCCCAGGAGTCCGTGACCGAGCAGGACTCCAAGGACAGCACCTACTCCCTG
AGCTCCACCCTGACCCTGTCCAAGGCTGATTATGAGAAGCACAAGGTGTATGCTTGCG
AGGTGACACACCAGGGCCTGTCCAGCCCTGTGACCAAGAGCTTCAACCGGGGCGAGT
GC
   Humanized antibody light chain coding sequence: FS002-C11-LC (Kappa-2) (SEQ ID NO: 8), which is used to express the humanized light chain variable region + human light chain constant region 
GACATCCAGATGATCCAGTCTCCTTCTTCCGTGTCTGCTTCTGTGGGAGACAGAGTCAC
CATCACTTGTCGGGCGAGTCAGGGTATCAGCAACTCGTTAGCCTGGTATCAGCACAAA
CCAGGTACAACCCCTAAGCTCCTGATCTATGCTGCATCCAGTTTGCAAGGTGGGGTCCC
ATCCAGGTTCAGCGGCAGTTCATCTGAGACAGATTTCACTCTCACCATCAGCAGCCTTC
AGCCTGAAGATTTTGCAACTTACTACTGTCAGCAGGCCAACAGTTTCCCGATCACCTTC
GGCCAGGGGACACGACTGGATATTAAACGAACTGTGGCTGCACCATCCGTGTTCATCT
TCCCTCCTAGCGACGAGCAGCTGAAGAGCGGCACCGCTAGCGTGGTGTGTCTGCTGAA
TAACTTCTATCCCAGGGAGGCCAAGGTGCAGTGGAAGGTGGATAACGCCCTGCAGAGC
GGCAACTCCCAGGAGTCCGTGACCGAGCAGGACTCCAAGGACAGCACCTACTCCCTG
AGCTCCACCCTGACCCTGTCCAAGGCTGATTATGAGAAGCACAAGGTGTATGCTTGCG
AGGTGACACACCAGGGCCTGTCCAGCCCTGTGACCAAGAGCTTCAACCGGGGCGAGT
GC
   Humanized antibody light chain coding sequence: FS002-C216-LC (Kappa-2) (SEQ ID NO: 9), which is used to express the humanized light chain variable region + human light chain constant region
      GAAATTGTGTTGACACAGTCTCCAGCCACTCTGTCTTTGTCTCCAGGGGAAAGAGCCA CCCTCTCCTGCAGGGCCAGTCAGAGTGTTAGCAGCTACTTAGCCTGGTACCAACAAAA 
ACCTGGCCAGGCTCCCAGACTCCTCATCTATGATGCATCCAACAGGGCCACTGGCATCC
CAGCCAGGTTCAGTGGCAGTGGGTCTGGGACAGACTTCACTCTCACCATCAGCAGCCT
AGAGCCTGAAGATTTTGCAGTTTATTACTGTCAGCAGCGTACCAACTGGCTTATGTACA
CTTTTGGCCAGGGGACCAAGCTGGAGATCAAACGAACTGTGGCTGCACCATCCGTGTT
CATCTTCCCTCCTAGCGACGAGCAGCTGAAGAGCGGCACCGCTAGCGTGGTGTGTCTG
CTGAATAACTTCTATCCCAGGGAGGCCAAGGTGCAGTGGAAGGTGGATAACGCCCTGC
AGAGCGGCAACTCCCAGGAGTCCGTGACCGAGCAGGACTCCAAGGACAGCACCTACT
CCCTGAGCTCCACCCTGACCCTGTCCAAGGCTGATTATGAGAAGCACAAGGTGTATGC
TTGCGAGGTGACACACCAGGGCCTGTCCAGCCCTGTGACCAAGAGCTTCAACCGGGG
CGAGTGC
   Humanized antibody light chain coding sequence: FS002-C375-LC (Kappa-2) (SEQ ID NO: 10), which is used to express the humanized light chain variable region + human light chain constant region 
GAAATTGTGTTGACACAGTCTCCAGCCACCCTGTCTTTGTCTCCAGGGGAAAGAGCCA
CCCTCTCCTGCAGGGCCAGTCAGAGTGTTAGCAGCTACTTAGCCTGGTACCAACAGAA
ACCTGGCCAGGCTCCCAGGCTCCTCATCTATGATGCATCCAACAGGGCCACTGGCATCC
CAGCCAGGTTCAGTGGCAGTGGGTCTGGGACAGACTTCACTCTCACCATCAGCAGCCT
AGAGCCTGAAGATTTTGCAGTTTATTACTGTCAGCAGCGTAGCAACTGGCCCCTCACTT
TCGGCGGAGGGACCAAGGTGGAGATCAAACGAACTGTGGCTGCACCATCCGTGTTCA
TCTTCCCTCCTAGCGACGAGCAGCTGAAGAGCGGCACCGCTAGCGTGGTGTGTCTGCT
GAATAACTTCTATCCCAGGGAGGCCAAGGTGCAGTGGAAGGTGGATAACGCCCTGCAG
AGCGGCAACTCCCAGGAGTCCGTGACCGAGCAGGACTCCAAGGACAGCACCTACTCC
CTGAGCTCCACCCTGACCCTGTCCAAGGCTGATTATGAGAAGCACAAGGTGTATGCTT
GCGAGGTGACACACCAGGGCCTGTCCAGCCCTGTGACCAAGAGCTTCAACCGGGGCG
AGTGC
   Humanized antibody light chain coding sequence: FS002-C417-LC (Kappa-2) (SEQ ID NO: 11), which is used to express the humanized light chain variable region + human light chain constant region 
AACATCCAGATGACCCAGTCTCCATCCTCCCTGTCTGCATCTGTAGGAGACAGAGTCAC
CATCACTTGCCGGGCAAGTCAGAGCATTAGCAGCTATTTAAATTGGTATCAGCAGAAAC
CAGGGAAAGCCCCTAAGCTCCTGATCTATGCTGCATCCAGTTTGCAAAGTGGGGTCCC
ATCAAGGTTCAGTGGCAGTGGATCTGGGACAGATTTCACTCTCACCATCAGCAGTCTG
CAACCTGAAGATTTTGCATCTTACTACTGTCAACAGAGTTACAGTTCGTGGGCGTTCGG
CCAAGGGACCAAGGTGGAAATCAAACGAACTGTGGCTGCACCATCCGTGTTCATCTTC
CCTCCTAGCGACGAGCAGCTGAAGAGCGGCACCGCTAGCGTGGTGTGTCTGCTGAATA
ACTTCTATCCCAGGGAGGCCAAGGTGCAGTGGAAGGTGGATAACGCCCTGCAGAGCG
GCAACTCCCAGGAGTCCGTGACCGAGCAGGACTCCAAGGACAGCACCTACTCCCTGA
GCTCCACCCTGACCCTGTCCAAGGCTGATTATGAGAAGCACAAGGTGTATGCTTGCGA
GGTGACACACCAGGGCCTGTCCAGCCCTGTGACCAAGAGCTTCAACCGGGGCGAGTG
C
   Humanized antibody light chain coding sequence: FS002-B27-LC (Kappa-2) (SEQ ID NO: 12), which is used to express the humanized light chain variable region + human light chain constant region 
AACATCCAGTTGACCCAGTCTCCATCTTCCGTGTCTGCATCTGTAGGAGACAGAGTCAC
CATCACTTGTCGGGCGAGTCAGGGTTTTAGTAGGTGGTTAGCCTGGTATCAGCAGAAA
CCAGGGAAAGCCCCTAAGCTCCTGATCTATGGTGCATCCAGTTTGCAAAGTGGGGTCC
CATCAAGATTCAGCGGCAGTGGATCTGGGACAGATTTCACTCTCACCATCAGCAGCCT
GCAGCCTGAAGATTTTGCAACTTACTATTGTCAACAGGCTAACAGTTTCCCTCTCACTT
TCGGCGGAGGGACCAAGCTGGAGATCAAACGAACTGTGGCTGCACCATCCGTGTTCAT
CTTCCCTCCTAGCGACGAGCAGCTGAAGAGCGGCACCGCTAGCGTGGTGTGTCTGCTG
AATAACTTCTATCCCAGGGAGGCCAAGGTGCAGTGGAAGGTGGATAACGCCCTGCAGA
GCGGCAACTCCCAGGAGTCCGTGACCGAGCAGGACTCCAAGGACAGCACCTACTCCC
 
TGAGCTCCACCCTGACCCTGTCCAAGGCTGATTATGAGAAGCACAAGGTGTATGCTTG
CGAGGTGACACACCAGGGCCTGTCCAGCCCTGTGACCAAGAGCTTCAACCGGGGCGA
GTGC
   Humanized antibody light chain coding sequence: FS002-C330-LC (Kappa-2) (SEQ ID NO: 13), which is used to express the humanized light chain variable region + human light chain constant region 
AACATCCAGTTGACCCAGTCTCCATCCTCCCTGTCTGCATCTGTAGGAGACAGAGTCAC
CATCACTTGCCGGGCAAGTCAGAGCATTAGCAGCTATTTAAATTGGTATCAGCAGAAAC
CAGGGAAAGCCCCTAAGCTCCTGATCTATGCTGCATCCAGTTTGCAAAGTGGGGTCCC
ATCAAGGTTCAGTGGCAGTGGATCTGGGACAGATTTCACTCTCACCATCAGCAGTCTG
CAACCTGAAGATTTTGCAACTTACTACTGTCAACAGAGTTACAGTACCCCAGACACTTT
CGGCCCTGGGACCAAAGTGGATATCAAACGAACTGTGGCTGCACCATCCGTGTTCATC
TTCCCTCCTAGCGACGAGCAGCTGAAGAGCGGCACCGCTAGCGTGGTGTGTCTGCTGA
ATAACTTCTATCCCAGGGAGGCCAAGGTGCAGTGGAAGGTGGATAACGCCCTGCAGAG
CGGCAACTCCCAGGAGTCCGTGACCGAGCAGGACTCCAAGGACAGCACCTACTCCCT
GAGCTCCACCCTGACCCTGTCCAAGGCTGATTATGAGAAGCACAAGGTGTATGCTTGC
GAGGTGACACACCAGGGCCTGTCCAGCCCTGTGACCAAGAGCTTCAACCGGGGCGAG
TGC
   Humanized antibody light chain coding sequence: FS002-C153-LC(LambdaC2)-2 (SEQ ID NO: 14), which is used to express the humanized light chain variable region + human light chain constant region 
CAGTCTGCCCTGACTCAGCCTGCCTCCGTGTCTGGGTCTCCTGGACAGTCGATCACCAT
CTCCTGCACTGGAACCAGCAGTGACGTTGGTGGTTATAACTATGTCTCCTGGTACCAAC
AACACCCAGGCAAAGCCCCCAAACTCATGATTTATGATGTCAGTAATCGGCCCTCAGG
GGTTTCTAATCGCTTCTCTGGCTCCAAGTCTGGCAACACGGCCTCCCTGACCATCTCTG
GGCTCCAGGCTGAGGACGAGGCTGATTATTATTGCAGCTCATTTACAACCATCACTACG
AGGGTGTTCGGCGGAGGGACCAAGCTGACCGTCCTAGGTCAGCCCAAGGCTGCCCCC
TCGGTGACACTGTTCCCTCCATCCTCTGAGGAACTGCAGGCCAACAAGGCTACCCTCG
TGTGCCTGATCTCCGACTTTTACCCTGGCGCTGTGACCGTGGCCTGGAAGGCTGATAGT
TCTCCTGTGAAGGCCGGCGTGGAAACCACCACACCTTCCAAGCAGTCCAACAACAAA
TACGCCGCCTCCTCCTACCTGTCTCTGACCCCTGAACAGTGGAAGTCCCACCGGTCCTA
CAGCTGCCAAGTGACCCATGAGGGCTCCACCGTGGAAAAGACCGTGGCTCCTACCGA
GTGCTCT
   Humanized antibody light chain coding sequence: FS002-C241-LC(LambdaC2)-2 (SEQ ID NO: 15), which is used to express the humanized light chain variable region + human light chain constant region 
CAGTCTGCCCTGACTCAGCCTGCCTCCGTGTCTGGGTCTCCTGGACAGTCGATCACCAT
CTCCTGCAGTGGAACCAGCAGTGACATTGGTCGTTATAACTATGTCTCCTGGTACCAAC
AACACCCAGGCAAAGCCCCCAAACTCCTGATTTTTGATGTCAGTAATCGGCCCTCAGG
GGTTTCTAATCGTTTCTCTGGCTCCAAGTCTGGCAACACGGCCTCCCTGACCATCTCTG
GGCTCCAGGCTGAGGACGAGGCTGATTATTACTGCAGCTCATATACAAGCACCGTCCCT
TATGTCTTCGGAACTGGGACGCGGGTCGCCGTCCAAGGTCAGCCCAAGGCTGCCCCCT
CGGTGACACTGTTCCCTCCATCCTCTGAGGAACTGCAGGCCAACAAGGCTACCCTCGT
GTGCCTGATCTCCGACTTTTACCCTGGCGCTGTGACCGTGGCCTGGAAGGCTGATAGTT
CTCCTGTGAAGGCCGGCGTGGAAACCACCACACCTTCCAAGCAGTCCAACAACAAAT
ACGCCGCCTCCTCCTACCTGTCTCTGACCCCTGAACAGTGGAAGTCCCACCGGTCCTA
CAGCTGCCAAGTGACCCATGAGGGCTCCACCGTGGAAAAGACCGTGGCTCCTACCGA
GTGCTCT
   Humanized antibody light chain coding sequence: FS002-C15-LC (Kappa-2) (SEQ ID NO: 16), which is used to express the humanized light chain variable region + human light chain constant region 
GAAATTGTATTGACACAGTCTCCAGCCACCCTGTCTTTGTCTCCAGGGGAAAGAGCCA
CCCTCTCCTGCCAGGCCAGTCAGACTTTTAGCGATTACTTAGCCTGGTACCAACAGAA
ACCTGGCCAGGCTCCCAGGCTCCTCATCTATGATGCATCCACCAGGGCCACTGGCATCC
CAGCCAGGTTCAGTGGCAGTGGGTCTGGGACAGACTTCACTCTCACCATCAGCAGCCT
AGAGCCTGAAGATTTTGCAGTTTATTACTGTCAGCAGCGTAGCCACTGGCCTCCAGGGT
ACACTTTTGGCCAAGGGACCAGGGTGGAGATCAAACGAACTGTGGCTGCACCATCCG
TGTTCATCTTCCCTCCTAGCGACGAGCAGCTGAAGAGCGGCACCGCTAGCGTGGTGTG
TCTGCTGAATAACTTCTATCCCAGGGAGGCCAAGGTGCAGTGGAAGGTGGATAACGCC
CTGCAGAGCGGCAACTCCCAGGAGTCCGTGACCGAGCAGGACTCCAAGGACAGCACC
TACTCCCTGAGCTCCACCCTGACCCTGTCCAAGGCTGATTATGAGAAGCACAAGGTGT
ATGCTTGCGAGGTGACACACCAGGGCCTGTCCAGCCCTGTGACCAAGAGCTTCAACC
GGGGCGAGTGC
   Humanized antibody light chain coding sequence: FS002-C58-LC (Kappa-2) (SEQ ID NO: 17), which is used to express the humanized light chain variable region + human light chain constant region 
GAAATTGTGTTGACACAGTCTCCAGCCACCCTGTCTTTGTCTCCAGGGGAAAGAGCCA
CCCTCTCCTGCAGGGCCAGTCAGAGTGTTTACAGTTCTTTAGCCTGGTACCAACAGAA
ACCTGGCCAGGCTCCCAAGCTCCTCATCTATGATGCATCCAACAGGGCCACTGGCGTC
CCAGCCAGGTTCAGTGGCAGTGGGTCTGGGACAGACTTCACTCTCACCATCAGCAGCC
TAGAGCCTGAGGATTTTGCACTTTATTACTGTCAGCACCGTCAGCATTGGCCTCTCACT
TTCGGCGGAGGGACCAAGGTGGAGATCAAACGAACTGTGGCTGCACCATCCGTGTTC
ATCTTCCCTCCTAGCGACGAGCAGCTGAAGAGCGGCACCGCTAGCGTGGTGTGTCTGC
TGAATAACTTCTATCCCAGGGAGGCCAAGGTGCAGTGGAAGGTGGATAACGCCCTGCA
GAGCGGCAACTCCCAGGAGTCCGTGACCGAGCAGGACTCCAAGGACAGCACCTACTC
CCTGAGCTCCACCCTGACCCTGTCCAAGGCTGATTATGAGAAGCACAAGGTGTATGCT
TGCGAGGTGACACACCAGGGCCTGTCCAGCCCTGTGACCAAGAGCTTCAACCGGGGC
GAGTGC
   Humanized antibody light chain coding sequence: FS002-A72-LC (Kappa-2) (SEQ ID NO: 18), which is used to express the humanized light chain variable region + human light chain constant region 
GAAATTGTGATGACGCAGTCTCCAGTCACCCTGTCTGTCTCTCCAGGGGACTCAGCCA
CCCTCTCCTGCAGGGCCAGTCAGAGTGTTGATAAAAACTTAGCCTGGTACCAGCAGAA
AACTGGCCAGGCTCCCAGGCTCCTCATCTATGGTGCAGCCACCAGGGCCACTGGTATC
CCAGCCAGGTTCAGTGGCAGTGGGTCTGGGACAGAATTCACTCTCACCATCAGCAGCC
TGCGGTCTGAAGATTTTGCAGTTTATTACTGTCAGCAGTATAGAAACTGGCCTTGGACG
TTCGGCCTAGGGACCAAGGTGGAGATCAAACGAACTGTGGCTGCACCATCCGTGTTCA
TCTTCCCTCCTAGCGACGAGCAGCTGAAGAGCGGCACCGCTAGCGTGGTGTGTCTGCT
GAATAACTTCTATCCCAGGGAGGCCAAGGTGCAGTGGAAGGTGGATAACGCCCTGCAG
AGCGGCAACTCCCAGGAGTCCGTGACCGAGCAGGACTCCAAGGACAGCACCTACTCC
CTGAGCTCCACCCTGACCCTGTCCAAGGCTGATTATGAGAAGCACAAGGTGTATGCTT
GCGAGGTGACACACCAGGGCCTGTCCAGCCCTGTGACCAAGAGCTTCAACCGGGGCG
AGTGC
Heavy chain:
   Humanized antibody heavy chain coding sequence: FS002-A1-HC (IgG1) (SEQ ID NO: 19), which is used to express the humanized heavy chain variable region + human heavy chain constant region 
CAGGTGCAGCTGGTGCAATCTGGGGCTGAGGTGAAGAAGCCTGGGGCCTCAGTGAAG
GTTTCCTGCAAGGCATCTGGATACACCTTCACCAGCTACTATATGCACTGGGTGCGACA
GGCCCCTGGACAAGGGCTTGAGTGGATGGGAATAATCAACCCTAGTGGTGGTAGCACA
 
AGCTACGCACAGAAGTTCCAGGGCAGAGTCACCATGACCAGGGACACGTCCACGAGC
ACAGTCTACATGGAGCTGAGCAGCCTGAGATCTGAGGACACGGCCGTGTATTACTGTG
CGAGAGATATTAGTGGGAGCTACCAAACTGATGCTTTTGATATCTGGGGCCAAGGGAC
AATGGTCACCGTCTCATCAGCTTCCACCAAGGGCCCCTCCGTGTTCCCCCTGGCTCCCT
CTTCCAAGAGCACCAGCGGCGGCACCGCTGCTCTGGGATGTCTGGTGAAGGACTACTT
CCCTGAGCCTGTGACCGTGTCCTGGAATTCCGGCGCCCTGACCTCCGGCGTGCACACA
TTCCCTGCTGTGCTGCAGTCCTCCGGCCTGTATAGCCTGTCCTCCGTGGTGACAGTGCC
TAGCTCCAGCCTGGGCACCCAGACCTATATCTGCAACGTGAACCACAAGCCTAGCAAT
ACCAAGGTGGACAAGAAGGTGGAGCCTAAGAGCTGCGACAAGACCCACACCTGTCCT
CCATGTCCTGCTCCAGAACTGCTCGGCGGACCTTCCGTGTTCCTGTTTCCTCCAAAGCC
TAAGGACACCCTGATGATCAGCAGAACCCCTGAAGTGACCTGCGTGGTGGTGGATGTG
TCCCACGAGGATCCCGAAGTGAAGTTCAATTGGTACGTGGACGGCGTGGAAGTGCAC
AACGCCAAGACCAAGCCTAGAGAGGAACAGTACAACAGCACCTACAGAGTGGTGTCC
GTGCTGACCGTGCTGCACCAGGATTGGCTGAACGGCAAAGAGTACAAGTGCAAGGTG
TCCAACAAGGCCCTGCCTGCTCCTATCGAGAAAACCATCAGCAAGGCCAAGGGCCAG
CCTAGGGAACCCCAGGTTTACACACTGCCTCCAAGCAGGGACGAGCTGACCAAGAAT
CAGGTGTCCCTGACCTGCCTGGTCAAGGGCTTCTACCCTTCCGATATCGCCGTGGAATG
GGAGAGCAATGGCCAGCCTGAGAACAACTACAAGACAACCCCTCCTGTGCTGGACAG
CGACGGCTCATTCTTCCTGTACAGCAAGCTGACAGTGGACAAGAGCAGATGGCAGCA
GGGCAACGTGTTCAGCTGCAGCGTGATGCACGAGGCCCTGCACAACCACTACACCCA
GAAGTCCCTGAGCCTGTCTCCTGGCAAA
   Humanized antibody heavy chain coding sequence: FS002-A5-HC (IgG1) (SEQ ID NO: 20), which is used to express the humanized heavy chain variable region + human heavy chain constant region 
CAGGTGCAGCTGCAGGACTCGGGCCCAGGACTGGTGAAGCCTTCACAGACCCTGTCC
CTCACCTGCACTGTCTCTGGTGGCTCCATCAGCAGTGGTGGTTACTACTGGAGCTGGAT
CCGCCAGCACCCAGGGAAGGGCCTGGAGTGGATTGGGTACATCTATTACAGTGGGAGC
ACCTACTACAACCCGTCCCTCAAGAGTCGAGTTACCATATCAGTAGACACGTCTAAGA
ACCAGTTCTCCCTGAAGCTGAGCTCTGTGACTGCCGCGGACACGGCCGTGTATTACTG
TGCGAGAGTGCCTGTCCCACTGGTGGGGGCTTTTGATATCTGGGGCCAAGGGACAATG
GTCACCGTCTCATCAGCTTCCACCAAGGGCCCCTCCGTGTTCCCCCTGGCTCCCTCTTC
CAAGAGCACCAGCGGCGGCACCGCTGCTCTGGGATGTCTGGTGAAGGACTACTTCCCT
GAGCCTGTGACCGTGTCCTGGAATTCCGGCGCCCTGACCTCCGGCGTGCACACATTCC
CTGCTGTGCTGCAGTCCTCCGGCCTGTATAGCCTGTCCTCCGTGGTGACAGTGCCTAGC
TCCAGCCTGGGCACCCAGACCTATATCTGCAACGTGAACCACAAGCCTAGCAATACCA
AGGTGGACAAGAAGGTGGAGCCTAAGAGCTGCGACAAGACCCACACCTGTCCTCCAT
GTCCTGCTCCAGAACTGCTCGGCGGACCTTCCGTGTTCCTGTTTCCTCCAAAGCCTAA
GGACACCCTGATGATCAGCAGAACCCCTGAAGTGACCTGCGTGGTGGTGGATGTGTCC
CACGAGGATCCCGAAGTGAAGTTCAATTGGTACGTGGACGGCGTGGAAGTGCACAAC
GCCAAGACCAAGCCTAGAGAGGAACAGTACAACAGCACCTACAGAGTGGTGTCCGTG
CTGACCGTGCTGCACCAGGATTGGCTGAACGGCAAAGAGTACAAGTGCAAGGTGTCC
AACAAGGCCCTGCCTGCTCCTATCGAGAAAACCATCAGCAAGGCCAAGGGCCAGCCT
AGGGAACCCCAGGTTTACACACTGCCTCCAAGCAGGGACGAGCTGACCAAGAATCAG
GTGTCCCTGACCTGCCTGGTCAAGGGCTTCTACCCTTCCGATATCGCCGTGGAATGGGA
GAGCAATGGCCAGCCTGAGAACAACTACAAGACAACCCCTCCTGTGCTGGACAGCGA
CGGCTCATTCTTCCTGTACAGCAAGCTGACAGTGGACAAGAGCAGATGGCAGCAGGG
CAACGTGTTCAGCTGCAGCGTGATGCACGAGGCCCTGCACAACCACTACACCCAGAA
GTCCCTGAGCCTGTCTCCTGGCAAA
   Humanized antibody heavy chain coding sequence: FS002-A15-HC (IgG1) (SEQ ID NO: 21), which is used to express the humanized heavy chain variable region + human heavy chain constant region 
CGGCTGCAGCTGCAGGAGTCGGGCCCAGGACTGGTGAAGCCTTCACAGACCCTGTCC
CTCACCTGCACTGTCTCTGGTGGCTCCATCAGCAGTGGTGGTTACTACTGGAGCTGGAT
 
CCGCCAGCACCCAGGGAAGGGCCTGGAGTGGATTGGGTACATCTATTACAGTGGGAGC
ACCTACTACAACCCGTCCCTCAAGAGTCGAGTTACCATATCAGTAGACACGTCTAAGA
ACCAGTTCTCCCTGAAGCTGAGCTCTGTGACTGCCGCGGACACGGCCGTGTATTACTG
TGCGAGAGCAACCATCTACGGTTACTTTGACTACTGGGGCCAGGGAACCCTGGTCACC
GTCTCATCAGCTTCCACCAAGGGCCCCTCCGTGTTCCCCCTGGCTCCCTCTTCCAAGAG
CACCAGCGGCGGCACCGCTGCTCTGGGATGTCTGGTGAAGGACTACTTCCCTGAGCCT
GTGACCGTGTCCTGGAATTCCGGCGCCCTGACCTCCGGCGTGCACACATTCCCTGCTG
TGCTGCAGTCCTCCGGCCTGTATAGCCTGTCCTCCGTGGTGACAGTGCCTAGCTCCAGC
CTGGGCACCCAGACCTATATCTGCAACGTGAACCACAAGCCTAGCAATACCAAGGTGG
ACAAGAAGGTGGAGCCTAAGAGCTGCGACAAGACCCACACCTGTCCTCCATGTCCTG
CTCCAGAACTGCTCGGCGGACCTTCCGTGTTCCTGTTTCCTCCAAAGCCTAAGGACAC
CCTGATGATCAGCAGAACCCCTGAAGTGACCTGCGTGGTGGTGGATGTGTCCCACGAG
GATCCCGAAGTGAAGTTCAATTGGTACGTGGACGGCGTGGAAGTGCACAACGCCAAG
ACCAAGCCTAGAGAGGAACAGTACAACAGCACCTACAGAGTGGTGTCCGTGCTGACC
GTGCTGCACCAGGATTGGCTGAACGGCAAAGAGTACAAGTGCAAGGTGTCCAACAAG
GCCCTGCCTGCTCCTATCGAGAAAACCATCAGCAAGGCCAAGGGCCAGCCTAGGGAA
CCCCAGGTTTACACACTGCCTCCAAGCAGGGACGAGCTGACCAAGAATCAGGTGTCC
CTGACCTGCCTGGTCAAGGGCTTCTACCCTTCCGATATCGCCGTGGAATGGGAGAGCA
ATGGCCAGCCTGAGAACAACTACAAGACAACCCCTCCTGTGCTGGACAGCGACGGCT
CATTCTTCCTGTACAGCAAGCTGACAGTGGACAAGAGCAGATGGCAGCAGGGCAACG
TGTTCAGCTGCAGCGTGATGCACGAGGCCCTGCACAACCACTACACCCAGAAGTCCCT
GAGCCTGTCTCCTGGCAAA
   Humanized antibody heavy chain coding sequence: FS002-36-HC (IgG1) (SEQ ID NO: 22), which is used to express the humanized heavy chain variable region + human heavy chain constant region 
CAGGTGCAGCTGCAGGAGTCGGGCCCAGGACTGGTGAAGCCTTCACAGACCCTGTCC
CTCACCTGCACTGTCTCTGGTGGCTCCATCAGCAGTGGTGGTTACTACTGGAGCTGGAT
CCGCCAGCACCCAGGGAAGGGCCTGGAGTGGATTGGGTACATCTATTACAGTGGGAGC
ACCTACTACAACCCGTCCCTCAAGAGTCGAGTTACCATATCAGTAGACACGTCTAAGA
ACCAGTTCTCCCTGAAGCTGAGCTCTGTGACTGCCGCGGACACGGCCGTGTACTACTG
TGCGAGAGTCCGCCTATTGGGTGGTGGTTTTGATATCTGGGGCCAAGGGACCACGGTC
ACCGTCTCATCAGCTTCCACCAAGGGCCCCTCCGTGTTCCCCCTGGCTCCCTCTTCCAA
GAGCACCAGCGGCGGCACCGCTGCTCTGGGATGTCTGGTGAAGGACTACTTCCCTGAG
CCTGTGACCGTGTCCTGGAATTCCGGCGCCCTGACCTCCGGCGTGCACACATTCCCTG
CTGTGCTGCAGTCCTCCGGCCTGTATAGCCTGTCCTCCGTGGTGACAGTGCCTAGCTCC
AGCCTGGGCACCCAGACCTATATCTGCAACGTGAACCACAAGCCTAGCAATACCAAGG
TGGACAAGAAGGTGGAGCCTAAGAGCTGCGACAAGACCCACACCTGTCCTCCATGTC
CTGCTCCAGAACTGCTCGGCGGACCTTCCGTGTTCCTGTTTCCTCCAAAGCCTAAGGA
CACCCTGATGATCAGCAGAACCCCTGAAGTGACCTGCGTGGTGGTGGATGTGTCCCAC
GAGGATCCCGAAGTGAAGTTCAATTGGTACGTGGACGGCGTGGAAGTGCACAACGCC
AAGACCAAGCCTAGAGAGGAACAGTACAACAGCACCTACAGAGTGGTGTCCGTGCTG
ACCGTGCTGCACCAGGATTGGCTGAACGGCAAAGAGTACAAGTGCAAGGTGTCCAAC
AAGGCCCTGCCTGCTCCTATCGAGAAAACCATCAGCAAGGCCAAGGGCCAGCCTAGG
GAACCCCAGGTTTACACACTGCCTCCAAGCAGGGACGAGCTGACCAAGAATCAGGTG
TCCCTGACCTGCCTGGTCAAGGGCTTCTACCCTTCCGATATCGCCGTGGAATGGGAGA
GCAATGGCCAGCCTGAGAACAACTACAAGACAACCCCTCCTGTGCTGGACAGCGACG
GCTCATTCTTCCTGTACAGCAAGCTGACAGTGGACAAGAGCAGATGGCAGCAGGGCA
ACGTGTTCAGCTGCAGCGTGATGCACGAGGCCCTGCACAACCACTACACCCAGAAGT
CCCTGAGCCTGTCTCCTGGCAAA
   Humanized antibody heavy chain coding sequence: FS002-A66-HC (IgG1) (SEQ ID NO: 23), which is used to express the humanized heavy chain variable region + human heavy chain constant region GAGGTACAACTGGTGGAGTCTGGGGGAGGCGTGGTCCAGCCTGGGAGGTCCCTGAGA 
CTCTCCTGTGCAGCCTCTGGATTCACCTTCAGTAGCTATGCTATGCACTGGGTCCGCCA
GGCTCCAGGCAAGGGGCTGGAGTGGGTGGCAGTTATATCATATGATGGAAGTAATAAAT
ACTACGTAGACTCCGTGAAGGGCCGATTCACCATCTCCAGAGACAATTCCAAGAACAC
GCTGTATCTGCAAATGAACAGCCTGAGAGCTGAGGACACGGCTGTGTATTACTGTGCG
AGAGATCGTATAACGTATCTTGACTACTACTACTACGGTATGGACGTCTGGGGCCAAGG
GACCACGGTCACCGTCTCATCAGCTTCCACCAAGGGCCCCTCCGTGTTCCCCCTGGCT
CCCTCTTCCAAGAGCACCAGCGGCGGCACCGCTGCTCTGGGATGTCTGGTGAAGGACT
ACTTCCCTGAGCCTGTGACCGTGTCCTGGAATTCCGGCGCCCTGACCTCCGGCGTGCA
CACATTCCCTGCTGTGCTGCAGTCCTCCGGCCTGTATAGCCTGTCCTCCGTGGTGACAG
TGCCTAGCTCCAGCCTGGGCACCCAGACCTATATCTGCAACGTGAACCACAAGCCTAG
CAATACCAAGGTGGACAAGAAGGTGGAGCCTAAGAGCTGCGACAAGACCCACACCTG
TCCTCCATGTCCTGCTCCAGAACTGCTCGGCGGACCTTCCGTGTTCCTGTTTCCTCCAA
AGCCTAAGGACACCCTGATGATCAGCAGAACCCCTGAAGTGACCTGCGTGGTGGTGGA
TGTGTCCCACGAGGATCCCGAAGTGAAGTTCAATTGGTACGTGGACGGCGTGGAAGTG
CACAACGCCAAGACCAAGCCTAGAGAGGAACAGTACAACAGCACCTACAGAGTGGTG
TCCGTGCTGACCGTGCTGCACCAGGATTGGCTGAACGGCAAAGAGTACAAGTGCAAG
GTGTCCAACAAGGCCCTGCCTGCTCCTATCGAGAAAACCATCAGCAAGGCCAAGGGC
CAGCCTAGGGAACCCCAGGTTTACACACTGCCTCCAAGCAGGGACGAGCTGACCAAG
AATCAGGTGTCCCTGACCTGCCTGGTCAAGGGCTTCTACCCTTCCGATATCGCCGTGGA
ATGGGAGAGCAATGGCCAGCCTGAGAACAACTACAAGACAACCCCTCCTGTGCTGGA
CAGCGACGGCTCATTCTTCCTGTACAGCAAGCTGACAGTGGACAAGAGCAGATGGCA
GCAGGGCAACGTGTTCAGCTGCAGCGTGATGCACGAGGCCCTGCACAACCACTACAC
CCAGAAGTCCCTGAGCCTGTCTCCTGGCAAA
   Humanized antibody heavy chain coding sequence: FS002-A10-HC (IgG1) (SEQ ID NO: 24), which is used to express the humanized heavy chain variable region + human heavy chain constant region 
CAGGTACAGCTGCAGCAGTCAGGTCCAGGACTGGTGAAGCCCTCGCAGACCCTCTCA
CTTACCTGTGCCATCTCCGGGGACAGTGTCTCTAGCGACAGTGCTGCTTGGAACTGGA
TCAGGCAGTCCCCATCGAGAGGCCTTGAGTGGCTGGGAAGGACATACTACAGGTCCA
AGTGGTATAATGATTATGCAGTATCTGTGAAAAGTCGAATAACCATCAACCCAGACACA
TCCAAGAACCAGTTCTCCCTGCAGCTAAATTCTGTGACTTCCGACGACACGGCTGTGT
ATTACTGTGCAAGAGGCTCTTGGGCGGCTCCATGGTTGTGGGGCCAGGGAACCCTGGT
CACCGTCTCATCAGCTTCCACCAAGGGCCCCTCCGTGTTCCCCCTGGCTCCCTCTTCCA
AGAGCACCAGCGGCGGCACCGCTGCTCTGGGATGTCTGGTGAAGGACTACTTCCCTGA
GCCTGTGACCGTGTCCTGGAATTCCGGCGCCCTGACCTCCGGCGTGCACACATTCCCT
GCTGTGCTGCAGTCCTCCGGCCTGTATAGCCTGTCCTCCGTGGTGACAGTGCCTAGCTC
CAGCCTGGGCACCCAGACCTATATCTGCAACGTGAACCACAAGCCTAGCAATACCAAG
GTGGACAAGAAGGTGGAGCCTAAGAGCTGCGACAAGACCCACACCTGTCCTCCATGT
CCTGCTCCAGAACTGCTCGGCGGACCTTCCGTGTTCCTGTTTCCTCCAAAGCCTAAGG
ACACCCTGATGATCAGCAGAACCCCTGAAGTGACCTGCGTGGTGGTGGATGTGTCCCA
CGAGGATCCCGAAGTGAAGTTCAATTGGTACGTGGACGGCGTGGAAGTGCACAACGC
CAAGACCAAGCCTAGAGAGGAACAGTACAACAGCACCTACAGAGTGGTGTCCGTGCT
GACCGTGCTGCACCAGGATTGGCTGAACGGCAAAGAGTACAAGTGCAAGGTGTCCAA
CAAGGCCCTGCCTGCTCCTATCGAGAAAACCATCAGCAAGGCCAAGGGCCAGCCTAG
GGAACCCCAGGTTTACACACTGCCTCCAAGCAGGGACGAGCTGACCAAGAATCAGGT
GTCCCTGACCTGCCTGGTCAAGGGCTTCTACCCTTCCGATATCGCCGTGGAATGGGAG
AGCAATGGCCAGCCTGAGAACAACTACAAGACAACCCCTCCTGTGCTGGACAGCGAC
GGCTCATTCTTCCTGTACAGCAAGCTGACAGTGGACAAGAGCAGATGGCAGCAGGGC
AACGTGTTCAGCTGCAGCGTGATGCACGAGGCCCTGCACAACCACTACACCCAGAAG
TCCCTGAGCCTGTCTCCTGGCAAA
   Humanized antibody heavy chain coding sequence: FS002-C9-HC (IgG1) (SEQ ID NO: 25), which is used to express the humanized heavy chain variable region + human heavy chain constant region 
CAGGTGCAGCTGTTGGAGTCTGGCCCAGGACTGGTGAAGCCTTCACAGACCGTGTCC
CTCACCTGCACTGTCTCTGGTGTCTCCATCAGCAGAGGTGTTTACTCCTGGAGCTGGAT
CCGCCAGCACCCAGGGAAGGGCCTCGAATGGATTGGGTTCATCGATTACAATGGGAGA
ACCGACTACAACCCGTCCCTCAAGAGTCGAGTTACCATATCACAAGACACGTCTAAGA
ACGACTTCTCCCTGAAGCTGACGTCTGTGACTGCCGCGGACACGGCCATATATTACTGT
GCGAGAGGTTTGCATGCTTTTGATATCTGGGGCCAAGGGACAATGGTCACCGTCTCATC
AGCTTCCACCAAGGGCCCCTCCGTGTTCCCCCTGGCTCCCTCTTCCAAGAGCACCAGC
GGCGGCACCGCTGCTCTGGGATGTCTGGTGAAGGACTACTTCCCTGAGCCTGTGACCG
TGTCCTGGAATTCCGGCGCCCTGACCTCCGGCGTGCACACATTCCCTGCTGTGCTGCA
GTCCTCCGGCCTGTATAGCCTGTCCTCCGTGGTGACAGTGCCTAGCTCCAGCCTGGGCA
CCCAGACCTATATCTGCAACGTGAACCACAAGCCTAGCAATACCAAGGTGGACAAGAA
GGTGGAGCCTAAGAGCTGCGACAAGACCCACACCTGTCCTCCATGTCCTGCTCCAGAA
CTGCTCGGCGGACCTTCCGTGTTCCTGTTTCCTCCAAAGCCTAAGGACACCCTGATGAT
CAGCAGAACCCCTGAAGTGACCTGCGTGGTGGTGGATGTGTCCCACGAGGATCCCGA
AGTGAAGTTCAATTGGTACGTGGACGGCGTGGAAGTGCACAACGCCAAGACCAAGCC
TAGAGAGGAACAGTACAACAGCACCTACAGAGTGGTGTCCGTGCTGACCGTGCTGCA
CCAGGATTGGCTGAACGGCAAAGAGTACAAGTGCAAGGTGTCCAACAAGGCCCTGCC
TGCTCCTATCGAGAAAACCATCAGCAAGGCCAAGGGCCAGCCTAGGGAACCCCAGGT
TTACACACTGCCTCCAAGCAGGGACGAGCTGACCAAGAATCAGGTGTCCCTGACCTGC
CTGGTCAAGGGCTTCTACCCTTCCGATATCGCCGTGGAATGGGAGAGCAATGGCCAGC
CTGAGAACAACTACAAGACAACCCCTCCTGTGCTGGACAGCGACGGCTCATTCTTCCT
GTACAGCAAGCTGACAGTGGACAAGAGCAGATGGCAGCAGGGCAACGTGTTCAGCTG
CAGCGTGATGCACGAGGCCCTGCACAACCACTACACCCAGAAGTCCCTGAGCCTGTCT
CCTGGCAAA
   Humanized antibody heavy chain coding sequence: FS002-C11-HC (IgG1) (SEQ ID NO: 26), which is used to express the humanized heavy chain variable region + human heavy chain constant region 
CGGCTGCAGCTGCAGGAGTCGGGCCCACGACTGGTGAAGCCTTCGGAGACCCTGTCC
CTCACCTGCAGCGTCTCTGGTGGCTCCATCAGCAGTAGGAATCACTACTGGGCCTGGA
TCCGCCAGCCCCCAGGGAAGGGCCTGGAGTGGATTGGGTACATCTATTACAGTGGGAG
CACCTACTACAACCCGTCCCTCAAGAGTCGAGTTACCATATCAGTAGACACGTCTAAGA
ACCAGTTCTCCCTGAAGCTGAGCTCTGTGACTGCCGCGGACACGGCCGTGTATTACTG
TGCGAGAGTAGGGCTGCTGCTCCGGGACTTTGACTACTGGGGCCAGGGAACCCTGGT
CACCGTCTCATCAGCTTCCACCAAGGGCCCCTCCGTGTTCCCCCTGGCTCCCTCTTCCA
AGAGCACCAGCGGCGGCACCGCTGCTCTGGGATGTCTGGTGAAGGACTACTTCCCTGA
GCCTGTGACCGTGTCCTGGAATTCCGGCGCCCTGACCTCCGGCGTGCACACATTCCCT
GCTGTGCTGCAGTCCTCCGGCCTGTATAGCCTGTCCTCCGTGGTGACAGTGCCTAGCTC
CAGCCTGGGCACCCAGACCTATATCTGCAACGTGAACCACAAGCCTAGCAATACCAAG
GTGGACAAGAAGGTGGAGCCTAAGAGCTGCGACAAGACCCACACCTGTCCTCCATGT
CCTGCTCCAGAACTGCTCGGCGGACCTTCCGTGTTCCTGTTTCCTCCAAAGCCTAAGG
ACACCCTGATGATCAGCAGAACCCCTGAAGTGACCTGCGTGGTGGTGGATGTGTCCCA
CGAGGATCCCGAAGTGAAGTTCAATTGGTACGTGGACGGCGTGGAAGTGCACAACGC
CAAGACCAAGCCTAGAGAGGAACAGTACAACAGCACCTACAGAGTGGTGTCCGTGCT
GACCGTGCTGCACCAGGATTGGCTGAACGGCAAAGAGTACAAGTGCAAGGTGTCCAA
CAAGGCCCTGCCTGCTCCTATCGAGAAAACCATCAGCAAGGCCAAGGGCCAGCCTAG
GGAACCCCAGGTTTACACACTGCCTCCAAGCAGGGACGAGCTGACCAAGAATCAGGT
GTCCCTGACCTGCCTGGTCAAGGGCTTCTACCCTTCCGATATCGCCGTGGAATGGGAG
AGCAATGGCCAGCCTGAGAACAACTACAAGACAACCCCTCCTGTGCTGGACAGCGAC
GGCTCATTCTTCCTGTACAGCAAGCTGACAGTGGACAAGAGCAGATGGCAGCAGGGC
AACGTGTTCAGCTGCAGCGTGATGCACGAGGCCCTGCACAACCACTACACCCAGAAG
TCCCTGAGCCTGTCTCCTGGCAAA
   Humanized antibody heavy chain coding sequence: FS002-C216-HC (IgG1) (SEQ ID NO: 27), which is used to express the humanized heavy chain variable region + human heavy chain constant region 
GAGGTGCAGCTGGTGGAGTCTGGGGGAGGCGTGGTCCAGCCTGGGAGGTCCCTGAGA
CTCTCCTGTGCAGCCTCTGGATTCACCTTCAGTAGCTATGCTATGCACTGGGTCCGCCA
GGCTCCAGGCAAGGGGCTGGAGTGGGTGGCAGTTATATCATATGATGGAAGTAATAAAT
ACTACGCAGACTCCGTGAAGGGCCGATTCACCATCTCCAGAGACAATTCCAAGAACAC
GCTGTATCTGCAAATGAACAGCCTGAGAGCTGAGGACACGGCTGTGTATTACTGTGCG
AGGGATTGTAGTGGTGGTAGCTGCTATGATGCTTTTGATATCTGGGGCCAAGGGACAAT
GGTCACCGTCTCATCAGCTTCCACCAAGGGCCCCTCCGTGTTCCCCCTGGCTCCCTCTT
CCAAGAGCACCAGCGGCGGCACCGCTGCTCTGGGATGTCTGGTGAAGGACTACTTCCC
TGAGCCTGTGACCGTGTCCTGGAATTCCGGCGCCCTGACCTCCGGCGTGCACACATTC
CCTGCTGTGCTGCAGTCCTCCGGCCTGTATAGCCTGTCCTCCGTGGTGACAGTGCCTAG
CTCCAGCCTGGGCACCCAGACCTATATCTGCAACGTGAACCACAAGCCTAGCAATACC
AAGGTGGACAAGAAGGTGGAGCCTAAGAGCTGCGACAAGACCCACACCTGTCCTCCA
TGTCCTGCTCCAGAACTGCTCGGCGGACCTTCCGTGTTCCTGTTTCCTCCAAAGCCTAA
GGACACCCTGATGATCAGCAGAACCCCTGAAGTGACCTGCGTGGTGGTGGATGTGTCC
CACGAGGATCCCGAAGTGAAGTTCAATTGGTACGTGGACGGCGTGGAAGTGCACAAC
GCCAAGACCAAGCCTAGAGAGGAACAGTACAACAGCACCTACAGAGTGGTGTCCGTG
CTGACCGTGCTGCACCAGGATTGGCTGAACGGCAAAGAGTACAAGTGCAAGGTGTCC
AACAAGGCCCTGCCTGCTCCTATCGAGAAAACCATCAGCAAGGCCAAGGGCCAGCCT
AGGGAACCCCAGGTTTACACACTGCCTCCAAGCAGGGACGAGCTGACCAAGAATCAG
GTGTCCCTGACCTGCCTGGTCAAGGGCTTCTACCCTTCCGATATCGCCGTGGAATGGGA
GAGCAATGGCCAGCCTGAGAACAACTACAAGACAACCCCTCCTGTGCTGGACAGCGA
CGGCTCATTCTTCCTGTACAGCAAGCTGACAGTGGACAAGAGCAGATGGCAGCAGGG
CAACGTGTTCAGCTGCAGCGTGATGCACGAGGCCCTGCACAACCACTACACCCAGAA
GTCCCTGAGCCTGTCTCCTGGCAAA
   Humanized antibody heavy chain coding sequence: FS002-C375-HC (IgG1) (SEQ ID NO: 28), which is used to express the humanized heavy chain variable region + human heavy chain constant region 
CAGGTGCAGCTACAGGAGTCGGGCCCAGGACTGGTGAAGCCTTCACAGACCCTGTCC
CTCACCTGCACTGTCTCTGGTGGCTCCATCAGCAGTGGTGGTTACTACTGGAGCTGGAT
CCGCCAGCACCCAGGGAAGGGCCTGGAGTGGATTGGGTACATCTATTACAGTGGGAGC
ACCTACTACAACCCGTCCCTCAAGAGTCGAGTTACCATATCAGTAGACACGTCTAAGA
ACCAGTTCTCCCTGAAGCTGAGCTCTGTGACTGCCGCGGACACGGCCGTGTATTACTG
TGCGAGAGTTGGAGTAGCCCTTCACTACGGTATGGACGTCTGGGGCCAAGGGACCACG
GTCACCGTCTCATCAGCTTCCACCAAGGGCCCCTCCGTGTTCCCCCTGGCTCCCTCTTC
CAAGAGCACCAGCGGCGGCACCGCTGCTCTGGGATGTCTGGTGAAGGACTACTTCCCT
GAGCCTGTGACCGTGTCCTGGAATTCCGGCGCCCTGACCTCCGGCGTGCACACATTCC
CTGCTGTGCTGCAGTCCTCCGGCCTGTATAGCCTGTCCTCCGTGGTGACAGTGCCTAGC
TCCAGCCTGGGCACCCAGACCTATATCTGCAACGTGAACCACAAGCCTAGCAATACCA
AGGTGGACAAGAAGGTGGAGCCTAAGAGCTGCGACAAGACCCACACCTGTCCTCCAT
GTCCTGCTCCAGAACTGCTCGGCGGACCTTCCGTGTTCCTGTTTCCTCCAAAGCCTAA
GGACACCCTGATGATCAGCAGAACCCCTGAAGTGACCTGCGTGGTGGTGGATGTGTCC
CACGAGGATCCCGAAGTGAAGTTCAATTGGTACGTGGACGGCGTGGAAGTGCACAAC
GCCAAGACCAAGCCTAGAGAGGAACAGTACAACAGCACCTACAGAGTGGTGTCCGTG
CTGACCGTGCTGCACCAGGATTGGCTGAACGGCAAAGAGTACAAGTGCAAGGTGTCC
AACAAGGCCCTGCCTGCTCCTATCGAGAAAACCATCAGCAAGGCCAAGGGCCAGCCT
AGGGAACCCCAGGTTTACACACTGCCTCCAAGCAGGGACGAGCTGACCAAGAATCAG
GTGTCCCTGACCTGCCTGGTCAAGGGCTTCTACCCTTCCGATATCGCCGTGGAATGGGA
GAGCAATGGCCAGCCTGAGAACAACTACAAGACAACCCCTCCTGTGCTGGACAGCGA
CGGCTCATTCTTCCTGTACAGCAAGCTGACAGTGGACAAGAGCAGATGGCAGCAGGG
CAACGTGTTCAGCTGCAGCGTGATGCACGAGGCCCTGCACAACCACTACACCCAGAA
GTCCCTGAGCCTGTCTCCTGGCAAA
   Humanized antibody heavy chain coding sequence: FS002-C417-HC (IgG1) (SEQ ID NO: 29), which is used to express the humanized heavy chain variable region + human heavy chain constant region 
GAGGTGCAGCTGGTGGAGTCTGGGGGAGTCGTGGTACAGCCTGGGGGGTCCCTGAGA
CTCTCCTGTGCAGCCTCTGGATTCACCTTTGATGATTATACCATGCACTGGGTCCGTCAA
GCTCCGGGGAAGGGTCTGGAGTGGGTCTCTCTTATTAGTTGGGATGGTGGTAGCACAT
ACTATGCAGACTCTGTGAAGGGCCGATTCACCATCTCCAGAGACAACGCCAAGAACAC
GCTGTATCTGCAAATGAACAGTCTGAGAGCCGAGGACACGGCTGTGTATTACTGTGCA
AGAGATACGGGGTACGCTATGGATGTCTGGGGCCAAGGGACAATGGTCACCGTCTCAT
CAGCTTCCACCAAGGGCCCCTCCGTGTTCCCCCTGGCTCCCTCTTCCAAGAGCACCAG
CGGCGGCACCGCTGCTCTGGGATGTCTGGTGAAGGACTACTTCCCTGAGCCTGTGACC
GTGTCCTGGAATTCCGGCGCCCTGACCTCCGGCGTGCACACATTCCCTGCTGTGCTGC
AGTCCTCCGGCCTGTATAGCCTGTCCTCCGTGGTGACAGTGCCTAGCTCCAGCCTGGGC
ACCCAGACCTATATCTGCAACGTGAACCACAAGCCTAGCAATACCAAGGTGGACAAGA
AGGTGGAGCCTAAGAGCTGCGACAAGACCCACACCTGTCCTCCATGTCCTGCTCCAGA
ACTGCTCGGCGGACCTTCCGTGTTCCTGTTTCCTCCAAAGCCTAAGGACACCCTGATGA
TCAGCAGAACCCCTGAAGTGACCTGCGTGGTGGTGGATGTGTCCCACGAGGATCCCG
AAGTGAAGTTCAATTGGTACGTGGACGGCGTGGAAGTGCACAACGCCAAGACCAAGC
CTAGAGAGGAACAGTACAACAGCACCTACAGAGTGGTGTCCGTGCTGACCGTGCTGC
ACCAGGATTGGCTGAACGGCAAAGAGTACAAGTGCAAGGTGTCCAACAAGGCCCTGC
CTGCTCCTATCGAGAAAACCATCAGCAAGGCCAAGGGCCAGCCTAGGGAACCCCAGG
TTTACACACTGCCTCCAAGCAGGGACGAGCTGACCAAGAATCAGGTGTCCCTGACCTG
CCTGGTCAAGGGCTTCTACCCTTCCGATATCGCCGTGGAATGGGAGAGCAATGGCCAG
CCTGAGAACAACTACAAGACAACCCCTCCTGTGCTGGACAGCGACGGCTCATTCTTCC
TGTACAGCAAGCTGACAGTGGACAAGAGCAGATGGCAGCAGGGCAACGTGTTCAGCT
GCAGCGTGATGCACGAGGCCCTGCACAACCACTACACCCAGAAGTCCCTGAGCCTGT
CTCCTGGCAAA
   Humanized antibody heavy chain coding sequence: FS002-B27-HC (IgG1) (SEQ ID NO: 30), which is used to express the humanized heavy chain variable region + human heavy chain constant region 
CAGGTCCAGCTGGTGCAGTCTGGGGCTGAGGTGAAGAAGCCTGGGTCCTCGGTGAAG
GTCTCCTGCAAGGCTTCTGGAGGCACCTTCAGCAGCTATGCTATCAGCTGGGTGCGAC
AGGCCCCTGGACAAGGGCTTGAGTGGATGGGAAGGATCATCCCTATCCTTGGTATAGC
AAACTACGCACAGAAGTTCCAGGGCAGAGTCACGATTACCGCGGACAAATCCACGAG
CACAGCCTACATGGAGCTGAGCAGCCTGAGATCTGAGGACACGGCCGTGTATTACTGT
GCGAGAGCGGAGGGCAGCAGCTGGTACTACTTTGACTACTGGGGCCAGGGAACCCTG
GTCACCGTCTCATCAGCTTCCACCAAGGGCCCCTCCGTGTTCCCCCTGGCTCCCTCTTC
CAAGAGCACCAGCGGCGGCACCGCTGCTCTGGGATGTCTGGTGAAGGACTACTTCCCT
GAGCCTGTGACCGTGTCCTGGAATTCCGGCGCCCTGACCTCCGGCGTGCACACATTCC
CTGCTGTGCTGCAGTCCTCCGGCCTGTATAGCCTGTCCTCCGTGGTGACAGTGCCTAGC
TCCAGCCTGGGCACCCAGACCTATATCTGCAACGTGAACCACAAGCCTAGCAATACCA
AGGTGGACAAGAAGGTGGAGCCTAAGAGCTGCGACAAGACCCACACCTGTCCTCCAT
GTCCTGCTCCAGAACTGCTCGGCGGACCTTCCGTGTTCCTGTTTCCTCCAAAGCCTAA
GGACACCCTGATGATCAGCAGAACCCCTGAAGTGACCTGCGTGGTGGTGGATGTGTCC
CACGAGGATCCCGAAGTGAAGTTCAATTGGTACGTGGACGGCGTGGAAGTGCACAAC
GCCAAGACCAAGCCTAGAGAGGAACAGTACAACAGCACCTACAGAGTGGTGTCCGTG
CTGACCGTGCTGCACCAGGATTGGCTGAACGGCAAAGAGTACAAGTGCAAGGTGTCC
AACAAGGCCCTGCCTGCTCCTATCGAGAAAACCATCAGCAAGGCCAAGGGCCAGCCT
AGGGAACCCCAGGTTTACACACTGCCTCCAAGCAGGGACGAGCTGACCAAGAATCAG
GTGTCCCTGACCTGCCTGGTCAAGGGCTTCTACCCTTCCGATATCGCCGTGGAATGGGA
GAGCAATGGCCAGCCTGAGAACAACTACAAGACAACCCCTCCTGTGCTGGACAGCGA
CGGCTCATTCTTCCTGTACAGCAAGCTGACAGTGGACAAGAGCAGATGGCAGCAGGG
CAACGTGTTCAGCTGCAGCGTGATGCACGAGGCCCTGCACAACCACTACACCCAGAA
GTCCCTGAGCCTGTCTCCTGGCAAA
   Humanized antibody heavy chain coding sequence: FS002-C330-HC (IgG1) (SEQ ID NO: 31), which is used to express the humanized heavy chain variable region + human heavy chain constant region 
CAGCTGCAGCTGCAGGAGTCGGGCCCAGGACTGGTGAAGCCTTCGGGGACCCTGTCC
CTCACCTGCGCTGTCTCTGGTGGCTCCATCAGCAGTAGTAACTGGTGGAGTTGGGTCC
GCCAGCCCCCAGGGAAGGGGCTGGAGTGGATTGGGGAAATCTATCATAGTGGGAGCA
CCAACTACAACCCGTCCCTCAAGAGTCGAGTCACCATATCAGTAGACAAGTCCAAGAA
CCAGTTCTCCCTGAAGCTGAGCTCTGTGACCGCCGCGGACACGGCCGTGTATTACTGT
GCGAGAGCCCACTCCCGATATTACTATGGCCTTGACTACTGGGGCCAGGGAACCCTGG
TCACCGTCTCATCAGCTTCCACCAAGGGCCCCTCCGTGTTCCCCCTGGCTCCCTCTTCC
AAGAGCACCAGCGGCGGCACCGCTGCTCTGGGATGTCTGGTGAAGGACTACTTCCCTG
AGCCTGTGACCGTGTCCTGGAATTCCGGCGCCCTGACCTCCGGCGTGCACACATTCCC
TGCTGTGCTGCAGTCCTCCGGCCTGTATAGCCTGTCCTCCGTGGTGACAGTGCCTAGCT
CCAGCCTGGGCACCCAGACCTATATCTGCAACGTGAACCACAAGCCTAGCAATACCAA
GGTGGACAAGAAGGTGGAGCCTAAGAGCTGCGACAAGACCCACACCTGTCCTCCATG
TCCTGCTCCAGAACTGCTCGGCGGACCTTCCGTGTTCCTGTTTCCTCCAAAGCCTAAG
GACACCCTGATGATCAGCAGAACCCCTGAAGTGACCTGCGTGGTGGTGGATGTGTCCC
ACGAGGATCCCGAAGTGAAGTTCAATTGGTACGTGGACGGCGTGGAAGTGCACAACG
CCAAGACCAAGCCTAGAGAGGAACAGTACAACAGCACCTACAGAGTGGTGTCCGTGC
TGACCGTGCTGCACCAGGATTGGCTGAACGGCAAAGAGTACAAGTGCAAGGTGTCCA
ACAAGGCCCTGCCTGCTCCTATCGAGAAAACCATCAGCAAGGCCAAGGGCCAGCCTA
GGGAACCCCAGGTTTACACACTGCCTCCAAGCAGGGACGAGCTGACCAAGAATCAGG
TGTCCCTGACCTGCCTGGTCAAGGGCTTCTACCCTTCCGATATCGCCGTGGAATGGGAG
AGCAATGGCCAGCCTGAGAACAACTACAAGACAACCCCTCCTGTGCTGGACAGCGAC
GGCTCATTCTTCCTGTACAGCAAGCTGACAGTGGACAAGAGCAGATGGCAGCAGGGC
AACGTGTTCAGCTGCAGCGTGATGCACGAGGCCCTGCACAACCACTACACCCAGAAG
TCCCTGAGCCTGTCTCCTGGCAAA
   Humanized antibody heavy chain coding sequence: FS002-C153-HC (IgG1) (SEQ ID NO: 32), which is used to express the humanized heavy chain variable region + human heavy chain constant region 
CAGGTGCAGCTACAACAGTGGGGGGCAGAGCTTGTGAAGCCAGGGGCCTCAGTCAAG
TTGTCCTGCACAGCTTCTGGCTTCAACATTAAAGACACCTATATGCACTGGGTGAAGCA
GAGGCCTGAACAGGGCCTGGAGTGGATTGGAAGGATTGATCCTGCGAATGGTAATACT
AAATATGACCCGAAGTTCCAGGGCAAGGCCACTATAACAGCAGACACATCCTCCAACA
CAGCCTACCTGCAGCTCAGCAGCCTGACATCTGAGGACACTGCCGTCTATTACTGTGCT
AGATCCTACTATAGGTACGACGGGTACTTCGATGTCTGGGGCGCAGGGACAATGGTCA
CCGTCTCATCAGCTTCCACCAAGGGCCCCTCCGTGTTCCCCCTGGCTCCCTCTTCCAAG
AGCACCAGCGGCGGCACCGCTGCTCTGGGATGTCTGGTGAAGGACTACTTCCCTGAGC
CTGTGACCGTGTCCTGGAATTCCGGCGCCCTGACCTCCGGCGTGCACACATTCCCTGC
TGTGCTGCAGTCCTCCGGCCTGTATAGCCTGTCCTCCGTGGTGACAGTGCCTAGCTCCA
GCCTGGGCACCCAGACCTATATCTGCAACGTGAACCACAAGCCTAGCAATACCAAGGT
GGACAAGAAGGTGGAGCCTAAGAGCTGCGACAAGACCCACACCTGTCCTCCATGTCC
TGCTCCAGAACTGCTCGGCGGACCTTCCGTGTTCCTGTTTCCTCCAAAGCCTAAGGAC
ACCCTGATGATCAGCAGAACCCCTGAAGTGACCTGCGTGGTGGTGGATGTGTCCCACG
AGGATCCCGAAGTGAAGTTCAATTGGTACGTGGACGGCGTGGAAGTGCACAACGCCA
AGACCAAGCCTAGAGAGGAACAGTACAACAGCACCTACAGAGTGGTGTCCGTGCTGA
CCGTGCTGCACCAGGATTGGCTGAACGGCAAAGAGTACAAGTGCAAGGTGTCCAACA
AGGCCCTGCCTGCTCCTATCGAGAAAACCATCAGCAAGGCCAAGGGCCAGCCTAGGG
AACCCCAGGTTTACACACTGCCTCCAAGCAGGGACGAGCTGACCAAGAATCAGGTGT
CCCTGACCTGCCTGGTCAAGGGCTTCTACCCTTCCGATATCGCCGTGGAATGGGAGAG
CAATGGCCAGCCTGAGAACAACTACAAGACAACCCCTCCTGTGCTGGACAGCGACGG
CTCATTCTTCCTGTACAGCAAGCTGACAGTGGACAAGAGCAGATGGCAGCAGGGCAA
CGTGTTCAGCTGCAGCGTGATGCACGAGGCCCTGCACAACCACTACACCCAGAAGTCC
CTGAGCCTGTCTCCTGGCAAA
   Humanized antibody heavy chain coding sequence: FS002-C241-HC (IgG1) (SEQ ID NO: 33), which is used to express the humanized heavy chain variable region + human heavy chain constant region 
CGGCTGCAGCTGCAGGAGTCGGGCCCAGGACTGGTGAAGCCTTCGGAGACCCTGTCC
CTCACCTGCACTGTCTCTGGTGGCTCCATCAGCAGTAGTAGTTACTACTGGGGCTGGAT
CCGCCAGCCCCCAGGGAAGGGGCTGGAGTGGATTGGGAGTATCTATTATAGTGGGAGC
ACCTACTACAACCCGTCCCTCAAGAGTCGAGTCACCATATCCGTAGACACGTCCAAGA
ACCAGTTCTCCCTGAAGCTGAGCTCTGTGACCGCCGCAGACACGGCTGTGTATTACTG
TGCGAGACGGTACAGCTATGGTAGAGATGCTTTTGATATCTGGGGCCAAGGGACAATG
GTCACCGTCTCATCAGCTTCCACCAAGGGCCCCTCCGTGTTCCCCCTGGCTCCCTCTTC
CAAGAGCACCAGCGGCGGCACCGCTGCTCTGGGATGTCTGGTGAAGGACTACTTCCCT
GAGCCTGTGACCGTGTCCTGGAATTCCGGCGCCCTGACCTCCGGCGTGCACACATTCC
CTGCTGTGCTGCAGTCCTCCGGCCTGTATAGCCTGTCCTCCGTGGTGACAGTGCCTAGC
TCCAGCCTGGGCACCCAGACCTATATCTGCAACGTGAACCACAAGCCTAGCAATACCA
AGGTGGACAAGAAGGTGGAGCCTAAGAGCTGCGACAAGACCCACACCTGTCCTCCAT
GTCCTGCTCCAGAACTGCTCGGCGGACCTTCCGTGTTCCTGTTTCCTCCAAAGCCTAA
GGACACCCTGATGATCAGCAGAACCCCTGAAGTGACCTGCGTGGTGGTGGATGTGTCC
CACGAGGATCCCGAAGTGAAGTTCAATTGGTACGTGGACGGCGTGGAAGTGCACAAC
GCCAAGACCAAGCCTAGAGAGGAACAGTACAACAGCACCTACAGAGTGGTGTCCGTG
CTGACCGTGCTGCACCAGGATTGGCTGAACGGCAAAGAGTACAAGTGCAAGGTGTCC
AACAAGGCCCTGCCTGCTCCTATCGAGAAAACCATCAGCAAGGCCAAGGGCCAGCCT
AGGGAACCCCAGGTTTACACACTGCCTCCAAGCAGGGACGAGCTGACCAAGAATCAG
GTGTCCCTGACCTGCCTGGTCAAGGGCTTCTACCCTTCCGATATCGCCGTGGAATGGGA
GAGCAATGGCCAGCCTGAGAACAACTACAAGACAACCCCTCCTGTGCTGGACAGCGA
CGGCTCATTCTTCCTGTACAGCAAGCTGACAGTGGACAAGAGCAGATGGCAGCAGGG
CAACGTGTTCAGCTGCAGCGTGATGCACGAGGCCCTGCACAACCACTACACCCAGAA
GTCCCTGAGCCTGTCTCCTGGCAAA
   Humanized antibody heavy chain coding sequence: FS002-C15-HC (IgG1) (SEQ ID NO: 34), which is used to express the humanized heavy chain variable region + human heavy chain constant region 
CAGCTGCAGCTGCAGGAGTCGGGCCCAGGACTGGTGAAGCCTTCGGAGACCCTGTCC
CTCACCTGCACTGTCTCTGGTGGCTCCATCAGCAGTAGTAGTTACTACTGGGGCTGGAT
CCGCCAGCCCCCAGGGAAGGGGCTGGAGTGGATTGGGAGTATCTATTATAGTGGGAGC
ACCTACTACAACCCGTCCCTCAAGAGTCGAGTCACCATATCCGTAGACACGTCCAAGA
ACCAGTTCTCCCTGAAGCTGAGCTCTGTGACCGCCGCAGACACGGCTGTGTATTACTG
TGCGAGACTGACGATTTTTGGAGTGGTGCTCTACTGGGGCCAGGGAACCCTGGTCACC
GTCTCATCAGCTTCCACCAAGGGCCCCTCCGTGTTCCCCCTGGCTCCCTCTTCCAAGAG
CACCAGCGGCGGCACCGCTGCTCTGGGATGTCTGGTGAAGGACTACTTCCCTGAGCCT
GTGACCGTGTCCTGGAATTCCGGCGCCCTGACCTCCGGCGTGCACACATTCCCTGCTG
TGCTGCAGTCCTCCGGCCTGTATAGCCTGTCCTCCGTGGTGACAGTGCCTAGCTCCAGC
CTGGGCACCCAGACCTATATCTGCAACGTGAACCACAAGCCTAGCAATACCAAGGTGG
ACAAGAAGGTGGAGCCTAAGAGCTGCGACAAGACCCACACCTGTCCTCCATGTCCTG
CTCCAGAACTGCTCGGCGGACCTTCCGTGTTCCTGTTTCCTCCAAAGCCTAAGGACAC
CCTGATGATCAGCAGAACCCCTGAAGTGACCTGCGTGGTGGTGGATGTGTCCCACGAG
GATCCCGAAGTGAAGTTCAATTGGTACGTGGACGGCGTGGAAGTGCACAACGCCAAG
ACCAAGCCTAGAGAGGAACAGTACAACAGCACCTACAGAGTGGTGTCCGTGCTGACC
GTGCTGCACCAGGATTGGCTGAACGGCAAAGAGTACAAGTGCAAGGTGTCCAACAAG
GCCCTGCCTGCTCCTATCGAGAAAACCATCAGCAAGGCCAAGGGCCAGCCTAGGGAA
CCCCAGGTTTACACACTGCCTCCAAGCAGGGACGAGCTGACCAAGAATCAGGTGTCC
CTGACCTGCCTGGTCAAGGGCTTCTACCCTTCCGATATCGCCGTGGAATGGGAGAGCA
ATGGCCAGCCTGAGAACAACTACAAGACAACCCCTCCTGTGCTGGACAGCGACGGCT
CATTCTTCCTGTACAGCAAGCTGACAGTGGACAAGAGCAGATGGCAGCAGGGCAACG
TGTTCAGCTGCAGCGTGATGCACGAGGCCCTGCACAACCACTACACCCAGAAGTCCCT
GAGCCTGTCTCCTGGCAAA
   Humanized antibody heavy chain coding sequence: FS002-C58-HC (IgG1) (SEQ ID NO: 35), which is used to express the humanized heavy chain variable region + human heavy chain constant region 
GAGGTGCAGCTGTTGGAGTCTGGGGGAGGCGTGGTCCAGCCTGGGAGGTCCCTGAGA
CTCTCCTGTGCAGCCTCTGGATTCACCTTCAGTAGCTATGCTATGCACTGGGTCCGCCA
GGCTCCAGGCAAGGGGCTGGAGTGGGTGGCAGTTATATCATATGATGGAAGTAATAAAT
ACTACGCAGACTCCGTGAAGGGCCGATTCACCATCTCCAGAGACAATTCCAAGAACAC
GCTGTATCTGCAAATGAACAGCCTGAGAGCTGAGGACACGGCTGTGTATTACTGTGCG
AGAGGAAAGTACAACTGGTTCGACCCCTGGGGCCAGGGCACCCTGGTCACCGTCTCA
TCAGCTTCCACCAAGGGCCCCTCCGTGTTCCCCCTGGCTCCCTCTTCCAAGAGCACCA
GCGGCGGCACCGCTGCTCTGGGATGTCTGGTGAAGGACTACTTCCCTGAGCCTGTGAC
CGTGTCCTGGAATTCCGGCGCCCTGACCTCCGGCGTGCACACATTCCCTGCTGTGCTG
CAGTCCTCCGGCCTGTATAGCCTGTCCTCCGTGGTGACAGTGCCTAGCTCCAGCCTGGG
CACCCAGACCTATATCTGCAACGTGAACCACAAGCCTAGCAATACCAAGGTGGACAAG
AAGGTGGAGCCTAAGAGCTGCGACAAGACCCACACCTGTCCTCCATGTCCTGCTCCAG
AACTGCTCGGCGGACCTTCCGTGTTCCTGTTTCCTCCAAAGCCTAAGGACACCCTGAT
GATCAGCAGAACCCCTGAAGTGACCTGCGTGGTGGTGGATGTGTCCCACGAGGATCCC
GAAGTGAAGTTCAATTGGTACGTGGACGGCGTGGAAGTGCACAACGCCAAGACCAAG
CCTAGAGAGGAACAGTACAACAGCACCTACAGAGTGGTGTCCGTGCTGACCGTGCTG
CACCAGGATTGGCTGAACGGCAAAGAGTACAAGTGCAAGGTGTCCAACAAGGCCCTG
CCTGCTCCTATCGAGAAAACCATCAGCAAGGCCAAGGGCCAGCCTAGGGAACCCCAG
GTTTACACACTGCCTCCAAGCAGGGACGAGCTGACCAAGAATCAGGTGTCCCTGACCT
GCCTGGTCAAGGGCTTCTACCCTTCCGATATCGCCGTGGAATGGGAGAGCAATGGCCA
GCCTGAGAACAACTACAAGACAACCCCTCCTGTGCTGGACAGCGACGGCTCATTCTTC
CTGTACAGCAAGCTGACAGTGGACAAGAGCAGATGGCAGCAGGGCAACGTGTTCAGC
TGCAGCGTGATGCACGAGGCCCTGCACAACCACTACACCCAGAAGTCCCTGAGCCTGT
CTCCTGGCAAA
   Humanized antibody heavy chain coding sequence: FS002-A72-HC (IgG1) (SEQ ID NO: 36), which is used to express the humanized heavy chain variable region + human heavy chain constant region

CAGGTGCAGCTGGTGGACTCTGGGGGGGGCTTGGTACAGCCTGGAGGGTCCCTGAGA
CTCTCCTGTGTAGCCTCTGGATTCGCCTTCAGTAATTATGAGATGAACTGGGTCCGCCA
GGCTCCAGGGAAGGGGCTGGAGTGGGTTTCATACATTAGTAGTAGTAGTAGTTACACA
AACTACGCAGACTCTGTGAAGGGCCGATTCACCATCTCCAGAGACAACGCCAAGAAC
TCACTGTATCTGCAAATGAACAGCCTGAGAGCCGAGGACACGGCCGTGTATTACTGTG
CGAGAGCCCAGGTTCGGGATGCTTTTGATATCTGGGGCCAAGGGACCACGGTCACCGT
CTCATCAGCTTCCACCAAGGGCCCCTCCGTGTTCCCCCTGGCTCCCTCTTCCAAGAGC
ACCAGCGGCGGCACCGCTGCTCTGGGATGTCTGGTGAAGGACTACTTCCCTGAGCCTG
TGACCGTGTCCTGGAATTCCGGCGCCCTGACCTCCGGCGTGCACACATTCCCTGCTGT
GCTGCAGTCCTCCGGCCTGTATAGCCTGTCCTCCGTGGTGACAGTGCCTAGCTCCAGCC
TGGGCACCCAGACCTATATCTGCAACGTGAACCACAAGCCTAGCAATACCAAGGTGGA
CAAGAAGGTGGAGCCTAAGAGCTGCGACAAGACCCACACCTGTCCTCCATGTCCTGCT
CCAGAACTGCTCGGCGGACCTTCCGTGTTCCTGTTTCCTCCAAAGCCTAAGGACACCC
TGATGATCAGCAGAACCCCTGAAGTGACCTGCGTGGTGGTGGATGTGTCCCACGAGGA
TCCCGAAGTGAAGTTCAATTGGTACGTGGACGGCGTGGAAGTGCACAACGCCAAGAC
CAAGCCTAGAGAGGAACAGTACAACAGCACCTACAGAGTGGTGTCCGTGCTGACCGT
GCTGCACCAGGATTGGCTGAACGGCAAAGAGTACAAGTGCAAGGTGTCCAACAAGGC
CCTGCCTGCTCCTATCGAGAAAACCATCAGCAAGGCCAAGGGCCAGCCTAGGGAACC
CCAGGTTTACACACTGCCTCCAAGCAGGGACGAGCTGACCAAGAATCAGGTGTCCCT
GACCTGCCTGGTCAAGGGCTTCTACCCTTCCGATATCGCCGTGGAATGGGAGAGCAAT
GGCCAGCCTGAGAACAACTACAAGACAACCCCTCCTGTGCTGGACAGCGACGGCTCA
TTCTTCCTGTACAGCAAGCTGACAGTGGACAAGAGCAGATGGCAGCAGGGCAACGTG
TTCAGCTGCAGCGTGATGCACGAGGCCCTGCACAACCACTACACCCAGAAGTCCCTGA
GCCTGTCTCCTGGCAAA

The amino acid sequences of the light chain variable region and the heavy chain variable region in the antibody expressed by the above vectors are as follows:
Light chain variable region (amino acid sequence):
FS002-A1-LC (Kappa) light chain variable region (SEQ ID NO:37)

DIQLTQSPSSLSASVGDRVTITCRASQSIISYLNWYQQKPGKAPKLLIYAASSLQSGVPSRFS
GSGSGTDFTLTISSLQPEDFATYYCQQSYSTPDTFGQGTKLEIK

The italic underlined parts respectively indicate VL-CDR1 (SEQ ID NO:38: ***RASQSIISYL**N*), VL-CDR2 (SEQ ID NO:39: ***AASSLQS***), VL-CDR3 (SEQ ID NO:40: ***QQSYSTPDT***)*.*
FS002-A5-LC (Kappa) light chain variable region (SEQ ID NO:41)

EIVLTQSPATLSLSPGERATLSCRASQSVSSNLGWYQQKPGQAPSLLIYGASNRATGIPARFS
GSGSGTDFTLTISSLEPEDFAVYYCQQRDTWPLTFGGGTKVEIK

The italic underlined parts respectively indicate VL-CDR1 (SEQ ID NO:42: ***RASQSVSSNLG***), VL-CDR2 (SEQ ID NO:43: ***GASNRAT***), VL-CDR3 (SEQ ID NO:44: ***QQRDTWPLT***)*.*
FS002-A15-LC (Kappa) light chain variable region (SEQ ID NO:45)

EIVLTQSPATLPLSPGERATLSCRASQSVGFYLAWYQQKPGQPPRLLFYDASKRATGIPARFS
ASGSGTDFTLTISGLEPEDFAVYYCQHRISWPLTFGQGTKVEVK

The italic underlined parts respectively indicate VL-CDR1 (SEQ ID NO:46: ***RASQSVGFYLA***), VL-CDR2(SEQ ID NO:47: ***DASKRAT*)*,*** VL-CDR3(SEQ ID NO:48: ***OHRISWPLT*)*.***
FS002-A36-LC (Kappa) light chain variable region (SEQ ID NO:49)

EIVLTQSPATLSLSPGERATLSCRASQSVSTYLAWYQQKPGQAPRLLIYDTSIRATGIPARFS
GSGSGTDFTLTISSLEPEDFAVYYCQQRSNWPLTFGGGTKVEIK

The italic underlined parts respectively indicate VL-CDR1 (SEQ ID NO:50: ***RASQSVSTYLA***), VL-CDR2(SEQ ID NO:51: ***DTSIRAT*),** VL-CDR3(SEQ ID NO:52: ***QQRSNWPLT***).
FS002-A66-LC (Kappa) light chain variable region (SEQ ID NO:53)

EIVLTQSPAILSLSPGERATLSCRASQSVSEYLAWYQQKPGQAPRLLIYDISNRATGIPARFS
GSGSETDFTLTISSLEPEDFAVYYCQQRRTWPLTFGPGTKVDIK

The italic underlined parts respectively indicate VL-CDR1 (SEQ ID NO:54: ***RASQSVSEYLA***), VL-CDR2(SEQ ID NO:55: ***DISNRAT*),** VL-CDR3(SEQ ID NO:56: ***QQRRTWPLT***).
FS002-A10-LC (Kappa-2) light chain variable region (SEQ ID NO:57)

NIQLTQSPPFLSASVGDRVTITCRASQDISSFLAWYQQKPGKAPNLLIHAATTLQSGVPSRF
SGSGSGTEFTLTISSLRPEDFATYYCQQLHTYPITFGQGTRLEIK

The italic underlined parts respectively indicate VL-CDR1 (SEQ ID NO:58: ***RASQDISSFLA***), VL-CDR2(SEQ ID NO:59: ***AATTLQS***), VL-CDR3(SEQ ID NO:60: ***QQLHTYPIT***).
FS002-C9-LC (Kappa-2) light chain variable region (SEQ ID NO:61)

NIQMTQSPPSLSASVGDRVAITCRASQDISNSLAWYQQKSGQPPKLLLYDVSKLERGVPSR
FSGSGSRTDYTLTISSLQPEDFATYYCQQYSSDPTFGQGSKLEFK

The italic underlined parts respectively indicate VL-CDR1 (SEQ ID NO:62: ***RASQDISNSLA***), VL-CDR2(SEQ ID NO:63: ***DVSKLER*),** VL-CDR3(SEQ ID NO:64: ***QQYSSDPT***).
FS002-C11-LC (Kappa-2) light chain variable region (SEQ ID NO:65)

DIQMIQSPSSVSASVGDRVTITC*RASQGISNSLA*WYQHKPGTTPKLLIY*AASSLQG*GVPSRF
SGSSSETDFTLTISSLQPEDFATYYC*QQANSFPIT*FGQGTRLDIK

The italic underlined parts respectively indicate VL-CDR1 (SEQ ID NO:66: ***RASQGISNSLA***), VL-CDR2(SEQ ID NO:67: ***AASSLQG***), VL-CDR3(SEQ ID NO:68: ***QQANSFPIT***).
FS002-C216-LC (Kappa-2) light chain variable region (SEQ ID NO:69)

EIVLTQSPATLSLSPGERATLSCRASQSVSSYLAWYQQKPGQAPRLLIYDASNRATGIPARFS

GSGSGTDFTLTISSLEPEDFAVYYCQQRTNWLMYTFGQGTKLEIK

The italic underlined parts respectively indicate VL-CDR1 (SEQ ID NO:70: ***RASQSVSSYLA***), VL-CDR2(SEQ ID NO:71: *DASNRAT*), VL-CDR3(SEQ ID NO:72: ***QQRTNWLMYT***).
FS002-C375-LC (Kappa-2) light chain variable region (SEQ ID NO:73)

EIVLTQSPATLSLSPGERATLSCRASQSVSSYLAWYQQKPGQAPRLLIYDASNRATGIPARFS
GSGSGTDFTLTISSLEPEDFAVYYCQQRSNWPLTFGGGTKVEIK

The italic underlined parts respectively indicate VL-CDR1 (SEQ ID NO:74: ***RASQSVSSYLA***), VL-CDR2(SEQ ID NO:75: ***DASNRAT*),** VL-CDR3(SEQ ID NO:76: ***OORSNWPLT*).**
FS002-C417-LC (Kappa-2) light chain variable region (SEQ ID NO:77)

NIQMTQSPSSLSASVGDRVTITCRASQSISSYLNWYQQKPGKAPKLLIYAASSLQSGVPSRF
SGSGSGTDFTLTISSLQPEDFASYYCQQSYSSWAFGQGTKVEIK

The italic underlined parts respectively indicate VL-CDR1 (SEQ ID NO:78: ***RASQSISSYLN***), VL-CDR2(SEQ ID NO:79: ***AASSLQS***), VL-CDR3(SEQ ID NO80: ***QQSYSSWA*).**
FS002-B27-LC (Kappa-2) light chain variable region (SEQ ID NO:81)

NIQLTQSPSSVSASVGDRVTITC**RASQGFSRWLA**WYQQKPGKAPKLLIY**GASSLQS**GVPSR
FSGSGSGTDFTLTISSLQPEDFATYYC**QQANSFPLT**FGGGTKLEIK

The italic underlined parts respectively indicate VL-CDR1 (SEQ ID NO:82: ***RASQGFSRWLA***), VL-CDR2(SEQ ID NO:83: ***GASSLQS***), VL-CDR3(SEQ ID NO:84: ***QQANSFPLT***).
FS002-C330-LC (Kappa-2) light chain variable region (SEQ ID NO:85)

NIQLTQSPSSLSASVGDRVTITCRASQSISSYLNWYQQKPGKAPKLLIYAASSLQSGVPSRFS
GSGSGTDFTLTISSLQPEDFATYYCQQSYSTPDTFGPGTKVDIK

The italic underlined parts respectively indicate VL-CDR1 (SEQ ID NO:86: ***RASQSISSYLN***), VL-CDR2(SEQ ID NO:87: ***AASSLOS*),** VL-CDR3(SEQ ID NO:88: ***QQSYSTPDT***).
FS002-C153-LC(LambdaC2)-2 light chain variable region (SEQ ID NO:89)

QSALTQPASVSGSPGQSITISCTGTSSDVGGYNYVSWYQQHPGKAPKLMIYDVSNRPSGVS
NRFSGSKSGNTASLTISGLQAEDEADYYCSSFTTITTRVFGGGTKLTVL

The italic underlined parts respectively indicate VL-CDR1 (SEQ ID NO:90: ***TGTSSDVGGYNYVS***), VL-CDR2(SEQ ID NO:91: ***DVSNRPS*),** VL-CDR3(SEQ ID NO:92: ***SSFTTITTRV***).
FS002-C241-LC(LambdaC2)-2 light chain variable region (SEQ ID NO:93)

QSALTQPASVSGSPGQSITISCSGTSSDIGRYNYVSWYQQHPGKAPKLLIFDVSNRPSGVSN
RFSGSKSGNTASLTISGLQAEDEADYYCSSYTSTVPYVFGTGTRVAVQ

The italic underlined parts respectively indicate VL-CDR1 (SEQ ID NO:94: ***SGTSSDIGRYNYVS*),** VL-CDR2(SEQ ID NO:95: ***DVSNRPS*),** VL-CDR3(SEQ ID NO:96: ***SSYTSTVPYV***).
FS002-C15-LC (Kappa-2) light chain variable region (SEQ ID NO:97)

EIVLTQSPATLSLSPGERATLSCQASQTFSDYLAWYQQKPGQAPRLLIYDASTRATGIPARFS
GSGSGTDFTLTISSLEPEDFAVYYCQQRSHWPPGYTFGQGTRVEIK

The italic underlined parts respectively indicate VL-CDR1 (SEQ ID NO:98: ***QASQTFSDYLA***), VL-CDR2(SEQ ID NO:99: ***DASTRAT***), VL-CDR3(SEQ ID NO:100: ***QQRSHWPPGYT***).
FS002-C58-LC (Kappa-2) light chain variable region (SEQ ID NO: 101)

EIVLTQSPATLSLSPGERATLSCRASQSVYSSLAWYQQKPGQAPKLLIYDASNRATGVPARF
SGSGSGTDFTLTISSLEPEDFALYYCQHRQHWPLTFGGGTKVEIK

The italic underlined parts respectively indicate VL-CDR1 (SEQ ID NO:102: ***RASQSVYSSLA***), VL-CDR2(SEQ ID NO:103: ***DASNRAT***), VL-CDR3(SEQ ID NO:104: *QHRQHWPLT*).
FS002-A72-LC (Kappa-2) light chain variable region (SEQ ID NO:105)

EIVMTQSPVTLSVSPGDSATLSCRASQSVDKNLAWYQQKTGQAPRLLIYGAATRATGIPAR
FSGSGSGTEFTLTISSLRSEDFAVYYCQQYRNWPWTFGLGTKVEIK

The italic underlined parts respectively indicate VL-CDR1 (SEQ ID NO:106: ***RASQSVDKNLA***), VL-CDR2(SEQ ID NO:107: ***GAATRAT***), VL-CDR3(SEQ ID NO:108: ***QQYRNWPWT***).
Light chain constant region (amino acid sequence):
   Kappa light chain constant region sequence (SEQ ID NO:109)
   Lambda light chain constant region sequence (SEQ ID NO:110) 
GQPKAAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS
Heavy chain variable region (amino acid sequence):
   FS002-A1-HC (IgG1) heavy chain variable region (SEQ ID NO:111)

QVQLVQSGAEVKKPGASVKVSCKASGYTFTSYYMHWVRQAPGQGLEWMGIINPSGGST
SYAQKFQGRVTMTRDTSTSTVYMELSSLRSEDTAVYYCARDISGSYQTDAFDIWGQGTM
VTVSS

The italic underlined parts respectively indicate VH-CDR1 (SEQ ID NO:112: ***GYTFTSYYMH***), VH-CDR2(SEQ ID NO:113: ***IINPSGGSTS*),** VH-CDR3(SEQ ID NO:114: ***DISGSYQTDAFDI***)*.*
FS002-A5-HC (IgG1) heavy chain variable region (SEQ ID NO:115)

QVQLQDSGPGLVKPSQTLSLTCTVSGGSISSGGYYWSWIRQHPGKGLEWIGYIYYSGSTYY
NPSLKSRVTISVDTSKNQFSLKLSSVTAADTAVYYCARVPVPLVGAFDIWGQGTMVTVSS

The italic underlined parts respectively indicate VH-CDR1 (SEQ ID NO:116: ***GGSISSGGYYWS),*** VH-CDR2(SEQ ID NO:117: ***YIYYSGSTY***), VH-CDR3(SEQ ID NO:118: ***VPVPLVGAFDI***).
FS002-A15-HC (IgG1) heavy chain variable region (SEQ ID NO:119)

RLQLQESGPGLVKPSQTLSLTCTVSGGSISSGGYYWSWIRQHPGKGLEWIGYIYYSGSTYYN
PSLKSRVTISVDTSKNQFSLKLSSVTAADTAVYYCARATIYGYFDYWGQGTLVTVSS

The italic underlined parts respectively indicate VH-CDR1 (SEQ ID NO:120: ***GGSISSGGYYWS),*** VH-CDR2(SEQ ID NO:121: ***YIYYSGSTY),*** VH-CDR3(SEQ ID NO:122: ***ATIYGYFDY*)*.***
FS002-A36-HC (IgG1) heavy chain variable region (SEQ ID NO:123)

QVQLQESGPGLVKPSQTLSLTCTVSGGSISSGGYYWSWIRQHPGKGLEWIGYIYYSGSTYY
NPSLKSRVTISVDTSKNQFSLKLSSVTAADTAVYYCARVRLLGGGFDIWGQGTTVTVSS

The italic underlined parts respectively indicate VH-CDR1 (SEQ ID NO:124: *GGSISSGGYYWS),* VH-CDR2(SEQ ID NO:125: ***YIYYSGSTY),*** VH-CDR3(SEQ ID NO:126: ***VRLLGGGFDI***).
FS002-A66-HC (IgG1) heavy chain variable region (SEQ ID NO:127)

EVQLVESGGGVVQPGRSLRLSCAAS**GFTFSSYAMH**WVRQAPGKGLEWVA**VISYDGSNKY**
YVDSVKGRFTISRDNSKNTLYLQMNSLRAEDTAVYYCAR**DRITYLDYYYYGMDV**WGQGT
TVTVSS

The italic underlined parts respectively indicate VH-CDR1 (SEQ ID NO:128: ***GFTFSSYAMH***), VH-CDR2(SEQ ID NO:129: ***VISYDGSNKY*),** VH-CDR3(SEQ ID NO:130: ***DRITYLDYYYYGMDV***).
FS002-A10-HC (IgG1) heavy chain variable region (SEQ ID NO:131)

QVQLQQSGPGLVKPSQTLSLTCAISGDSVSSDSAAWNWIRQSPSRGLEWLGRTYYRSKWYN
DYAVSVKSRITINPDTSKNQFSLQLNSVTSDDTAVYYCARGSWAAPWLWGQGTLVTVSS

The italic underlined parts respectively indicate VH-CDR1 (SEQ ID NO:132: ***GDSVSSDSAAWN***), VH-CDR2(SEQ ID NO:133: ***RTYYRSKWYND*),** VH-CDR3(SEQ ID NO:134: ***GSWAAPWL*).**
FS002-C9-HC (IgG1) heavy chain variable region (SEQ ID NO:135)

QVQLLESGPGLVKPSQTVSLTCTVSGVSISRGVYSWSWIRQHPGKGLEWIGFIDYNGRTDY
NPSLKSRVTISQDTSKNDFSLKLTSVTAADTAIYYCARGLHAFDIWGQGTMVTVSS

The italic underlined parts respectively indicate VH-CDR1 (SEQ ID NO:136: ***GVSISRGVYSWS***), VH-CDR2(SEQ ID NO:137: ***FIDYNGRTD*),** VH-CDR3(SEQ ID NO:138: ***GLHAFDI***).
FS002-C11-HC (IgG1) heavy chain variable region (SEQ ID NO: 139)

RLQLQESGPRLVKPSETLSLTCSVSGGSISSRNHYWAWIRQPPGKGLEWIGYIYYSGSTYYN
PSLKSRVTISVDTSKNQFSLKLSSVTAADTAVYYCARVGLLLRDFDYWGQGTLVTVSS

The italic underlined parts respectively indicate VH-CDR1 (SEQ ID NO:140: ***GGSISSRNHYWA*),** VH-CDR2(SEQ ID NO:141: ***YIYYSGSTY),*** VH-CDR3(SEQ ID NO:142: ***VGLLLRDFDY***).
FS002-C216-HC (IgG1) heavy chain variable region (SEQ ID NO:143)

EVQLVESGGGVVQPGRSLRLSCAASGFTFSSYAMHWVRQAPGKGLEWVAVISYDGSNKY
YADSVKGRFTISRDNSKNTLYLQMNSLRAEDTAVYYCARDCSGGSCYDAFDIWGQGTMV
TVSS

The italic underlined parts respectively indicate VH-CDR1 (SEQ ID NO:144: ***GFTFSSYAMH***), VH-CDR2(SEQ ID NO:145: ***VISYDGSNKY*),** VH-CDR3(SEQ ID NO:146: ***DCSGGSCYDAFDI***).
FS002-C375-HC (IgG1) heavy chain variable region (SEQ ID NO: 147)

QVQLQESGPGLVKPSQTLSLTCTVSGGSISSGGYYWSWIRQHPGKGLEWIGYIYYSGSTYY
NPSLKSRVTISVDTSKNQFSLKLSSVTAADTAVYYCARVGVALHYGMDVWGQGTTVTVSS

The italic underlined parts respectively indicate VH-CDR1 (SEQ ID NO:148: ***GGSISSGGYYWS*),** VH-CDR2(SEQ ID NO:149: ***YIYYSGSTY),*** VH-CDR3(SEQ ID NO:150: ***VGTVALHYGMDV***).
FS002-C417-HC (IgG1) heavy chain variable region (SEQ ID NO: 151)

EVQLVESGGVVVQPGGSLRLSCAASGFTFDDYTMHWVRQAPGKGLEWVSLISWDGGST
YYADSVKGRFTISRDNAKNTLYLQMNSLRAEDTAVYYCARDTGYAMDVWGQGTMVTVS
S

The italic underlined parts respectively indicate VH-CDR1 (SEQ ID NO:152: ***GFTFDDYTMH***), VH-CDR2(SEQ ID NO: 153: ***LISWDGGSTY*),** VH-CDR3(SEQ ID NO: 154: ***DTGYAMDV***).
FS002-B27-HC (IgG1) heavy chain variable region (SEQ ID NO: 155)

QVQLVQSGAEVKKPGSSVKVSCKASGGTFSSYAISWVRQAPGQGLEWMGRIIPILGIANY
AQKFQGRVTITADKSTSTAYMELSSLRSEDTAVYYCARAEGSSWYYFDYWGQGTLVTVSS

The italic underlined parts respectively indicate VH-CDR1 (SEQ ID NO:156: ***GGTFSSYAIS***), VH-CDR2(SEQ ID NO:157: ***RIIPILGIAN***), VH-CDR3(SEQ ID NO:158: ***AEGSSWYYFDY*).**
FS002-C330-HC (IgG1) heavy chain variable region (SEQ ID NO: 159)

QLQLQESGPGLVKPSGTLSLTCAVSGGSISSSNWWSWVRQPPGKGLEWIGEIYHSGSTNYN
PSLKSRVTISVDKSKNQFSLKLSSVTAADTAVYYCARAHSRYYYGLDYWGQGTLVTVSS

The italic underlined parts respectively indicate VH-CDR1 (SEQ ID NO:160: ***GGSISSSNWWS*),** VH-CDR2(SEQ ID NO:161: ***EIYHSGSTN***), VH-CDR3(SEQ ID NO:162: ***AHSRYYYGLDY***).
FS002-C153-HC (IgG1) heavy chain variable region (SEQ ID NO: 163)

QVQLQQWGAELVKPGASVKLSCTASGFNIKDTYMHWVKQRPEQGLEWIGRIDPANGNT
KYDPKFQGKATITADTSSNTAYLQLSSLTSEDTAVYYCARSYYRYDGYFDVWGAGTMVTV
SS

The italic underlined parts respectively indicate VH-CDR1 (SEQ ID NO:164: ***GFNIKDTYMH***), VH-CDR2(SEQ ID NO:165: ***RIDPANGNTK*),** VH-CDR3(SEQ ID NO:166: ***SYYRYDGYFDV***).
FS002-C241-HC (IgG1) heavy chain variable region (SEQ ID NO: 167)

RLQLQESGPGLVKPSETLSLTCTVSGGSISSSSYYWGWIRQPPGKGLEWIGSIYYSGSTYYNP
SLKSRVTISVDTSKNQFSLKLSSVTAADTAVYYCARRYSYGRDAFDIWGQGTMVTVSS

The italic underlined parts respectively indicate VH-CDR1 (SEQ ID NO: 168: ***GGSISSSSYYWG*),** VH-CDR2 (SEQ ID NO:169: ***SIYYSGSTY*)*,*** VH-CDR3(SEQ ID NO:170: ***RYSYGRDAFDI***).
FS002-C15-HC (IgG1) heavy chain variable region (SEQ ID NO:171)

QLQLQESGPGLVKPSETLSLTCTVSGGSISSSSYYWGWIRQPPGKGLEWIGSIYYSGSTYYNP
SLKSRVTISVDTSKNQFSLKLSSVTAADTAVYYCARLTIFGVVLYWGQGTLVTVSS

The italic underlined parts respectively indicate VH-CDR1 (SEQ ID NO: 172: ***GGSISSSSYYWG*),** VH-CDR2 (SEQ ID NO:173: ***SIYYSGSTY*),** VH-CDR3 (SEQ ID NO:174: ***LTIFGVVLY***).
FS002-C58-HC (IgG1) heavy chain variable region (SEQ ID NO:175)

EVQLLESGGGVVQPGRSLRLSCAASGFTFSSYAMHWVRQAPGKGLEWVAVISYDGSNKY
YADSVKGRFTISRDNSKNTLYLQMNSLRAEDTAVYYCARGKYNWFDPWGQGTLVTVSS

The italic underlined parts respectively indicate VH-CDR1 (SEQ ID NO:176: ***GFTFSSYAMH***), VH-CDR2 (SEQ ID NO:177: ***VISYDGSNKY*),** VH-CDR3 (SEQ ID NO:178: ***GKYNWFDP*).**
FS002-A72-HC (IgG1) heavy chain variable region (SEQ ID NO:179)

QVQLVDSGGGLVQPGGSLRLSCVASGFAFSNYEMNWVRQAPGKGLEWVSYISSSSSYTNY
ADSVKGRFTISRDNAKNSLYLQMNSLRAEDTAVYYCARAQVRDAFDIWGQGTTVTVSS

The italic underlined parts respectively indicate VH-CDR1 (SEQ ID NO:180: ***GFAFSNYEMN***), VH-CDR2 (SEQ ID NO:181: ***YISSSSSYTN***), VH-CDR3 (SEQ ID NO:182: ***AQVRDAFDI***).
IgG1 heavy chain constant region (SEQ ID NO: 183)

The full-length amino acid sequences of the light chain of the antibody are as follows (the shaded parts correspond to the constant region sequences):
FS002-A1-LC (Kappa) light chain (SEQ ID NO:184) DIQLTQSPSSLSASVGDRVTITCRASQSIISYLNWYQQKPGKAPKLLIYAASSLQSGVPSRFS 
GSGSGTDFTLTISSLQPEDFATYYCQQSYSTPDTFGQGTKLEIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC
FS002-A5-LC (Kappa) light chain (SEQ ID NO:185) 
EIVLTQSPATLSLSPGERATLSCRASQSVSSNLGWYQQKPGQAPSLLIYGASNRATGIPARFS
GSGSGTDFTLTISSLEPEDFAVYYCQQRDTWPLTFGGGTKVEIKRTVAAPSVFIFPPSDEQL
KSGTASVVCLLNNFYPREAKVQWKVDNALQSGNSQESVTEQDSKDSTYSLSSTLTLSKA
DYEKHKVYACEVTHQGLSSPVTKSFNRGEC
FS002-A15-LC (Kappa) light chain (SEQ ID NO:186) 
EIVLTQSPATLPLSPGERATLSCRASQSVGFYLAWYQQKPGQPPRLLFYDASKRATGIPARFS
ASGSGTDFTLTISGLEPEDFAVYYCQHRISWPLTFGQGTKVEVKRTVAAPSVFIFPPSDEQL
KSGTASVVCLLNNFYPREAKVQWKVDNALQSGNSQESVTEQDSKDSTYSLSSTLTLSKA
DYEKHKVYACEVTHQGLSSPVTKSFNRGEC
FS002-A36-LC (Kappa) light chain (SEQ ID NO: 187) 
EIVLTQSPATLSLSPGERATLSCRASQSVSTYLAWYQQKPGQAPRLLIYDTSIRATGIPARFS
GSGSGTDFTLTISSLEPEDFAVYYCQQRSNWPLTFGGGTKVEIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC
FS002-A66-LC (Kappa) light chain (SEQ ID NO:188) 
EIVLTQSPAILSLSPGERATLSCRASQSVSEYLAWYQQKPGQAPRLLIYDISNRATGIPARFS
GSGSETDFTLTISSLEPEDFAVYYCQQRRTWPLTFGPGTKVDIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC
FS002-A10-LC (Kappa-2) light chain (SEQ ID NO:189) 
NIQLTQSPPFLSASVGDRVTITCRASQDISSFLAWYQQKPGKAPNLLIHAATTLQSGVPSRF
SGSGSGTEFTLTISSLRPEDFATYYCQQLHTYPITFGQGTRLEIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC
FS002-C9-LC (Kappa-2) light chain (SEQ ID NO:190) 
NIQMTQSPPSLSASVGDRVAITCRASQDISNSLAWYQQKSGQPPKLLLYDVSKLERGVPSR
FSGSGSRTDYTLTISSLQPEDFATYYCQQYSSDPTFGQGSKLEFKRTVAAPSVFIFPPSDEQL
KSGTASVVCLLNNFYPREAKVQWKVDNALQSGNSQESVTEQDSKDSTYSLSSTLTLSKA
DYEKHKVYACEVTHQGLSSPVTKSFNRGEC
FS002-C11-LC (Kappa-2) light chain (SEQ ID NO:191) 
DIQMIQSPSSVSASVGDRVTITC RASQGISNSLA WYQHKPGTTPKLLIY AASSLQG GVPSRF
SGSSSETDFTLTISSLQPEDFATYYC QQANSFPIT FGQGTRLDIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC
FS002-C216-LC (Kappa-2) light chain (SEQ ID NO:192) 
EIVLTQSPATLSLSPGERATLSCRASQSVSSYLAWYQQKPGQAPRLLIYDASNRATGIPARFS
GSGSGTDFTLTISSLEPEDFAVYYCQQRTNWLMYTFGQGTKLEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC
FS002-C375-LC (Kappa-2) light chain (SEQ ID NO:193) 
EIVLTQSPATLSLSPGERATLSCRASQSVSSYLAWYQQKPGQAPRLLIYDASNRATGIPARFS
GSGSGTDFTLTISSLEPEDFAVYYCQQRSNWPLTFGGGTKVEIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC
FS002-C417-LC (Kappa-2) light chain (SEQ ID NO:194) 
NIQMTQSPSSLSASVGDRVTITCRASQSISSYLNWYQQKPGKAPKLLIYAASSLQSGVPSRF
SGSGSGTDFTLTISSLQPEDFASYYCQQSYSSWAFGQGTKVEIKRTVAAPSVFIFPPSDEQLK
 
SGTASVVCLLNNFYPREAKVQWKVDNALQSGNSQESVTEQDSKDSTYSLSSTLTLSKAD
YEKHKVYACEVTHQGLSSPVTKSFNRGEC
FS002-B27-LC (Kappa-2) light chain (SEQ ID NO:195) 
NIQLTQSPSSVSASVGDRVTITCRASQGFSRWLAWYQQKPGKAPKLLIYGASSLQSGVPSR
FSGSGSGTDFTLTISSLQPEDFATYYCQQANSFPLTFGGGTKLEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC
FS002-C330-LC (Kappa-2) light chain (SEQ ID NO:196) 
NIQLTQSPSSLSASVGDRVTITCRASQSISSYLNWYQQKPGKAPKLLIYAASSLQSGVPSRFS
GSGSGTDFTLTISSLQPEDFATYYCQQSYSTPDTFGPGTKVDIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC
FS002-C153-LC (LambdaC2)-2 light chain (SEQ ID NO: 197) 
QSALTQPASVSGSPGQSITISCTGTSSDVGGYNYVSWYQQHPGKAPKLMIYDVSNRPSGVS
NRFSGSKSGNTASLTISGLQAEDEADYYCSSFTTITTRVFGGGTKLTVLGQPKAAPSVTLFP
PSSEELQANKATLVCLISDFYPGAVTVAWKADSSPVKAGVETTTPSKQSNNKYAASSYLSL
TPEQWKSHRSYSCQVTHEGSTVEKTVAPTECS
FS002-C241-LC(LambdaC2)-2 light chain (SEQ ID NO:198) 
QSALTQPASVSGSPGQSITISCSGTSSDIGRYNYVSWYQQHPGKAPKLLIFDVSNRPSGVSN
RFSGSKSGNTASLTISGLQAEDEADYYCSSYTSTVPYVFGTGTRVAVQGQPKAAPSVTLFPP
SSEELQANKATLVCLISDFYPGAVTVAWKADSSPVKAGVETTTPSKQSNNKYAASSYLSLT
PEQWKSHRSYSCQVTHEGSTVEKTVAPTECS
FS002-C15-LC (Kappa-2) light chain (SEQ ID NO:199) 
EIVLTQSPATLSLSPGERATLSCQASQTFSDYLAWYQQKPGQAPRLLIYDASTRATGIPARFS
GSGSGTDFTLTISSLEPEDFAVYYCQQRSHWPPGYTFGQGTRVEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC
FS002-C58-LC (Kappa-2) light chain (SEQ ID NO:200) 
EIVLTQSPATLSLSPGERATLSCRASQSVYSSLAWYQQKPGQAPKLLIYDASNRATGVPARF
SGSGSGTDFTLTISSLEPEDFALYYCQHRQHWPLTFGGGTKVEIKRTVAAPSVFIFPPSDEQL
KSGTASVVCLLNNFYPREAKVQWKVDNALQSGNSQESVTEQDSKDSTYSLSSTLTLSKA
DYEKHKVYACEVTHQGLSSPVTKSFNRGEC
FS002-A72-LC (Kappa-2) light chain (SEQ ID NO:201)

EIVMTQSPVTLSVSPGDSATLSCRASQSVDKNLAWYQQKTGQAPRLLIYGAATRATGIPAR
FSGSGSGTEFTLTISSLRSEDFAVYYCQQYRNWPWTFGLGTKVEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC

The full-length amino acid sequences of the heavy chain of the antibody are as follows (the shaded parts correspond to the constant region sequences):
FS002-A1-HC (IgG1) heavy chain (SEQ ID NO:202) 
QVQLVQSGAEVKKPGASVKVSCKASGYTFTSYYMHWVRQAPGQGLEWMGIINPSGGST
SYAQKFQGRVTMTRDTSTSTVYMELSSLRSEDTAVYYCARDISGSYQTDAFDIWGQGTM
VTVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSKLTVDKS
RWQQGNVFSCSVMHEALHNHYTQKSLSLSPGK
FS002-A5-HC (IgG1) heavy chain (SEQ ID NO:203) QVQLQDSGPGLVKPSQTLSLTCTVSGGSISSGGYYWSWIRQHPGKGLEWIGYIYYSGSTYY 
NPSLKSRVTISVDTSKNQFSLKLSSVTAADTAVYYCARVPVPLVGAFDIWGQGTMVTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGK
FS002-A15-HC (IgG1) heavy chain (SEQ ID NO:204) 
RLQLQESGPGLVKPSQTLSLTCTVSGGSISSGGYYWSWIRQHPGKGLEWIGYIYYSGSTYYN
PSLKSRVTISVDTSKNQFSLKLSSVTAADTAVYYCARATIYGYFDYWGQGTLVTVSSASTK
GPSVFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFS
CSVMHEALHNHYTQKSLSLSPGK
FS002-A36-HC (IgG1) heavy chain (SEQ ID NO:205) 
QVQLQESGPGLVKPSQTLSLTCTVSGGSISSGGYYWSWIRQHPGKGLEWIGYIYYSGSTYY
NPSLKSRVTISVDTSKNQFSLKLSSVTAADTAVYYCARVRLLGGGFDIWGQGTTVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQG
NVFSCSVMHEALHNHYTQKSLSLSPGK
FS002-A66-HC (IgG1) heavy chain (SEQ ID NO:206) 
EVQLVESGGGVVQPGRSLRLSCAASGFTFSSYAMHWVRQAPGKGLEWVAVISYDGSNKY
YVDSVKGRFTISRDNSKNTLYLQMNSLRAEDTAVYYCARDRITYLDYYYYGMDVWGQGT
TVTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYSKLTVDK
SRWQQGNVFSCSVMHEALHNHYTQKSLSLSPGK
FS002-A10-HC (IgG1) heavy chain (SEQ ID NO:207) 
QVQLQQSGPGLVKPSQTLSLTCAISGDSVSSDSAAWNWIRQSPSRGLEWLGRTYYRSKWYN
DYAVSVKSRITINPDTSKNQFSLQLNSVTSDDTAVYYCARGSWAAPWLWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRWQQ
GNVFSCSVMHEALHNHYTQKSLSLSPGK
FS002-C9-HC (IgG1) heavy chain (SEQ ID NO:208) 
QVQLLESGPGLVKPSQTVSLTCTVSGVSISRGVYSWSWIRQHPGKGLEWIGFIDYNGRTDY
NPSLKSRVTISQDTSKNDFSLKLTSVTAADTAIYYCARGLHAFDIWGQGTMVTVSSASTK
GPSVFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFS
CSVMHEALHNHYTQKSLSLSPGK
FS002-C11-HC (IgG1) heavy chain (SEQ ID NO:209) 
RLQLQESGPRLVKPSETLSLTCSVSGGSISSRNHYWAWIRQPPGKGLEWIGYIYYSGSTYYN
PSLKSRVTISVDTSKNQFSLKLSSVTAADTAVYYCARVGLLLRDFDYWGQGTLVTVSSAST
KGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGN
VFSCSVMHEALHNHYTQKSLSLSPGK
FS002-C216-HC (IgG1) heavy chain (SEQ ID NO:210) 
EVQLVESGGGVVQPGRSLRLSCAASGFTFSSYAMHWVRQAPGKGLEWVAVISYDGSNKY
YADSVKGRFTISRDNSKNTLYLQMNSLRAEDTAVYYCARDCSGGSCYDAFDIWGQGTMV
TVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSKLTVDKS
RWQQGNVFSCSVMHEALHNHYTQKSLSLSPGK
FS002-C375-HC (IgG1) heavy chain (SEQ ID NO:211) 
QVQLQESGPGLVKPSQTLSLTCTVSGGSISSGGYYWSWIRQHPGKGLEWIGYIYYSGSTYY
NPSLKSRVTISVDTSKNQFSLKLSSVTAADTAVYYCARVGVALHYGMDVWGQGTTVTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGK
FS002-C417-HC (IgG1) heavy chain (SEQ ID NO:212) 
EVQLVESGGVVVQPGGSLRLSCAASGFTFDDYTMHWVRQAPGKGLEWVSLISWDGGST
YYADSVKGRFTISRDNAKNTLYLQMNSLRAEDTAVYYCARDTGYAMDVWGQGTMVTVS
SASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGP
SVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGK
FS002-B27-HC (IgG1) heavy chain (SEQ ID NO:213) 
QVQLVQSGAEVKKPGSSVKVSCKASGGTFSSYAISWVRQAPGQGLEWMGRIIPILGIANY
AQKFQGRVTITADKSTSTAYMELSSLRSEDTAVYYCARAEGSSWYYFDYWGQGTLVTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGK
FS002-C330-HC (IgG1) heavy chain (SEQ ID NO:214) 
QLQLQESGPGLVKPSGTLSLTCAVSGGSISSSNWWSWVRQPPGKGLEWIGEIYHSGSTNYN
PSLKSRVTISVDKSKNQFSLKLSSVTAADTAVYYCARAHSRYYYGLDYWGQGTLVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQG
NVFSCSVMHEALHNHYTQKSLSLSPGK
FS002-C153-HC (IgG1) heavy chain (SEQ ID NO:215) 
QVQLQQWGAELVKPGASVKLSCTASGFNIKDTYMHWVKQRPEQGLEWIGRIDPANGNT
KYDPKFQGKATITADTSSNTAYLQLSSLTSEDTAVYYCARSYYRYDGYFDVWGAGTMVTV
SSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGP
SVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGK
FS002-C241-HC (IgG1) heavy chain (SEQ ID NO:216) 
RLQLQESGPGLVKPSETLSLTCTVSGGSISSSSYYWGWIRQPPGKGLEWIGSIYYSGSTYYNP
SLKSRVTISVDTSKNQFSLKLSSVTAADTAVYYCARRYSYGRDAFDIWGQGTMVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQG
NVFSCSVMHEALHNHYTQKSLSLSPGK
FS002-C15-HC (IgG1) heavy chain (SEQ ID NO:217) 
QLQLQESGPGLVKPSETLSLTCTVSGGSISSSSYYWGWIRQPPGKGLEWIGSIYYSGSTYYNP
SLKSRVTISVDTSKNQFSLKLSSVTAADTAVYYCARLTIFGVVLYWGQGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPP
KPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFS
CSVMHEALHNHYTQKSLSLSPGK
FS002-C58-HC (IgG1) heavy chain (SEQ ID NO:218) 
EVQLLESGGGVVQPGRSLRLSCAASGFTFSSYAMHWVRQAPGKGLEWVAVISYDGSNKY
YADSVKGRFTISRDNSKNTLYLQMNSLRAEDTAVYYCARGKYNWFDPWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRWQQ
GNVFSCSVMHEALHNHYTQKSLSLSPGK
FS002-A72-HC (IgG1) heavy chain (SEQ ID NO:219)

QVQLVDSGGGLVQPGGSLRLSCVASGFAFSNYEMNWVRQAPGKGLEWVSYISSSSSYTNY
ADSVKGRFTISRDNAKNSLYLQMNSLRAEDTAVYYCARAQVRDAFDIWGQGTTVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRWQQ
GNVFSCSVMHEALHNHYTQKSLSLSPGK

**Table 5: The sequence numberings of the heavy chains, the light chains, the constant regions, the variable regions, and the CDRs as the above are summarized as follows (SEQ ID NO:)**

| **Antibody** | **Light Chain** | **Light Chain Sequence** | **VL CDR1 -3** | **Light Chain Constant Region** | **Light Chain Variable Region** | **Heavy Chain** | **Heavy Chain Sequence** | **VH CDR1-3** | **Heavy Chain Constant Region** | **Heavy Chain Variable Region** |
|---|---|---|---|---|---|---|---|---|---|---|
| FS002-A1 | FS002-A1-LC (Kappa) | 184 | 38-40 | 109 | 37 | FS002-A1-H C (IgG1) | 202 | 112-114 | 183 | 111 |
| FS002-A5 | FS002-A5-LC (Kappa) | 185 | 42-44 | 109 | 41 | FS002-A5-H C (IgG1) | 203 | 116-118 | 183 | 115 |
| FS002-A15 | FS002-A15 -LC (Kappa) | 186 | 46-48 | 109 | 45 | FS002-A15-HC (IgG1) | 204 | 120-122 | 183 | 119 |
| FS002-A36 | FS002-A36 -LC (Kappa) | 187 | 50-52 | 109 | 49 | FS002-A36-HC (IgG1) | 205 | 124-126 | 183 | 123 |
| FS002-A66 | FS002-A66 -LC (Kappa) | 188 | 54-56 | 109 | 53 | FS002-A66-HC (IgG1) | 206 | 128-130 | 183 | 127 |
| FS002-A10 | FS002-A10 -LC (Kappa-2) | 189 | 58-60 | 109 | 57 | FS002-A10-HC (IgG1) | 207 | 132-134 | 183 | 131 |
| FS002-C9 | FS002-C9-LC (Kappa-2) | 190 | 62-64 | 109 | 61 | FS002-C9-H C (IgG1) | 208 | 136-138 | 183 | 135 |
| FS002-C11 | FS002-C11 -LC (Kappa-2) | 191 | 66-68 | 109 | 65 | FS002-C11-HC (IgG1) | 209 | 140-142 | 183 | 139 |
| FS002-C21 6 | FS002-C21 6-LC (Kappa-2) | 192 | 70-72 | 109 | 69 | FS002-C216 -HC (IgG1) | 210 | 144-146 | 183 | 143 |
| FS002-C37 5 | FS002-C37 5-LC (Kappa-2) | 193 | 74-76 | 109 | 73 | FS002-C375 -HC (IgG1) | 211 | 148-150 | 183 | 147 |
| FS002-C41 7 | FS002-C41 7-LC (Kappa-2) | 194 | 78-80 | 109 | 77 | FS002-C417 -HC (IgG1) | 212 | 152-154 | 183 | 151 |
| FS002-B27 | FS002-B27 -LC (Kappa-2) | 195 | 82-84 | 109 | 81 | FS002-B27-HC (IgG1) | 213 | 156-158 | 183 | 155 |
| FS002-C33 0 | FS002-C33 0-LC (Kappa-2) | 196 | 86-88 | 109 | 85 | FS002-C330 -HC (IgG1) | 214 | 160-162 | 183 | 159 |
| FS002-C15 3 | FS002-C15 3-LC(Lamb daC2)-2 | 197 | 90-92 | 110 | 89 | FS002-C153 -HC (IgG1) | 215 | 164-166 | 183 | 163 |
| FS002-C24 1 | FS002-C24 1-LC(Lamb daC2)-2 | 198 | 94-96 | 110 | 93 | FS002-C241 -HC (IgG1) | 216 | 168-170 | 183 | 167 |
| FS002-C15 | FS002-C15 -LC (Kappa-2) | 199 | 98-10 0 | 109 | 97 | FS002-C15-HC (IgG1) | 217 | 172-174 | 183 | 171 |
| FS002-C58 | FS002-C58 -LC (Kappa-2) | 200 | 102-1 04 | 109 | 101 | FS002-C58-HC (IgG1) | 218 | 176-178 | 183 | 175 |
| FS002-A72 | FS002-A72 -LC (Kappa-2) | 201 | 106-1 08 | 109 | 105 | FS002-A72-HC (IgG1) | 219 | 180-182 | 183 | 179 |

### 2. Antibody expression and purification, and SDS-PAGE identification

The above plasmids were expressed in CHO cells for 7 days as required using the ExpiCHO-s expression system, and the antibody was obtained by purifying with Protein A affinity chromatography on the 6th day of expression.

Subsequently, the purity of the resulting antibody was measured by SDS-PAGE. In SDS-PAGE, the purity of the reduced band or the sum of the reduced heavy chain plus light chain was calculated with ImageJ through peak area normalization, wherein the non-reduced band of the reference IPI had a molecular weight of about 150 kDa, and a purity of greater than 90%, and the reduced heavy chain had a molecular weight of about 55 kDa, the light chain had a molecular weight of about 25 kDa, and the heavy chain plus light chain had a purity of greater than 90%.

### Example 3 - Evaluating the affinity of the anti-ITGA2 antibody to the ITGA2 protein at the protein level by ELISA method

The affinity of the ITGA2 antibody to the ITGA2 protein at the protein level was determined using the following method, comprising coating the ELISA plate with 2 ug/mL ITGA2-His. The plate was washed 3 times with PBST and blocked with 5% PBS-Milk for 2 h at room temperature. The plate was washed 3 times with PBST, then the ELISA plate was added with the diluted antibody at 30 µL per well, and left standing for 60 min at room temperature. The plate was washed 3 times with PBST, then the ELISA plate was added with anti-human IgG-Fc-HRP (abcam; ab97225) and left standing for 60 min at room temperature It was added with TMB. The reaction was stopped with 2 M stop solution, and the OD450 was read. Finally, EC50 was used to compare the binding capacity of each antibody to ITGA2-his. The antibody Vatelizumab (purchased from Biointron) was used as a positive control. The results are shown in Table 6 below.

**Table 6**

| Determined Anti-ITGA2 antibody | EC50 of ITGA2 Determined by ELISA (nM) |
|---|---|
| FS002-A1 | 0.029 |
| FS002-A10 | 0.021 |
| FS002-A15 | 0.028 |
| FS002-A36 | 0.022 |
| FS002-A5 | 0.032 |
| FS002-A66 | 0.031 |
| FS002-A72 | 0.259 |
| FS002-B27 | 0.571 |
| FS002-C11 | 0.035 |
| FS002-C15 | 0.044 |
| FS002-C153 | 0.087 |
| FS002-C216 | 0.073 |
| FS002-C241 | 0.116 |
| FS002-C330 | 0.103 |
| FS002-C375 | 0.073 |
| FS002-C417 | 0.156 |
| FS002-C58 | 0.279 |
| FS002-C9 | 12.080 |
| Vatelizumab | 0.023 |

As shown in the table above, 17 of the 18 tested antibodies have good binding activity to ITGA2-His (EC50<1 nM).

### Example 4 - Determining affinity of anti-ITGA2 antibody to ITGA2 at the cellular level by flow cytometry

Human bile duct cancer HuCCT1 cells were seeded into a 96-well plate at 50,000 cells per well, and blown evenly and washed twice with PBS. The PBS was then drawn and discarded. The ITGA2 antibody to be tested was used as a primary antibody, and was prepared to a working concentration of 30 nM with 1% FBS/PBS solution. It was diluted by 3 folds in sequence, with a total of 5 specific concentration points. The prepared antibody dilution was added to the corresponding 96-well plate and incubated at 4°C for 30 min. It was then blown and washed twice with 1% FBS/PBS solution. After the PBS was drawn and discarded, the solution was centrifuged, and the antibody supernatant was drawn and discarded. A goat anti-human secondary antibody IgG (H+L) with cross-recognition ability from other species removed was prepared with a PBS solution comprising 10% FBS at 1:300. It was then added to a 96-well plate, and incubated at 4 °C for 30 min. Flow cytometry was used for collection and analysis, and finally the affinity of the antibody to the ITGA2 target was evaluated by EC50. The antibody Vatelizumab (purchased from Biointron) was used as a positive control antibody for binding to the ITGA2.

The results of the determination of EC50 are shown in Table 7. As shown in Table 7, 14 of these 18 antibodies have EC50 of less than 5 nM and bind well to ITGA2-positive HuCCT1 cells.

**Table 7**

| Determined Anti-ITGA2 antibody | EC50 of HuCCT1 Determined by Flow Cytometry (nM) |
|---|---|
| FS002-A1 | 0.653 |
| FS002-A10 | 1.127 |
| FS002-A15 | 0.927 |
| FS002-A36 | 0.793 |
| FS002-A5 | 1.267 |
| FS002-A66 | 2.353 |
| FS002-A72 | 17.213 |
| FS002-B27 | 2.753 |
| FS002-C11 | 0.747 |
| FS002-C15 | 2.133 |
| FS002-C153 | 3.627 |
| FS002-C216 | 0.987 |
| FS002-C241 | 2.413 |
| FS002-C330 | 2.067 |
| FS002-C375 | 2.167 |
| FS002-C417 | 68.400 |
| FS002-C58 | 9.480 |
| FS002-C9 | 34.560 |
| Vatelizumab | 0.793 |

### Example 5 - Preparing an antibody-drug conjugate (ADC)

The antibody-drug conjugate was prepared by the following method.

### Materials and Methods:

Conjugating buffer: 25 mM Na₂B₄O₇, 25 mM NaCl, 1 mM DPTA, pH 7.4
Dialysis buffer: 1 × PBS, pH 7.4
Antibody reduction concentration: 5 mg/mL
Tris(2-chloroethyl)phosphate (TCEP): 5 mM, binding buffer
TCEP:antibody = 2.3:1
Payload: 10 mM linker-payload, dimethyl sulfoxide (DMSO)
DMSO in final reaction: 10%
Payload:antibody =10:1
Reduction temperature and time: 25°C and 2 hours
Conjugating temperature and time: 25°C and 4 hours

### Conjugating Steps:

Thaw and dialyze the antibody using a conjugation buffer at 4 °C for > 4 h;
If needed, concentrate the antibody solution to 2-5 mg/ml;
Add a TCEP working solution to reduce the antibody and incubate it in a water bath at 25°C;
Prepare a linker-payload solution in DMSO (10 mM);
Calculate the volume of the effective linker-payload solution for each conjugation based on molar concentration;
Mix the antibody with the linker-payload at the desired molar ratio and incubate it in a 25 °C water bath;
Dialyze the conjugate for more than 4 h at 4 °C using an ADC storage buffer, with the buffer changed at least every 4 h; and
Extract and filter the ADC using a 0.22 µm filter, and send the samples for SEC-HPLC and HIC-HPLC analysis.

Through the above method, the antibody-drug conjugate of antibodies FS002-A1, FS002-A15, FS002-A36 of the present invention and the positive control antibody Vatelizumab was prepared in the present example using vc-MMAE (mc-vc-PAB-MMAE, C68H105N11O15, provided by ChemPartner) as the linker-payload.

**Table 8**

| ADC Name | Drug purity (%, SEC) | Drug-antibody ratio (DAR) |
|---|---|---|
| FS002-A1-vc-MMAE | 98.8 | 3.4 |
| FS002-A15-vc-MMAE | 97.1 | 2.9 |
| FS002-A36-vc-MMAE | 98.0 | 3.8 |
| Vatelizumab-ve-MMAE | 98.9 | 3.9 |

### Example 6 - Biological activity of antibody-drug conjugate (ADC) for killing cancer cells

The example tests the biological activity of the ADC prepared from the antibody-conjugated toxin of the present invention for killing cancer cells.

Human bile duct cancer cells HuCC-T1 available from Zhejiang MeisenCTCC, human epidermal cancer cells A431 available from Procell Life Science&Technology Co.,Ltd. (Wuhan) and Chinese hamster ovary cells CHO from negative control ATCC were seeded into a 96-well plate at 2500 cells/well. It was then incubated in a 5% CO2 incubator overnight at 37°C. Half of the medium was removed from the cell plate on the next day. A 2x hIgG1 (negative control antibody, provided by Biointron), Vatelizumab, FS002-A1-vc-MMAE, FS002-A15-vc-MMAE, FS002-A36-vc-MMAE, Vatelizumab-ve-MMAE, hIgG1-vcMMAE (negative control ADC, provided by ChemPartner) was prepared with a complete culture medium, and then diluted at a 3-fold gradient with 11 specific concentration points in total. Serial dilutions of the above molecules were added to the appropriate wells, and it was then incubated in a 5% CO2 incubator for 5 days at 37°C. After the incubation, 100 uL of Cell-Titer-Glo 2.0 reagent was added to the 96-well plate. It was then well mixed on a plate shaker for 2 minutes, and finally equilibrated at room temperature for 10 minutes. Readings were taken using a microplate reader. Finally, IC50 was used to compare the biological activity of the ADC against each cell line.

The results are shown in Table 9. As shown in Table 9, FS002-A1-vc-MMAE, FS002-A15-vc-MMAE, FS002-A36-vc-MMAE and Vatelizumab-ve-MMAE had good killing effect on HuCC-T1 and A431, but had no killing effect on negative control cells CHO. The negative control antibody hIgG1, the ITGA2 antibody Vatelizumab not coupled to ve-MMAE, and the negative control hIgG1-vc-MMAE cannot effectively kill HuCC-T1 and A431 cells.

**Table 9**

| ADC Name | HuCC-T1 cell killing IC50 (nM) | A431 cell killing IC50 (nM) | CHO cell killing IC50 (nM) |
|---|---|---|---|
| hIgG1 | >207 | >207 | >207 |
| Vatelizumab | >207 | >207 | >207 |
| hIgG 1-vc-MMAE | 86.60 | 26.90 | >207 |
| FS002-A1-vc-MMAE | 0.14 | 0.14 | >207 |
| FS002-A15-vc-MMAE | 0.20 | 0.20 | >207 |
| FS002-A36-vc-MMAE | 0.08 | 0.02 | >207 |
| Vatelizumab-ve-MMAE | 0.89 | 0.08 | >207 |

These results suggest that ITGA2 antibody cannot be used alone to kill tumor cells in vitro, but surprisingly, the ITGA2 protein has been found to be an effective target for the antibody-drug conjugate. The antibody-drug conjugate obtained by Conjugating the antibody to micromolecule toxins can produce excellent killing effect on tumors expressing the ITGA2 protein.

### Sets of clauses

1. An isolated antibody specifically binding to an ITGA2 protein or an antigen-binding fragment thereof, comprising a heavy chain variable region comprising HCDR1, HCDR2, and HCDR3 sequences, and a light chain variable region comprising LCDR1, LCDR2, and LCDR3 sequences, wherein the antibody or the antigen-binding fragment thereof comprises a heavy chain variable region and a light chain variable region selected from at least one of the following groups:
   1) a heavy chain variable region that comprises (a) HCDR1 as set forth in SEQ ID NO: 112, (b) HCDR2 as set forth in SEQ ID NO: 113, and (c) HCDR3 as set forth in SEQ ID NO: 114, and a light chain variable region that comprises (d) LCDR1 as set forth in SEQ ID NO: 38, (e) LCDR2 as set forth in SEQ ID NO: 39, and (f) LCDR3 as set forth in SEQ ID NO: 40;
   2) a heavy chain variable region that comprises (a) HCDR1 as set forth in SEQ ID NO: 116, (b) HCDR2 as set forth in SEQ ID NO: 117, and (c) HCDR3 as set forth in SEQ ID NO: 118, and a light chain variable region that comprises (d) LCDR1 as set forth in SEQ ID NO: 42, (e) LCDR2 as set forth in SEQ ID NO: 43, and (f) LCDR3 as set forth in SEQ ID NO: 44;
   3) a heavy chain variable region that comprises (a) HCDR1 as set forth in SEQ ID NO: 120, (b) HCDR2 as set forth in SEQ ID NO: 121, and (c) HCDR3 as set forth in SEQ ID NO: 122, and a light chain variable region that comprises (d) LCDR1 as set forth in SEQ ID NO: 46, (e) LCDR2 as set forth in SEQ ID NO: 47, and (f) LCDR3 as set forth in SEQ ID NO: 48;
   4) a heavy chain variable region that comprises (a) HCDR1 as set forth in SEQ ID NO: 124, (b) HCDR2 as set forth in SEQ ID NO: 125, and (c) HCDR3 as set forth in SEQ ID NO: 126, and a light chain variable region that comprises (d) LCDR1 as set forth in SEQ ID NO: 50, (e) LCDR2 as set forth in SEQ ID NO: 51, and (f) LCDR3 as set forth in SEQ ID NO: 52;
   5) a heavy chain variable region that comprises (a) HCDR1 as set forth in SEQ ID NO: 128, (b) HCDR2 as set forth in SEQ ID NO: 129, and (c) HCDR3 as set forth in SEQ ID NO: 130, and a light chain variable region that comprises (d) LCDR1 as set forth in SEQ ID NO: 54, (e) LCDR2 as set forth in SEQ ID NO: 55, and (f) LCDR3 as set forth in SEQ ID NO: 56;
   6) a heavy chain variable region that comprises (a) HCDR1 as set forth in SEQ ID NO: 132, (b) HCDR2 as set forth in SEQ ID NO: 133, and (c) HCDR3 as set forth in SEQ ID NO: 134, and a light chain variable region that comprises (d) LCDR1 as set forth in SEQ ID NO: 58, (e) LCDR2 as set forth in SEQ ID NO: 59, and (f) LCDR3 as set forth in SEQ ID NO: 60;
   7) a heavy chain variable region that comprises (a) HCDR1 as set forth in SEQ ID NO: 136, (b) HCDR2 as set forth in SEQ ID NO: 137, and (c) HCDR3 as set forth in SEQ ID NO: 138, and a light chain variable region that comprises (d) LCDR1 as set forth in SEQ ID NO: 62, (e) LCDR2 as set forth in SEQ ID NO: 63, and (f) LCDR3 as set forth in SEQ ID NO: 64;
   8) a heavy chain variable region that comprises (a) HCDR1 as set forth in SEQ ID NO: 140, (b) HCDR2 as set forth in SEQ ID NO: 141, and (c) HCDR3 as set forth in SEQ ID NO: 142, and a light chain variable region that comprises (d) LCDR1 as set forth in SEQ ID NO: 66, (e) LCDR2 as set forth in SEQ ID NO: 67, and (f) LCDR3 as set forth in SEQ ID NO: 68;
   9) a heavy chain variable region that comprises (a) HCDR1 as set forth in SEQ ID NO: 144, (b) HCDR2 as set forth in SEQ ID NO: 145, and (c) HCDR3 as set forth in SEQ ID NO: 146, and a light chain variable region that comprises (d) LCDR1 as set forth in SEQ ID NO: 70, (e) LCDR2 as set forth in SEQ ID NO: 71, and (f) LCDR3 as set forth in SEQ ID NO: 72;
   10) a heavy chain variable region that comprises (a) HCDR1 as set forth in SEQ ID NO: 148, (b) HCDR2 as set forth in SEQ ID NO: 149, and (c) HCDR3 as set forth in SEQ ID NO: 150, and a light chain variable region that comprises (d) LCDR1 as set forth in SEQ ID NO: 74, (e) LCDR2 as set forth in SEQ ID NO: 75, and (f) LCDR3 as set forth in SEQ ID NO: 76;
   11) a heavy chain variable region that comprises (a) HCDR1 as set forth in SEQ ID NO: 152, (b) HCDR2 as set forth in SEQ ID NO: 153, and (c) HCDR3 as set forth in SEQ ID NO: 154, and a light chain variable region that comprises (d) LCDR1 as set forth in SEQ ID NO: 78, (e) LCDR2 as set forth in SEQ ID NO: 79, and (f) LCDR3 as set forth in SEQ ID NO: 80;
   12) a heavy chain variable region that comprises (a) HCDR1 as set forth in SEQ ID NO: 156, (b) HCDR2 as set forth in SEQ ID NO: 157, and (c) HCDR3 as set forth in SEQ ID NO: 158, and a light chain variable region that comprises (d) LCDR1 as set forth in SEQ ID NO: 82, (e) LCDR2 as set forth in SEQ ID NO: 83, and (f) LCDR3 as set forth in SEQ ID NO: 84;
   13) a heavy chain variable region that comprises (a) HCDR1 as set forth in SEQ ID NO: 160, (b) HCDR2 as set forth in SEQ ID NO: 161, and (c) HCDR3 as set forth in SEQ ID NO: 162, and a light chain variable region that comprises (d) LCDR1 as set forth in SEQ ID NO: 86, (e) LCDR2 as set forth in SEQ ID NO: 87, and (f) LCDR3 as set forth in SEQ ID NO: 88;
   14) a heavy chain variable region that comprises (a) HCDR1 as set forth in SEQ ID NO: 164, (b) HCDR2 as set forth in SEQ ID NO: 165, and (c) HCDR3 as set forth in SEQ ID NO: 166, and a light chain variable region that comprises (d) LCDR1 as set forth in SEQ ID NO: 90, (e) LCDR2 as set forth in SEQ ID NO: 91, and (f) LCDR3 as set forth in SEQ ID NO: 92;
   15) a heavy chain variable region that comprises (a) HCDR1 as set forth in SEQ ID NO: 168, (b) HCDR2 as set forth in SEQ ID NO: 169, and (c) HCDR3 as set forth in SEQ ID NO: 170, and a light chain variable region that comprises (d) LCDR1 as set forth in SEQ ID NO: 94, (e) LCDR2 as set forth in SEQ ID NO: 95, and (f) LCDR3 as set forth in SEQ ID NO: 96;
   16) a heavy chain variable region that comprises (a) HCDR1 as set forth in SEQ ID NO: 172, (b) HCDR2 as set forth in SEQ ID NO: 173, and (c) HCDR3 as set forth in SEQ ID NO: 174, and a light chain variable region that comprises (d) LCDR1 as set forth in SEQ ID NO: 98, (e) LCDR2 as set forth in SEQ ID NO: 99, and (f) LCDR3 as set forth in SEQ ID NO: 100;
   17) a heavy chain variable region that comprises (a) HCDR1 as set forth in SEQ ID NO: 176, (b) HCDR2 as set forth in SEQ ID NO: 177, and (c) HCDR3 as set forth in SEQ ID NO: 178, and a light chain variable region that comprises (d) LCDR1 as set forth in SEQ ID NO: 102, (e) LCDR2 as set forth in SEQ ID NO: 103, and (f) LCDR3 as set forth in SEQ ID NO: 104; and
   18) a heavy chain variable region that comprises (a) HCDR1 as set forth in SEQ ID NO: 180, (b) HCDR2 as set forth in SEQ ID NO: 181, and (c) HCDR3 as set forth in SEQ ID NO: 182, and a light chain variable region that comprises (d) LCDR1 as set forth in SEQ ID NO: 106, (e) LCDR2 as set forth in SEQ ID NO: 107, and (f) LCDR3 as set forth in SEQ ID NO: 108.
2. The isolated antibody or the antigen binding fragment thereof according to clause 1, wherein the isolated antibody or the antigen binding fragment thereof comprises a heavy chain variable region and a light chain variable region selected from at least one of the following groups:
   1) a heavy chain variable region that comprises an amino acid sequence as set forth in SEQ ID NO:111, or an amino acid sequence that is at least 85%, at least 90%, at least 95%, at least 96%, at least 97%, at least 98%, or at least 99% identical to the amino acid sequence as set forth in SEQ ID NO:111, and a light chain variable region that comprises an amino acid sequence as set forth in SEQ ID NO:37, or an amino acid sequence that is at least 85%, at least 90%, at least 95%, at least 96%, at least 97%, at least 98%, or at least 99% identical to the amino acid sequence as set forth in SEQ ID NO:37;
   2) a heavy chain variable region that comprises an amino acid sequence as set forth in SEQ ID NO:115, or an amino acid sequence that is at least 85%, at least 90%, at least 95%, at least 96%, at least 97%, at least 98%, or at least 99% identical to the amino acid sequence as set forth in SEQ ID NO:115, and a light chain variable region that comprises an amino acid sequence as set forth in SEQ ID NO:41, or an amino acid sequence that is at least 85%, at least 90%, at least 95%, at least 96%, at least 97%, at least 98%, or at least 99% identical to the amino acid sequence as set forth in SEQ ID NO:41;
   3) a heavy chain variable region that comprises an amino acid sequence as set forth in SEQ ID NO:119, or an amino acid sequence that is at least 85%, at least 90%, at least 95%, at least 96%, at least 97%, at least 98%, or at least 99% identical to the amino acid sequence as set forth in SEQ ID NO:119, and a light chain variable region that comprises an amino acid sequence as set forth in SEQ ID NO:45, or an amino acid sequence that is at least 85%, at least 90%, at least 95%, at least 96%, at least 97%, at least 98%, or at least 99% identical to the amino acid sequence as set forth in SEQ ID NO:45;
   4) a heavy chain variable region that comprises an amino acid sequence as set forth in SEQ ID NO:123, or an amino acid sequence that is at least 85%, at least 90%, at least 95%, at least 96%, at least 97%, at least 98%, or at least 99% identical to the amino acid sequence as set forth in SEQ ID NO:123, and a light chain variable region that comprises an amino acid sequence as set forth in SEQ ID NO:49, or an amino acid sequence that is at least 85%, at least 90%, at least 95%, at least 96%, at least 97%, at least 98%, or at least 99% identical to the amino acid sequence as set forth in SEQ ID NO:49;
   5) a heavy chain variable region that comprises an amino acid sequence as set forth in SEQ ID NO:127, or an amino acid sequence that is at least 85%, at least 90%, at least 95%, at least 96%, at least 97%, at least 98%, or at least 99% identical to the amino acid sequence as set forth in SEQ ID NO: 127, and a light chain variable region that comprises an amino acid sequence as set forth in SEQ ID NO:53, or an amino acid sequence that is at least 85%, at least 90%, at least 95%, at least 96%, at least 97%, at least 98%, or at least 99% identical to the amino acid sequence as set forth in SEQ ID NO:53;
   6) a heavy chain variable region that comprises an amino acid sequence as set forth in SEQ ID NO:131, or an amino acid sequence that is at least 85%, at least 90%, at least 95%, at least 96%, at least 97%, at least 98%, or at least 99% identical to the amino acid sequence as set forth in SEQ ID NO:131, and a light chain variable region that comprises an amino acid sequence as set forth in SEQ ID NO:57, or an amino acid sequence that is at least 85%, at least 90%, at least 95%, at least 96%, at least 97%, at least 98%, or at least 99% identical to the amino acid sequence as set forth in SEQ ID NO:57;
   7) a heavy chain variable region that comprises an amino acid sequence as set forth in SEQ ID NO:135, or an amino acid sequence that is at least 85%, at least 90%, at least 95%, at least 96%, at least 97%, at least 98%, or at least 99% identical to the amino acid sequence as set forth in SEQ ID NO: 135, and a light chain variable region that comprises an amino acid sequence as set forth in SEQ ID NO:61, or an amino acid sequence that is at least 85%, at least 90%, at least 95%, at least 96%, at least 97%, at least 98%, or at least 99% identical to the amino acid sequence as set forth in SEQ ID NO:61;
   8) a heavy chain variable region that comprises an amino acid sequence as set forth in SEQ ID NO:139, or an amino acid sequence that is at least 85%, at least 90%, at least 95%, at least 96%, at least 97%, at least 98%, or at least 99% identical to the amino acid sequence as set forth in SEQ ID NO: 139, and a light chain variable region that comprises an amino acid sequence as set forth in SEQ ID NO:65, or an amino acid sequence that is at least 85%, at least 90%, at least 95%, at least 96%, at least 97%, at least 98%, or at least 99% identical to the amino acid sequence as set forth in SEQ ID NO:65;
   9) a heavy chain variable region that comprises an amino acid sequence as set forth in SEQ ID NO:143, or an amino acid sequence that is at least 85%, at least 90%, at least 95%, at least 96%, at least 97%, at least 98%, or at least 99% identical to the amino acid sequence as set forth in SEQ ID NO: 143, and a light chain variable region that comprises an amino acid sequence as set forth in SEQ ID NO:69, or an amino acid sequence that is at least 85%, at least 90%, at least 95%, at least 96%, at least 97%, at least 98%, or at least 99% identical to the amino acid sequence as set forth in SEQ ID NO:69;
   10) a heavy chain variable region that comprises an amino acid sequence as set forth in SEQ ID NO:147, or an amino acid sequence that is at least 85%, at least 90%, at least 95%, at least 96%, at least 97%, at least 98%, or at least 99% identical to the amino acid sequence as set forth in SEQ ID NO: 147, and a light chain variable region that comprises an amino acid sequence as set forth in SEQ ID NO:73, or an amino acid sequence that is at least 85%, at least 90%, at least 95%, at least 96%, at least 97%, at least 98%, or at least 99% identical to the amino acid sequence as set forth in SEQ ID NO:73;
   11) a heavy chain variable region that comprises an amino acid sequence as set forth in SEQ ID NO:151, or an amino acid sequence that is at least 85%, at least 90%, at least 95%, at least 96%, at least 97%, at least 98%, or at least 99% identical to the amino acid sequence as set forth in SEQ ID NO:151, and a light chain variable region that comprises an amino acid sequence as set forth in SEQ ID NO:77, or an amino acid sequence that is at least 85%, at least 90%, at least 95%, at least 96%, at least 97%, at least 98%, or at least 99% identical to the amino acid sequence as set forth in SEQ ID NO:77;
   12) a heavy chain variable region that comprises an amino acid sequence as set forth in SEQ ID NO:155, or an amino acid sequence that is at least 85%, at least 90%, at least 95%, at least 96%, at least 97%, at least 98%, or at least 99% identical to the amino acid sequence as set forth in SEQ ID NO:155, and a light chain variable region that comprises an amino acid sequence as set forth in SEQ ID NO:81, or an amino acid sequence that is at least 85%, at least 90%, at least 95%, at least 96%, at least 97%, at least 98%, or at least 99% identical to the amino acid sequence as set forth in SEQ ID NO:81;
   13) a heavy chain variable region that comprises an amino acid sequence as set forth in SEQ ID NO:159, or an amino acid sequence that is at least 85%, at least 90%, at least 95%, at least 96%, at least 97%, at least 98%, or at least 99% identical to the amino acid sequence as set forth in SEQ ID NO:159, and a light chain variable region that comprises an amino acid sequence as set forth in SEQ ID NO:85, or an amino acid sequence that is at least 85%, at least 90%, at least 95%, at least 96%, at least 97%, at least 98%, or at least 99% identical to the amino acid sequence as set forth in SEQ ID NO:85;
   14) a heavy chain variable region that comprises an amino acid sequence as set forth in SEQ ID NO:163, or an amino acid sequence that is at least 85%, at least 90%, at least 95%, at least 96%, at least 97%, at least 98%, or at least 99% identical to the amino acid sequence as set forth in SEQ ID NO: 163, and a light chain variable region that comprises an amino acid sequence as set forth in SEQ ID NO:89, or an amino acid sequence that is at least 85%, at least 90%, at least 95%, at least 96%, at least 97%, at least 98%, or at least 99% identical to the amino acid sequence as set forth in SEQ ID NO:89;
   15) a heavy chain variable region that comprises an amino acid sequence as set forth in SEQ ID NO:167, or an amino acid sequence that is at least 85%, at least 90%, at least 95%, at least 96%, at least 97%, at least 98%, or at least 99% identical to the amino acid sequence as set forth in SEQ ID NO: 167, and a light chain variable region that comprises an amino acid sequence as set forth in SEQ ID NO:93, or an amino acid sequence that is at least 85%, at least 90%, at least 95%, at least 96%, at least 97%, at least 98%, or at least 99% identical to the amino acid sequence as set forth in SEQ ID NO:93;
   16) a heavy chain variable region that comprises an amino acid sequence as set forth in SEQ ID NO:171, or an amino acid sequence that is at least 85%, at least 90%, at least 95%, at least 96%, at least 97%, at least 98%, or at least 99% identical to the amino acid sequence as set forth in SEQ ID NO: 171, and a light chain variable region that comprises an amino acid sequence as set forth in SEQ ID NO:97, or an amino acid sequence that is at least 85%, at least 90%, at least 95%, at least 96%, at least 97%, at least 98%, or at least 99% identical to the amino acid sequence as set forth in SEQ ID NO:97;
   17) a heavy chain variable region that comprises an amino acid sequence as set forth in SEQ ID NO:175, or an amino acid sequence that is at least 85%, at least 90%, at least 95%, at least 96%, at least 97%, at least 98%, or at least 99% identical to the amino acid sequence as set forth in SEQ ID NO: 175, and a light chain variable region that comprises an amino acid sequence as set forth in SEQ ID NO:101, or an amino acid sequence that is at least 85%, at least 90%, at least 95%, at least 96%, at least 97%, at least 98%, or at least 99% identical to the amino acid sequence as set forth in SEQ ID NO:101; and
   18) a heavy chain variable region that comprises an amino acid sequence as set forth in SEQ ID NO:179, or an amino acid sequence that is at least 85%, at least 90%, at least 95%, at least 96%, at least 97%, at least 98%, or at least 99% identical to the amino acid sequence as set forth in SEQ ID NO: 179, and a light chain variable region that comprises an amino acid sequence as set forth in SEQ ID NO:105, or an amino acid sequence that is at least 85%, at least 90%, at least 95%, at least 96%, at least 97%, at least 98%, or at least 99% identical to the amino acid sequence as set forth in SEQ ID NO: 105.
3. The isolated antibody or the antigen binding fragment thereof according to clause 1 or 2, wherein the isolated antibody or the antigen binding fragment thereof further comprises a heavy chain constant region and/or a light chain constant region,
   the heavy chain constant region comprises an amino acid sequence as set forth in SEQ ID NO: 183, or an amino acid sequence that is at least 85%, at least 90%, at least 95%, at least 96%, at least 97%, at least 98%, or at least 99% identical to the amino acid sequence as set forth in SEQ ID NO:183,
   the light chain constant region comprises an amino acid sequence as set forth in SEQ ID NO: 109, or an amino acid sequence that is at least 85%, at least 90%, at least 95%, at least 96%, at least 97%, at least 98%, or at least 99% identical to the amino acid sequence as set forth in SEQ ID NO: 109, or the light chain constant region comprises an amino acid sequence as set forth in SEQ ID NO:110, or an amino acid sequence that is at least 85%, at least 90%, at least 95%, at least 96%, at least 97%, at least 98%, or at least 99% identical to the amino acid sequence as set forth in SEQ ID NO:110.
4. The isolated antibody or the antigen binding fragment thereof according to any one of clauses 1 to 3, wherein the isolated antibody or the antigen binding fragment thereof comprises a heavy chain and a light chain selected from at least one of the following groups:
   1) a heavy chain that comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202, and a light chain that comprises an amino acid sequence as set forth in SEQ ID NO: 184, or an amino acid sequence that is at least 85%, at least 90%, at least 95%, at least 96%, at least 97%, at least 98%, or at least 99% identical to the amino acid sequence as set forth in SEQ ID NO: 184;
   2) a heavy chain that comprises an amino acid sequence as set forth in SEQ ID NO:203, or an amino acid sequence that is at least 85%, at least 90%, at least 95%, at least 96%, at least 97%, at least 98%, or at least 99% identical to the amino acid sequence as set forth in SEQ ID NO:203, and a light chain that comprises an amino acid sequence as set forth in SEQ ID NO:185, or an amino acid sequence that is at least 85%, at least 90%, at least 95%, at least 96%, at least 97%, at least 98%, or at least 99% identical to the amino acid sequence as set forth in SEQ ID NO: 185;
   3) a heavy chain that comprises an amino acid sequence as set forth in SEQ ID NO:204, or an amino acid sequence that is at least 85%, at least 90%, at least 95%, at least 96%, at least 97%, at least 98%, or at least 99% identical to the amino acid sequence as set forth in SEQ ID NO:204, and a light chain that comprises an amino acid sequence as set forth in SEQ ID NO: 186, or an amino acid sequence that is at least 85%, at least 90%, at least 95%, at least 96%, at least 97%, at least 98%, or at least 99% identical to the amino acid sequence as set forth in SEQ ID NO: 186;
   4) a heavy chain that comprises an amino acid sequence as set forth in SEQ ID NO:205, or an amino acid sequence that is at least 85%, at least 90%, at least 95%, at least 96%, at least 97%, at least 98%, or at least 99% identical to the amino acid sequence as set forth in SEQ ID NO:205, and a light chain that comprises an amino acid sequence as set forth in SEQ ID NO: 187, or an amino acid sequence that is at least 85%, at least 90%, at least 95%, at least 96%, at least 97%, at least 98%, or at least 99% identical to the amino acid sequence as set forth in SEQ ID NO: 187;
   5) a heavy chain that comprises an amino acid sequence as set forth in SEQ ID NO:206, or an amino acid sequence that is at least 85%, at least 90%, at least 95%, at least 96%, at least 97%, at least 98%, or at least 99% identical to the amino acid sequence as set forth in SEQ ID NO:206, and a light chain that comprises an amino acid sequence as set forth in SEQ ID NO:188, or an amino acid sequence that is at least 85%, at least 90%, at least 95%, at least 96%, at least 97%, at least 98%, or at least 99% identical to the amino acid sequence as set forth in SEQ ID NO:188;
   6) a heavy chain that comprises an amino acid sequence as set forth in SEQ ID NO:207, or an amino acid sequence that is at least 85%, at least 90%, at least 95%, at least 96%, at least 97%, at least 98%, or at least 99% identical to the amino acid sequence as set forth in SEQ ID NO:207, and a light chain that comprises an amino acid sequence as set forth in SEQ ID NO:189, or an amino acid sequence that is at least 85%, at least 90%, at least 95%, at least 96%, at least 97%, at least 98%, or at least 99% identical to the amino acid sequence as set forth in SEQ ID NO: 189;
   7) a heavy chain that comprises an amino acid sequence as set forth in SEQ ID NO:208, or an amino acid sequence that is at least 85%, at least 90%, at least 95%, at least 96%, at least 97%, at least 98%, or at least 99% identical to the amino acid sequence as set forth in SEQ ID NO:208, and a light chain that comprises an amino acid sequence as set forth in SEQ ID NO:190, or an amino acid sequence that is at least 85%, at least 90%, at least 95%, at least 96%, at least 97%, at least 98%, or at least 99% identical to the amino acid sequence as set forth in SEQ ID NO:190;
   8) a heavy chain that comprises an amino acid sequence as set forth in SEQ ID NO:209, or an amino acid sequence that is at least 85%, at least 90%, at least 95%, at least 96%, at least 97%, at least 98%, or at least 99% identical to the amino acid sequence as set forth in SEQ ID NO:209, and a light chain that comprises an amino acid sequence as set forth in SEQ ID NO:191, or an amino acid sequence that is at least 85%, at least 90%, at least 95%, at least 96%, at least 97%, at least 98%, or at least 99% identical to the amino acid sequence as set forth in SEQ ID NO:191;
   9) a heavy chain that comprises an amino acid sequence as set forth in SEQ ID NO:210, or an amino acid sequence that is at least 85%, at least 90%, at least 95%, at least 96%, at least 97%, at least 98%, or at least 99% identical to the amino acid sequence as set forth in SEQ ID NO:210, and a light chain that comprises an amino acid sequence as set forth in SEQ ID NO:192, or an amino acid sequence that is at least 85%, at least 90%, at least 95%, at least 96%, at least 97%, at least 98%, or at least 99% identical to the amino acid sequence as set forth in SEQ ID NO:192;
   10) a heavy chain that comprises an amino acid sequence as set forth in SEQ ID NO:211, or an amino acid sequence that is at least 85%, at least 90%, at least 95%, at least 96%, at least 97%, at least 98%, or at least 99% identical to the amino acid sequence as set forth in SEQ ID NO:211, and a light chain that comprises an amino acid sequence as set forth in SEQ ID NO:193, or an amino acid sequence that is at least 85%, at least 90%, at least 95%, at least 96%, at least 97%, at least 98%, or at least 99% identical to the amino acid sequence as set forth in SEQ ID NO: 193;
   11) a heavy chain that comprises an amino acid sequence as set forth in SEQ ID NO:212, or an amino acid sequence that is at least 85%, at least 90%, at least 95%, at least 96%, at least 97%, at least 98%, or at least 99% identical to the amino acid sequence as set forth in SEQ ID NO:212, and a light chain that comprises an amino acid sequence as set forth in SEQ ID NO:194, or an amino acid sequence that is at least 85%, at least 90%, at least 95%, at least 96%, at least 97%, at least 98%, or at least 99% identical to the amino acid sequence as set forth in SEQ ID NO:194;
   12) a heavy chain that comprises an amino acid sequence as set forth in SEQ ID NO:213, or an amino acid sequence that is at least 85%, at least 90%, at least 95%, at least 96%, at least 97%, at least 98%, or at least 99% identical to the amino acid sequence as set forth in SEQ ID NO:213, and a light chain that comprises an amino acid sequence as set forth in SEQ ID NO:195, or an amino acid sequence that is at least 85%, at least 90%, at least 95%, at least 96%, at least 97%, at least 98%, or at least 99% identical to the amino acid sequence as set forth in SEQ ID NO:195;
   13) a heavy chain that comprises an amino acid sequence as set forth in SEQ ID NO:214, or an amino acid sequence that is at least 85%, at least 90%, at least 95%, at least 96%, at least 97%, at least 98%, or at least 99% identical to the amino acid sequence as set forth in SEQ ID NO:214, and a light chain that comprises an amino acid sequence as set forth in SEQ ID NO:196, or an amino acid sequence that is at least 85%, at least 90%, at least 95%, at least 96%, at least 97%, at least 98%, or at least 99% identical to the amino acid sequence as set forth in SEQ ID NO: 196;
   14) a heavy chain that comprises an amino acid sequence as set forth in SEQ ID NO:215, or an amino acid sequence that is at least 85%, at least 90%, at least 95%, at least 96%, at least 97%, at least 98%, or at least 99% identical to the amino acid sequence as set forth in SEQ ID NO:215, and a light chain that comprises an amino acid sequence as set forth in SEQ ID NO:197, or an amino acid sequence that is at least 85%, at least 90%, at least 95%, at least 96%, at least 97%, at least 98%, or at least 99% identical to the amino acid sequence as set forth in SEQ ID NO:197;
   15) a heavy chain that comprises an amino acid sequence as set forth in SEQ ID NO:216, or an amino acid sequence that is at least 85%, at least 90%, at least 95%, at least 96%, at least 97%, at least 98%, or at least 99% identical to the amino acid sequence as set forth in SEQ ID NO:216, and a light chain that comprises an amino acid sequence as set forth in SEQ ID NO:198, or an amino acid sequence that is at least 85%, at least 90%, at least 95%, at least 96%, at least 97%, at least 98%, or at least 99% identical to the amino acid sequence as set forth in SEQ ID NO: 198;
   16) a heavy chain that comprises an amino acid sequence as set forth in SEQ ID NO:217, or an amino acid sequence that is at least 85%, at least 90%, at least 95%, at least 96%, at least 97%, at least 98%, or at least 99% identical to the amino acid sequence as set forth in SEQ ID NO:217, and a light chain that comprises an amino acid sequence as set forth in SEQ ID NO:199, or an amino acid sequence that is at least 85%, at least 90%, at least 95%, at least 96%, at least 97%, at least 98%, or at least 99% identical to the amino acid sequence as set forth in SEQ ID NO:199;
   17) a heavy chain that comprises an amino acid sequence as set forth in SEQ ID NO:218, or an amino acid sequence that is at least 85%, at least 90%, at least 95%, at least 96%, at least 97%, at least 98%, or at least 99% identical to the amino acid sequence as set forth in SEQ ID NO:218, and a light chain that comprises an amino acid sequence as set forth in SEQ ID NO:200, or an amino acid sequence that is at least 85%, at least 90%, at least 95%, at least 96%, at least 97%, at least 98%, or at least 99% identical to the amino acid sequence as set forth in SEQ ID NO:200; and
   18) a heavy chain that comprises an amino acid sequence as set forth in SEQ ID NO:219, or an amino acid sequence that is at least 85%, at least 90%, at least 95%, at least 96%, at least 97%, at least 98%, or at least 99% identical to the amino acid sequence as set forth in SEQ ID NO:219, and a light chain that comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201.
5. The isolated antibody or the antigen binding fragment thereof according to any one of clauses 1 to 4, wherein the isolated antibody or the antigen binding fragment thereof consists of a heavy chain and a light chain selected from at least one of the following groups:
   1) a heavy chain that consists of an amino acid sequence as set forth in SEQ ID NO:202, and a light chain that consists of an amino acid sequence as set forth in SEQ ID NO:184;
   2) a heavy chain that consists of an amino acid sequence as set forth in SEQ ID NO:203, and a light chain that consists of an amino acid sequence as set forth in SEQ ID NO: 185;
   3) a heavy chain that consists of an amino acid sequence as set forth in SEQ ID NO:204, and a light chain that consists of an amino acid sequence as set forth in SEQ ID NO: 186;
   4) a heavy chain that consists of an amino acid sequence as set forth in SEQ ID NO:205, and a light chain that consists of an amino acid sequence as set forth in SEQ ID NO:187;
   5) a heavy chain that consists of an amino acid sequence as set forth in SEQ ID NO:206, and a light chain that consists of an amino acid sequence as set forth in SEQ ID NO: 188;
   6) a heavy chain that consists of an amino acid sequence as set forth in SEQ ID NO:207, and a light chain that consists of an amino acid sequence as set forth in SEQ ID NO: 189;
   7) a heavy chain that consists of an amino acid sequence as set forth in SEQ ID NO:208, and a light chain that consists of an amino acid sequence as set forth in SEQ ID NO:190;
   8) a heavy chain that consists of an amino acid sequence as set forth in SEQ ID NO:209, and a light chain that consists of an amino acid sequence as set forth in SEQ ID NO:191;
   9) a heavy chain that consists of an amino acid sequence as set forth in SEQ ID NO:210, and a light chain that consists of an amino acid sequence as set forth in SEQ ID NO:192;
   10) a heavy chain that consists of an amino acid sequence as set forth in SEQ ID NO: 211, and a light chain that consists of an amino acid sequence as set forth in SEQ ID NO: 193;
   11) a heavy chain that consists of an amino acid sequence as set forth in SEQ ID NO:212, and a light chain that consists of an amino acid sequence as set forth in SEQ ID NO: 194;
   12) a heavy chain that consists of an amino acid sequence as set forth in SEQ ID NO:213, and a light chain that consists of an amino acid sequence as set forth in SEQ ID NO:195;
   13) a heavy chain that consists of an amino acid sequence as set forth in SEQ ID NO:214, and a light chain that consists of an amino acid sequence as set forth in SEQ ID NO:196;
   14) a heavy chain that consists of an amino acid sequence as set forth in SEQ ID NO:215, and a light chain that consists of an amino acid sequence as set forth in SEQ ID NO:197;
   15) a heavy chain that consists of an amino acid sequence as set forth in SEQ ID NO:216, and a light chain that consists of an amino acid sequence as set forth in SEQ ID NO:198;
   16) a heavy chain that consists of an amino acid sequence as set forth in SEQ ID NO:217, and a light chain that consists of an amino acid sequence as set forth in SEQ ID NO:199;
   17) a heavy chain that consists of an amino acid sequence as set forth in SEQ ID NO:218, and a light chain that consists of an amino acid sequence as set forth in SEQ ID NO:200; and
   18) a heavy chain that consists of an amino acid sequence as set forth in SEQ ID NO:219, and a light chain that consists of an amino acid sequence as set forth in SEQ ID NO:201.
6. An isolated antibody specifically binding to an ITGA2 protein or an antigen-binding fragment thereof, wherein the isolated antibody or the antigen-binding fragment thereof comprises a heavy chain and a light chain selected from at least one of the following groups:
   1) a heavy chain that comprises an amino acid sequence encoded by a nucleotide sequence as set forth in SEQ ID NO:19, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:19, or consists of an amino acid sequence encoded by the nucleotide sequence as set forth in SEQ ID NO:19, and
      a light chain that comprises an amino acid sequence encoded by a nucleotide sequence as set forth in SEQ ID NO:1, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:1, or consists of an amino acid sequence encoded by the nucleotide sequence as set forth in SEQ ID NO: 1;
   2) a heavy chain that comprises an amino acid sequence encoded by a nucleotide sequence as set forth in SEQ ID NO:20, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:20, or consists of an amino acid sequence encoded by the nucleotide sequence as set forth in SEQ ID NO:20, and
      a light chain that comprises an amino acid sequence encoded by a nucleotide sequence as set forth in SEQ ID NO:2, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:2, or consists of an amino acid sequence encoded by the nucleotide sequence as set forth in SEQ ID NO:2;
   3) a heavy chain that comprises an amino acid sequence encoded by a nucleotide sequence as set forth in SEQ ID NO:21, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:21, or consists of an amino acid sequence encoded by the nucleotide sequence as set forth in SEQ ID NO:21, and
      a light chain that comprises an amino acid sequence encoded by a nucleotide sequence as set forth in SEQ ID NO:3, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:3, or consists of an amino acid sequence encoded by the nucleotide sequence as set forth in SEQ ID NO:3;
   4) a heavy chain that comprises an amino acid sequence encoded by a nucleotide sequence as set forth in SEQ ID NO:22, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:22, or consists of an amino acid sequence encoded by the nucleotide sequence as set forth in SEQ ID NO:22, and
      a light chain that comprises an amino acid sequence encoded by a nucleotide sequence as set forth in SEQ ID NO:4, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:4, or consists of an amino acid sequence encoded by the nucleotide sequence as set forth in SEQ ID NO:4;
   5) a heavy chain that comprises an amino acid sequence encoded by a nucleotide sequence as set forth in SEQ ID NO:23, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:23, or consists of an amino acid sequence encoded by the nucleotide sequence as set forth in SEQ ID NO:23, and
      a light chain that comprises an amino acid sequence encoded by a nucleotide sequence as set forth in SEQ ID NO:5, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:5, or consists of an amino acid sequence encoded by the nucleotide sequence as set forth in SEQ ID NO:5;
   6) a heavy chain that comprises an amino acid sequence encoded by a nucleotide sequence as set forth in SEQ ID NO:24, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:24, or consists of an amino acid sequence encoded by the nucleotide sequence as set forth in SEQ ID NO:24, and
      a light chain that comprises an amino acid sequence encoded by a nucleotide sequence as set forth in SEQ ID NO:6, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:6, or consists of an amino acid sequence encoded by the nucleotide sequence as set forth in SEQ ID NO:6;
   7) a heavy chain that comprises an amino acid sequence encoded by a nucleotide sequence as set forth in SEQ ID NO:25, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:25, or consists of an amino acid sequence encoded by the nucleotide sequence as set forth in SEQ ID NO:25, and
      a light chain that comprises an amino acid sequence encoded by a nucleotide sequence as set forth in SEQ ID NO:7, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:7, or consists of an amino acid sequence encoded by the nucleotide sequence as set forth in SEQ ID NO:7;
   8) a heavy chain that comprises an amino acid sequence encoded by a nucleotide sequence as set forth in SEQ ID NO:26, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:26, or consists of an amino acid sequence encoded by the nucleotide sequence as set forth in SEQ ID NO:26, and
      a light chain that comprises an amino acid sequence encoded by a nucleotide sequence as set forth in SEQ ID NO:8, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:8, or consists of an amino acid sequence encoded by the nucleotide sequence as set forth in SEQ ID NO:8;
   9) a heavy chain that comprises an amino acid sequence encoded by a nucleotide sequence as set forth in SEQ ID NO:27, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:27, or consists of an amino acid sequence encoded by the nucleotide sequence as set forth in SEQ ID NO:27, and
      a light chain that comprises an amino acid sequence encoded by a nucleotide sequence as set forth in SEQ ID NO:9, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:9, or consists of an amino acid sequence encoded by the nucleotide sequence as set forth in SEQ ID NO:9;
   10) a heavy chain that comprises an amino acid sequence encoded by a nucleotide sequence as set forth in SEQ ID NO:28, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:28, or consists of an amino acid sequence encoded by the nucleotide sequence as set forth in SEQ ID NO:28, and
      a light chain that comprises an amino acid sequence encoded by a nucleotide sequence as set forth in SEQ ID NO:10, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:10, or consists of an amino acid sequence encoded by the nucleotide sequence as set forth in SEQ ID NO:10;
   11) a heavy chain that comprises an amino acid sequence encoded by a nucleotide sequence as set forth in SEQ ID NO:29, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:29, or consists of an amino acid sequence encoded by the nucleotide sequence as set forth in SEQ ID NO:29, and
      a light chain that comprises an amino acid sequence encoded by a nucleotide sequence as set forth in SEQ ID NO: 11, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:11, or consists of an amino acid sequence encoded by the nucleotide sequence as set forth in SEQ ID NO: 11;
   12) a heavy chain that comprises an amino acid sequence encoded by a nucleotide sequence as set forth in SEQ ID NO:30, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:30, or consists of an amino acid sequence encoded by the nucleotide sequence as set forth in SEQ ID NO:30, and
      a light chain that comprises an amino acid sequence encoded by a nucleotide sequence as set forth in SEQ ID NO:12, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:12, or consists of an amino acid sequence encoded by the nucleotide sequence as set forth in SEQ ID NO:12;
   13) a heavy chain that comprises an amino acid sequence encoded by a nucleotide sequence as set forth in SEQ ID NO:31, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:31, or consists of an amino acid sequence encoded by the nucleotide sequence as set forth in SEQ ID NO:31, and
      a light chain that comprises an amino acid sequence encoded by a nucleotide sequence as set forth in SEQ ID NO:13, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:13, or consists of an amino acid sequence encoded by the nucleotide sequence as set forth in SEQ ID NO:13;
   14) a heavy chain that comprises an amino acid sequence encoded by a nucleotide sequence as set forth in SEQ ID NO:32, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:32, or consists of an amino acid sequence encoded by the nucleotide sequence as set forth in SEQ ID NO:32, and
      a light chain that comprises an amino acid sequence encoded by a nucleotide sequence as set forth in SEQ ID NO:14, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:14, or consists of an amino acid sequence encoded by the nucleotide sequence as set forth in SEQ ID NO: 14;
   15) a heavy chain that comprises an amino acid sequence encoded by a nucleotide sequence as set forth in SEQ ID NO:33, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:33, or consists of an amino acid sequence encoded by the nucleotide sequence as set forth in SEQ ID NO:33, and
      a light chain that comprises an amino acid sequence encoded by a nucleotide sequence as set forth in SEQ ID NO:15, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:15, or consists of an amino acid sequence encoded by the nucleotide sequence as set forth in SEQ ID NO:15;
   16) a heavy chain that comprises an amino acid sequence encoded by a nucleotide sequence as set forth in SEQ ID NO:34, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:34, or consists of an amino acid sequence encoded by the nucleotide sequence as set forth in SEQ ID NO:34, and
      a light chain that comprises an amino acid sequence encoded by a nucleotide sequence as set forth in SEQ ID NO: 16, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO: 16, or consists of an amino acid sequence encoded by the nucleotide sequence as set forth in SEQ ID NO:16;
   17) a heavy chain that comprises an amino acid sequence encoded by a nucleotide sequence as set forth in SEQ ID NO:35, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:35, or consists of an amino acid sequence encoded by the nucleotide sequence as set forth in SEQ ID NO:35, and
      a light chain that comprises an amino acid sequence encoded by a nucleotide sequence as set forth in SEQ ID NO: 17, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO: 17, or consists of an amino acid sequence encoded by the nucleotide sequence as set forth in SEQ ID NO:17; and
   18) a heavy chain that comprises an amino acid sequence encoded by a nucleotide sequence as set forth in SEQ ID NO:36, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:36, or consists of an amino acid sequence encoded by the nucleotide sequence as set forth in SEQ ID NO:36, and
      a light chain that comprises an amino acid sequence encoded by a nucleotide sequence as set forth in SEQ ID NO: 18, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:18, or consists of an amino acid sequence encoded by the nucleotide sequence as set forth in SEQ ID NO:18.
7. The isolated antibody or the antigen binding fragment thereof according to any one of clauses 1 to 6,
   wherein the antibody is capable of binding to an ITGA2 protein in vitro at an EC50 of less than 1 nM; and/or
   wherein the antibody is capable of binding to an ITGA2 protein on the surface of HuCCT1 cells with an EC50 of less than 5 nM;
   and/or wherein the antibody is a monoclonal antibody, a polyclonal antibody, a chimeric antibody, or a humanized antibody.
8. An isolated nucleic acid molecule encoding the antibody or the antigen-binding fragment thereof according to any one of clauses 1 to 7; or
   the nucleic acid molecule comprises or consists of a nucleotide sequence selected from any of the following nucleotide sequences as set forth in SEQ ID NOs: 1-18, and/or the nucleic acid molecule comprises or consists of a nucleotide sequence selected from any of the following nucleotide sequences as set forth in SEQ ID NOs: 19-36; or
   the nucleic acid molecule comprises or consists of a nucleotide sequence combination selected from any one of the following nucleotide sequences: SEQ ID NO: 1 and SEQ ID NO: 19, SEQ ID NO: 2 and SEQ ID NO: 20, SEQ ID NO: 3 and SEQ ID NO: 21, SEQ ID NO: 4 and SEQ ID NO: 22, SEQ ID NO: 5 and SEQ ID NO: 23, SEQ ID NO: 6 and SEQ ID NO: 24, SEQ ID NO: 7 and SEQ ID NO: 25, SEQ ID NO: 8 and SEQ ID NO: 26, SEQ ID NO: 9 and SEQ ID NO: 27, SEQ ID NO: 10 and SEQ ID NO: 28, SEQ ID NO: 11 and SEQ ID NO: 29, SEQ ID NO: 12 and SEQ ID NO: 30, SEQ ID NO: 13 and SEQ ID NO: 31, SEQ ID NO: 14 and SEQ ID NO: 32, SEQ ID NO: 15 and SEQ ID NO: 33, SEQ ID NO: 16 and SEQ ID NO: 34, SEQ ID NO: 17 and SEQ ID NO: 35, and SEQ ID NO: 18 and SEQ ID NO: 36.
9. The isolated nucleic acid molecule according to clause 8, wherein the nucleic acid molecule is operably linked to an expression regulatory sequence.
10. An expression vector comprising the nucleic acid molecule according to clause 8 or 9.
11. A host cell comprising the nucleic acid molecule according to clause 8 or 9 or the expression vector according to clause 10.
12. A hybridoma cell expressing the antibody or the antigen-binding fragment thereof according to any one of clauses 1 to 7.
13. An antibody-drug conjugate of the formula:
   Ab-L-D, wherein:
      "Ab" represents at least one antibody or antigen-binding fragment thereof according to the present invention described above;
      "L" represents the linker that connects the antibody moiety to the partner molecule; and
      "D" represents the partner molecule;
      or
   Ab-(L-D)p, wherein:
      "Ab" represents at least one antibody or antigen-binding fragment thereof according to the present invention described above;
      "L" represents the linker that connects the antibody moiety to the partner molecule;
      "D" represents the partner molecule; and
      "p" represents the number of linker/partner molecules (preferably drug payloads) conjugated to the antibody or the antigen-binding fragment thereof according to the present invention described above; optionally, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.
14. The antibody-drug conjugate according to clause 13, wherein the linker is selected from one of the following: ve, mc-GGFG, AcLysve, mc, MalPeg6, m(H20)c and m(H20)cvc; and/or the linker is cleavable.
15. The antibody-drug conjugate according to clause 13 or 14, wherein
   the partner molecule is a drug payload, wherein the drug payload is a therapeutic molecule; optionally, the drug payload is selected from: a cytotoxic agent, a cytostatic agent, an immunomodulator and a chemotherapeutic agent; optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD; or
   the partner molecule is a marker molecule; optionally, the marker molecule is selected from one of: a radioactive label (for example, an isotope), a fluorescent label (for example, a fluorescent dye), a chemical substance, and an enzyme or label that can detect a modification.
16. A pharmaceutical composition comprising the antibody or the antigen-binding fragment thereof according to any one of clauses 1-7, or the antibody-drug conjugate according to any one of clauses 13 to 15, and optionally a pharmaceutically acceptable carrier.
17. Use of the antibody or the antigen-binding fragment thereof according to any one of the clauses 1-7 or the antibody-drug conjugate according to any one of the clauses 13 to 15 in preparing a pharmaceutical composition; optionally, the pharmaceutical composition is used for treating a malignant tumor, preventing and/or treating metastasis or relapse of a malignant tumor in a patient; optionally, the pharmaceutical composition is used for detecting presence of a malignant tumor in a patient; and optionally, the pharmaceutical composition is used for prognosticating relapse or progression of a malignant tumor in a patient.
18. A method for treating a malignant tumor, preventing and/or treating metastasis or relapse of a malignant tumor in a patient, comprising administering to the patient an effective amount of the antibody or the antigen-binding fragment thereof according to any one of clauses 1 to 7, the antibody-drug conjugate according to any one of clauses 13 to 15, or the pharmaceutical composition according to clause 16.
19. The method according to clause 18, wherein the malignant tumor is selected from a group consisting of the following: breast cancer, large intestine cancer, skin cancer, pancreatic cancer, prostate cancer, liver cancer, lung cancer, gastric cancer, leukemia, bile duct cancer, cervical cancer, colorectal cancer, endometrial cancer, esophageal cancer, head and neck cancer, lymphoma, medullary thyroid cancer, Non-Hodgkin's lymphoma, ovarian cancer, glioma, melanoma, and bladder cancer; preferably, the malignant tumor is selected from a group consisting of the following: colorectal cancer, bile duct cancer, pancreatic cancer, and skin cancer.
20. The method according to clause 18 or 19, further comprising administering to the patient other anti-tumor treatments, such as administering a chemotherapeutic agent, an antibody targeting other tumor-specific antigens, or a radiation therapy.
21. A method for detecting presence of tumor cells in a biological sample, comprising:
   a) contacting the biological sample with the antibody or antigen-binding fragment thereof according to any one of clauses 1 to 7;
   b) detecting binding of the antibody or antigen-binding fragment thereof to a target antigen in the biological sample,
   wherein the detected binding represents presence of the tumor cells in the biological sample.
22. A method for detecting presence of a malignant tumor in a patient, comprising:
   a) contacting the biological sample obtained from the patient with the antibody or antigen-binding fragment thereof according to any one of clauses 1 to 7;
   b) detecting binding of the antibody or the antigen-binding fragment thereof to a target antigen in the biological sample, wherein the detected binding represents presence of the malignant tumor in the patient.
23. A method for prognosticating relapse or progression of a malignant tumor in a patient, the method comprising:
   (a) isolating a biological sample comprising tumor cells from the patient;
   (b) contacting the biological sample comprising tumor cells with the antibody or antigen-binding fragment thereof according to any one of clauses 1 to 7; and
   (c) identifying presence of the tumor cells that bind to the antibody or the antigen-binding fragment thereof,
   thereby prognosticating the relapse or progression of the malignant tumor in the patient.
24. The method according to clause 23, wherein the progression of the malignant tumor includes metastasis of the malignant tumor in the patient.
25. The method according to clause 23 or 24, wherein the biological sample is selected from one of the following: a blood sample, a tissue sample, and a lymph sample.
26. The method according to any one of clauses 23 to 25, wherein the malignant tumor is selected from a group consisting of the following: breast cancer, colorectal cancer, large intestine cancer, skin cancer, pancreatic cancer, prostate cancer, liver cancer, lung cancer, gastric cancer, leukemia, bile duct cancer, cervical cancer, endometrial cancer, esophageal cancer, head and neck cancer, lymphoma, medullary thyroid cancer, Non-Hodgkin's lymphoma, ovarian cancer, glioma, melanoma, and bladder cancer, preferably, the malignant tumor is selected from a group consisting of the following: colorectal cancer, bile duct cancer, pancreatic cancer, and skin cancer.
27. A method for producing an antibody or an antigen-binding fragment thereof targeting human tumor cells that express ITGA2, the method comprising:
   (i) culturing the host cell according to clause 11 in a condition suitable for expressing the nucleic acid molecule or the expression vector, and
   (ii) isolating and purifying the antibody or the antigen-binding fragment thereof expressed by the nucleic acid molecule or the expression vector.

## Claims

1. An isolated antibody specifically binding to an ITGA2 protein or an antigen-binding fragment thereof, comprising a heavy chain variable region comprising HCDR1, HCDR2, and HCDR3 sequences, and a light chain variable region comprising LCDR1, LCDR2, and LCDR3 sequences, wherein the antibody or the antigen-binding fragment thereof comprises a heavy chain variable region and a light chain variable region selected from at least one of the following groups:
1) a heavy chain variable region that comprises (a) HCDR1 as set forth in SEQ ID NO: 112, (b) HCDR2 as set forth in SEQ ID NO: 113, and (c) HCDR3 as set forth in SEQ ID NO: 114, and a light chain variable region that comprises (d) LCDR1 as set forth in SEQ ID NO: 38, (e) LCDR2 as set forth in SEQ ID NO: 39, and (f) LCDR3 as set forth in SEQ ID NO: 40;
2) a heavy chain variable region that comprises (a) HCDR1 as set forth in SEQ ID NO: 116, (b) HCDR2 as set forth in SEQ ID NO: 117, and (c) HCDR3 as set forth in SEQ ID NO: 118, and a light chain variable region that comprises (d) LCDR1 as set forth in SEQ ID NO: 42, (e) LCDR2 as set forth in SEQ ID NO: 43, and (f) LCDR3 as set forth in SEQ ID NO: 44;
3) a heavy chain variable region that comprises (a) HCDR1 as set forth in SEQ ID NO: 120, (b) HCDR2 as set forth in SEQ ID NO: 121, and (c) HCDR3 as set forth in SEQ ID NO: 122, and a light chain variable region that comprises (d) LCDR1 as set forth in SEQ ID NO: 46, (e) LCDR2 as set forth in SEQ ID NO: 47, and (f) LCDR3 as set forth in SEQ ID NO: 48;
4) a heavy chain variable region that comprises (a) HCDR1 as set forth in SEQ ID NO: 124, (b) HCDR2 as set forth in SEQ ID NO: 125, and (c) HCDR3 as set forth in SEQ ID NO: 126, and a light chain variable region that comprises (d) LCDR1 as set forth in SEQ ID NO: 50, (e) LCDR2 as set forth in SEQ ID NO: 51, and (f) LCDR3 as set forth in SEQ ID NO: 52;
5) a heavy chain variable region that comprises (a) HCDR1 as set forth in SEQ ID NO: 128, (b) HCDR2 as set forth in SEQ ID NO: 129, and (c) HCDR3 as set forth in SEQ ID NO: 130, and a light chain variable region that comprises (d) LCDR1 as set forth in SEQ ID NO: 54, (e) LCDR2 as set forth in SEQ ID NO: 55, and (f) LCDR3 as set forth in SEQ ID NO: 56;
6) a heavy chain variable region that comprises (a) HCDR1 as set forth in SEQ ID NO: 132, (b) HCDR2 as set forth in SEQ ID NO: 133, and (c) HCDR3 as set forth in SEQ ID NO: 134, and a light chain variable region that comprises (d) LCDR1 as set forth in SEQ ID NO: 58, (e) LCDR2 as set forth in SEQ ID NO: 59, and (f) LCDR3 as set forth in SEQ ID NO: 60;
7) a heavy chain variable region that comprises (a) HCDR1 as set forth in SEQ ID NO: 136, (b) HCDR2 as set forth in SEQ ID NO: 137, and (c) HCDR3 as set forth in SEQ ID NO: 138, and a light chain variable region that comprises (d) LCDR1 as set forth in SEQ ID NO: 62, (e) LCDR2 as set forth in SEQ ID NO: 63, and (f) LCDR3 as set forth in SEQ ID NO: 64;
8) a heavy chain variable region that comprises (a) HCDR1 as set forth in SEQ ID NO: 140, (b) HCDR2 as set forth in SEQ ID NO: 141, and (c) HCDR3 as set forth in SEQ ID NO: 142, and a light chain variable region that comprises (d) LCDR1 as set forth in SEQ ID NO: 66, (e) LCDR2 as set forth in SEQ ID NO: 67, and (f) LCDR3 as set forth in SEQ ID NO: 68;
9) a heavy chain variable region that comprises (a) HCDR1 as set forth in SEQ ID NO: 144, (b) HCDR2 as set forth in SEQ ID NO: 145, and (c) HCDR3 as set forth in SEQ ID NO: 146, and a light chain variable region that comprises (d) LCDR1 as set forth in SEQ ID NO: 70, (e) LCDR2 as set forth in SEQ ID NO: 71, and (f) LCDR3 as set forth in SEQ ID NO: 72;
10) a heavy chain variable region that comprises (a) HCDR1 as set forth in SEQ ID NO: 148, (b) HCDR2 as set forth in SEQ ID NO: 149, and (c) HCDR3 as set forth in SEQ ID NO: 150, and a light chain variable region that comprises (d) LCDR1 as set forth in SEQ ID NO: 74, (e) LCDR2 as set forth in SEQ ID NO: 75, and (f) LCDR3 as set forth in SEQ ID NO: 76;
11) a heavy chain variable region that comprises (a) HCDR1 as set forth in SEQ ID NO: 152, (b) HCDR2 as set forth in SEQ ID NO: 153, and (c) HCDR3 as set forth in SEQ ID NO: 154, and a light chain variable region that comprises (d) LCDR1 as set forth in SEQ ID NO: 78, (e) LCDR2 as set forth in SEQ ID NO: 79, and (f) LCDR3 as set forth in SEQ ID NO: 80;
12) a heavy chain variable region that comprises (a) HCDR1 as set forth in SEQ ID NO: 156, (b) HCDR2 as set forth in SEQ ID NO: 157, and (c) HCDR3 as set forth in SEQ ID NO: 158, and a light chain variable region that comprises (d) LCDR1 as set forth in SEQ ID NO: 82, (e) LCDR2 as set forth in SEQ ID NO: 83, and (f) LCDR3 as set forth in SEQ ID NO: 84;
13) a heavy chain variable region that comprises (a) HCDR1 as set forth in SEQ ID NO: 160, (b) HCDR2 as set forth in SEQ ID NO: 161, and (c) HCDR3 as set forth in SEQ ID NO: 162, and a light chain variable region that comprises (d) LCDR1 as set forth in SEQ ID NO: 86, (e) LCDR2 as set forth in SEQ ID NO: 87, and (f) LCDR3 as set forth in SEQ ID NO: 88;
14) a heavy chain variable region that comprises (a) HCDR1 as set forth in SEQ ID NO: 164, (b) HCDR2 as set forth in SEQ ID NO: 165, and (c) HCDR3 as set forth in SEQ ID NO: 166, and a light chain variable region that comprises (d) LCDR1 as set forth in SEQ ID NO: 90, (e) LCDR2 as set forth in SEQ ID NO: 91, and (f) LCDR3 as set forth in SEQ ID NO: 92;
15) a heavy chain variable region that comprises (a) HCDR1 as set forth in SEQ ID NO: 168, (b) HCDR2 as set forth in SEQ ID NO: 169, and (c) HCDR3 as set forth in SEQ ID NO: 170, and a light chain variable region that comprises (d) LCDR1 as set forth in SEQ ID NO: 94, (e) LCDR2 as set forth in SEQ ID NO: 95, and (f) LCDR3 as set forth in SEQ ID NO: 96;
16) a heavy chain variable region that comprises (a) HCDR1 as set forth in SEQ ID NO: 172, (b) HCDR2 as set forth in SEQ ID NO: 173, and (c) HCDR3 as set forth in SEQ ID NO: 174, and a light chain variable region that comprises (d) LCDR1 as set forth in SEQ ID NO: 98, (e) LCDR2 as set forth in SEQ ID NO: 99, and (f) LCDR3 as set forth in SEQ ID NO: 100;
17) a heavy chain variable region that comprises (a) HCDR1 as set forth in SEQ ID NO: 176, (b) HCDR2 as set forth in SEQ ID NO: 177, and (c) HCDR3 as set forth in SEQ ID NO: 178, and a light chain variable region that comprises (d) LCDR1 as set forth in SEQ ID NO: 102, (e) LCDR2 as set forth in SEQ ID NO: 103, and (f) LCDR3 as set forth in SEQ ID NO: 104; and
18) a heavy chain variable region that comprises (a) HCDR1 as set forth in SEQ ID NO: 180, (b) HCDR2 as set forth in SEQ ID NO: 181, and (c) HCDR3 as set forth in SEQ ID NO: 182, and a light chain variable region that comprises (d) LCDR1 as set forth in SEQ ID NO: 106, (e) LCDR2 as set forth in SEQ ID NO: 107, and (f) LCDR3 as set forth in SEQ ID NO: 108.

2. The isolated antibody or the antigen binding fragment thereof according to claim 1, wherein the isolated antibody or the antigen binding fragment thereof comprises a heavy chain variable region and a light chain variable region selected from at least one of the following groups:
1) a heavy chain variable region that comprises an amino acid sequence as set forth in SEQ ID NO:111, or an amino acid sequence that is at least 85%, at least 90%, at least 95%, at least 96%, at least 97%, at least 98%, or at least 99% identical to the amino acid sequence as set forth in SEQ ID NO:111, and a light chain variable region that comprises an amino acid sequence as set forth in SEQ ID NO:37, or an amino acid sequence that is at least 85%, at least 90%, at least 95%, at least 96%, at least 97%, at least 98%, or at least 99% identical to the amino acid sequence as set forth in SEQ ID NO:37;
2) a heavy chain variable region that comprises an amino acid sequence as set forth in SEQ ID NO:115, or an amino acid sequence that is at least 85%, at least 90%, at least 95%, at least 96%, at least 97%, at least 98%, or at least 99% identical to the amino acid sequence as set forth in SEQ ID NO:115, and a light chain variable region that comprises an amino acid sequence as set forth in SEQ ID NO:41, or an amino acid sequence that is at least 85%, at least 90%, at least 95%, at least 96%, at least 97%, at least 98%, or at least 99% identical to the amino acid sequence as set forth in SEQ ID NO:41;
3) a heavy chain variable region that comprises an amino acid sequence as set forth in SEQ ID NO:119, or an amino acid sequence that is at least 85%, at least 90%, at least 95%, at least 96%, at least 97%, at least 98%, or at least 99% identical to the amino acid sequence as set forth in SEQ ID NO:119, and a light chain variable region that comprises an amino acid sequence as set forth in SEQ ID NO:45, or an amino acid sequence that is at least 85%, at least 90%, at least 95%, at least 96%, at least 97%, at least 98%, or at least 99% identical to the amino acid sequence as set forth in SEQ ID NO:45;
4) a heavy chain variable region that comprises an amino acid sequence as set forth in SEQ ID NO:123, or an amino acid sequence that is at least 85%, at least 90%, at least 95%, at least 96%, at least 97%, at least 98%, or at least 99% identical to the amino acid sequence as set forth in SEQ ID NO: 123, and a light chain variable region that comprises an amino acid sequence as set forth in SEQ ID NO:49, or an amino acid sequence that is at least 85%, at least 90%, at least 95%, at least 96%, at least 97%, at least 98%, or at least 99% identical to the amino acid sequence as set forth in SEQ ID NO:49;
5) a heavy chain variable region that comprises an amino acid sequence as set forth in SEQ ID NO:127, or an amino acid sequence that is at least 85%, at least 90%, at least 95%, at least 96%, at least 97%, at least 98%, or at least 99% identical to the amino acid sequence as set forth in SEQ ID NO: 127, and a light chain variable region that comprises an amino acid sequence as set forth in SEQ ID NO:53, or an amino acid sequence that is at least 85%, at least 90%, at least 95%, at least 96%, at least 97%, at least 98%, or at least 99% identical to the amino acid sequence as set forth in SEQ ID NO:53;
6) a heavy chain variable region that comprises an amino acid sequence as set forth in SEQ ID NO:131, or an amino acid sequence that is at least 85%, at least 90%, at least 95%, at least 96%, at least 97%, at least 98%, or at least 99% identical to the amino acid sequence as set forth in SEQ ID NO:131, and a light chain variable region that comprises an amino acid sequence as set forth in SEQ ID NO:57, or an amino acid sequence that is at least 85%, at least 90%, at least 95%, at least 96%, at least 97%, at least 98%, or at least 99% identical to the amino acid sequence as set forth in SEQ ID NO:57;
7) a heavy chain variable region that comprises an amino acid sequence as set forth in SEQ ID NO:135, or an amino acid sequence that is at least 85%, at least 90%, at least 95%, at least 96%, at least 97%, at least 98%, or at least 99% identical to the amino acid sequence as set forth in SEQ ID NO: 135, and a light chain variable region that comprises an amino acid sequence as set forth in SEQ ID NO:61, or an amino acid sequence that is at least 85%, at least 90%, at least 95%, at least 96%, at least 97%, at least 98%, or at least 99% identical to the amino acid sequence as set forth in SEQ ID NO:61;
8) a heavy chain variable region that comprises an amino acid sequence as set forth in SEQ ID NO:139, or an amino acid sequence that is at least 85%, at least 90%, at least 95%, at least 96%, at least 97%, at least 98%, or at least 99% identical to the amino acid sequence as set forth in SEQ ID NO:139, and a light chain variable region that comprises an amino acid sequence as set forth in SEQ ID NO:65, or an amino acid sequence that is at least 85%, at least 90%, at least 95%, at least 96%, at least 97%, at least 98%, or at least 99% identical to the amino acid sequence as set forth in SEQ ID NO:65;
9) a heavy chain variable region that comprises an amino acid sequence as set forth in SEQ ID NO:143, or an amino acid sequence that is at least 85%, at least 90%, at least 95%, at least 96%, at least 97%, at least 98%, or at least 99% identical to the amino acid sequence as set forth in SEQ ID NO: 143, and a light chain variable region that comprises an amino acid sequence as set forth in SEQ ID NO:69, or an amino acid sequence that is at least 85%, at least 90%, at least 95%, at least 96%, at least 97%, at least 98%, or at least 99% identical to the amino acid sequence as set forth in SEQ ID NO:69;
10) a heavy chain variable region that comprises an amino acid sequence as set forth in SEQ ID NO:147, or an amino acid sequence that is at least 85%, at least 90%, at least 95%, at least 96%, at least 97%, at least 98%, or at least 99% identical to the amino acid sequence as set forth in SEQ ID NO: 147, and a light chain variable region that comprises an amino acid sequence as set forth in SEQ ID NO:73, or an amino acid sequence that is at least 85%, at least 90%, at least 95%, at least 96%, at least 97%, at least 98%, or at least 99% identical to the amino acid sequence as set forth in SEQ ID NO:73;
11) a heavy chain variable region that comprises an amino acid sequence as set forth in SEQ ID NO:151, or an amino acid sequence that is at least 85%, at least 90%, at least 95%, at least 96%, at least 97%, at least 98%, or at least 99% identical to the amino acid sequence as set forth in SEQ ID NO: 151, and a light chain variable region that comprises an amino acid sequence as set forth in SEQ ID NO:77, or an amino acid sequence that is at least 85%, at least 90%, at least 95%, at least 96%, at least 97%, at least 98%, or at least 99% identical to the amino acid sequence as set forth in SEQ ID NO:77;
12) a heavy chain variable region that comprises an amino acid sequence as set forth in SEQ ID NO:155, or an amino acid sequence that is at least 85%, at least 90%, at least 95%, at least 96%, at least 97%, at least 98%, or at least 99% identical to the amino acid sequence as set forth in SEQ ID NO:155, and a light chain variable region that comprises an amino acid sequence as set forth in SEQ ID NO:81, or an amino acid sequence that is at least 85%, at least 90%, at least 95%, at least 96%, at least 97%, at least 98%, or at least 99% identical to the amino acid sequence as set forth in SEQ ID NO:81;
13) a heavy chain variable region that comprises an amino acid sequence as set forth in SEQ ID NO:159, or an amino acid sequence that is at least 85%, at least 90%, at least 95%, at least 96%, at least 97%, at least 98%, or at least 99% identical to the amino acid sequence as set forth in SEQ ID NO:159, and a light chain variable region that comprises an amino acid sequence as set forth in SEQ ID NO:85, or an amino acid sequence that is at least 85%, at least 90%, at least 95%, at least 96%, at least 97%, at least 98%, or at least 99% identical to the amino acid sequence as set forth in SEQ ID NO:85;
14) a heavy chain variable region that comprises an amino acid sequence as set forth in SEQ ID NO:163, or an amino acid sequence that is at least 85%, at least 90%, at least 95%, at least 96%, at least 97%, at least 98%, or at least 99% identical to the amino acid sequence as set forth in SEQ ID NO: 163, and a light chain variable region that comprises an amino acid sequence as set forth in SEQ ID NO:89, or an amino acid sequence that is at least 85%, at least 90%, at least 95%, at least 96%, at least 97%, at least 98%, or at least 99% identical to the amino acid sequence as set forth in SEQ ID NO:89;
15) a heavy chain variable region that comprises an amino acid sequence as set forth in SEQ ID NO:167, or an amino acid sequence that is at least 85%, at least 90%, at least 95%, at least 96%, at least 97%, at least 98%, or at least 99% identical to the amino acid sequence as set forth in SEQ ID NO: 167, and a light chain variable region that comprises an amino acid sequence as set forth in SEQ ID NO:93, or an amino acid sequence that is at least 85%, at least 90%, at least 95%, at least 96%, at least 97%, at least 98%, or at least 99% identical to the amino acid sequence as set forth in SEQ ID NO:93;
16) a heavy chain variable region that comprises an amino acid sequence as set forth in SEQ ID NO:171, or an amino acid sequence that is at least 85%, at least 90%, at least 95%, at least 96%, at least 97%, at least 98%, or at least 99% identical to the amino acid sequence as set forth in SEQ ID NO: 171, and a light chain variable region that comprises an amino acid sequence as set forth in SEQ ID NO:97, or an amino acid sequence that is at least 85%, at least 90%, at least 95%, at least 96%, at least 97%, at least 98%, or at least 99% identical to the amino acid sequence as set forth in SEQ ID NO:97;
17) a heavy chain variable region that comprises an amino acid sequence as set forth in SEQ ID NO:175, or an amino acid sequence that is at least 85%, at least 90%, at least 95%, at least 96%, at least 97%, at least 98%, or at least 99% identical to the amino acid sequence as set forth in SEQ ID NO: 175, and a light chain variable region that comprises an amino acid sequence as set forth in SEQ ID NO:101, or an amino acid sequence that is at least 85%, at least 90%, at least 95%, at least 96%, at least 97%, at least 98%, or at least 99% identical to the amino acid sequence as set forth in SEQ ID NO:101; and
18) a heavy chain variable region that comprises an amino acid sequence as set forth in SEQ ID NO:179, or an amino acid sequence that is at least 85%, at least 90%, at least 95%, at least 96%, at least 97%, at least 98%, or at least 99% identical to the amino acid sequence as set forth in SEQ ID NO: 179, and a light chain variable region that comprises an amino acid sequence as set forth in SEQ ID NO:105, or an amino acid sequence that is at least 85%, at least 90%, at least 95%, at least 96%, at least 97%, at least 98%, or at least 99% identical to the amino acid sequence as set forth in SEQ ID NO: 105.

3. The isolated antibody or the antigen binding fragment thereof according to claim 1 or 2, wherein the isolated antibody or the antigen binding fragment thereof further comprises a heavy chain constant region and/or a light chain constant region,
the heavy chain constant region comprises an amino acid sequence as set forth in SEQ ID NO: 183, or an amino acid sequence that is at least 85%, at least 90%, at least 95%, at least 96%, at least 97%, at least 98%, or at least 99% identical to the amino acid sequence as set forth in SEQ ID NO:183,
the light chain constant region comprises an amino acid sequence as set forth in SEQ ID NO: 109, or an amino acid sequence that is at least 85%, at least 90%, at least 95%, at least 96%, at least 97%, at least 98%, or at least 99% identical to the amino acid sequence as set forth in SEQ ID NO: 109, or the light chain constant region comprises an amino acid sequence as set forth in SEQ ID NO:110, or an amino acid sequence that is at least 85%, at least 90%, at least 95%, at least 96%, at least 97%, at least 98%, or at least 99% identical to the amino acid sequence as set forth in SEQ ID NO:110.

4. The isolated antibody or the antigen binding fragment thereof according to claim 3, wherein the isolated antibody or the antigen binding fragment thereof comprises a heavy chain and a light chain selected from at least one of the following groups:
1) a heavy chain that comprises an amino acid sequence as set forth in SEQ ID NO:202, or an amino acid sequence that is at least 85%, at least 90%, at least 95%, at least 96%, at least 97%, at least 98%, or at least 99% identical to the amino acid sequence as set forth in SEQ ID NO:202, and a light chain that comprises an amino acid sequence as set forth in SEQ ID NO: 184, or an amino acid sequence that is at least 85%, at least 90%, at least 95%, at least 96%, at least 97%, at least 98%, or at least 99% identical to the amino acid sequence as set forth in SEQ ID NO: 184;
2) a heavy chain that comprises an amino acid sequence as set forth in SEQ ID NO:203, or an amino acid sequence that is at least 85%, at least 90%, at least 95%, at least 96%, at least 97%, at least 98%, or at least 99% identical to the amino acid sequence as set forth in SEQ ID NO:203, and a light chain that comprises an amino acid sequence as set forth in SEQ ID NO: 185, or an amino acid sequence that is at least 85%, at least 90%, at least 95%, at least 96%, at least 97%, at least 98%, or at least 99% identical to the amino acid sequence as set forth in SEQ ID NO: 185;
3) a heavy chain that comprises an amino acid sequence as set forth in SEQ ID NO:204, or an amino acid sequence that is at least 85%, at least 90%, at least 95%, at least 96%, at least 97%, at least 98%, or at least 99% identical to the amino acid sequence as set forth in SEQ ID NO:204, and a light chain that comprises an amino acid sequence as set forth in SEQ ID NO: 186, or an amino acid sequence that is at least 85%, at least 90%, at least 95%, at least 96%, at least 97%, at least 98%, or at least 99% identical to the amino acid sequence as set forth in SEQ ID NO: 186;
4) a heavy chain that comprises an amino acid sequence as set forth in SEQ ID NO:205, or an amino acid sequence that is at least 85%, at least 90%, at least 95%, at least 96%, at least 97%, at least 98%, or at least 99% identical to the amino acid sequence as set forth in SEQ ID NO:205, and a light chain that comprises an amino acid sequence as set forth in SEQ ID NO:187, or an amino acid sequence that is at least 85%, at least 90%, at least 95%, at least 96%, at least 97%, at least 98%, or at least 99% identical to the amino acid sequence as set forth in SEQ ID NO: 187;
5) a heavy chain that comprises an amino acid sequence as set forth in SEQ ID NO:206, or an amino acid sequence that is at least 85%, at least 90%, at least 95%, at least 96%, at least 97%, at least 98%, or at least 99% identical to the amino acid sequence as set forth in SEQ ID NO:206, and a light chain that comprises an amino acid sequence as set forth in SEQ ID NO:188, or an amino acid sequence that is at least 85%, at least 90%, at least 95%, at least 96%, at least 97%, at least 98%, or at least 99% identical to the amino acid sequence as set forth in SEQ ID NO: 188;
6) a heavy chain that comprises an amino acid sequence as set forth in SEQ ID NO:207, or an amino acid sequence that is at least 85%, at least 90%, at least 95%, at least 96%, at least 97%, at least 98%, or at least 99% identical to the amino acid sequence as set forth in SEQ ID NO:207, and a light chain that comprises an amino acid sequence as set forth in SEQ ID NO:189, or an amino acid sequence that is at least 85%, at least 90%, at least 95%, at least 96%, at least 97%, at least 98%, or at least 99% identical to the amino acid sequence as set forth in SEQ ID NO: 189;
7) a heavy chain that comprises an amino acid sequence as set forth in SEQ ID NO:208, or an amino acid sequence that is at least 85%, at least 90%, at least 95%, at least 96%, at least 97%, at least 98%, or at least 99% identical to the amino acid sequence as set forth in SEQ ID NO:208, and a light chain that comprises an amino acid sequence as set forth in SEQ ID NO:190, or an amino acid sequence that is at least 85%, at least 90%, at least 95%, at least 96%, at least 97%, at least 98%, or at least 99% identical to the amino acid sequence as set forth in SEQ ID NO: 190;
8) a heavy chain that comprises an amino acid sequence as set forth in SEQ ID NO:209, or an amino acid sequence that is at least 85%, at least 90%, at least 95%, at least 96%, at least 97%, at least 98%, or at least 99% identical to the amino acid sequence as set forth in SEQ ID NO:209, and a light chain that comprises an amino acid sequence as set forth in SEQ ID NO:191, or an amino acid sequence that is at least 85%, at least 90%, at least 95%, at least 96%, at least 97%, at least 98%, or at least 99% identical to the amino acid sequence as set forth in SEQ ID NO:191;
9) a heavy chain that comprises an amino acid sequence as set forth in SEQ ID NO:210, or an amino acid sequence that is at least 85%, at least 90%, at least 95%, at least 96%, at least 97%, at least 98%, or at least 99% identical to the amino acid sequence as set forth in SEQ ID NO:210, and a light chain that comprises an amino acid sequence as set forth in SEQ ID NO:192, or an amino acid sequence that is at least 85%, at least 90%, at least 95%, at least 96%, at least 97%, at least 98%, or at least 99% identical to the amino acid sequence as set forth in SEQ ID NO:192;
10) a heavy chain that comprises an amino acid sequence as set forth in SEQ ID NO:211, or an amino acid sequence that is at least 85%, at least 90%, at least 95%, at least 96%, at least 97%, at least 98%, or at least 99% identical to the amino acid sequence as set forth in SEQ ID NO:211, and a light chain that comprises an amino acid sequence as set forth in SEQ ID NO:193, or an amino acid sequence that is at least 85%, at least 90%, at least 95%, at least 96%, at least 97%, at least 98%, or at least 99% identical to the amino acid sequence as set forth in SEQ ID NO:193;
11) a heavy chain that comprises an amino acid sequence as set forth in SEQ ID NO:212, or an amino acid sequence that is at least 85%, at least 90%, at least 95%, at least 96%, at least 97%, at least 98%, or at least 99% identical to the amino acid sequence as set forth in SEQ ID NO:212, and a light chain that comprises an amino acid sequence as set forth in SEQ ID NO:194, or an amino acid sequence that is at least 85%, at least 90%, at least 95%, at least 96%, at least 97%, at least 98%, or at least 99% identical to the amino acid sequence as set forth in SEQ ID NO:194;
12) a heavy chain that comprises an amino acid sequence as set forth in SEQ ID NO:213, or an amino acid sequence that is at least 85%, at least 90%, at least 95%, at least 96%, at least 97%, at least 98%, or at least 99% identical to the amino acid sequence as set forth in SEQ ID NO:213, and a light chain that comprises an amino acid sequence as set forth in SEQ ID NO:195, or an amino acid sequence that is at least 85%, at least 90%, at least 95%, at least 96%, at least 97%, at least 98%, or at least 99% identical to the amino acid sequence as set forth in SEQ ID NO:195;
13) a heavy chain that comprises an amino acid sequence as set forth in SEQ ID NO:214, or an amino acid sequence that is at least 85%, at least 90%, at least 95%, at least 96%, at least 97%, at least 98%, or at least 99% identical to the amino acid sequence as set forth in SEQ ID NO:214, and a light chain that comprises an amino acid sequence as set forth in SEQ ID NO:196, or an amino acid sequence that is at least 85%, at least 90%, at least 95%, at least 96%, at least 97%, at least 98%, or at least 99% identical to the amino acid sequence as set forth in SEQ ID NO:196;
14) a heavy chain that comprises an amino acid sequence as set forth in SEQ ID NO:215, or an amino acid sequence that is at least 85%, at least 90%, at least 95%, at least 96%, at least 97%, at least 98%, or at least 99% identical to the amino acid sequence as set forth in SEQ ID NO:215, and a light chain that comprises an amino acid sequence as set forth in SEQ ID NO:197, or an amino acid sequence that is at least 85%, at least 90%, at least 95%, at least 96%, at least 97%, at least 98%, or at least 99% identical to the amino acid sequence as set forth in SEQ ID NO:197;
15) a heavy chain that comprises an amino acid sequence as set forth in SEQ ID NO:216, or an amino acid sequence that is at least 85%, at least 90%, at least 95%, at least 96%, at least 97%, at least 98%, or at least 99% identical to the amino acid sequence as set forth in SEQ ID NO:216, and a light chain that comprises an amino acid sequence as set forth in SEQ ID NO:198, or an amino acid sequence that is at least 85%, at least 90%, at least 95%, at least 96%, at least 97%, at least 98%, or at least 99% identical to the amino acid sequence as set forth in SEQ ID NO: 198;
16) a heavy chain that comprises an amino acid sequence as set forth in SEQ ID NO:217, or an amino acid sequence that is at least 85%, at least 90%, at least 95%, at least 96%, at least 97%, at least 98%, or at least 99% identical to the amino acid sequence as set forth in SEQ ID NO:217, and a light chain that comprises an amino acid sequence as set forth in SEQ ID NO:199, or an amino acid sequence that is at least 85%, at least 90%, at least 95%, at least 96%, at least 97%, at least 98%, or at least 99% identical to the amino acid sequence as set forth in SEQ ID NO:199;
17) a heavy chain that comprises an amino acid sequence as set forth in SEQ ID NO:218, or an amino acid sequence that is at least 85%, at least 90%, at least 95%, at least 96%, at least 97%, at least 98%, or at least 99% identical to the amino acid sequence as set forth in SEQ ID NO:218, and a light chain that comprises an amino acid sequence as set forth in SEQ ID NO:200, or an amino acid sequence that is at least 85%, at least 90%, at least 95%, at least 96%, at least 97%, at least 98%, or at least 99% identical to the amino acid sequence as set forth in SEQ ID NO:200; and
18) a heavy chain that comprises an amino acid sequence as set forth in SEQ ID NO:219, or an amino acid sequence that is at least 85%, at least 90%, at least 95%, at least 96%, at least 97%, at least 98%, or at least 99% identical to the amino acid sequence as set forth in SEQ ID NO:219, and a light chain that comprises an amino acid sequence as set forth in SEQ ID NO:201, or an amino acid sequence that is at least 85%, at least 90%, at least 95%, at least 96%, at least 97%, at least 98%, or at least 99% identical to the amino acid sequence as set forth in SEQ ID NO:201.

5. The isolated antibody or the antigen binding fragment thereof according to claim 4, wherein the isolated antibody or the antigen binding fragment thereof consists of a heavy chain and a light chain selected from at least one of the following groups:
1) a heavy chain that consists of an amino acid sequence as set forth in SEQ ID NO:202, and a light chain that consists of an amino acid sequence as set forth in SEQ ID NO: 184;
2) a heavy chain that consists of an amino acid sequence as set forth in SEQ ID NO:203, and a light chain that consists of an amino acid sequence as set forth in SEQ ID NO: 185;
3) a heavy chain that consists of an amino acid sequence as set forth in SEQ ID NO:204, and a light chain that consists of an amino acid sequence as set forth in SEQ ID NO:186;
4) a heavy chain that consists of an amino acid sequence as set forth in SEQ ID NO:205, and a light chain that consists of an amino acid sequence as set forth in SEQ ID NO: 187;
5) a heavy chain that consists of an amino acid sequence as set forth in SEQ ID NO:206, and a light chain that consists of an amino acid sequence as set forth in SEQ ID NO:188;
6) a heavy chain that consists of an amino acid sequence as set forth in SEQ ID NO:207, and a light chain that consists of an amino acid sequence as set forth in SEQ ID NO:189;
7) a heavy chain that consists of an amino acid sequence as set forth in SEQ ID NO:208, and a light chain that consists of an amino acid sequence as set forth in SEQ ID NO:190;
8) a heavy chain that consists of an amino acid sequence as set forth in SEQ ID NO:209, and a light chain that consists of an amino acid sequence as set forth in SEQ ID NO:191;
9) a heavy chain that consists of an amino acid sequence as set forth in SEQ ID NO:210, and a light chain that consists of an amino acid sequence as set forth in SEQ ID NO:192;
10) a heavy chain that consists of an amino acid sequence as set forth in SEQ ID NO:211, and a light chain that consists of an amino acid sequence as set forth in SEQ ID NO:193;
11) a heavy chain that consists of an amino acid sequence as set forth in SEQ ID NO:212, and a light chain that consists of an amino acid sequence as set forth in SEQ ID NO:194;
12) a heavy chain that consists of an amino acid sequence as set forth in SEQ ID NO:213, and a light chain that consists of an amino acid sequence as set forth in SEQ ID NO:195;
13) a heavy chain that consists of an amino acid sequence as set forth in SEQ ID NO:214, and a light chain that consists of an amino acid sequence as set forth in SEQ ID NO:196;
14) a heavy chain that consists of an amino acid sequence as set forth in SEQ ID NO:215, and a light chain that consists of an amino acid sequence as set forth in SEQ ID NO:197;
15) a heavy chain that consists of an amino acid sequence as set forth in SEQ ID NO:216, and a light chain that consists of an amino acid sequence as set forth in SEQ ID NO:198;
16) a heavy chain that consists of an amino acid sequence as set forth in SEQ ID NO:217, and a light chain that consists of an amino acid sequence as set forth in SEQ ID NO:199;
17) a heavy chain that consists of an amino acid sequence as set forth in SEQ ID NO:218, and a light chain that consists of an amino acid sequence as set forth in SEQ ID NO:200; and
18) a heavy chain that consists of an amino acid sequence as set forth in SEQ ID NO:219, and a light chain that consists of an amino acid sequence as set forth in SEQ ID NO:201.

6. An isolated antibody specifically binding to an ITGA2 protein or an antigen-binding fragment thereof, wherein the isolated antibody or the antigen-binding fragment thereof comprises a heavy chain and a light chain selected from at least one of the following groups:
1) a heavy chain that comprises an amino acid sequence encoded by a nucleotide sequence as set forth in SEQ ID NO:19, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:19, or consists of an amino acid sequence encoded by the nucleotide sequence as set forth in SEQ ID NO:19, and
a light chain that comprises an amino acid sequence encoded by a nucleotide sequence as set forth in SEQ ID NO:1, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:1, or consists of an amino acid sequence encoded by the nucleotide sequence as set forth in SEQ ID NO:1;
2) a heavy chain that comprises an amino acid sequence encoded by a nucleotide sequence as set forth in SEQ ID NO:20, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:20, or consists of an amino acid sequence encoded by the nucleotide sequence as set forth in SEQ ID NO:20, and
a light chain that comprises an amino acid sequence encoded by a nucleotide sequence as set forth in SEQ ID NO:2, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:2, or consists of an amino acid sequence encoded by the nucleotide sequence as set forth in SEQ ID NO:2;
3) a heavy chain that comprises an amino acid sequence encoded by a nucleotide sequence as set forth in SEQ ID NO:21, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:21, or consists of an amino acid sequence encoded by the nucleotide sequence as set forth in SEQ ID NO:21, and
a light chain that comprises an amino acid sequence encoded by a nucleotide sequence as set forth in SEQ ID NO:3, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:3, or consists of an amino acid sequence encoded by the nucleotide sequence as set forth in SEQ ID NO:3;
4) a heavy chain that comprises an amino acid sequence encoded by a nucleotide sequence as set forth in SEQ ID NO:22, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:22, or consists of an amino acid sequence encoded by the nucleotide sequence as set forth in SEQ ID NO:22, and
a light chain that comprises an amino acid sequence encoded by a nucleotide sequence as set forth in SEQ ID NO:4, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:4, or consists of an amino acid sequence encoded by the nucleotide sequence as set forth in SEQ ID NO:4;
5) a heavy chain that comprises an amino acid sequence encoded by a nucleotide sequence as set forth in SEQ ID NO:23, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:23, or consists of an amino acid sequence encoded by the nucleotide sequence as set forth in SEQ ID NO:23, and
a light chain that comprises an amino acid sequence encoded by a nucleotide sequence as set forth in SEQ ID NO:5, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:5, or consists of an amino acid sequence encoded by the nucleotide sequence as set forth in SEQ ID NO:5;
6) a heavy chain that comprises an amino acid sequence encoded by a nucleotide sequence as set forth in SEQ ID NO:24, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:24, or consists of an amino acid sequence encoded by the nucleotide sequence as set forth in SEQ ID NO:24, and
a light chain that comprises an amino acid sequence encoded by a nucleotide sequence as set forth in SEQ ID NO:6, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:6, or consists of an amino acid sequence encoded by the nucleotide sequence as set forth in SEQ ID NO:6;
7) a heavy chain that comprises an amino acid sequence encoded by a nucleotide sequence as set forth in SEQ ID NO:25, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:25, or consists of an amino acid sequence encoded by the nucleotide sequence as set forth in SEQ ID NO:25, and
a light chain that comprises an amino acid sequence encoded by a nucleotide sequence as set forth in SEQ ID NO:7, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:7, or consists of an amino acid sequence encoded by the nucleotide sequence as set forth in SEQ ID NO:7;
8) a heavy chain that comprises an amino acid sequence encoded by a nucleotide sequence as set forth in SEQ ID NO:26, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:26, or consists of an amino acid sequence encoded by the nucleotide sequence as set forth in SEQ ID NO:26, and
a light chain that comprises an amino acid sequence encoded by a nucleotide sequence as set forth in SEQ ID NO:8, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:8, or consists of an amino acid sequence encoded by the nucleotide sequence as set forth in SEQ ID NO:8;
9) a heavy chain that comprises an amino acid sequence encoded by a nucleotide sequence as set forth in SEQ ID NO:27, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:27, or consists of an amino acid sequence encoded by the nucleotide sequence as set forth in SEQ ID NO:27, and
a light chain that comprises an amino acid sequence encoded by a nucleotide sequence as set forth in SEQ ID NO:9, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:9, or consists of an amino acid sequence encoded by the nucleotide sequence as set forth in SEQ ID NO:9;
10) a heavy chain that comprises an amino acid sequence encoded by a nucleotide sequence as set forth in SEQ ID NO:28, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:28, or consists of an amino acid sequence encoded by the nucleotide sequence as set forth in SEQ ID NO:28, and
a light chain that comprises an amino acid sequence encoded by a nucleotide sequence as set forth in SEQ ID NO:10, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:10, or consists of an amino acid sequence encoded by the nucleotide sequence as set forth in SEQ ID NO:10;
11) a heavy chain that comprises an amino acid sequence encoded by a nucleotide sequence as set forth in SEQ ID NO:29, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:29, or consists of an amino acid sequence encoded by the nucleotide sequence as set forth in SEQ ID NO:29, and
a light chain that comprises an amino acid sequence encoded by a nucleotide sequence as set forth in SEQ ID NO:11, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:11, or consists of an amino acid sequence encoded by the nucleotide sequence as set forth in SEQ ID NO:11;
12) a heavy chain that comprises an amino acid sequence encoded by a nucleotide sequence as set forth in SEQ ID NO:30, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:30, or consists of an amino acid sequence encoded by the nucleotide sequence as set forth in SEQ ID NO:30, and
a light chain that comprises an amino acid sequence encoded by a nucleotide sequence as set forth in SEQ ID NO:12, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:12, or consists of an amino acid sequence encoded by the nucleotide sequence as set forth in SEQ ID NO:12;
13) a heavy chain that comprises an amino acid sequence encoded by a nucleotide sequence as set forth in SEQ ID NO:31, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:31, or consists of an amino acid sequence encoded by the nucleotide sequence as set forth in SEQ ID NO:31, and
a light chain that comprises an amino acid sequence encoded by a nucleotide sequence as set forth in SEQ ID NO:13, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:13, or consists of an amino acid sequence encoded by the nucleotide sequence as set forth in SEQ ID NO:13;
14) a heavy chain that comprises an amino acid sequence encoded by a nucleotide sequence as set forth in SEQ ID NO:32, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:32, or consists of an amino acid sequence encoded by the nucleotide sequence as set forth in SEQ ID NO:32, and
a light chain that comprises an amino acid sequence encoded by a nucleotide sequence as set forth in SEQ ID NO:14, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:14, or consists of an amino acid sequence encoded by the nucleotide sequence as set forth in SEQ ID NO:14;
15) a heavy chain that comprises an amino acid sequence encoded by a nucleotide sequence as set forth in SEQ ID NO:33, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:33, or consists of an amino acid sequence encoded by the nucleotide sequence as set forth in SEQ ID NO:33, and
a light chain that comprises an amino acid sequence encoded by a nucleotide sequence as set forth in SEQ ID NO:15, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:15, or consists of an amino acid sequence encoded by the nucleotide sequence as set forth in SEQ ID NO:15;
16) a heavy chain that comprises an amino acid sequence encoded by a nucleotide sequence as set forth in SEQ ID NO:34, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:34, or consists of an amino acid sequence encoded by the nucleotide sequence as set forth in SEQ ID NO:34, and
a light chain that comprises an amino acid sequence encoded by a nucleotide sequence as set forth in SEQ ID NO:16, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:16, or consists of an amino acid sequence encoded by the nucleotide sequence as set forth in SEQ ID NO:16;
17) a heavy chain that comprises an amino acid sequence encoded by a nucleotide sequence as set forth in SEQ ID NO:35, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:35, or consists of an amino acid sequence encoded by the nucleotide sequence as set forth in SEQ ID NO:35, and
a light chain that comprises an amino acid sequence encoded by a nucleotide sequence as set forth in SEQ ID NO:17, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:17, or consists of an amino acid sequence encoded by the nucleotide sequence as set forth in SEQ ID NO:17; and
18) a heavy chain that comprises an amino acid sequence encoded by a nucleotide sequence as set forth in SEQ ID NO:36, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:36, or consists of an amino acid sequence encoded by the nucleotide sequence as set forth in SEQ ID NO:36, and
a light chain that comprises an amino acid sequence encoded by a nucleotide sequence as set forth in SEQ ID NO:18, or an amino acid sequence that is at least 85%, at least 90%, at least 95%, at least 96%, at least 97%, at least 98%, or at least 99% identical to the amino acid sequence encoded by the nucleotide sequence as set forth in SEQ ID NO:18, or consists of an amino acid sequence encoded by the nucleotide sequence as set forth in SEQ ID NO:18.

7. The isolated antibody or the antigen binding fragment thereof according to any one of claims 1 to 6,
wherein the antibody is capable of binding to an ITGA2 protein in vitro at an EC50 of less than 1 nM; and/or
wherein the antibody is capable of binding to an ITGA2 protein on the surface of HuCCT1 cells with an EC50 of less than 5 nM; and/or
wherein the antibody is a monoclonal antibody, a polyclonal antibody, a chimeric antibody, or a humanized antibody.

8. An isolated nucleic acid molecule encoding the antibody or the antigen-binding fragment thereof according to any one of claims 1 to 7; or
the nucleic acid molecule comprises or consists of a nucleotide sequence selected from any of the following nucleotide sequences as set forth in SEQ ID NOs: 1-18, and/or the nucleic acid molecule comprises or consists of a nucleotide sequence selected from any of the following nucleotide sequences as set forth in SEQ ID NOs: 19-36; or
the nucleic acid molecule comprises or consists of a nucleotide sequence combination selected from any one of the following nucleotide sequences: SEQ ID NO: 1 and SEQ ID NO: 19, SEQ ID NO: 2 and SEQ ID NO: 20, SEQ ID NO: 3 and SEQ ID NO: 21, SEQ ID NO: 4 and SEQ ID NO: 22, SEQ ID NO: 5 and SEQ ID NO: 23, SEQ ID NO: 6 and SEQ ID NO: 24, SEQ ID NO: 7 and SEQ ID NO: 25, SEQ ID NO: 8 and SEQ ID NO: 26, SEQ ID NO: 9 and SEQ ID NO: 27, SEQ ID NO: 10 and SEQ ID NO: 28, SEQ ID NO: 11 and SEQ ID NO: 29, SEQ ID NO: 12 and SEQ ID NO: 30, SEQ ID NO: 13 and SEQ ID NO: 31, SEQ ID NO: 14 and SEQ ID NO: 32, SEQ ID NO: 15 and SEQ ID NO: 33, SEQ ID NO: 16 and SEQ ID NO: 34, SEQ ID NO: 17 and SEQ ID NO: 35, and SEQ ID NO: 18 and SEQ ID NO: 36.

9. An antibody-drug conjugate of the formula:
Ab-L-D, wherein:
"Ab" represents at least one antibody or antigen-binding fragment thereof according to the present invention described above;
"L" represents a linker that connects the antibody moiety to a partner molecule; and
"D" represents the partner molecule;
or
Ab-(L-D)p, wherein:
"Ab" represents at least one antibody or antigen-binding fragment thereof according to the present invention described above;
"L" represents a linker that connects the antibody moiety to a partner molecule;
"D" represents the partner molecule; and
"p" represents the number of linker/partner molecules (preferably drug payloads) conjugated to the antibody or the antigen-binding fragment thereof according to the present invention described above; optionally, p is selected from the following values: 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0.

10. The antibody-drug conjugate according to claim 9, wherein the linker is selected from one of the following: ve, mc-GGFG, AcLysve, mc, MalPeg6, m(H20)c and m(H20)cvc; and/or the linker is cleavable.

11. The antibody-drug conjugate according to claim 9 or 10, wherein
the partner molecule is a drug payload, wherein the drug payload is a therapeutic molecule; optionally, the drug payload is selected from: a cytotoxic agent, a cytostatic agent, an immunomodulator and a chemotherapeutic agent; optionally, the drug payload is selected from: MMAE, MMAF, DM1, DM4, Dxd, SN-38, Topotecan and a derivative thereof, Exatecan and a derivative thereof, Belotecan and a derivative thereof, Camptothecin, Calicheamicin, and PBD; or
the partner molecule is a marker molecule; optionally, the marker molecule is selected from one of: a radioactive label (for example, an isotope), a fluorescent label (for example, a fluorescent dye), a chemical substance, and an enzyme or label that is capable of detecting a modification.

12. A pharmaceutical composition comprising the antibody or the antigen-binding fragment thereof according to any one of claims 1 to 7, or the antibody-drug conjugate according to any one of claims 9 to 11, and optionally a pharmaceutically acceptable carrier.

13. Use of the antibody or the antigen-binding fragment thereof according to any one of claims 1 to 7 or the antibody-drug conjugate according to any one of claims 9 to 11 in preparing a pharmaceutical composition; optionally, the pharmaceutical composition is used for treating a malignant tumor, preventing and/or treating metastasis or relapse of a malignant tumor in a patient; optionally, the pharmaceutical composition is used for detecting presence of a malignant tumor in a patient; and optionally, the pharmaceutical composition is used for prognosticating relapse or progression of a malignant tumor in a patient.

14. A method for treating a malignant tumor, preventing and/or treating metastasis or relapse of a malignant tumor in a patient, the method comprising administering to the patient an effective amount of the antibody or the antigen-binding fragment thereof according to any one of claims 1 to 7, the antibody-drug conjugate according to any one of claims 9 to 11, or the pharmaceutical composition according to claim 16.

15. The method according to claim 14, wherein the malignant tumor is selected from a group consisting of the following: breast cancer, large intestine cancer, skin cancer, pancreatic cancer, prostate cancer, liver cancer, lung cancer, gastric cancer, leukemia, bile duct cancer, cervical cancer, colorectal cancer, endometrial cancer, esophageal cancer, head and neck cancer, lymphoma, medullary thyroid cancer, Non-Hodgkin's lymphoma, ovarian cancer, glioma, melanoma, and bladder cancer, preferably, the malignant tumor is selected from a group consisting of the following: colorectal cancer, bile duct cancer, pancreatic cancer, and skin cancer.

16. The method according to claim 14 or 15, further comprising administering to the patient other anti-tumor treatments, such as administering a chemotherapeutic agent, an antibody targeting other tumor-specific antigens, or a radiation therapy.
